(19) Europäisches Patentamt
European Patent Office
Office européen des brevets

(11) EP 4 495 126 A1

(12) **EUROPEAN PATENT APPLICATION**
published in accordance with Art. 153(4) EPC

(43) Date of publication:
**22.01.2025 Bulletin 2025/04**

(21) Application number: **23787822.8**

(22) Date of filing: **14.04.2023**

(51) International Patent Classification (IPC):
***C07F 9/6558*** *(2006.01)* ***C07D 401/14*** *(2006.01)*
***C07D 417/14*** *(2006.01)* ***A61K 31/675*** *(2006.01)*
***A61P 35/00*** *(2006.01)*

(52) Cooperative Patent Classification (CPC):
**A61K 31/675; A61P 35/00; C07D 401/14; C07D 417/14; C07F 9/6558**

(86) International application number:
**PCT/CN2023/088336**

(87) International publication number:
**WO 2023/198180 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.04.2022 CN 202210398246**
**28.03.2023 CN 202310317846**

(71) Applicant: **Beijing Tide Pharmaceutical Co., Ltd.**
**Beijing 100176 (CN)**

(72) Inventors:
- **WANG, Hongjun**
**Beijing 100176 (CN)**
- **WANG, Yeming**
**Beijing 100176 (CN)**
- **WANG, Xiaoqian**
**Beijing 100176 (CN)**
- **ZHAO, Yanping**
**Beijing 100176 (CN)**

(74) Representative: **Novitas Patent AB**
**P.O. Box 55557**
**102 04 Stockholm (SE)**

(54) **EGFR DEGRADATION AGENT**

(57) The present invention relates to a novel compound of general formula (A) capable of inhibiting and inducing degradation of EGFR, and a pharmaceutical composition comprising the compound, which can be used for treating diseases associated with EGFR kinase, such as cancer. The present invention further relates to preparation and use of the compound described above.

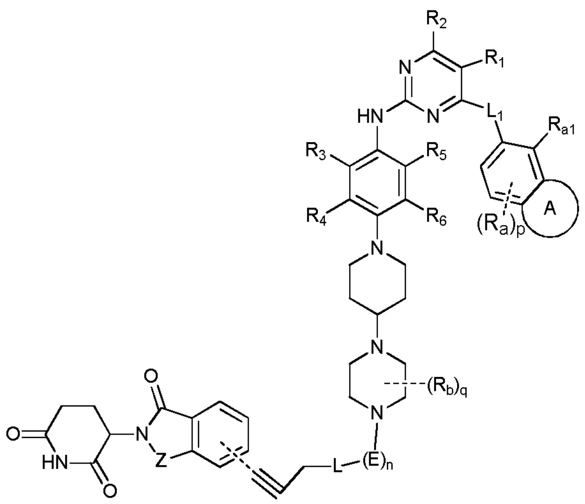

(A)

## Description

This application claims the priority of Chinese application No. 202210398246.3 filed on April 15, 2022 and Chinese application No. 202310317846.7 filed on March 28, 2023, which are incorporated herein by reference in their entirety.

## FIELD OF THE INVENTION

The present disclosure relates to the field of medicine. Specifically, the present disclosure provides compounds capable of inducing EGFR degradation, and a preparation method and use thereof.

## BACKGROUND OF THE INVENTION

Lung cancer is one of the most common malignant tumors. In 2020, there were 2.2 million new cases of lung cancer and 1.8 million deaths worldwide. Among them, non-small cell lung cancer (NSCLC) accounts for 80%-85% of all lung cancers. In non-small cell lung cancer, about 50% of patients in China and 11-16% of patients in Western countries have EGFR gene mutation, in which the most common types of mutations are exon 19 deletion mutation (del E746-A750) and exon 21 L858R point mutation, accounting for about 90% of all EGFR mutations in populations. EGFR small molecule inhibitors are the standard first-line treatment for non-small cell lung cancer with EGFR gene mutation and have been widely used in the field of lung cancer treatment. They inhibit the activation of tyrosine kinase by competing with endogenous ligands for binding to EGFR, thereby blocking the EGFR signaling pathway, inhibiting the proliferation and metastasis of tumor cells and promoting a series of biological effects such as tumor cell apoptosis.

Currently, there are five EGFR small molecule inhibitors approved by the FDA for the treatment of non-small cell lung cancer, namely Gefitinib, Erlotinib, Afatinib, Dacomitinib and Osimertinib. The first-generation EGFR small-molecule inhibitors Gefitinib and Erlotinib have been used to treat advanced non-small cell lung cancer with activating EGFR mutations (L858R, del E746-A750). However, patients develop resistance after 10-12 months of administration of Gefitinib and Erlotinib, wherein more than 50 % of drug-resistant patients are due to the secondary mutation of T790M in EGFR. Afatinib, a second-generation irreversible EGFR inhibitor, is effective for advanced non-small cell lung cancer patients with activating EGFR mutations (L858R, or del E746-A750), but cannot resolve the clinical drug resistance caused by EGFR T790M mutation. Moreover, Afatinib lacks selectivity to wild-type EGFR and has great toxicity. Osimertinib, a third-generation irreversible inhibitor, overcomes the drug resistance caused by EGFR T790M and can effectively treat advanced non-small cell lung cancer patients with drug resistance caused by EGFR T790M mutation. Although Osimertinib has achieved great success in the clinical treatment of non-small cell lung cancer with EGFR T790M mutant, some patients who benefits from the treatment develops drug resistance after 9-14 months of treatment. Studies have shown that 15-22% of patients with drug resistance of Osimertinib are due to EGFR C797S mutation. As a type of cancer with high morbidity and mortality, lung cancer has a huge and unmet demand for clinical drugs. Therefore, the development of new lung cancer drugs to meet clinical treatment needs is an urgent problem to be solved.

Ubiquitin-proteasome system (UPS) is a multi-component system for intracellular protein degradation, which is involved in important physiological and biochemical processes such as cell growth and differentiation, DNA replication and repair, cell metabolism, immune response and so on. Protein degradation mediated by the ubiquitin-proteasome pathway is an important mechanism of the body for regulating intracellular protein level and function, and plays an important role in maintaining protein homeostasis in vivo. Inducing the degradation of EGFR through the intracellular ubiquitin-proteasome pathway provides a new idea for the treatment of cancers.

The present disclosure provides compounds capable of inducing degradation of EGFR, and preparation methods and use thereof.

## SUMMARY OF THE INVENTION

In one aspect, the present disclosure provides a compound of formula (A) or (X), or a pharmaceutically acceptable salt, enantiomer, diastereomer, racemate, solvate, hydrate, polymorph, prodrug, or isotopic variant thereof, or a mixture thereof, which can be used to treat EGFR kinase-mediated diseases, such as diseases.

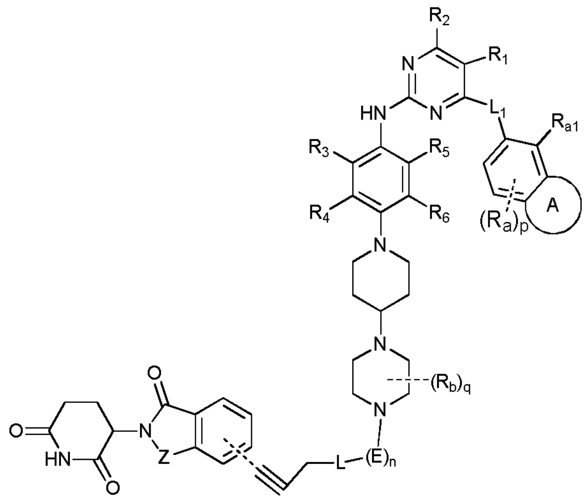

(A)

wherein

----- represents that the point of attachment to the rest of the molecule can be located at an available point of a ring where it is located;
Ring A is absent, or is selected from $C_{4-10}$ cycloalkyl, 5-8 membered heterocyclyl, $C_{6-10}$ aryl and 5-14 membered heteroaryl;
$R_1$ is selected from H, halogen, -OH, $-NH_2$, -CN, $C_{1-6}$ alkyl and $C_{1-6}$ haloalkyl;
$R_2$ is selected from H, halogen, -OH, $-NH_2$, -CN, $C_{1-6}$ alkyl and $C_{1-6}$ haloalkyl;
$R_3$ is selected from H, halogen, -OH, $-NH_2$, -CN, $C_{1-6}$ alkyl, $C_{1-6}$ haloalkyl, $-OC_{1-6}$ alkyl, $-OC_{1-6}$ haloalkyl, $C_{2-6}$ alkenyl and $C_{2-6}$ alkynyl;
$R_4$ is selected from H, halogen, -OH, $-NH_2$, -CN, $C_{1-6}$ alkyl and $C_{1-6}$ haloalkyl;
$R_5$ is selected from H, halogen, -OH, $-NH_2$, -CN, $C_{1-6}$ alkyl and $C_{1-6}$ haloalkyl;
$R_6$ is selected from $C_{1-6}$ alkyl, $C_{1-6}$ haloalkyl, -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, $-OR_c$, $-NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_{a1}$ is selected from

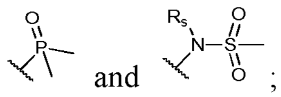

$R_s$ is selected from H, $C_{1-6}$ alkyl and $C_{1-6}$ haloalkyl;
$R_a$ is selected from H, halogen, -OH, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$;
alternatively, two $R_a$ on the same carbon atom or on different carbon atoms are taken together to form a $C_{4-10}$ cycloalkyl, a 5-8 membered heterocyclyl, a $C_{6-10}$ aryl, or a 5-10 membered heteroaryl;
$R_b$ is selected from H, halogen, -OH, $-NH_2$, -CN, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl, a 3-10 membered heterocyclyl, a $C_{6-10}$ aryl, or a 5-10 membered heteroaryl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;

L is selected from a chemical bond, -O-, -NR#-, -CR#R#'-, -S-, -SO- and $-S(O)_2-$;
$L_1$ is selected from a chemical bond, -O-, -NR#-, -CR#R#'-, -S-, -SO- and $-S(O)_2-$;
E is selected from a chemical bond and -CR#R#'-;
wherein R# and R#' are independently selected from H, halogen, -OH, $-NH_2$, -CN, $C_{1-6}$ alkyl, $C_{1-6}$ haloalkyl, $C_{2-6}$ alkenyl, and $C_{2-6}$ alkynyl;
p is 0, 1, 2, 3, or 4;
q is 0, 1, 2, 3, or 4;
n is 0, 1, 2, 3, 4, or 5.

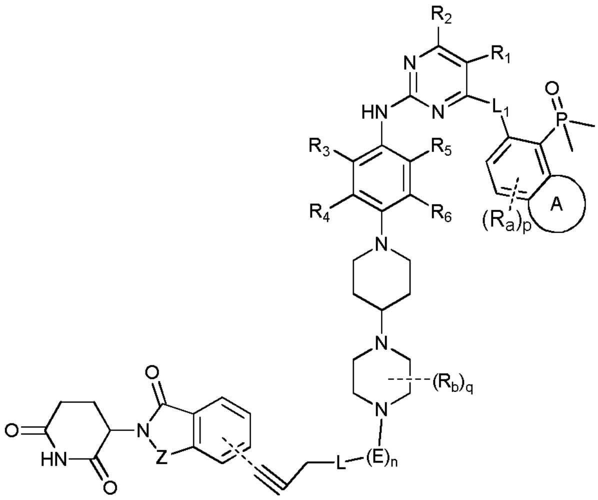

(X)

wherein

----- represents that the point of attachment to the rest of the molecule can be located at an available point of a ring where it is located;
Ring A is absent, or is selected from $C_{4-10}$ cycloalkyl, 5-8 membered heterocyclyl, $C_{6-10}$ aryl and 5-14 membered heteroaryl;
$R_1$ is selected from H, halogen, -OH, $-NH_2$, -CN, $C_{1-6}$ alkyl and $C_{1-6}$ haloalkyl;
$R_2$ is selected from H, halogen, -OH, $-NH_2$, -CN, $C_{1-6}$ alkyl and $C_{1-6}$ haloalkyl;
$R_3$ is selected from H, halogen, -OH, $-NH_2$, -CN, $C_{1-6}$ alkyl, $C_{1-6}$ haloalkyl, $-OC_{1-6}$ alkyl, $-OC_{1-6}$ haloalkyl, $C_{2-6}$ alkenyl and $C_{2-6}$ alkynyl;
$R_4$ is selected from H, halogen, -OH, $-NH_2$, -CN, $C_{1-6}$ alkyl and $C_{1-6}$ haloalkyl;
$R_5$ is selected from H, halogen, -OH, $-NH_2$, -CN, $C_{1-6}$ alkyl and $C_{1-6}$ haloalkyl;
$R_6$ is selected from -NH-C(O)-CR=CR'R" and -NH-C(O)-C=CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, $-OR_c$, $-NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_a$ is selected from H, halogen, -OH, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$;
alternatively, two $R_a$ on the same carbon atom or on different carbon atoms are taken together to form a $C_{4-10}$ cycloalkyl, a 5-8 membered heterocyclyl, a $C_{6-10}$ aryl, or a 5-10 membered heteroaryl
$R_b$ is selected from H, halogen, -OH, $-NH_2$, -CN, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl, a 3-10 membered heterocyclyl, a $C_{6-10}$ aryl, or a 5-10 membered heteroaryl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;

alternatively, $R_c$ and $R_d$ are taken together with the N atom to which they are attached to form 3-10 membered heterocyclyl;

L is selected from a chemical bond, -O-, -NR#-, -CR#R#'-, -S-, -SO- and $-S(O)_2-$;

$L_1$ is selected from a chemical bond, -O-, -NR#-, -CR#R#'-, -S-, -SO- and $-S(O)_2-$;

E is selected from a chemical bond and -CR#R#'-;

wherein R# and R#' are independently selected from H, halogen, -OH, $-NH_2$, -CN, $C_{1-6}$ alkyl, $C_{1-6}$ haloalkyl, $C_{2-6}$ alkenyl, and $C_{2-6}$ alkynyl;

p is 0, 1, 2, 3, or 4;

q is 0, 1, 2, 3, or 4;

n is 0, 1, 2, 3, 4, or 5.

In another aspect, the present disclosure provides a pharmaceutical composition comprising a compound disclosed herein, and optionally a pharmaceutically acceptable excipient.

In another aspect, the present disclosure provides a pharmaceutical composition comprising a compound disclosed herein and a pharmaceutically acceptable excipient, which further comprises other therapeutic agent (s).

In another aspect, the present disclosure provides a kit comprising a compound disclosed herein, other therapeutic agent (s), and a pharmaceutically acceptable carrier, adjuvant or vehicle.

In another aspect, the present disclosure provides a use of a compound disclosed herein in the manufacture of a medicament for treating and/or preventing a disease mediated by EGFR kinase.

In another aspect, the present disclosure provides a method of treating and/or preventing a disease mediated by EGFR kinase in a subject, comprising administering to the subject a compound disclosed herein or a composition disclosed herein.

In another aspect, the present disclosure provides a compound disclosed herein or a composition disclosed herein, for use in treating and/or preventing a disease mediated by EGFR kinase.

In a specific embodiment, the diseases treated by the present disclosure include cancer, such as ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular cancer, stomach cancer, gastrointestinal stromal tumor (GIST), thyroid cancer, cancer of bile duct, endometrial cancer, kidney cancer, anaplastic large cell lymphoma, acute myeloid leukemia (AML), multiple myeloma, melanoma, and mesothelioma.

Other objects and advantages of the present disclosure will be apparent to those skilled in the art from specific embodiments, examples and claims below.

Definition

Chemical definitions

Definitions of specific functional groups and chemical terms are described in more detail below.

When a range of values is listed, it is intended to encompass each value and sub-range within the range. For example, "$C_{1-6}$ alkyl" is intended to include $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

"$C_{1-6}$ alkyl" refers to a radical of a straight or branched, saturated hydrocarbon group having 1 to 6 carbon atoms. In some embodiments, $C_{1-4}$ alkyl is alternative. Examples of $C_{1-6}$ alkyl include methyl ($C_1$), ethyl ($C_2$), n-propyl ($C_3$), iso-propyl ($C_3$), n-butyl ($C_4$), tert-butyl ($C_4$), sec-butyl ($C_4$), isobutyl ($C_4$), n-pentyl ($C_5$), 3-pentyl ($C_5$), pentyl ($C_5$), neopentyl ($C_5$), 3-methyl-2-butyl ($C_5$), tert-pentyl ($C_5$) and n-hexyl ($C_6$). The term "$C_{1-6}$ alkyl" also includes heteroalkyl, wherein one or more (e.g., 1, 2, 3 or 4) carbon atoms are subsituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me ($-CH_3$), Et ($-CH_2CH_3$), iPr (-CH $(CH_3)_2$), nPr ($-CH_2CH_2CH_3$), n-Bu ($-CH_2CH_2CH_2CH_3$) or i-Bu ($-CH_2CH$ $(CH_3)_2$).

"$C_{2-6}$ alkenyl" refers to a radical of a straight or branched hydrocarbon group having 2 to 6 carbon atoms and at least one carbon-carbon double bond. In some embodiments, $C_{2-4}$ alkenyl is alternative. Examples of $C_{2-6}$ alkenyl include vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. The term "$C_{2-6}$ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"$C_{2-6}$ alkynyl" refers to a radical of a straight or branched hydrocarbon group having 2 to 6 carbon atoms, at least one carbon-carbon triple bond and optionally one or more carbon-carbon double bonds. In some embodiments, $C_{2-4}$ alkynyl is alternative. Examples of $C_{2-6}$ alkynyl include, but are not limited to, ethynyl ($C_2$), 1-propynyl ($C_3$), 2-propynyl ($C_3$),

1-butynyl ($C_4$), 2-butynyl ($C_4$), pentynyl ($C_5$), hexynyl ($C_6$), etc. The term "$C_{2-6}$ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"Halo" or "halogen" refers to fluorine (F), chlorine (Cl), bromine (Br) and iodine (I).

Thus, "$C_{1-6}$ haloalkyl" refers to the above "$C_{1-6}$ alkyl", which is substituted with one or more halogen. In some embodiments, $C_{1-4}$ haloalkyl is yet alternative, and still alternatively $C_{1-2}$ haloalkyl. Exemplary haloalkyl groups include, but are not limited to, $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, and the like. The haloalkyl can be substituted at any available point of attachment, for example, with 1 to 5 substituents, 1 to 3 substituents or 1 substituent.

"$C_{3-10}$ cycloalkyl" refers to a radical of a non-aromatic cyclic hydrocarbon group having from 3 to 10 ring carbon atoms and zero heteroatoms. In some embodiments, $C_{4-10}$ cycloalkyl, $C_{3-7}$ cycloalkyl and $C_{3-6}$ cycloalkyl are yet alternative, and still alternatively $C_{5-6}$ cycloalkyl. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl can be substituted with one or more substituents, for example, with 1 to 5 substituents, 1 to 3 substituents or 1 substituent.

"3-10 membered heterocyclyl" refers to a radical of 3-10 membered non-aromatic ring system having ring carbon atoms and 1 to 5 ring heteroatoms, wherein each of the heteroatoms is independently selected from nitrogen, oxygen, sulfur, boron, phosphorus and silicon. In the heterocyclyl containing one or more nitrogen atoms, the point of attachment can be a carbon or nitrogen atom as long as the valence permits. In some embodiments, 3-7 membered heterocyclyl is alternative, which is a radical of 3-7 membered non-aromatic ring system having ring carbon atoms and 1 to 4 ring heteroatoms. 3-6 membered heterocyclyl is alternative, which is a radical of 3-6 membered non-aromatic ring system having ring carbon atoms and 1 to 3 ring heteroatoms. 5-8 membered heterocyclyl is alternative, which is a radical of 5-8 membered non-aromatic ring system having ring carbon atoms and 1 to 3 ring heteroatoms. 5-6 membered heterocyclyl is yet alternative, which is a radical of 5-6 membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc. The heterocyclyl can be substituted with one or more substituents, for example, with 1 to 5 substituents, 1 to 3 substituents or 1 substituent.

"$C_{6-10}$ aryl" refers to a radical of monocyclic or polycyclic (e.g., bicyclic) 4n+2 aromatic ring system having 6-10 ring carbon atoms and zero heteroatoms (e.g., having 6 or 10 shared $\pi$ electrons in a cyclic array). In some embodiments, the aryl group has six ring carbon atoms ("$C_6$ aryl"; for example, phenyl). In some embodiments, the aryl group has ten ring carbon atoms ("$C_{10}$ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"5-14 membered heteroaryl" refers to a radical of 5-14 membered monocyclic or bicyclic 4n+2 aromatic ring

system (e.g., having 6, 10 or 14 shared π electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5-10 membered heteroaryl groups are alternative, which are radicals of 5-10 membered monocyclic or bicyclic 4n+2 aromatic ring systems having ring carbon atoms and 1-4 ring heteroatoms. In other embodiments, 5-6 membered heteroaryl groups are yet alternative, which are radicals of 5-6 membered monocyclic or bicyclic 4n+2 aromatic ring systems having ring carbon atoms and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4- oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

"Oxo" represents =O.

Alkyl, alkenyl, alkynyl, cycloalkyl, heterocyclyl, aryl and heteroaryl as defined herein are optionally substituted groups.

Exemplary substituents on carbon atoms include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 $R^{dd}$ groups;

or two geminal hydrogen on a carbon atom are replaced with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$ groups;

each of the $R^{aa}$ is independently selected from alkyl, haloalkyl, alkenyl, alkynyl, cycloalkyl, heterocyclyl, aryl and heteroaryl, or two of the $R^{aa}$ groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{bb}$ is independently selected from hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{bb}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, cycloalkyl, heterocyclyl, aryl and heteroaryl, or two $R^{cc}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{dd}$ is independently selected from halogen, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3{}^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, -C(=O)

N $(R^{ff})_2$, -OC(=O)N $(R^{ff})_2$, $-NR^{ff}C(=O)R^{ee}$, $-NR^{ff}CO_2R^{ee}$, - $NR^{ff}C(=O)N$ $(R^{ff})_2$, $-C(=NR^{ff})OR^{ee}$, $-OC(=NR^{ff})R^{ee}$, $-OC(=NR^{ff})OR^{ee}$, $-C(=NR^{ff})N$ $(R^{ff})_2$, - $OC(=NR^{ff})N$ $(R^{ff})_2$, $-NR^{ff}C(=NR^{ff})N$ $(R^{ff})_2$, $-NR^{ff}SO_2R^{ee}$, $-SO_2N$ $(R^{ff})_2$, $-SO_2R^{ee}$, $-SO_2OR^{ee}$, $-OSO_2R^{ee}$, $-S(=O)R^{ee}$, -Si $(R^{ee})_3$, -OSi $(R^{ee})_3$, -C(=S)N $(R^{ff})_2$, $-C(=O)SR^{ee}$, $-C(=S)SR^{ee}$, $-SC(=S)SR^{ee}$, -P $(=O)_2R^{ee}$, -P $(=O)(R^{ee})_2$, -OP $(=O)(R^{ee})_2$, -OP $(=O)(OR^{ee})_2$, alkyl, haloalkyl, alkenyl, alkynyl, cycloalkyl, heterocyclyl, aryl, heteroaryl, wherein each of the alkyl, alkenyl, alkynyl, cycloalkyl, heterocyclyl, aryl and heteroaryl is independently substituted with 0, 1, 2, 3, 4 or 5 $R^{gg}$ groups, or two geminal $R^{dd}$ substituents can be combined to form =O or =S;

each of the $R^{ee}$ is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, cycloalkyl, heterocyclyl, aryl and heteroaryl, or two $R^{ff}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{gg}$ is independently selected from halogen, -CN, $-NO_2$, $-N_3$, $-SO_2H$, $-SO_3H$, -OH, $-OC_{1-6}$ alkyl, -ON ($C_{1-6}$ alkyl)$_2$, -N ($C_{1-6}$ alkyl)$_2$, -N ($C_{1-6}$ alkyl)$_3{}^+X^-$, -NH ($C_{1-6}$ alkyl)$_2{}^+X^-$, $-NH_2$ ($C_{1-6}$ alkyl)$^+X^-$, $-NH_3{}^+X^-$, -N ($OC_{1-6}$ alkyl)($C_{1-6}$ alkyl), -N (OH)($C_{1-6}$ alkyl), -NH (OH), -SH, $-SC_{1-6}$ alkyl, - SS($C_{1-6}$ alkyl), -C(=O)($C_{1-6}$ alkyl), $-CO_2H$, $-CO_2$ ($C_{1-6}$ alkyl), -OC(=O)($C_{1-6}$ alkyl), $-OCO_2$ ($C_{1-6}$ alkyl), $-C(=O)NH_2$, -C(=O)N ($C_{1-6}$ alkyl)$_2$, -OC(=O)NH ($C_{1-6}$ alkyl), -NHC(=O)($C_{1-6}$ alkyl), -N ($C_{1-6}$ alkyl)C(=O)($C_{1-6}$ alkyl), $-NHCO_2$ ($C_{1-6}$ alkyl), -NHC(=O)N ($C_{1-6}$ alkyl)$_2$, -NHC(=O)NH ($C_{1-6}$ alkyl), - $NHC(=O)NH_2$, -C(=NH)O($C_{1-6}$ alkyl), -OC(=NH)($C_{1-6}$ alkyl), -OC(=NH)$OC_{1-6}$ alkyl, -C(=NH)N ($C_{1-6}$ alkyl)$_2$, -C(=NH) NH ($C_{1-6}$ alkyl), $-C(=NH)NH_2$, -OC(=NH)N ($C_{1-6}$ alkyl)$_2$, -OC(NH)NH ($C_{1-6}$ alkyl), $-OC(NH)NH_2$, -NHC(NH)N ($C_{1-6}$ alkyl)$_2$, $-NHC(=NH)NH_2$, $-NHSO_2$ ($C_{1-6}$ alkyl), $-SO_2N$ ($C_{1-6}$ alkyl)$_2$, $-SO_2NH$ ($C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, -Si ($C_{1-6}$ alkyl)$_3$, -OSi ($C_{1-6}$ alkyl)$_3$, -C(=S)N ($C_{1-6}$ alkyl)$_2$, C(=S)NH ($C_{1-6}$ alkyl), $C(=S)NH_2$, -C(=O)S($C_{1-6}$ alkyl), $-C(=S)SC_{1-6}$ alkyl, $-SC(=S)SC_{1-6}$ alkyl, -P $(=O)_2$ ($C_{1-6}$ alkyl), -P (=O)($C_{1-6}$ alkyl)$_2$, -OP (=O)($C_{1-6}$ alkyl)$_2$, -OP (=O)($OC_{1-6}$ alkyl)$_2$, $C_{1-6}$ alkyl, $C_{1-6}$ haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, $C_5$-$C_{10}$ heteroaryl; or two geminal $R^{gg}$ substituents may combine to form =O or =S; wherein $X^-$ is a counter-ion.

Exemplary substituents on nitrogen atoms include, but are not limited to, hydrogen, -OH, - $OR^{aa}$, -N $(R^{cc})_2$, -CN, $-C(=O)R^{aa}$, -C(=O)N $(R^{cc})_2$, $-CO_2R^{aa}$, $-SO_2R^{aa}$, $-C(=NR^{bb})R^{aa}$, $-C(=NR^{cc})OR^{aa}$, - $C(=NR^{cc})N$ $(R^{cc})_2$, $-SO_2N$ $(R^{cc})_2$, $-SO_2R^{cc}$, $-SO_2OR^{cc}$, $-SOR^{aa}$, -C(=S)N $(R^{cc})_2$, $-C(=O)SR^{cc}$, - $C(=S)SR^{cc}$, -P $(=O)_2R^{aa}$, -P $(=O)(R^{aa})_2$, -P $(=O)_2N$ $(R^{cc})_2$, -P $(=O)(NR^{cc})_2$, alkyl, haloalkyl, alkenyl, alkynyl, cycloalkyl, heterocyclyl, aryl and heteroaryl, or two $R^{cc}$ groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

Other Definitions

The term "cancer" includes, but is not limited to, the following cancers: breast, ovary, cervix, prostate, testis, esophagus, stomach, skin, lung, bone, colon, pancreas, thyroid, biliary tract, buccal cavity and pharynx (mouth), lips, tongue, oral cavity, pharynx, small intestine, colorectal, large intestine, rectum, cancer of brain and central nervous system, glioblastoma, neuroblastoma, keratoacanthoma, epidermoid carcinoma, large cell carcinoma, adenocarcinoma, adenoma, follicular carcinoma, undifferentiated carcinoma, papillary carcinoma, seminoma, melanoma, sarcoma, bladder cancer, liver cancer, kidney cancer, bone marrow disorder, lymphatic disorder, Hodgkin's disease, hairy cell carcinoma and leukemia.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient

amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "humam", "patient" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment ").

Generally, the "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

## BRIEF DESCRIPTION OF THE DRAWINGS

Fig. 1 is a graph showing the effects of compounds D078, Osimertinib and Brigatinib on the protein levels of $EGFR^{L858R/T790M/C797S}$ proteins.

Fig. 2 is a graph showing the effects of compound D552 and comparative compound L527 on the protein levels of $EGFR^{L858R/T790M/C797S}$ proteins.

Fig. 3 is a graph showing the effects of compounds D058, D552 and Osimertinib on the protein levels of $EGFR^{Del19/T790M/C797S}$ proteins.

## DETAILED DESCRIPTION OF THE INVENTION

As used herein, the term "compound disclosed herein" refers to the following compounds of formula (A) or (X) (including sub general formulas, such as formula (I), (II), (III) etc), or a pharmaceutically acceptable salt, enantiomer, diastereomer, racemate, solvate, hydrate, polymorph, prodrug, or isotopic variant thereof, or a mixture thereof.

In the present disclosure, compounds are named using standard nomenclature. For compounds having an asymmetric center, it should be understood, unless otherwise stated, that all optical isomers and mixtures thereof are included. Furthermore, unless otherwise specified, all isomer compounds and carbon-carbon double bonds included in the present disclosure may occur in the form of Z and E. Compounds which exist in different tautomeric forms, one of which is not limited to any particular tautomer, but is intended to cover all tautomeric forms.

In one embodiment, the present disclosure relates to a compound of general formula (A) or (X), or a pharmaceutically acceptable salt, enantiomer, diastereomer, racemate, solvate, hydrate, polymorph, prodrug, or isotopic variant thereof, or a mixture thereof:

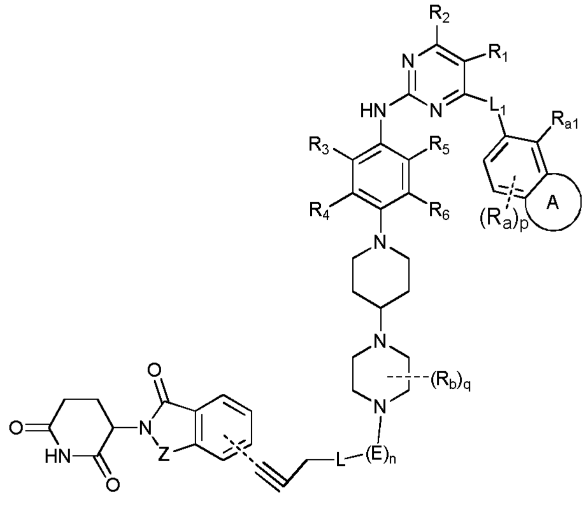

(A)

wherein

----- represents that the point of attachment to the rest of the molecule can be located at an available point of a ring where it is located;

Ring A is absent, or is selected from $C_{4-10}$ cycloalkyl, 5-8 membered heterocyclyl, $C_{6-10}$ aryl and 5-14 membered heteroaryl;

$R_1$ is selected from H, halogen, -OH, $-NH_2$, -CN, $C_{1-6}$ alkyl and $C_{1-6}$ haloalkyl;

$R_2$ is selected from H, halogen, -OH, $-NH_2$, -CN, $C_{1-6}$ alkyl and $C_{1-6}$ haloalkyl;

$R_3$ is selected from H, halogen, -OH, $-NH_2$, -CN, $C_{1-6}$ alkyl, $C_{1-6}$ haloalkyl, $-OC_{1-6}$ alkyl, $-OC_{1-6}$ haloalkyl, $C_{2-6}$ alkenyl and $C_{2-6}$ alkynyl;

$R_4$ is selected from H, halogen, -OH, $-NH_2$, -CN, $C_{1-6}$ alkyl and $C_{1-6}$ haloalkyl;

$R_5$ is selected from H, halogen, -OH, $-NH_2$, -CN, $C_{1-6}$ alkyl and $C_{1-6}$ haloalkyl;

$R_6$ is selected from $C_{1-6}$ alkyl, $C_{1-6}$ haloalkyl, -NH-C(O)-CR=CR'R" and -NH-C(O)-C≡CR';

wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10

membered heteroaryl are each optionally substituted with 0, 1, 2, 3, 4 or 5 R*, as long as the valence permits;
R* is selected from H, halogen, $-OR_c$, $-NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_{a1}$ is selected from

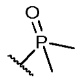
and
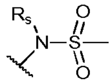
;

$R_s$ is selected from H, $C_{1-6}$ alkyl and $C_{1-6}$ haloalkyl;
$R_a$ is selected from H, halogen, -OH, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$;
alternatively, two $R_a$ on the same carbon atom or on different carbon atoms are taken together to form a $C_{4-10}$ cycloalkyl, a 5-8 membered heterocyclyl, a $C_{6-10}$ aryl, or a 5-10 membered heteroaryl
$R_b$ is selected from H, halogen, -OH, $-NH_2$, -CN, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl, a 3-10 membered heterocyclyl, a $C_{6-10}$ aryl, or a 5-10 membered heteroaryl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;
L is selected from a chemical bond, -O-, -NR#-, -CR#R#'-, -S-, -SO- and $-S(O)_2-$;
$L_1$ is selected from a chemical bond, -O-, -NR#-, -CR#R#'-, -S-, -SO- and $-S(O)_2-$;
E is selected from a chemical bond and -CR#R#'-;
wherein R# and R#' are independently selected from H, halogen, -OH, $-NH_2$, -CN, $C_{1-6}$ alkyl, $C_{1-6}$ haloalkyl, $C_{2-6}$ alkenyl, and $C_{2-6}$ alkynyl;
p is 0, 1, 2, 3, or 4;
q is 0, 1, 2, 3, or 4;
n is 0, 1, 2, 3, 4, or 5.

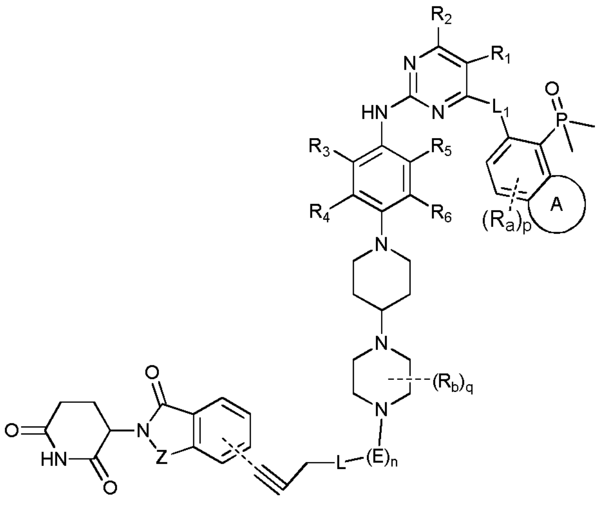
(X)

wherein

----- represents that the point of attachment to the rest of the molecule can be located at an available point of a ring where it is located;
Ring A is absent, or is selected from $C_{4-10}$ cycloalkyl, 5-8 membered heterocyclyl, $C_{6-10}$ aryl and 5-14 membered heteroaryl;
$R_1$ is selected from H, halogen, -OH, $-NH_2$, -CN, $C_{1-6}$ alkyl and $C_{1-6}$ haloalkyl;

$R_2$ is selected from H, halogen, -OH, $-NH_2$, -CN, $C_{1-6}$ alkyl and $C_{1-6}$ haloalkyl;
$R_3$ is selected from H, halogen, -OH, $-NH_2$, -CN, $C_{1-6}$ alkyl, $C_{1-6}$ haloalkyl, $-OC_{1-6}$ alkyl, $-OC_{1-6}$ haloalkyl, $C_{2-6}$ alkenyl and $C_{2-6}$ alkynyl;
$R_4$ is selected from H, halogen, -OH, $-NH_2$, -CN, $C_{1-6}$ alkyl and $C_{1-6}$ haloalkyl;
$R_5$ is selected from H, halogen, -OH, $-NH_2$, -CN, $C_{1-6}$ alkyl and $C_{1-6}$ haloalkyl;
$R_6$ is selected from -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, $-OR_c$, $-NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_a$ is selected from H, halogen, -OH, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$;
alternatively, two $R_a$ on the same carbon atom or on different carbon atoms are taken together to form a $C_{4-10}$ cycloalkyl, a 5-8 membered heterocyclyl, a $C_{6-10}$ aryl, or a 5-10 membered heteroaryl
$R_b$ is selected from H, halogen, -OH, $-NH_2$, -CN, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl, a 3-10 membered heterocyclyl, a $C_{6-10}$ aryl, or a 5-10 membered heteroaryl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;
L is selected from a chemical bond, -O-, -NR#-, -CR#R#'-, -S-, -SO- and $-S(O)_2-$;
$L_1$ is selected from a chemical bond, -O-, -NR#-, -CR#R#'-, -S-, -SO- and $-S(O)_2-$;
E is selected from a chemical bond and -CR#R#'-;
wherein R# and R#' are independently selected from H, halogen, -OH, $-NH_2$, -CN, $C_{1-6}$ alkyl, $C_{1-6}$ haloalkyl, $C_{2-6}$ alkenyl, and $C_{2-6}$ alkynyl;
p is 0, 1, 2, 3, or 4;
q is 0, 1, 2, 3, or 4;
n is 0, 1, 2, 3, 4, or 5.

## Ring A

In one embodiment, ring A is absent; in another embodiment, ring A is $C_{4-10}$ cycloalkyl; in another embodiment, ring A is a 5-8 membered heterocyclyl; in another embodiment, ring A is $C_{6-10}$ aryl; in another embodiment, ring A is a 5-14 membered heteroaryl; in another embodiment, ring A is a 5-10 membered heteroaryl; in another embodiment, ring A is a 5-6 membered heteroaryl; in another embodiment, ring A is a 5-6 membered heterocyclyl; in another embodiment, ring A is

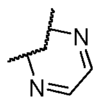

;

in another embodiment, ring A is

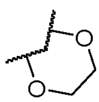

.

## $R_1$

In one embodiment, $R_1$ is H; in another embodiment, $R_1$ is halogen; in another embodiment, $R_1$ is -OH; in another embodiment, $R_1$ is $-NH_2$; in another embodiment, $R_1$ is -CN; in another embodiment, $R_1$ is $C_{1-6}$ alkyl; in another

embodiment, $R_1$ is $C_{1-4}$ alkyl; in another embodiment, $R_1$ is $C_{1-6}$ haloalkyl; in another embodiment, $R_1$ is $C_{1-4}$ haloalkyl; in another embodiment, $R_1$ is Cl; in another embodiment, $R_1$ is Br; in another embodiment, $R_1$ is $-CF_3$.

$R_2$

In one embodiment, $R_2$ is H; in another embodiment, $R_2$ is halogen; in another embodiment, $R_2$ is -OH; in another embodiment, $R_2$ is $-NH_2$; in another embodiment, $R_2$ is -CN; in another embodiment, $R_2$ is $C_{1-6}$ alkyl; in another embodiment, $R_2$ is $C_{1-4}$ alkyl; in another embodiment, $R_2$ is $C_{1-6}$ haloalkyl; in another embodiment, $R_2$ is $C_{1-4}$ haloalkyl.

$R_3$

In one embodiment, $R_3$ is H; in another embodiment, $R_3$ is halogen; in another embodiment, $R_3$ is -OH; in another embodiment, $R_3$ is $-NH_2$; in another embodiment, $R_3$ is -CN; in another embodiment, $R_3$ is $C_{1-6}$ alkyl; in another embodiment, $R_3$ is $C_{1-4}$ alkyl; in another embodiment, $R_3$ is $C_{1-6}$ haloalkyl; in another embodiment, $R_3$ is $C_{1-4}$ haloalkyl; in another embodiment, $R_3$ is $-OC_{1-6}$ alkyl; in another embodiment, $R_3$ is $-OC_{1-4}$ alkyl; in another embodiment, $R_3$ is $-OC_{1-6}$ haloalkyl; in another embodiment, $R_3$ is $-OC_{1-4}$ haloalkyl; in another embodiment, $R_3$ is $C_{2-6}$ alkenyl; in another embodiment, $R_3$ is $C_{2-6}$ alkynyl.

In one embodiment, $R_3$ is F; in another embodiment, $R_3$ is Cl; in another embodiment, $R_3$ is $- OCH_3$; in another embodiment, $R_3$ is $-OCF_3$.

$R_4$

In one embodiment, $R_4$ is H; in another embodiment, $R_4$ is halogen; in another embodiment, $R_4$ is -OH; in another embodiment, $R_4$ is $-NH_2$; in another embodiment, $R_4$ is -CN; in another embodiment, $R_4$ is $C_{1-6}$ alkyl; in another embodiment, $R_4$ is $C_{1-4}$ alkyl; in another embodiment, $R_4$ is $C_{1-6}$ haloalkyl; in another embodiment, $R_4$ is $C_{1-4}$ haloalkyl.

$R_5$

In one embodiment, $R_5$ is H; in another embodiment, $R_5$ is halogen; in another embodiment, $R_5$ is -OH; in another embodiment, $R_5$ is $-NH_2$; in another embodiment, $R_5$ is -CN; in another embodiment, $R_5$ is $C_{1-6}$ alkyl; in another embodiment, $R_5$ is $C_{1-4}$ alkyl; in another embodiment, $R_5$ is $C_{1-6}$ haloalkyl; in another embodiment, $R_5$ is $C_{1-4}$ haloalkyl.

$R_6$

In one embodiment, $R_6$ is $C_{1-6}$ alkyl; in another embodiment, $R_6$ is $C_{1-6}$ haloalkyl; in another embodiment, $R_6$ is -NH-C(O)-CR=CR'R"; in another embodiment, $R_6$ is -NH-C(O)-C≡CR'.

In one embodiment, $R_6$ is

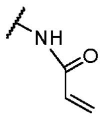

;

in another embodiment, $R_6$ is

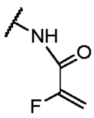

;

in another embodiment, $R_6$ is

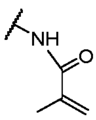

;

in another embodiment, $R_6$ is

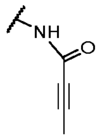;

in another embodiment, $R_6$ is

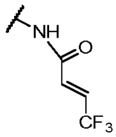;

in another embodiment, $R_6$ is

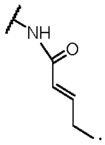;

in another embodiment, $R_6$ is

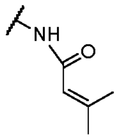;

in another embodiment, $R_6$ is

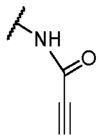;

in another embodiment, $R_6$ is

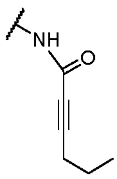;

in another embodiment, $R_6$ is

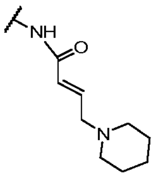;

in another embodiment, $R_6$ is

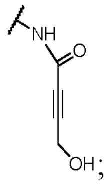

in another embodiment, $R_6$ is

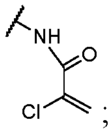

in another embodiment, $R_6$ is

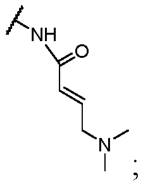

in another embodiment, $R_6$ is

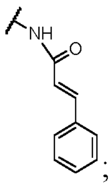

in another embodiment, $R_6$ is

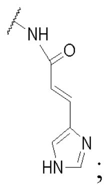

in another embodiment, $R_6$ is

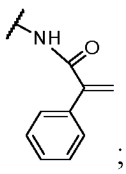

in another embodiment, $R_6$ is

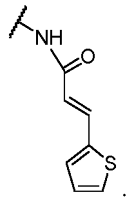

R

In one embodiment, R is H; in another embodiment, R is halogen; in another embodiment, R is F; in another embodiment, R is optionally substituted $C_{1-6}$ alkyl; in another embodiment, R is optionally substituted $C_{1-4}$ alkyl; in another embodiment, R is optionally substituted $C_{1-6}$ haloalkyl; in another embodiment, R is optionally substituted $C_{1-4}$ haloalkyl; in another embodiment, R is optionally substituted $C_{2-6}$ alkenyl; in another embodiment, R is optionally substituted $C_{2-6}$ alkynyl; in another embodiment, R is optionally substituted $C_{3-10}$ cycloalkyl; in another embodiment, R is optionally substituted $C_{3-7}$ cycloalkyl; in another embodiment, R is optionally substituted 3-10 membered heterocyclyl; in another embodiment, R is optionally substituted 3-7 membered heterocyclyl; in another embodiment, R is optionally substituted 5-6 membered heterocyclyl; in another embodiment, R is optionally substituted $C_{6-10}$ aryl; in another embodiment, R is an optionally substituted 5-10 membered heteroaryl; in another embodiment, R is an optionally substituted 5-6 membered heteroaryl.

In one embodiment, R is unsubstituted; in another embodiment, R is substituted with 1 R*; in another embodiment, R is substituted with 2 R*; in another embodiment, R is substituted with 3 R*; in another embodiment, R is substituted with 4 R*; in another embodiment, R is substituted with 5 R*; as long as the valence permits.

R'

In one embodiment, R' is H; in another embodiment, R' is halogen; in another embodiment, R' is F; in another embodiment, R' is optionally substituted $C_{1-6}$ alkyl; in another embodiment, R' is optionally substituted $C_{1-4}$ alkyl; in another embodiment, R' is optionally substituted $C_{1-6}$ haloalkyl; in another embodiment, R' is optionally substituted $C_{1-4}$ haloalkyl; in another embodiment, R' is optionally substituted $C_{2-6}$ alkenyl; in another embodiment, R' is optionally substituted $C_{2-6}$ alkynyl; in another embodiment, R' is optionally substituted $C_{3-10}$ cycloalkyl; in another embodiment, R' is optionally substituted $C_{3-7}$ cycloalkyl; in another embodiment, R' is an optionally substituted 3-10 membered heterocyclyl; in another embodiment, R' is an optionally substituted 3-7 membered heterocyclyl; in another embodiment, R' is an optionally substituted 5-6 membered heterocyclyl; in another embodiment, R' is an optionally substituted $C_{6-10}$ aryl; in another embodiment, R' is an optionally substituted 5-10 membered heteroaryl; in another embodiment, R' is an optionally substituted 5-6 membered heteroaryl.

In one embodiment, R' is unsubstituted; in another embodiment, R' is substituted with 1 R*; in another embodiment, R' is substituted with 2 R*; in another embodiment, R' is substituted with 3 R*; in another embodiment, R' is substituted with 4 R*; in another embodiment, R' is substituted with 5 R*; as long as the valence permits.

R"

In one embodiment, R" is H; in another embodiment, R" is halogen; in another embodiment, R" is F; in another embodiment, R" is optionally substituted $C_{1-6}$ alkyl; in another embodiment, R" is optionally substituted $C_{1-4}$ alkyl; in another embodiment, R" is optionally substituted $C_{1-6}$ haloalkyl; in another embodiment, R" is optionally substituted $C_{1-4}$ haloalkyl; in another embodiment, R" is optionally substituted $C_{2-6}$ alkenyl; in another embodiment, R" is optionally substituted $C_{2-6}$ alkynyl; in another embodiment, R" is optionally substituted $C_{3-10}$ cycloalkyl; in another embodiment, R" is optionally substituted $C_{3-7}$ cycloalkyl; in another embodiment, R" is an optionally substituted 3-10 membered heterocyclyl; in another embodiment, R" is an optionally substituted 3-7 membered heterocyclyl; in another embodiment, R" is an optionally substituted 5-6 membered heterocyclyl; in another embodiment, R" is an optionally substituted $C_{6-10}$ aryl; in another embodiment, R" is an optionally substituted 5-10 membered heteroaryl; in another embodiment, R" is an optionally substituted 5-6 membered heteroaryl.

In one embodiment, R" is unsubstituted; in another embodiment, R" is substituted with 1 R*; in another embodiment, R" is substituted with 2 R*; in another embodiment, R" is substituted with 3 R*; in another embodiment, R" is substituted with 4 R*; in another embodiment, R" is substituted with 5 R*; as long as the valence permits.

R*

In one embodiment, R* is H; in another embodiment, R* is halogen; in another embodiment, R* is -$OR_c$; in another embodiment, R* is -OH; in another embodiment, R* is -$NR_cR_d$; in another embodiment, R* is -$NH_2$; in another embodiment, R* is -CN; in another embodiment, R* is $C_{1-6}$ alkyl; in another embodiment, R* is $C_{1-4}$ alkyl; in another embodiment, R* is $C_{1-6}$ haloalkyl; in another embodiment, R * is $C_{1-4}$ haloalkyl; in another embodiment, R* is $C_{2-6}$ alkenyl; in another embodiment, R* is $C_{2-6}$ alkynyl; in another embodiment, R* is $C_{3-10}$ cycloalkyl; in another embodiment, R* is $C_{3-7}$ cycloalkyl; in another embodiment, R* is 3-10 membered heterocyclyl; in another embodiment, R* is 5-6 membered heterocyclyl; in another embodiment, R* is $C_{6-10}$ aryl; In another specific embodiment, R* is a 5-10 membered heteroaryl; in another specific embodiment, R* is a 5-6 membered heteroaryl.

Z

In one embodiment, Z is $CH_2$; in another embodiment, Z is C=O; in another embodiment, Z is C=S; in another embodiment, Z is C NH.

$R_{a1}$

In one specific embodiment, $R_{a1}$ is

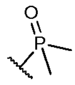

;

in another specific embodiment, $R_{a1}$ is

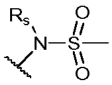

;

in another specific embodiment, $R_{a1}$ is

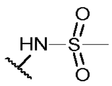

;

in another specific embodiment, $R_{a1}$ is

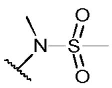

;

in another specific embodiment, $R_{a1}$ is

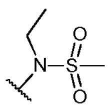

.

$R_a$

In one embodiment, $R_a$ is H; in another embodiment, $R_a$ is halogen; in another embodiment, $R_a$ is -OH; in another embodiment, $R_a$ is $C_{1-6}$ alkyl; in another embodiment, $R_a$ is $C_{1-4}$ alkyl; in another embodiment, $R_a$ is $C_{1-6}$ haloalkyl; in another embodiment, $R_a$ is $C_{1-4}$ haloalkyl; in another embodiment, $R_a$ is $C_{2-6}$ alkenyl; in another embodiment, $R_a$ is $C_{2-6}$ alkynyl; in another embodiment, $R_a$ is -CN; in another embodiment, $R_a$ is -$NR_cR_d$; in another embodiment, $R_a$ is -$NH(CH_2)_{0-5}R_c$; in another embodiment, $R_a$ is -$NHC(O)(CH_2)_{0-5}R_c$.

In one embodiment, two $R_a$ on the same carbon atom or on different carbon atoms are taken together to form a

$C_{4-10}$ cycloalkyl; in one embodiment, two $R_a$ on the same carbon atom or on different carbon atoms are taken together to form a $C_{3-7}$ cycloalkyl; in one embodiment, two $R_a$ on the same carbon atom or on different carbon atoms are taken together to form a 5-8 membered heterocyclyl; in one embodiment, two $R_a$ on the same carbon atom or on different carbon atoms are taken together to form a $C_{6-10}$ aryl; in one embodiment, two $R_a$ on the same carbon atom or on different carbon atoms are taken together to form a 5-10 membered heteroaryl.

$R_b$

In one embodiment, $R_b$ is H; in another embodiment, $R_b$ is halogen; in another embodiment, $R_b$ is -OH; in another embodiment, $R_b$ is $-NH_2$; in another embodiment, $R_b$ is -CN; in another embodiment, $R_b$ is $C_{1-6}$ alkyl; in another embodiment, $R_b$ is $C_{1-4}$ alkyl; in another embodiment, $R_b$ is $C_{1-6}$ haloalkyl; in another embodiment, $R_b$ is $C_{1-4}$ haloalkyl; in another embodiment, $R_b$ is $C_{2-6}$ alkenyl; in another embodiment, $R_b$ is $C_{2-6}$ alkynyl; in another embodiment, $R_b$ is $-CH_3$.

In one embodiment, two $R_b$ on the same carbon atom or on different carbon atoms are taken together to form $C_{3-10}$ cycloalkyl; in one embodiment, two $R_b$ on the same carbon atom or on different carbon atoms are taken together to form $C_{3-7}$ cycloalkyl; in one embodiment, two $R_b$ on the same carbon atom or on different carbon atoms are taken together to form 3-10 membered heterocyclyl; in one embodiment, two $R_b$ on the same carbon atom or on different carbon atoms are taken together to form 5-8 membered heterocyclyl; in one embodiment, two $R_b$ on the same carbon atom or on different carbon atoms are taken together to form 5-6 membered heterocyclyl; in one embodiment, two $R_b$ on the same carbon atom or on different carbon atoms are taken together to form $C_{6-10}$ aryl; in one embodiment, two $R_b$ on the same carbon atom or on different carbon atoms are taken together to form 5-10 membered heteroaryl; in one embodiment, two $R_b$ on the same carbon atom or on different carbon atoms are taken together to form 5-6 membered heteroaryl; in one embodiment, two $R_b$ on the same carbon atom or on different carbon atoms are taken together to form cyclopropyl.

$R_c$ and $R_d$

In one embodiment, $R_c$ is H; in another embodiment, $R_c$ is -OH; in another embodiment, $R_c$ is $-NH_2$; in another embodiment, $R_c$ is halogen; in another embodiment, $R_c$ is $C_{1-6}$ alkyl; in another embodiment, $R_c$ is $C_{1-4}$ alkyl; in another embodiment, $R_c$ is $C_{1-6}$ haloalkyl; in another embodiment, $R_c$ is $C_{1-4}$ haloalkyl; in another embodiment, $R_c$ is $C_{3-10}$ cycloalkyl; in another embodiment, $R_c$ is $C_{3-7}$ cycloalkyl; in another embodiment, $R_c$ is 3-10 membered heterocyclyl; in another embodiment, $R_c$ is 5-6 membered heterocyclyl; in another embodiment, $R_c$ is $C_{6-10}$ aryl; in another embodiment, $R_c$ is 5-10 membered heteroaryl; in another embodiment, $R_c$ is 5-6 membered heteroaryl.

In one embodiment, $R_d$ is H; in another embodiment, $R_d$ is -OH; in another embodiment, $R_d$ is $-NH_2$; in another embodiment, $R_d$ is halogen; in another embodiment, $R_d$ is $C_{1-6}$ alkyl; in another embodiment, $R_d$ is $C_{1-4}$ alkyl; in another embodiment, $R_d$ is $C_{1-6}$ haloalkyl; in another embodiment, $R_d$ is $C_{1-4}$ haloalkyl; in another embodiment, $R_d$ is $C_{3-10}$ cycloalkyl; in another embodiment, $R_d$ is $C_{3-7}$ cycloalkyl; in another embodiment, $R_d$ is 3-10 membered heterocyclyl; in another embodiment, $R_d$ is 5-6 membered heterocyclyl; in another embodiment, $R_d$ is $C_{6-10}$ aryl; in another embodiment, $R_d$ is 5-10 membered heteroaryl; in another embodiment, $R_d$ is 5-6 membered heteroaryl.

In one embodiment, $R_c$ and $R_d$ are taken together with the N atom to which they are attached to form a 3-10 membered heterocyclyl; in another embodiment, $R_c$ and $R_d$ are taken together with the N atom to which they are attached to form a 3-7 membered heterocyclyl; in another embodiment, $R_c$ and $R_d$ are taken together with the N atom to which they are attached to form a 5-6 membered heterocyclyl.

L

In one embodiment, L is a chemical bond; in another embodiment, L is -O-; in another embodiment, L is -NR#-; in another embodiment, L is -CR#R#'-; in another embodiment, L is -S-; in another embodiment, L is -SO-; in another embodiment, L is $-S(O)_2-$.

$L_1$

In one embodiment, $L_1$ is a chemical bond; in another embodiment, $L_1$ is -O-; in another embodiment, $L_1$ is -NR#-; in another embodiment, $L_1$ is -CR#R#'-; in another embodiment, $L_1$ is -S-; in another embodiment, $L_1$ is -SO-; in another embodiment, $L_1$ is $-S(O)_2-$.

E

In one embodiment, E is a chemical bond; in another embodiment, E is -CR#R#'-.

R# and R#'

In one embodiment, R# is H; in another embodiment, R# is halogen; in another embodiment, R# is -OH; in another embodiment, R# is -$NH_2$; in another embodiment, R# is -CN; in another embodiment, R# is $C_{1-6}$ alkyl; in another embodiment, R# is $C_{1-4}$ alkyl; in another embodiment, R# is $C_{1-6}$ haloalkyl; in another embodiment, R# is $C_{1-4}$ haloalkyl; in another embodiment, R# is $C_{2-6}$ alkenyl; in another embodiment, R# is $C_{2-6}$ alkynyl.

In one embodiment, R#' is H; in another embodiment, R#' is halogen; in another embodiment, R#' is -OH; in another embodiment, R#' is -$NH_2$; in another embodiment, R#' is -CN; in another embodiment, R#' is $C_{1-6}$ alkyl; in another embodiment, R#' is $C_{1-4}$ alkyl; in another embodiment, R#' is $C_{1-6}$ haloalkyl; in another embodiment, R#' is $C_{1-4}$ haloalkyl; in another embodiment, R#' is $C_{2-6}$ alkenyl; in another embodiment, R#' is $C_{2-6}$ alkynyl.

p

In one embodiment, p is 0; in another embodiment, p is 1; in another embodiment, p is 2; in another embodiment, p is 3; in another embodiment, p is 4; in another embodiment, p is 5.

q

In one embodiment, q is 0; in another embodiment, q is 1; in another embodiment, p is 2; in another embodiment, q is 3; in another embodiment, q is 4; in another embodiment, q is 5.

n

In one embodiment, n is 0; in another embodiment, n is 1; in another embodiment, n is 2; in another embodiment, n is 3; in another embodiment, n is 4; in another embodiment, n is 5.

Any technical solution in any of the above specific embodiments or any combination thereof can be combined with any technical solution in other specific embodiments or any combination thereof. For example, any technical solution or any combination thereof of Ring A can be combined with any technical solution or any combination thereof of L, $L_1$, E, $R_1$-$R_6$, $R_{a1}$, $R_s$, $R_a$, $R_b$, p, q and n, etc. The present disclosure is intended to include all combinations of these technical solutions, which are not listed one by one due to space limitations.

In a more specific embodiment, the present disclosure provides a compound of the above-mentioned general formula (X), or a pharmaceutically acceptable salt, enantiomer, diastereomer, racemate, solvate, hydrate, polymorph, prodrug or isotopic variant thereof, or a mixture thereof, wherein the compound has the following general structure:

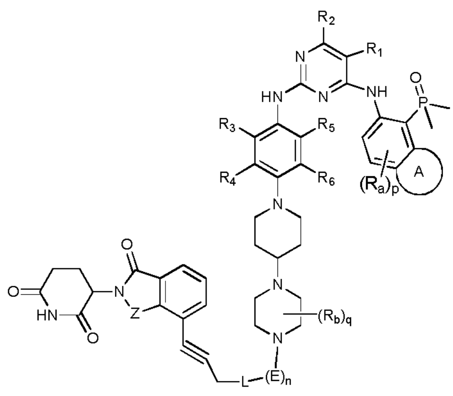
(I),

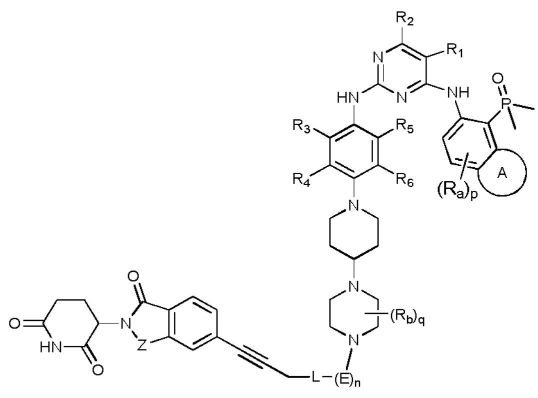
(II),

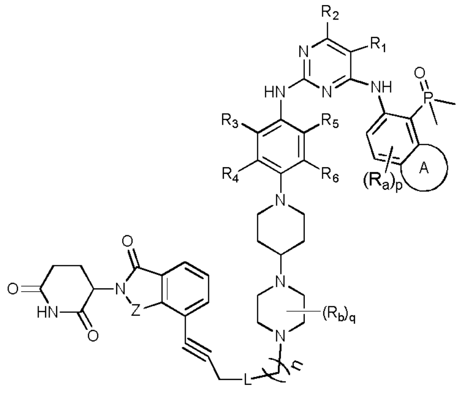

(III),

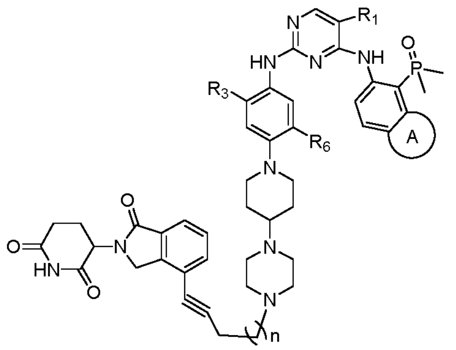

(IV),

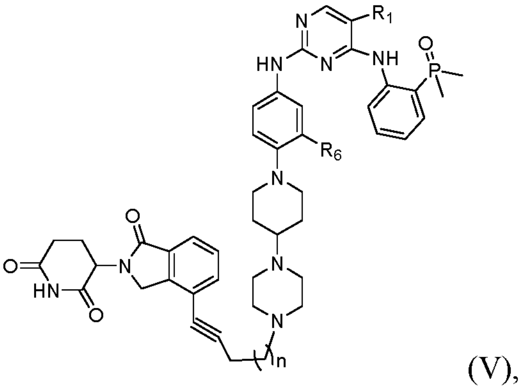

(V),

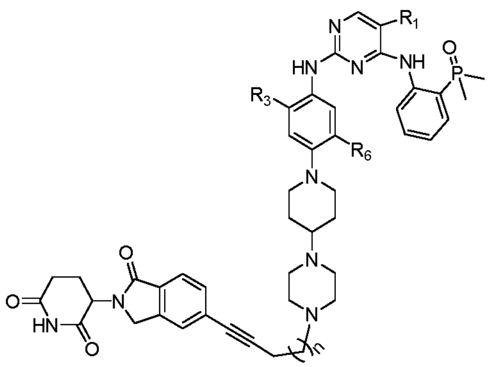

(VI),

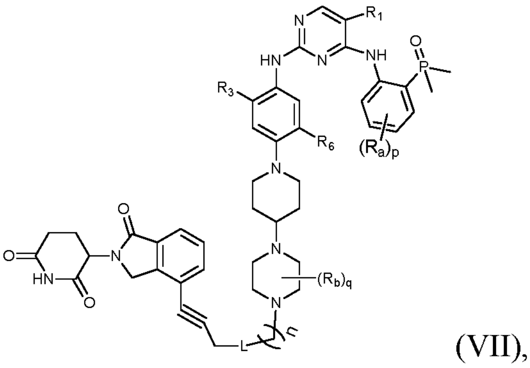

(VII),

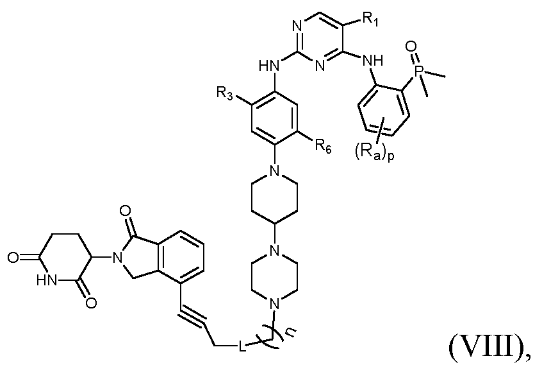

(VIII),

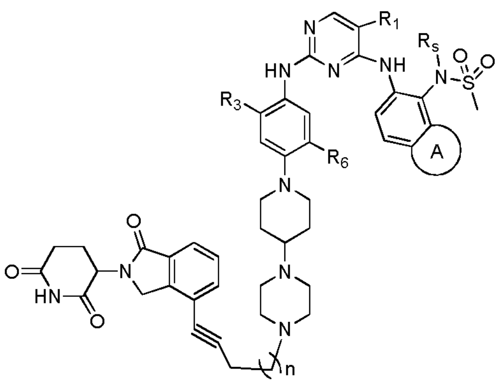

(IX)

wherein each group is as defined above.

In a more specific embodiment, the present disclosure provides a compound of formula (III), or a pharmaceutically acceptable salt, enantiomer, diastereomer, racemate, solvate, hydrate, polymorph, prodrug or isotopic variant

thereof, or a mixture thereof:

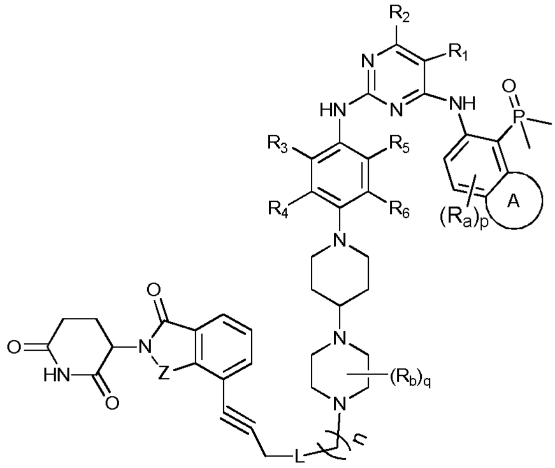

(III)

wherein

Ring A is absent, or is selected from $C_{4-10}$ cycloalkyl, 5-8 membered heterocyclyl, $C_{6-10}$ aryl and 5-10 membered heteroaryl;
$R_1$ is selected from H, halogen, $C_{1-6}$ alkyl and $C_{1-6}$ haloalkyl;
$R_2$ is selected from H, halogen, $C_{1-6}$ alkyl and $C_{1-6}$ haloalkyl;
$R_3$ is selected from H, halogen, $C_{1-6}$ alkyl, $C_{1-6}$ haloalkyl, -O$C_{1-6}$ alkyl, -O$C_{1-6}$ haloalkyl, $C_{2-6}$ alkenyl and $C_{2-6}$ alkynyl,
$R_4$ is selected from H, halogen, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from -NH-C(O)-CR=CR'R" and -NH-C(O)-C=CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, -$OR_c$, -$NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_a$ is selected from H, halogen, $C_{1-6}$ alkyl, $C_{1-6}$ haloalkyl, $C_{2-6}$ alkenyl, $C_{2-6}$ alkynyl, -CN, - $NR_cR_d$, -NH$(CH_2)_{0-5}R_c$ and -NHC(O)$(CH_2)_{0-5}R_c$;
$R_b$ is selected from H, halogen, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl or a 3-10 membered heterocyclyl;
$R_c$ is selected from H, -OH, -$NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, -$NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;
L is selected from a chemical bond, -O-, -NR#-, -S-, -SO- and -$S(O)_2$-;
wherein R# is selected from H, halogen, $C_{1-6}$ alkyl, $C_{1-6}$ haloalkyl, $C_{2-6}$ alkenyl and $C_{2-6}$ alkynyl;
p is 0, 1, 2, 3, or 4;
q is 0, 1, 2, 3, or 4;
n is 0, 1, 2, 3, 4, or 5.

In a more specific embodiment, the present disclosure provides a compound of the above-mentioned general formula (III), or a pharmaceutically acceptable salt, enantiomer, diastereomer, racemate, solvate, hydrate, polymorph, prodrug or isotopic variant thereof, or a mixture thereof, wherein:

Ring A is absent, or is selected from $C_{4-10}$ cycloalkyl, 5-8 membered heterocyclyl, $C_{6-10}$ aryl and 5-10 membered

heteroaryl;
$R_1$ is selected from H, halogen and $C_{1-6}$ haloalkyl;
$R_2$ is selected from H and halogen;
$R_3$ is selected from H, halogen, -O$C_{1-6}$ alkyl and -O$C_{1-6}$ haloalkyl;
$R_4$ is selected from H and halogen;
$R_5$ is selected from H and halogen;
$R_6$ is selected from -NH-C(O)-CR=CR'R" and -NH-C(O)-C=CR';
wherein R, R' and R" are each independently selected from H, halogen, $C_{1-6}$ alkyl, $C_{1-6}$ haloalkyl, $C_{3-10}$ cycloalkyl, 3-7 membered heterocyclyl, $C_{6-10}$ aryl and 5-10 membered heteroaryl, wherein the $C_{1-6}$ alkyl, $C_{1-6}$ haloalkyl, $C_{3-10}$ cycloalkyl, 3-7 membered heterocyclyl, $C_{6-10}$ aryl and 5-10 membered heteroaryl are each optionally substituted with 0, 1, 2 or 3 R*, as long as the valence permits;
R* is selected from H, halogen, -O$R_c$, -N$R_c$$R_d$, -CN, $C_{1-6}$ alkyl, $C_{1-6}$ haloalkyl, $C_{3-7}$ cycloalkyl, 5-6 membered heterocyclyl, $C_{6-10}$ aryl and 5-6 membered heteroaryl;
Z is selected from $CH_2$, C=O and C=S;
$R_a$ is selected from H, halogen, $C_{1-6}$ alkyl, $C_{1-6}$ haloalkyl, $C_{2-6}$ alkenyl, $C_{2-6}$ alkynyl, -CN, - N$R_c$$R_d$, -NH$(CH_2)_{0-5}$$R_c$ and -NHC(O)$(CH_2)_{0-5}$$R_c$;
$R_b$ is selected from H, $C_{1-6}$ alkyl and $C_{1-6}$ haloalkyl;
alternatively, two $R_b$ on the same carbon atom are taken together to form a $C_{3-7}$ cycloalkyl or a 5-6 membered heterocyclyl;
$R_c$ is selected from H, -OH, -$NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, -$NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
or $R_c$ and $R_d$ are taken together with the N atom to which they are attached to form 3-7 membered heterocyclyl;
L is selected from a chemical bond, -O-, -S- and -NR#-;
wherein R# is selected from H, halogen, $C_{1-4}$ alkyl and $C_{1-4}$ haloalkyl;
p is 0, 1, 2 or 3;
q is 0, 1, 2 or 3;
n is 1, 2, 3, 4 or 5.

In a more specific embodiment, the present disclosure provides a compound of the above-mentioned general formula (III), or a pharmaceutically acceptable salt, enantiomer, diastereomer, racemate, solvate, hydrate, polymorph, prodrug or isotopic variant thereof, or a mixture thereof, wherein:

Ring A is absent, or ring A is selected from a 5-6 membered heteroaryl and a 5-6 membered heterocyclyl;
$R_1$ is selected from halogen and $C_{1-4}$ haloalkyl;
$R_2$ is H;
$R_3$ is selected from H, halogen, -O$C_{1-4}$ alkyl and -O$C_{1-4}$ haloalkyl;
$R_4$ is H;
$R_5$ is H;
$R_6$ is selected from -NH-C(O)-CR=CR'R" and -NH-C(O)-C=CR';
wherein R is selected from H, halogen, $C_{1-4}$ alkyl and $C_{6-10}$ aryl, wherein the $C_{1-4}$ alkyl and $C_{6-10}$ aryl are each optionally substituted with 0, 1, or 2 R*;
R' and R" are each independently selected from H, $C_{1-4}$ alkyl, $C_{1-4}$ haloalkyl, $C_{3-7}$ cycloalkyl, 5-6 membered heterocyclyl, $C_{6-10}$ aryl and 5-6 membered heteroaryl, wherein the $C_{1-4}$ alkyl, $C_{1-4}$ haloalkyl, $C_{3-7}$ cycloalkyl, 5-6 membered heterocyclyl, $C_{6-10}$ aryl and 5-6 membered heteroaryl are each optionally substituted with 0, 1 or 2 R*, as long as the valence permits;
R* is selected from H, halogen, -O$R_c$, -N$R_c$$R_d$, $C_{1-4}$ alkyl, $C_{1-4}$ haloalkyl, $C_{3-7}$ cycloalkyl, 5-6 membered heterocyclyl, $C_{6-10}$ aryl and 5-6 membered heteroaryl;
Z is $CH_2$ or C=O;
$R_a$ is selected from H, halogen, $C_{1-6}$ alkyl, $C_{1-6}$ haloalkyl, $C_{2-6}$ alkenyl, $C_{2-6}$ alkynyl, -CN, - N$R_c$$R_d$, -NH$(CH_2)_{0-5}$$R_c$ and -NHC(O)$(CH_2)_{0-5}$Rc, alternatively H, halogen, -N$R_c$$R_d$ and -NH$(CH_2)_{0-5}$$R_c$, still alternatively H and halogen,
$R_b$ is selected from H and $C_{1-4}$ alkyl;
alternatively, two $R_b$ on the same carbon atom are taken together to form a $C_{3-7}$ cycloalkyl group;
$R_c$ is selected from H, -OH, -$NH_2$, halogen, $C_{1-4}$ alkyl, $C_{3-10}$ cycloalkyl, 3-10 membered heterocyclyl, $C_{6-10}$ aryl and 5-10 membered heteroaryl, alternatively H, $C_{1-4}$ alkyl, $C_{3-10}$ cycloalkyl and 3-10 membered heterocyclyl, still alternatively H and $C_{1-4}$ alkyl;
$R_d$ is selected from H, -OH, -$NH_2$, halogen, $C_{1-4}$ alkyl, $C_{3-10}$ cycloalkyl, 3-10 membered heterocyclyl, $C_{6-10}$ aryl and

5-10 membered heteroaryl, alternatively H, $C_{1-4}$ alkyl, $C_{3-10}$ cycloalkyl and 3-10 membered heterocyclyl, still alternatively H and $C_{1-4}$ alkyl;
or $R_c$ and $R_d$ are taken together with the N atom to which they are attached to form 5-6 membered heterocyclyl;
L is selected from a chemical bond and -O-;
p is 0, 1, or 2;
q is 0, 1, or 2;
n is 1, 2, 3, 4 or 5.

In a more specific embodiment, the present disclosure provides a compound of the above-mentioned general formula (III), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from

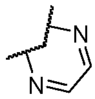 and 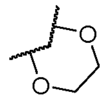;

$R_1$ is selected from Cl, Br and $-CF_3$;
$R_2$ is H;
$R_3$ is selected from H, F, Cl, $-OCH_3$ and $-OCF_3$, alternatively, $R_3$ is selected from H, F, $-OCH_3$ and $-OCF_3$;
$R_4$ is H;
$R_5$ is H;
$R_6$ is selected from

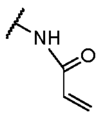, 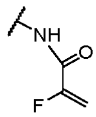, 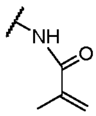, 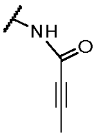, 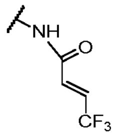, 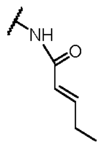, 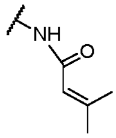,

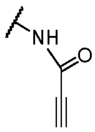, 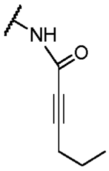, 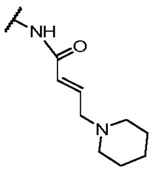, 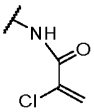, 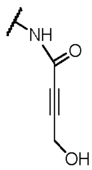, 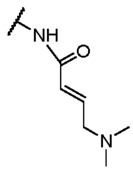, 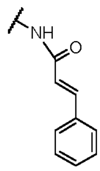, 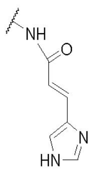, 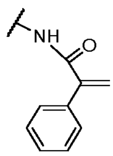,

and

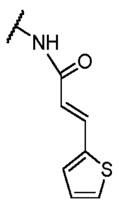;

Z is $CH_2$ or C=O;
$R_a$ is selected from H, halogen, $C_{1-6}$ alkyl, $C_{1-6}$ haloalkyl, $C_{2-6}$ alkenyl, $C_{2-6}$ alkynyl, -CN, - $NR_cR_d$, $-NH(CH_2)_{0-5}R_c$ and $-NHC(O)(CH_2)_{0-5}R_c$, alternatively H, F, Cl, $-NR_cR_d$ and $-NH(CH_2)_{0-5}R_c$, still alternatively H and F;
$R_b$ is selected from H and $-CH_3$;
alternatively, two $R_b$ on the same carbon atom are taken together to form a cyclopropyl group;
$R_c$ is selected from H, -OH, $-NH_2$, halogen, $C_{1-4}$ alkyl, $C_{3-7}$ cycloalkyl, 5-6 membered heterocyclyl, $C_{6-10}$ aryl and 5-6 membered heteroaryl, alternatively H, $C_{1-4}$ alkyl, $C_{3-7}$ cycloalkyl and 5-6 membered heterocyclyl, still alternatively H and $C_{1-4}$ alkyl;
$R_d$ is selected from H, -OH, $-NH_2$, halogen, $C_{1-4}$ alkyl, $C_{3-7}$ cycloalkyl, 5-6 membered heterocyclyl, $C_{6-10}$ aryl and 5-6

membered heteroaryl, alternatively H, $C_{1-4}$ alkyl, $C_{3-7}$ cycloalkyl and 5-6 membered heterocyclyl, still alternatively H and $C_{1-4}$ alkyl;
L is selected from a chemical bond and -O-;
p is 0 or 1;
q is 0, 1, or 2;
n is 2, 3 or 4.

In a more specific embodiment, the present disclosure provides a compound of formula (IV), or a pharmaceutically acceptable salt, enantiomer, diastereomer, racemate, solvate, hydrate, polymorph, prodrug or isotopic variant thereof, or a mixture thereof:

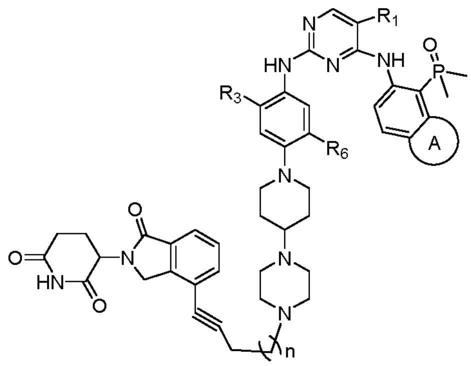

(IV)

wherein

Ring A is absent, or is selected from $C_{4-10}$ cycloalkyl, 5-8 membered heterocyclyl, $C_{6-10}$ aryl and 5-10 membered heteroaryl;
$R_1$ is selected from H, halogen and $C_{1-4}$ haloalkyl, alternatively H and halogen;
$R_3$ is selected from H, halogen, $-OC_{1-6}$ alkyl and $-OC_{1-6}$ haloalkyl, alternatively H and $-OC_{1-6}$ alkyl;
$R_6$ is selected from -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$alkynyl, 3-10 membered heterocyclyl and $C_{3-10}$ cycloalkyl, wherein the $C_{1-6}$ alkyl, $C_{1-6}$ haloalkyl, $C_{2-6}$ alkenyl, $C_{2-6}$ alkynyl, 3-10 membered heterocyclyl and $C_{3-10}$ cycloalkyl are each optionally substituted with 0, 1, 2 or 3 R*;
R* is selected from H, halogen, -OH, $-NH_2$, -CN, 3-10 membered heterocyclyl, and $C_{3-10}$ cycloalkyl;
n is 1, 2, 3 or 4.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-10 membered heteroaryl and 5-8 membered heterocyclyl;
$R_1$ is selected from halogen and $C_{1-4}$ haloalkyl, alternatively halogen;
$R_3$ is selected from H, $-OC_{1-6}$ alkyl and $-OC_{1-6}$ haloalkyl, alternatively H and $-OC_{1-6}$ alkyl;
$R_6$ is selected from -NH-C(O)-CR=CR'R" and -NH-C(O)-C≡CR';
wherein R, R' and R" are each independently selected from H, halogen, $C_{1-6}$ alkyl and $C_{1-6}$ haloalkyl, wherein the $C_{1-6}$ alkyl and $C_{1-6}$ haloalkyl are each optionally substituted with 0, 1 or 2 R*;
R* is selected from H, halogen, -OH and 3-10 membered heterocyclyl;
n is 1, 2, 3 or 4.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-6 membered heteroaryl and 5-6 membered heterocyclyl;
$R_1$ is selected from halogen and $C_{1-4}$ haloalkyl, alternatively halogen;
$R_3$ is selected from H, $-OC_{1-4}$ alkyl and $-OC_{1-4}$ haloalkyl, alternatively H and $-OC_{1-4}$ alkyl;
$R_6$ is selected from -NH-C(O)-CR=CR'R" and -NH-C(O)-C≡CR';

wherein R is selected from H, halogen and $C_{1-4}$ alkyl;
R' and R" are each independently selected from H, $C_{1-4}$ alkyl and $C_{1-4}$ haloalkyl, wherein the $C_{1-4}$ alkyl and $C_{1-4}$haloalkyl are each optionally substituted with 0, 1 or 2 R*;
R* is selected from H, halogen, -OH and 5-6 membered heterocyclyl;
n is 1, 2, 3 or 4.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from

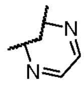 and 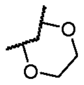;

$R_1$ is selected from Cl, Br and $-CF_3$, alternatively Cl and Br;
$R_3$ is selected from Hand $-OCH_3$;
$R_6$ is selected from

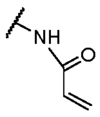, 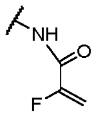, 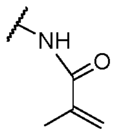, 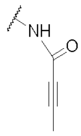, 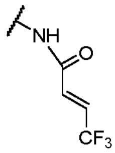, 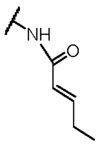, 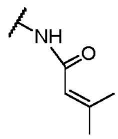,

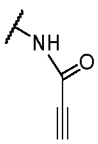, 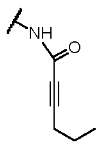, 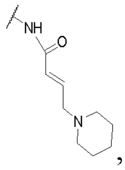, 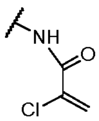 and 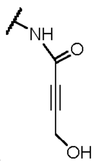;

n is 2, 3 or 4.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-10 membered heteroaryl and 5-8 membered heterocyclyl;
$R_1$ is selected from halogen and $C_{1-4}$ haloalkyl, alternatively halogen;
$R_3$ is selected from H, $-OC_{1-6}$ alkyl and $-OC_{1-6}$ haloalkyl, alternatively H and $-OC_{1-6}$ alkyl;
$R_6$ is selected from -NH-C(O)-CR=CR'R" and -NH-C(O)-C≡CR';
wherein R, R' and R" are each independently selected from H, F, $C_{1-6}$ alkyl and $C_{1-6}$ haloalkyl;
n is 1, 2, 3, 4 or 5.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-6 membered heteroaryl and 5-6 membered heterocyclyl;
$R_1$ is selected from halogen and $C_{1-4}$ haloalkyl, alternatively halogen;
$R_3$ is selected from H and $-OC_{1-4}$alkyl;
$R_6$ is selected from -NH-C(O)-CR=CR'R" and -NH-C(O)-C≡CR';
wherein R is selected from H, F and $C_{1-4}$ alkyl;
R' and R" are each independently selected from H, $C_{1-4}$ alkyl and $C_{1-4}$ haloalkyl;
n is 1, 2, 3 or 4.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from

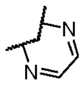 and 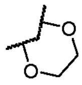;

$R_1$ is selected from Cl, Br and $-CF_3$, alternatively Cl and Br;
$R_3$ is selected from H and $-OCH_3$;
$R_6$ is selected from

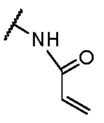, 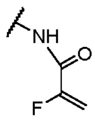, 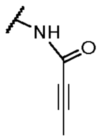, 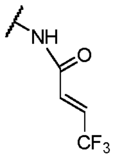, 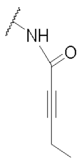, 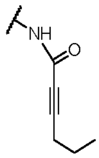, 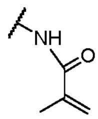,

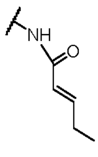, 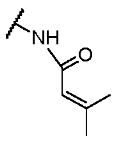 and 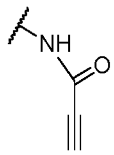;

n is 1, 2 or 3.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is selected from 5-10 membered heteroaryl and 5-8 membered heterocyclyl;
$R_1$ is selected from halogen and $C_{1-4}$ haloalkyl, alternatively halogen;
$R_3$ is selected from $-OC_{1-6}$ alkyl and $-OC_{1-6}$ haloalkyl, alternatively $-OC_{1-6}$ alkyl;
$R_6$ is selected from -NH-C(O)-CR=CR'R" and -NH-C(O)-C≡CR';
wherein R, R' and R" are each independently selected from H, halogen, $C_{1-6}$ alkyl and $C_{1-6}$ haloalkyl;
n is 1, 2 or 3.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is selected from 5-6 membered heteroaryl and 5-6 membered heterocyclyl;
$R_1$ is selected from halogen and $C_{1-4}$ haloalkyl, alternatively halogen;
$R_3$ is $-OC_{1-4}$alkyl;
$R_6$ is selected from -NH-C(O)-CR=CR'R" and -NH-C(O)-C≡CR';
wherein R is selected from H and halogen;
R' and R" are each independently selected from H, $C_{1-4}$ alkyl and $C_{1-4}$ haloalkyl;
n is 1, 2 or 3.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is selected from

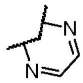 and 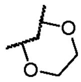;

$R_1$ is selected from Cl, Br and $-CF_3$, alternatively Cl and Br, still alternatively Cl;
$R_3$ is $-OCH_3$;
$R_6$ is selected from

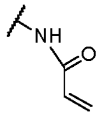, 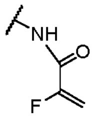, 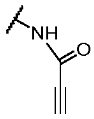, 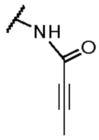, 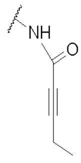, 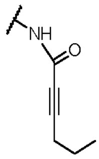 and 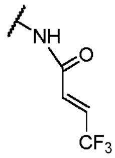;

n is 2.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is halogen, $C_{1-4}$ alkyl or $C_{1-4}$ haloalkyl;
$R_3$ is selected from $-OC_{1-6}$ alkyl and $-OC_{1-6}$ haloalkyl;
$R_6$ is selected from -NH-C(O)-CR=CR'R" and -NH-C(O)-C≡CR';
wherein R is H, F or Me;
R' and R" are each independently selected from H, $C_{1-6}$ alkyl and $C_{1-6}$ haloalkyl;
n is 2.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is Br or $C_{1-2}$ haloalkyl;
$R_3$ is selected from $-OC_{1-2}$ alkyl and $-OC_{1-2}$ haloalkyl;
$R_6$ is selected from -NH-C(O)-CR=CR'R" and -NH-C(O)-C≡CR';
wherein R is H or F; R' is H, $C_{1-4}$ alkyl or $C_{1-4}$ haloalkyl; R" is H;
n is 2.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is Br or $CF_3$;
$R_3$ is selected from -OMe and $-OCH_2CF_3$;
$R_6$ is selected from -NH-C(O)-CR=CR'R" and -NH-C(O)-C≡CR';
wherein R is H or F; R' is H, Me, iPr or $CF_3$; R" is H;
n is 2.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is $C_{1-4}$ haloalkyl;
$R_3$ is selected from $-OC_{1-6}$ alkyl and $-OC_{1-6}$ haloalkyl;

$R_6$ is selected from -NH-C(O)-CR=CR'R" and -NH-C(O)-C≡CR';
wherein R is H or halogen; R' is H, $C_{1-2}$ alkyl or $C_{1-2}$ haloalkyl; R" is H;
n is 2.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is $C_{1-2}$ haloalkyl;
$R_3$ is selected from $-OC_{1-2}$ alkyl and $-OC_{1-2}$ haloalkyl;
$R_6$ is selected from -NH-C(O)-CR=CR'R" and -NH-C(O)-C≡CR';
wherein R is H, F or Br; R' is H, $C_{1-2}$ alkyl or $C_{1-2}$ haloalkyl; R" is H;
n is 2.

In a more specific embodiment, the present disclosure provides a compound of the above-mentioned general formula (IV), or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is $CF_3$;
$R_3$ is selected from -OMe and $-OCH_2CF_3$;
$R_6$ is selected from -NH-C(O)-CR=CR'R" and -NH-C(O)-C≡CR';
wherein R is H or F; R' is H, Me or $CF_3$; R" is H;
n is 2.

In a more specific embodiment, the present disclosure provides a compound of formula (V), or a pharmaceutically acceptable salt, enantiomer, diastereomer, racemate, solvate, hydrate, polymorph, prodrug or isotopic variant thereof, or a mixture thereof:

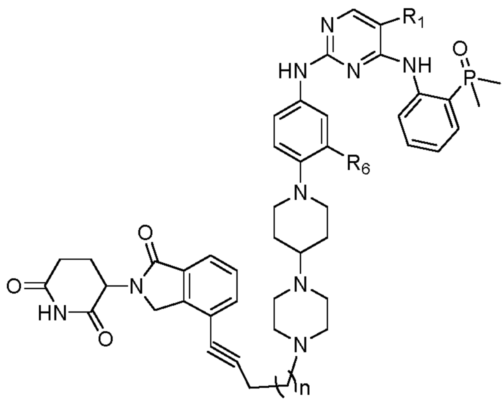

(V)

wherein

$R_1$ is halogen;
$R_6$ is selected from -NH-C(O)-CR=CR'R" and -NH-C(O)-C≡CR';
wherein R, R' and R" are each independently selected from H, halogen, $C_{1-6}$ alkyl and $C_{1-6}$ haloalkyl;
n is 0, 1, 2, 3, 4, or 5.

In a more specific embodiment, the present disclosure provides a compound of the above-mentioned general formula (V), or a pharmaceutically acceptable salt, enantiomer, diastereomer, racemate, solvate, hydrate, polymorph, prodrug or isotopic variant thereof, or a mixture thereof, wherein:

$R_1$ is halogen;
$R_6$ is selected from -NH-C(O)-CR=CR'R" and -NH-C(O)-C≡CR';
wherein R is selected from H and halogen;

R' and R" are each independently selected from H, $C_{1-4}$ alkyl and $C_{1-4}$ haloalkyl;
n is 1, 2, 3 or 4.

In a more specific embodiment, the present disclosure provides a compound of the above-mentioned general formula (V), or a pharmaceutically acceptable salt, enantiomer, diastereomer, racemate, solvate, hydrate, polymorph, prodrug or isotopic variant thereof, or a mixture thereof, wherein:

$R_1$ is selected from Br and Cl;

$R_6$ is selected from

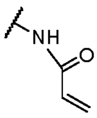, 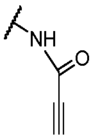, 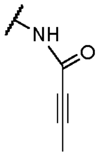, 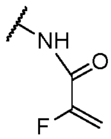, 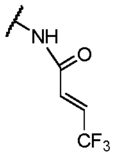, 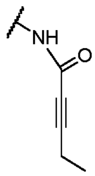 and 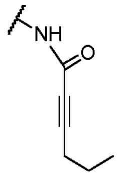;

n is 2, 3 or 4.

In a more specific embodiment, the present disclosure provides a compound of formula (VI), or a pharmaceutically acceptable salt, enantiomer, diastereomer, racemate, solvate, hydrate, polymorph, prodrug or isotopic variant thereof, or a mixture thereof:
wherein

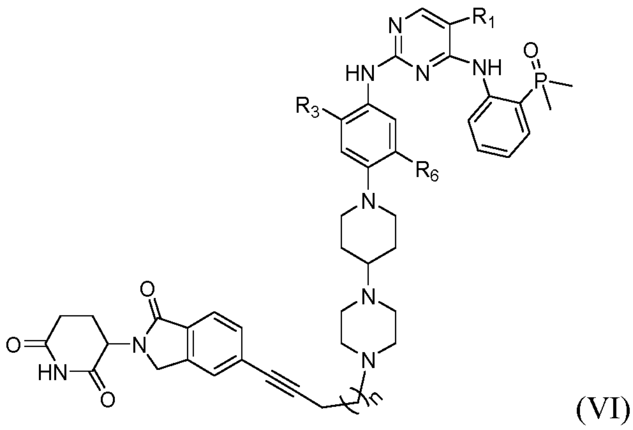

(VI)

$R_1$ is halogen;
$R_3$ is $-OC_{1-6}$ alkyl;
$R_6$ is selected from -NH-C(O)-CR=CR'R" and -NH-C(O)-C≡CR';
wherein R, R' and R" are independently selected from H, halogen, $C_{1-6}$ alkyl and $C_{1-6}$ haloalkyl;
n is 1, 2, 3 or 4.

In a more specific embodiment, the present disclosure provides a compound of the above-mentioned general formula (VI), or a pharmaceutically acceptable salt, enantiomer, diastereomer, racemate, solvate, hydrate, polymorph, prodrug or isotopic variant thereof, or a mixture thereof, wherein:

$R_1$ is halogen;
$R_3$ is $-OC_{1-6}$ alkyl;
$R_6$ is selected from -NH-C(O)-CR=CR'R" and -NH-C(O)-C≡CR';
wherein R, R' and R" are independently selected from H, $C_{1-6}$ alkyl and $C_{1-6}$ haloalkyl;
n is 2, 3 or 4.

In a more specific embodiment, the present disclosure provides a compound of the above-mentioned general

formula (VI), or a pharmaceutically acceptable salt, enantiomer, diastereomer, racemate, solvate, hydrate, polymorph, prodrug or isotopic variant thereof, or a mixture thereof, wherein:

$R_1$ is halogen;
$R_3$ is $-OC_{1-4}$alkyl;
$R_6$ is selected from -NH-C(O)-CR=CR'R" and -NH-C(O)-C≡CR';
wherein R is H;
R' and R" are independently selected from H, $C_{1-4}$alkyl and $C_{1-4}$haloalkyl;
n is 3.

In a more specific embodiment, the present disclosure provides a compound of the above-mentioned general formula (VI), or a pharmaceutically acceptable salt, enantiomer, diastereomer, racemate, solvate, hydrate, polymorph, prodrug or isotopic variant thereof, or a mixture thereof, wherein:

$R_1$ is Cl;
$R_3$ is $-OCH_3$;
$R_6$ is selected from

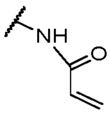 , 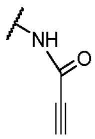 , 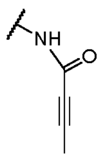 , 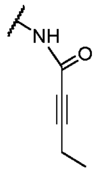 , 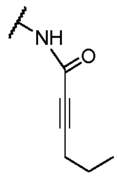 , 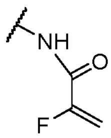

and

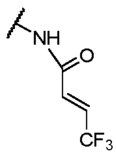 ;

n is 3.

In a more specific embodiment, the present disclosure provides a compound of formula (IX), or a pharmaceutically acceptable salt, enantiomer, diastereomer, racemate, solvate, hydrate, polymorph, prodrug or isotopic variant thereof, or a mixture thereof:

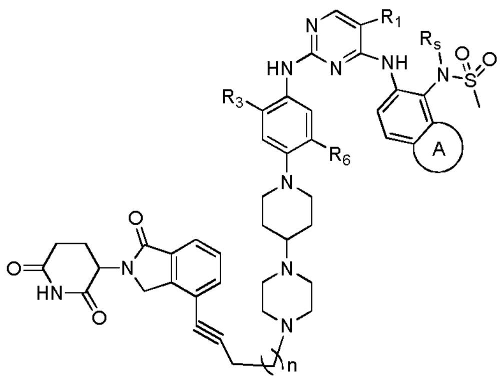

(IX)

wherein

Ring A is selected from $C_{4-10}$ cycloalkyl, 5-8 membered heterocyclyl, $C_{6-10}$ aryl and 5-14 membered heteroaryl;
$R_1$ is H, halogen, $C_{1-4}$ alkyl or $C_{1-4}$ haloalkyl; alternatively, $R_1$ is halogen, $C_{1-4}$ alkyl or $C_{1-4}$ haloalkyl;
$R_3$ is selected from $-OC_{1-6}$ alkyl and $-OC_{1-6}$ haloalkyl;

$R_6$ is selected from $C_{1-6}$ alkyl, $C_{1-6}$ haloalkyl, -NH-C(O)-CR=CR'R" and -NH-C(O)-C=CR';
wherein R is H, halogen, $C_{1-6}$ alkyl or $C_{1-6}$ haloalkyl;
R' and R" are each independently selected from H, $C_{1-6}$ alkyl and $C_{1-6}$ haloalkyl;
$R_s$ is selected from H, $C_{1-6}$ alkyl and $C_{1-6}$ haloalkyl;
n is 2.

In a more specific embodiment, the present disclosure provides a compound of the above-mentioned general formula (IX), or a pharmaceutically acceptable salt, enantiomer, diastereomer, racemate, solvate, hydrate, polymorph, prodrug or isotopic variant thereof, or a mixture thereof, wherein:

Ring A is selected from $C_{6-10}$ aryl and 5-6 membered heteroaryl;
$R_1$ is H, halogen, $C_{1-4}$ alkyl or $C_{1-4}$ haloalkyl; alternatively, $R_1$ is halogen, $C_{1-4}$ alkyl or $C_{1-4}$ haloalkyl;
$R_3$ is selected from -O$C_{1-4}$ alkyl and -O$C_{1-4}$ haloalkyl;
$R_6$ is selected from $C_{1-4}$ alkyl, $C_{1-4}$ haloalkyl, -NH-C(O)-CR=CR'R" and -NH-C(O)-C=CR';
wherein R is H or halogen; R' is H, $C_{1-4}$ alkyl or $C_{1-4}$ haloalkyl; R" is H;
$R_s$ is selected from H, $C_{1-4}$ alkyl and $C_{1-4}$ haloalkyl;
n is 2.

In a more specific embodiment, the present disclosure provides a compound of the above-mentioned general formula (IX), or a pharmaceutically acceptable salt, enantiomer, diastereomer, racemate, solvate, hydrate, polymorph, prodrug or isotopic variant thereof, or a mixture thereof, wherein:

Ring A is selected from phenyl and 5-6 membered heteroaryl;
$R_1$ is H, halogen or $C_{1-4}$ haloalkyl; alternatively, $R_1$ is halogen or $C_{1-4}$ haloalkyl;
$R_3$ is selected from -O$C_{1-4}$ alkyl and -O$C_{1-4}$ haloalkyl;
$R_6$ is selected from $C_{1-4}$ alkyl, $C_{1-4}$ haloalkyl, -NH-C(O)-CR=CR'R" and -NH-C(O)-C=CR';
wherein R is H or halogen; R' is H, $C_{1-4}$ alkyl or $C_{1-4}$ haloalkyl; R" is H;
$R_s$ is selected from H, $C_{1-4}$ alkyl and $C_{1-4}$ haloalkyl;
n is 2.

In a more specific embodiment, the present disclosure provides a compound of the above-mentioned general formula (IX), or a pharmaceutically acceptable salt, enantiomer, diastereomer, racemate, solvate, hydrate, polymorph, prodrug or isotopic variant thereof, or a mixture thereof, wherein:

Ring A is a 5-6 membered heteroaryl group;
$R_1$ is H, Cl, Br or $CF_{3;}$ alternatively, $R_1$ is Br or $CF_{3;}$
$R_3$ is selected from -O$C_{1-2}$ alkyl and -O$C_{1-2}$ haloalkyl;
$R_6$ is selected from $C_{1-4}$ alkyl, $C_{1-4}$ haloalkyl and -NH-C(O)-CR=CR'R";
wherein R is H or halogen; R' and R" are H;
$R_s$ is selected from H and $C_{1-4}$ alkyl;
n is 2.

In a more specific embodiment, the present disclosure provides a compound of the above-mentioned general formula (IX), or a pharmaceutically acceptable salt, enantiomer, diastereomer, racemate, solvate, hydrate, polymorph, prodrug or isotopic variant thereof, or a mixture thereof, wherein:

Ring A is pyrazinyl;
$R_1$ is H, Cl, Br or $CF_{3;}$ alternatively, $R_1$ is Br or $CF_{3;}$
$R_3$ is selected from -O$C_{1-2}$ alkyl and -O$C_{1-2}$ haloalkyl;
$R_6$ is selected from $C_{1-4}$ alkyl and -NH-C(O)-CR=CR'R";
wherein R is H or F; R' and R" are H;
$R_s$ is H;
n is 2.

In a more specific embodiment, the present disclosure relates to a compound, or a pharmaceutically acceptable salt, enantiomer, diastereomer, racemate, solvate, hydrate, polymorph, prodrug or isotopic variant thereof, or a mixture thereof, wherein the compound is selected from:

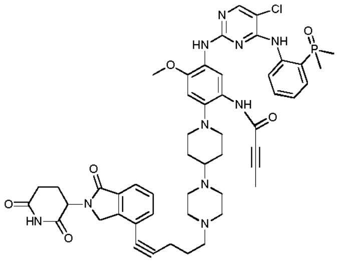
,
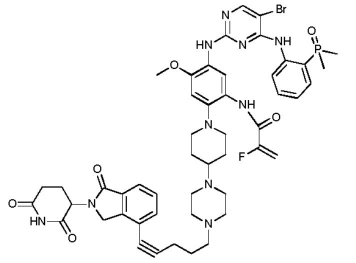
,
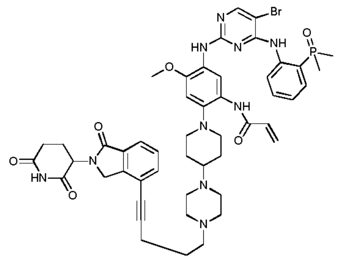
,
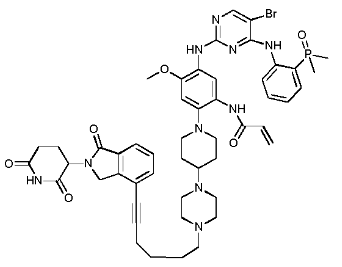
,
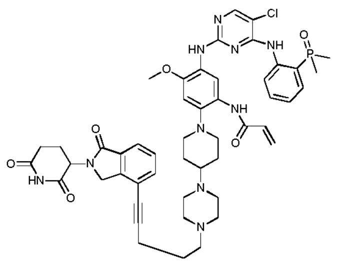
,
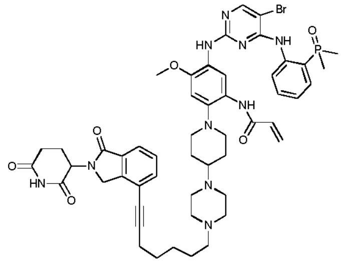
,
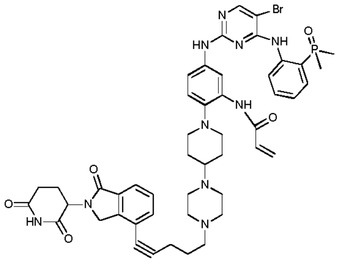
,
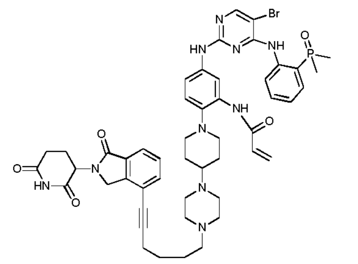
,
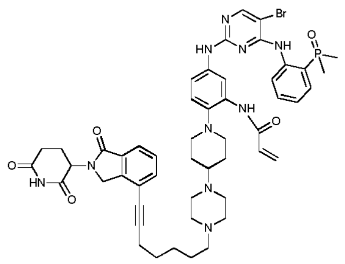
,
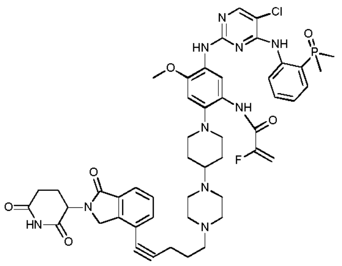
,
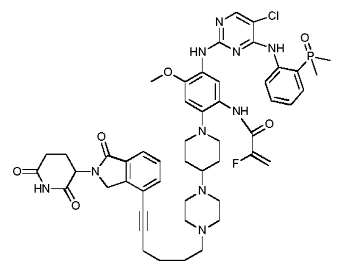
,
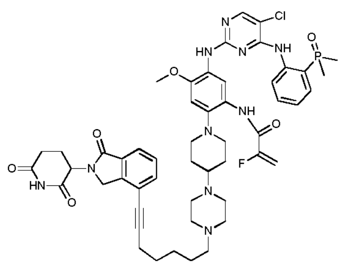
,
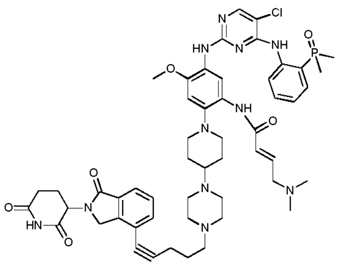
,
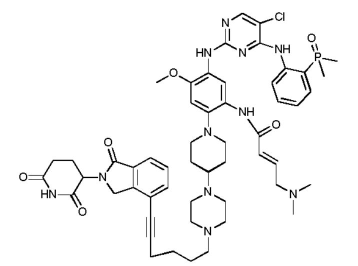
,
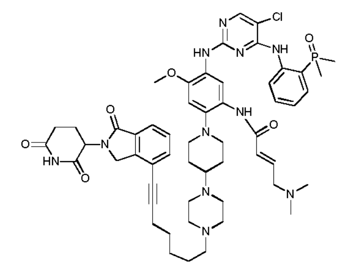
,

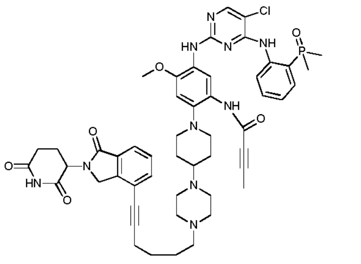 , 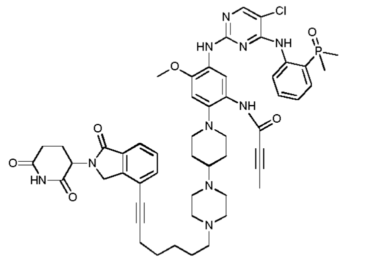 , 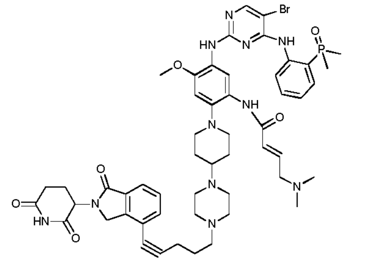 ,

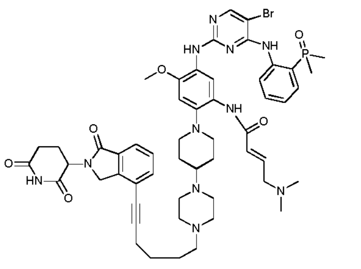 , 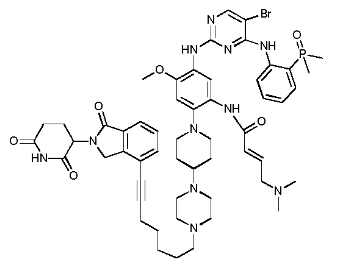 , 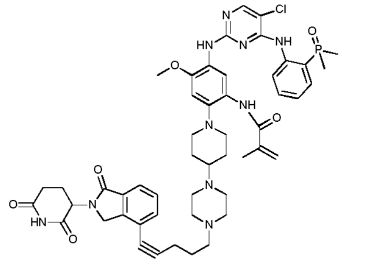 ,

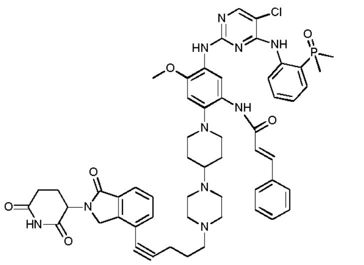 , 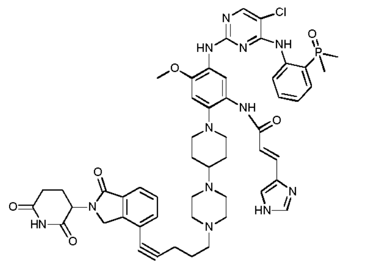 , 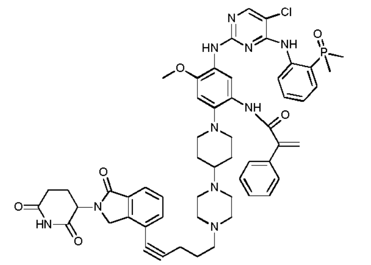 ,

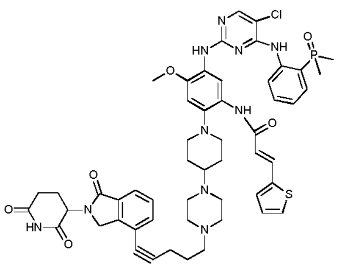 , 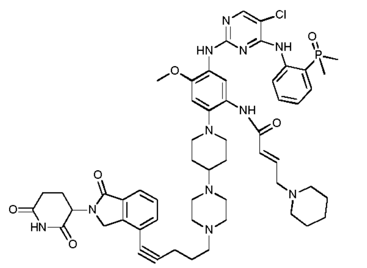 , 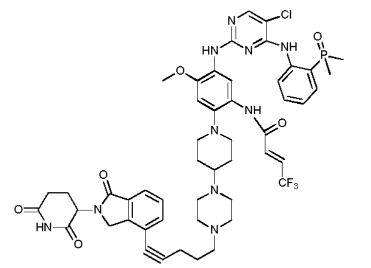 ,

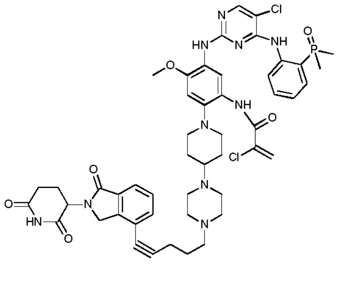 , 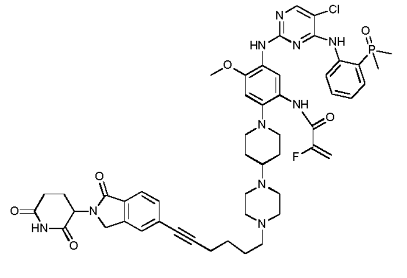 , 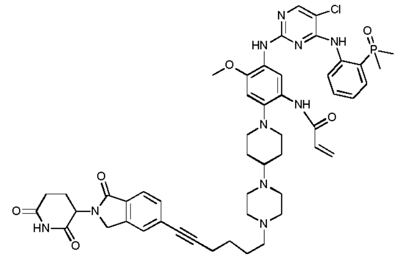 ,

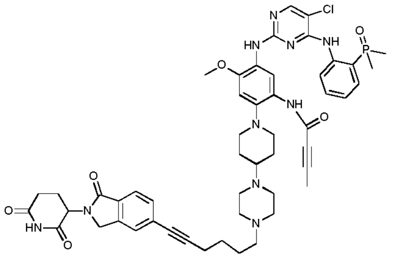 , 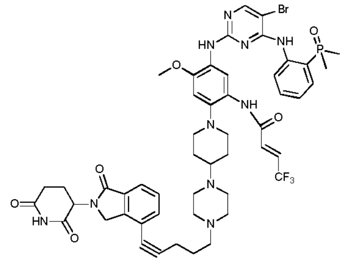 , 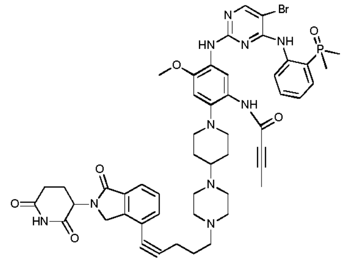 ,

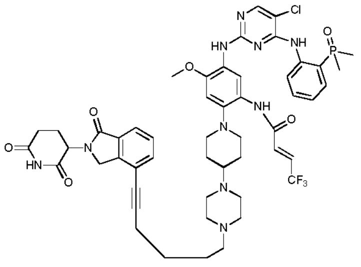,
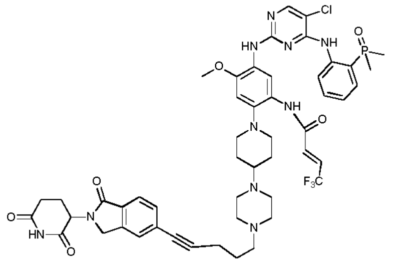,
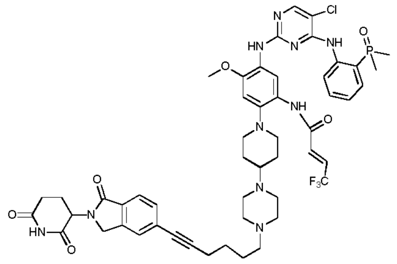,

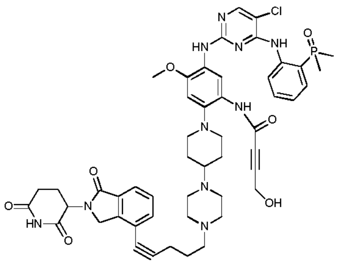,
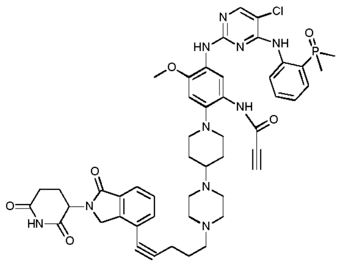,
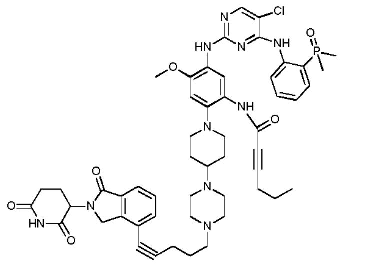,

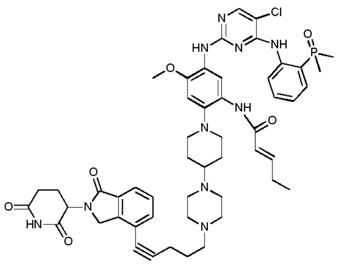,
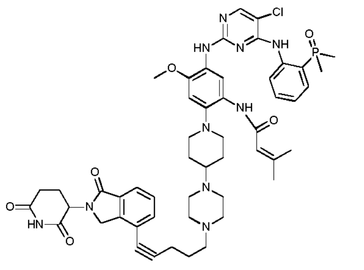,
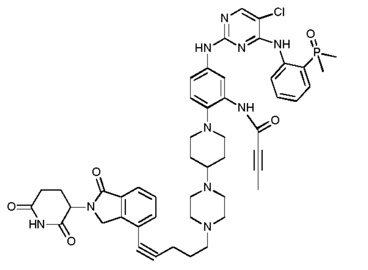,

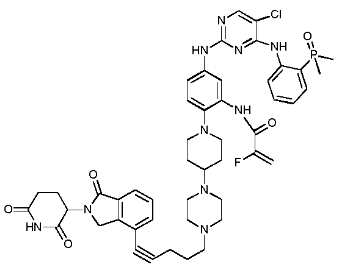,
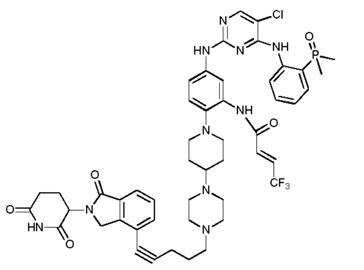,
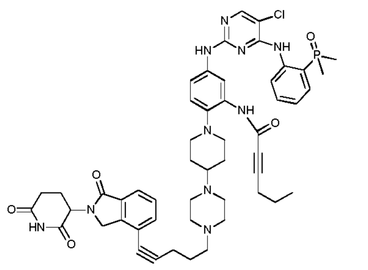,

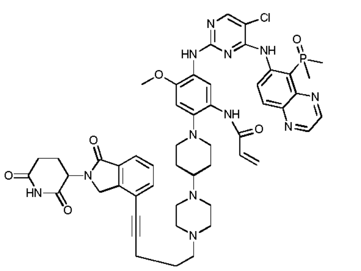,
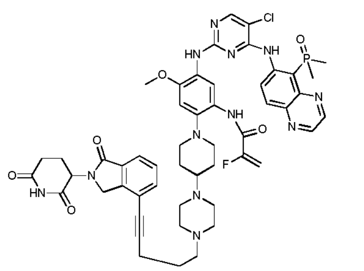,
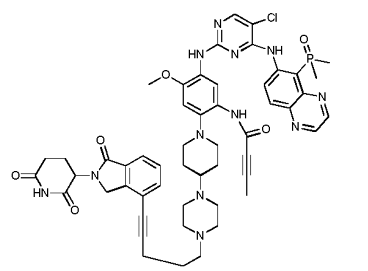,

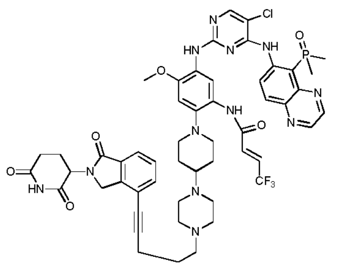,

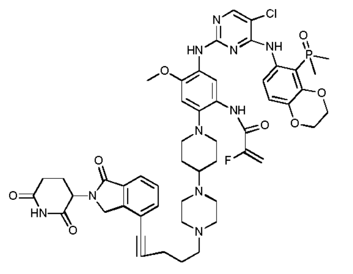,

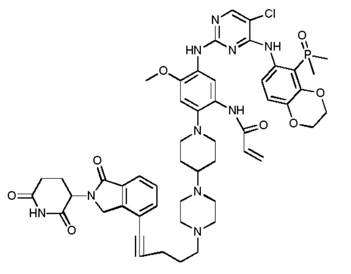,

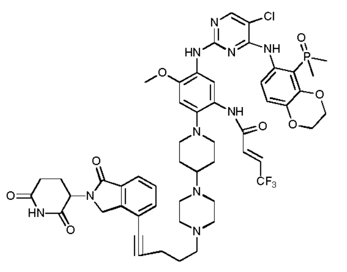,

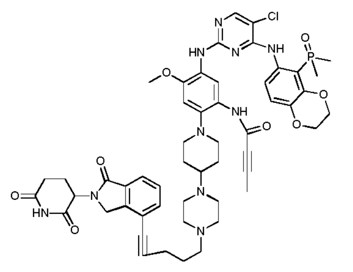,

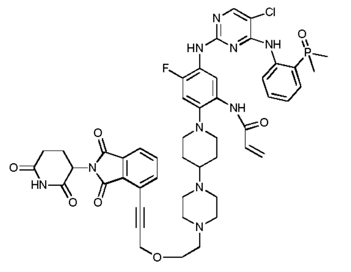,

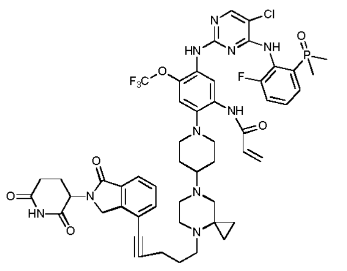,

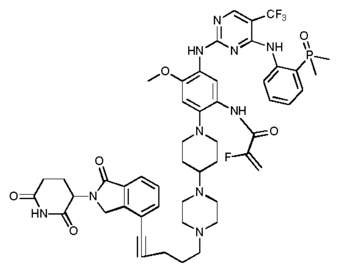,

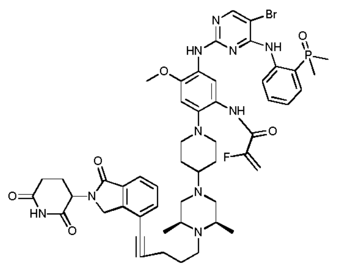,

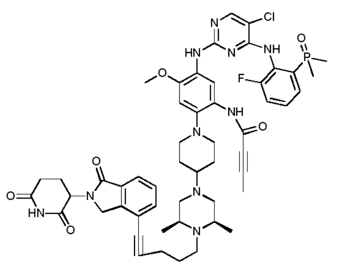,

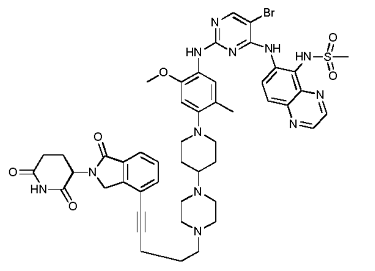,

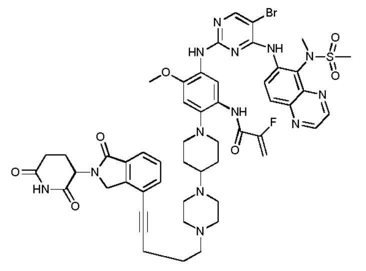,

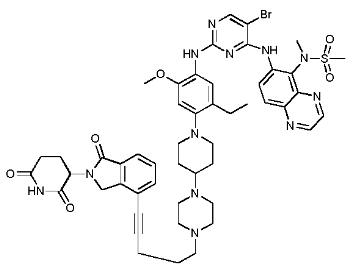,

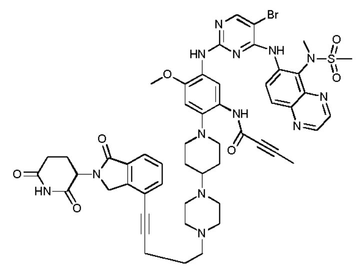,

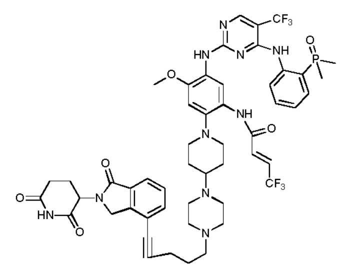,

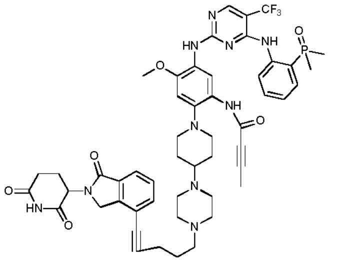 ,

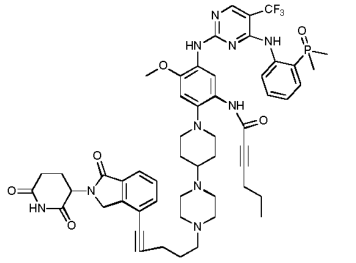 ,

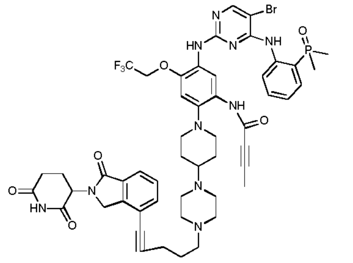 ,

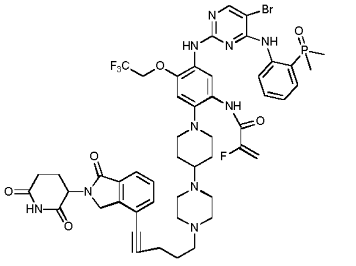 ,

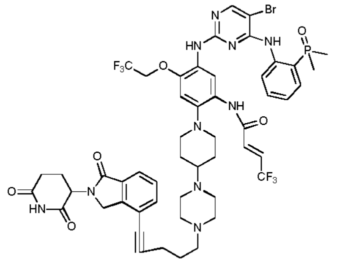 ,

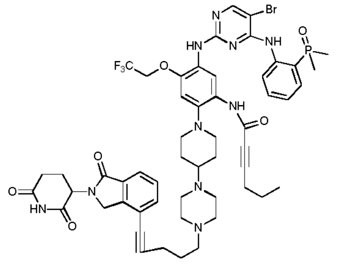 ,

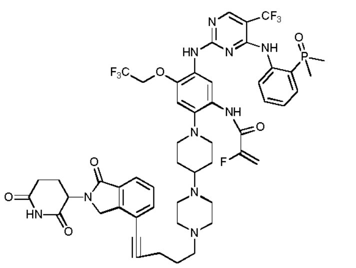 ,

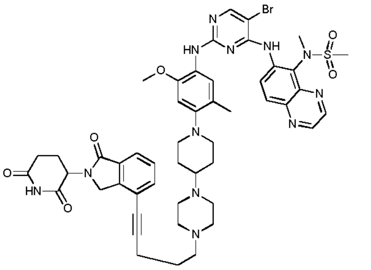 ,

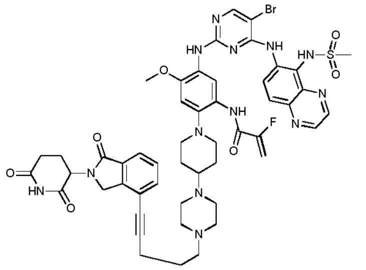 ,

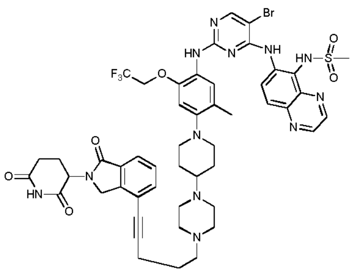 ,

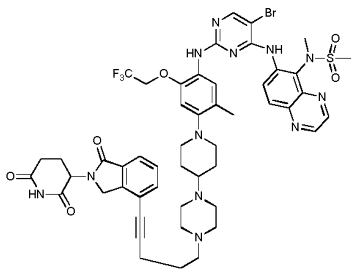 ,

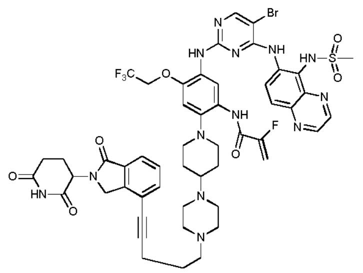 ,

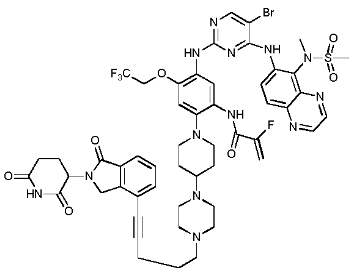 ,

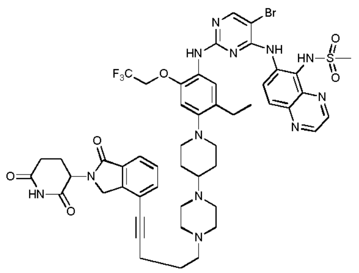 ,

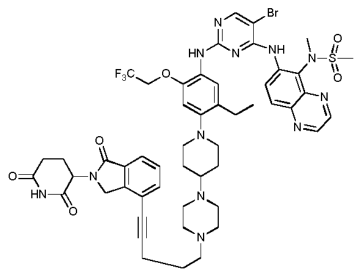 ,

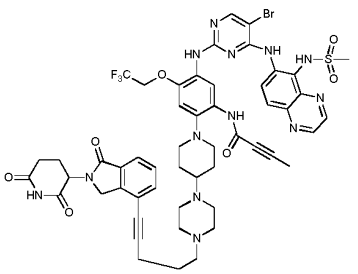,
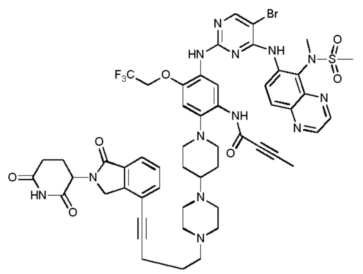,
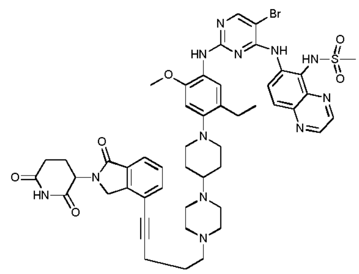,
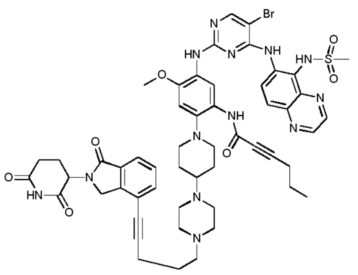,
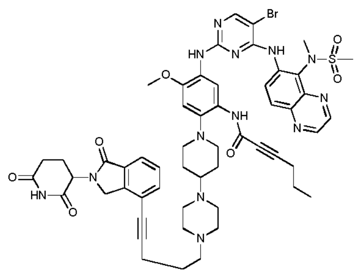,
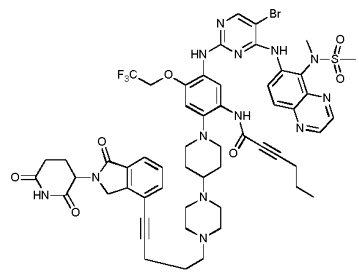,
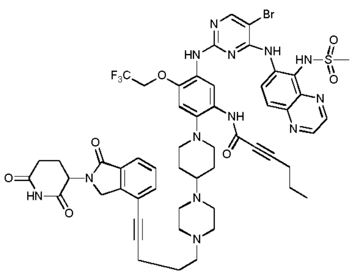,
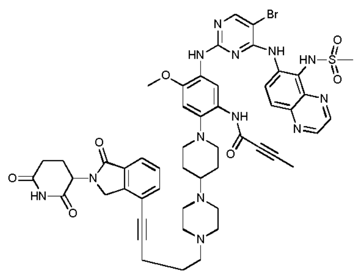,
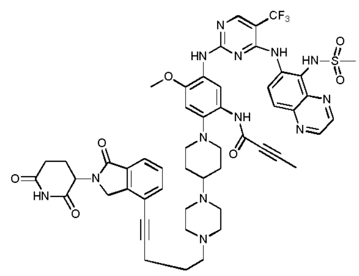,
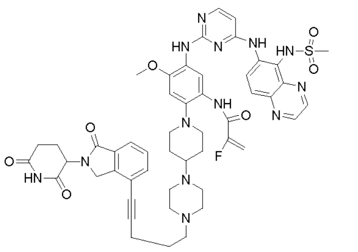,
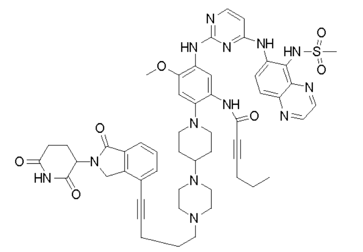,
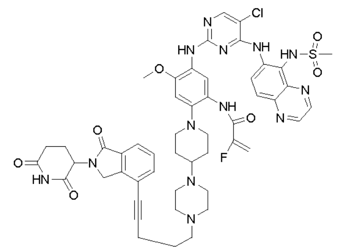,
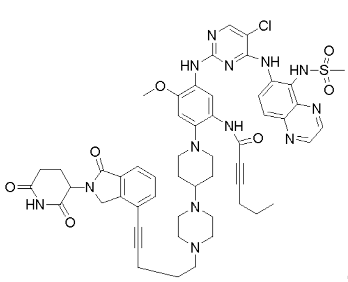
and
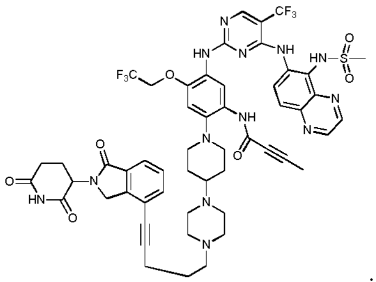.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

It will be understood by those skilled in the art that the organic compounds can form complexes with solvents in

which they are reacted or from which they are precipitated or crystallized. These complexes are known as "solvates." Where the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotope variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are yet alternative, because they are easy to prepare and detect. Furthermore, replaced by heavier isotopes, such as deuterium, which is $^{2}H$, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects *in vivo* by, for example, hydrolysis in blood. Pharmaceutically acceptable prodrugs are described in T. Higuchi and V Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19 (2) 115-130, each of which are incorporated herein by reference.

The prodrugs are any covalently bonded compounds of the present disclosure, which release the parent compound *in vivo* when the prodrug is administered to a patient. Prodrugs are typically prepared by modifying functional groups in such a way that the modifications can be cleaved either by routine manipulation or decompose *in vivo* to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, amino or sulfhydryl functional groups of the compounds of formula (I). Furthermore, in the case of carboxylic acid (-COOH), esters such as methyl esters and ethyl esters, etc. can be employed. The ester itself may be active in their own and/or hydrolyzable under *in vivo* conditions in the

human body. Suitable pharmaceutically acceptable *in vivo* hydrolysable ester groups include those groups that can readily break down in the human body to release the parent acids or salts thereof.

The present disclosure also provides a pharmaceutical formulation comprising a therapeutically effective amount of a compound of formula (I), or therapeutically acceptable salts thereof, and pharmaceutically acceptable carriers, diluents or excipients thereof. All of these forms belong to the present disclosure.

Pharmaceutical compositions and kits

In another aspect, the present disclosure provides a pharmaceutical composition comprising a compound of the present disclosure (also referred to as the "active ingredient") and a pharmaceutically acceptable excipient. In certain embodiments, the pharmaceutical composition comprises an effective amount of the compound of the present disclosure. In certain embodiments, the pharmaceutical composition comprises a therapeutically effective amount of the compound of the present disclosure. In certain embodiments, the pharmaceutical composition comprises a prophylactically effective amount of the compound of the present disclosure.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g.*, within the therapeutic window over the extended period of time.

The pharmaceutical compositions of the present disclosure may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the

systemic levels of the active ingredient desired throughout the body, *e.g.,* an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, *e.g.*, by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and still alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient (s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of *Remington's Pharmaceutical Sciences,* 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are $\alpha$-, $\beta$- and $\gamma$- cyclodextrins consisting of 6, 7 and 8 $\alpha$-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether $\beta$-cyclodextrin, *e.g.*, for example, sulfobutyl ether $\beta$-cyclodextrin, also

known as Captisol. See, *e.g.,* U.S. 5,376,645. In certain embodiments, the formulation comprises hexapropyl-β-cyclodextrin (*e.g.*, 10-50% in water).

Treatment

As stated herein, it is known that EGFR kinase have roles in tumourigenesis as well as numerous other diseases. We have found that the compounds of the present disclosure possess potent anti-tumour activity which it is believed is afforded by way of inhibition of EGFR kinase.

The compounds of the present disclosure are of value as anti-tumour agents. Particularly, the compounds of the present disclosure are of value as anti-proliferative, apoptotic and/or anti-invasive agents in the containment and/or treatment of solid and/or liquid tumour disease. Particularly, the compounds of the present disclosure are expected to be useful in the prevention or treatment of those tumours which are sensitive to inhibition of EGFR. Further, the compounds of the present disclosure are expected to be useful in the prevention or treatment of those tumours which are mediated alone or in part by EGFR. The compounds may thus be used to produce an EGFR enzyme inhibitory effect in a warm-blooded animal in need of such treatment.

As stated herein, inhibitors of EGFR kinase should be of therapeutic value for the treatment of cancer, such as ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular cancer, stomach cancer, gastrointestinal stromal tumor (GIST), thyroid cancer, cancer of bile duct, endometrial cancer, kidney cancer, anaplastic large cell lymphoma, acute myeloid leukemia (AML), multiple myeloma, melanoma, and mesothelioma.

Anti-cancer effects which are accordingly useful in the treatment of cancer in a patient include, but are not limited to, anti-tumour effects, the response rate, the time to disease progression and the survival rate. Anti-tumour effects of a method of treatment of the present disclosure include but are not limited to, inhibition of tumour growth, tumour growth delay, regression of tumour, shrinkage of tumour, increased time to regrowth of tumour on cessation of treatment, slowing of disease progression. Anti-cancer effects include prophylactic treatment as well as treatment of existing disease.

A EGFR kinase inhibitor, or a pharmaceutically acceptable salt thereof, may also be useful for the treatment patients with cancers, including, but not limited to, haematologic malignancies such as leukaemia, multiple myeloma, lymphomas such as Hodgkin's disease, non-Hodgkin's lymphomas (including mantle cell lymphoma), and myelodysplastic syndromes, and also solid tumours and their metastases such as breast cancer, lung cancer (non-small cell lung cancer (NSCL), small cell lung cancer (SCLC), squamous cell carcinoma), endometrial cancer, tumours of the central nervous system such as gliomas, dysembryoplastic neuroepithelial tumour, glioblastoma multiforme, mixed gliomas, medulloblastoma, retinoblastoma, neuroblastoma, germinoma and teratoma, cancers of the gastrointestinal tract such as gastric cancer, oesophagal cancer, hepatocellular (liver) carcinoma, cholangiocarcinomas, colon and rectal carcinomas, cancers of the small intestine, pancreatic cancers, cancers of the skin such as melanomas (in particular metastatic melanoma), thyroid cancers, cancers of the head and neck and cancers of the salivary glands, prostate, testis, ovary, cervix, uterus, vulva, bladder, kidney (including renal cell carcinoma, clear cell and renal oncocytoma), squamous cell carcinomas, sarcomas such as osteosarcoma, chondrosarcoma, leiomyosarcoma, soft tissue sarcoma, Ewing's sarcoma, gastrointestinal stromal tumour (GIST), Kaposi's sarcoma, and paediatric cancers such as rhabdomyosarcomas and neuroblastomas.

The effective dose of the compound of the present disclosure is usually at an average daily dose of 0.01 mg to 50 mg compound/kg of patient weight, alternatively 0.1 mg to 25 mg compound/kg of patient weight, in single or multiple administrations. Generally, the compound of the present disclosure can be administered to the patient who needs this treatment in the daily dose range of about 1 mg to about 3500 mg per patient, alternatively 10 mg to 1000 mg. For example, the daily dose per patient can be 10, 20, 30, 40, 50, 60, 70, 80, 90, 100, 150, 200, 250, 300, 350, 400, 500, 600, 700, 800, 900 or 1000 mg. It can be administered once or several times a day, weekly (or several days apart) or on an intermittent schedule. For example, on a weekly basis (e.g. every Monday), the compound can be administered one or more times a day, variably for several weeks, for example 4-10 weeks. Or, the compound may be administered daily for several days (e.g. 2-10 days), and then a few days (e.g. 1-30 days) without administering the compound, repeating the cycle arbitrarily or repeating a given number of times, e.g. 4-10 cycles. For example, the compound of the present disclosure can be administered daily for 5 days, and then interrupted for 9 days, and then administered daily for 5 days, then interrupted for 9 days, and so on, repeating the cycle arbitrarily or repeating 4-10 times in total.

Combination therapy

The treatment defined herein may be applied as a sole therapy or may involve, in addition to the compounds of the present disclosure, conventional surgery or radiotherapy or chemotherapy. Accordingly, the compounds of the present disclosure can also be used in combination with existing therapeutic agents for the treatment of cancer.

In addition to the compound disclosed herein, conventional surgery, radiotherapy, chemotherapy, or immunotherapy can be used for the treatment. Such chemotherapy can be administered simultaneously, sequentially or separately with the compound disclosed herein and may include one or more of the following categories of anti-tumour agents:

(i) antiproliferative/antineoplastic drugs and combinations thereof, as used in medical oncology, such as alkylating agents (for example czs-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin- C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);

(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5a-reductase such as finasteride;

(iii) anti-invasion agents [for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline [AZD0530 (saracatinib)], N-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825) and bosutinib (SKI-606), and metalloproteinase inhibitors like marimastat, inhibitors of urokinase plasminogen activator receptor function or antibodies to heparanase];

(iv) inhibitors of growth factor function: for example such inhibitors include growth factor antibodies and growth factor receptor antibodies (for example the anti-erbB2 antibody trastuzumab [Herceptin], the anti-EGFR antibody panitumumab and the anti-erbBl antibody cetuximab [Erbitux, C225]); such inhibitors also include tyrosine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)-quinazolin-4-amine (gefitinib, ZD 1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib (BAY 43-9006), tipifarnib (Rl 15777) and lonafarnib (SCH66336)), inhibitors of cell signalling through MEK and/or AKT kinases, c-kit inhibitors, abl kinase inhibitors, PI3 kinase inhibitors, Plt3 kinase inhibitors, CSF-1R kinase inhibitors, IGF receptor (insulinlike growth factor) kinase inhibitors; aurora kinase inhibitors (for example AZD1152, PH739358, VX-680, MLN8054, R763, MP235, MP529, VX-528 and AX39459) and cyclin dependent kinase inhibitors such as CDK2 and/or CDK4 inhibitors;

(v) antiangiogenic agents such as those which inhibit the effects of vascular endothelial growth factor, [for example the anti-human vascular endothelial cell growth factor antibody bevacizumab (Avastin) and for example, a VEGF receptor tyrosine kinase inhibitor such as vandetanib (ZD6474), vatalanib (PTK787), sunitinib (SUI 1248), axitinib (AG-013736), pazopanib (GW 786034) and 4-(4-fiuoro-2-methylindol-5-yloxy)-6-methoxy-7-(3-pyrrolidin-1-ylpropoxy)quinazoline (AZD2171) and compounds that work by other mechanisms (for example linomide, inhibitors of integrin $\alpha\nu\beta3$ function and angiostatin)];

(vi) vascular damaging agents such as combretastatin A4;

(vii)an endothelin receptor antagonist, for example zibotentan (ZD4054) or atrasentan;

(viii) antisense therapies, for example those which are directed to the targets listed above, such as ISIS 2503, an anti-ras antisense;

(ix) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and

(x) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines, approaches using anti-idiotypic antibodies, approaches to decrease the function of immune suppressive cells such as regulatory T cells, myeloid-derived suppressor cells or IDO (indoleamine 2,3,-deoxygenase)-expressing dendritic

cells, and approaches using cancer vaccines consisting of proteins or peptides derived from tumour-associated antigens such as NY-ESO-1, MAGE-3, WTI or Her2/neu.

## Examples

### Preparation of 5-(2-(2,6-dioxopiperidin-3-yl)-1-oxoisoindolin-4-yl)pent-4-ynal (M1)

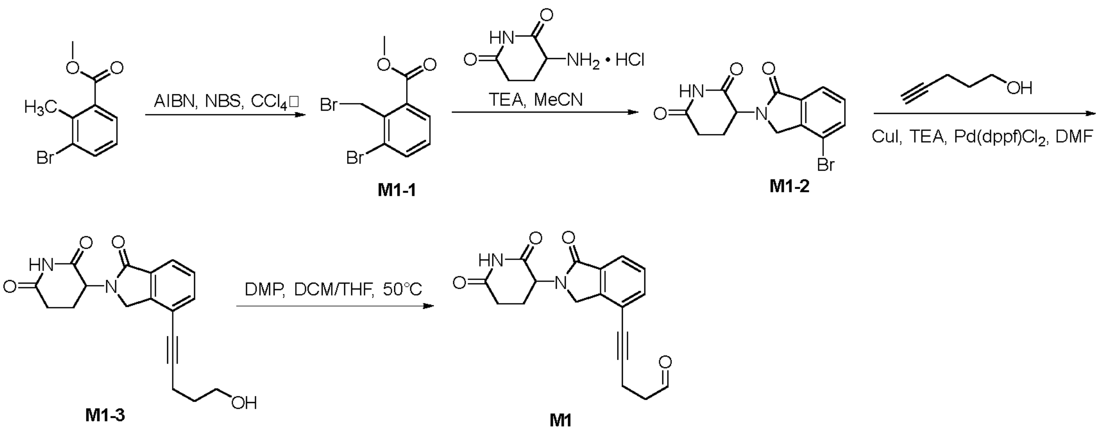


Step 1

Methyl 3-bromo-2-methylbenzoate (1.14 g, 5.0 mmol) was dissolved in 20.0 mL of $CCl_4$. Under nitrogen protection, NBS (1.34 g, 7.5 mmol) and AIBN (164 mg, 1.0 mmol) were added. The temperature was raised to 85°C, and the reaction was refluxed for 20 h. TLC showed that there was no raw material remaining. The reaction solution was cooled to room temperature, and filtered with suction. The filtrate was concentrated under reduced pressure to give a crude product, which was purified by Flash to give 1.35 g of product **M1-1** as light-yellow oil. LCMS: $[M + H]^+$ = 307, 309.

Step 2

**M1-1** (1.35 g, 4.41 mmol) and 3-aminopiperidine-2,6-dione hydrochloride (941 mg, 5.74 mmol) were dispersed in 25.0 mL of anhydrous MeCN, and TEA (580 mg, 5.74 mmol) was added. The temperature was raised to 80°C, and the reaction was refluxed for 16 h. The reaction was completed as detected by LCMS. The reaction solution was cooled to room temperature, and filtered with suction. The filter cake was rinsed three times with MeCN, and the solid was dried to give compound **M1-2** (1.31 g, yield 92.3%). LCMS: $[M + H]^+$ = 323, 325.

Step 3

**M1-2** (322 mg, 1.0 mmol), CuI (19 mg, 0.1 mmol) and Pd(dppf)$Cl_2$ (73.1 mg, 0.1 mmol) were dispersed in 10.0 mL of anhydrous DMF. Under $N_2$ protection, 4-alkynyl-1-pentanol (210 mg, 2.5 mmol) and TEA (303 mg, 3.0 mmol) were added in sequence. The reaction was heated to 70°C for 20 h. The reaction was completed as detected by LCMS. The reaction solution was cooled to room temperature and purified by RP-Flash to give a crude product **M1-3** as white solid (410 mg, yield 94.1%). LCMS: $[M + H]^+$ = 327.

Step 4

**M1-3** (400 mg, 0.920 mmol) was dissolved in a mixed solvent of 150 mL of anhydrous dichloromethane and 10 mL of anhydrous tetrahydrofuran. Under $N_2$ protection, Dess-Martin reagent (1.04 g, 2.454 mmol) was added, the temperature was raised to 50°C, and the reaction was refluxed for 2.0 h. The reaction was completed as determined by thin layer chromatography. The reaction solution was cooled to room temperature. 20 mL of saturated $NaHCO_3$ solution and 20 mL of saturated $Na_2S_2O_3$ solution were added to the reaction solution. The mixture was stirred vigorously at room temperature for 5 min. The organic layer was separated, dried with anhydrous sodium sulfate, and filtered with suction. The filtrate was concentrated under reduced pressure to give a crude product. The crude product was purified by Flash to give product **M1** as a light yellow solid (280 mg, 93.3%). LCMS: $[M + H]^+$ = 325.

**Preparation of 6-(2-(2,6-dioxopiperidin-3-yl)-1-oxoisoindolin-4-yl)hex-5-ynal (M2)**

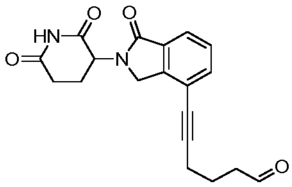

M2

It was prepared by the same method as for the preparation of **M1,** except that 4-alkynyl-1-pentanol in step 3 was replaced by 5-alkynyl-1-hexanol to finally give compound **M2.** LCMS: $[M + H]^+$ = 339.

**Preparation of 7-(2-(2,6-dioxopiperidin-3-yl)-1-oxoisoindolin-4-yl)hept-6-ynal (M3)**

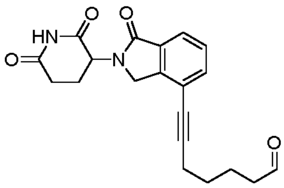

M3

It was prepared by the same method as for the preparation of **M1,** except that 4-alkynyl-1-pentanol in step 3 was replaced by 6-alkynyl-1-heptanol to finally give compound **M3**. LCMS: $[M + H]^+$ = 353.

**Preparation of 6-(2-(2,6-dioxopiperidin-3-yl)-1-oxoisoindolin-5-yl)hex-5-ynal (M4)**

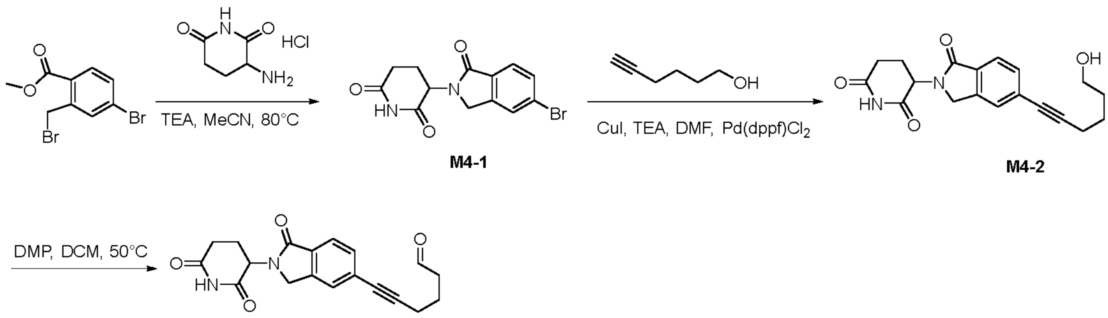


M4

Step 1

Methyl 4-bromo-2-(bromomethyl)benzoate (300 mg, 0.974 mmol), 3-aminopiperidine-2,6-dione hydrochloride (208 mg, 1.266 mmol), and TEA (128 mg, 1.266 mmol) were dissolved in 5 mL of acetonitrile. Under nitrogen protection, the reaction was stirred at 80 °C overnight. The mixture was cooled to room temperature, and the solvent was removed under reduced pressure to give a crude product, which was purified by Flash to give a white solid compound **M4-1** (180 mg). LCMS: $[M + H]^+$ = 323, 325.

Step 2

Compound **M4-1** (100 mg, 0.31 mmol), hexyl-5-yn-1-ol (76 mg, 0.78 mmol), cuprous iodide (12 mg, 0.06 mmol), $Pd(dppf)Cl_2$ (87 mg, 0.12 mmol) and TEA (94 mg, 0.93 mmol) were dissolved in 4 mL of anhydrous DMF. Under nitrogen

protection, the mixture was stirred at 70°C for 5 hours. The solvent was removed under reduced pressure to give a crude product, which was purified by thin layer chromatography to give an off-white solid compound **M4-2** (78 mg, yield 72.2%), LCMS: $[M + H]^+$ = 341.

Step 3

Compound **M4-2** (39 mg, 0.115 mmol) and Dess-Martin reagent (73 mg, 0.172 mmol) were dissolved in 15 mL of dichloromethane. The mixture was stirred at 50°C for 2 hours. The mixture was cooled to room temperature and quenched with saturated sodium bicarbonate aqueous solution. The organic phase was separated. The aqueous phase was extracted three times with dichloromethane. The organic phases were combined, dried over anhydrous sodium sulfate, and filtered. The filtrate was concentrated to give a crude product. The crude product was purified by thin layer chromatography to give a white solid compound **M4** (30 mg, yield 77.5%), LCMS: $[M + H]^+$ = 339.

**Preparation of 5-(2-(2,6-dioxopiperidin-3-yl)-1-oxoisoindolin-5-yl)pent-4-ynal (M5)**

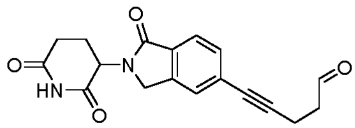

**M5**

It was prepared by the same method as for the preparation of **M4,** except that hexyl-5-yn-1-ol in step 2 was replaced by pentyl-4-yn-1-ol to finally give compound **M5.** LCMS: $[M + H]^+$ = 325.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)but-2-ynamide (D058)**

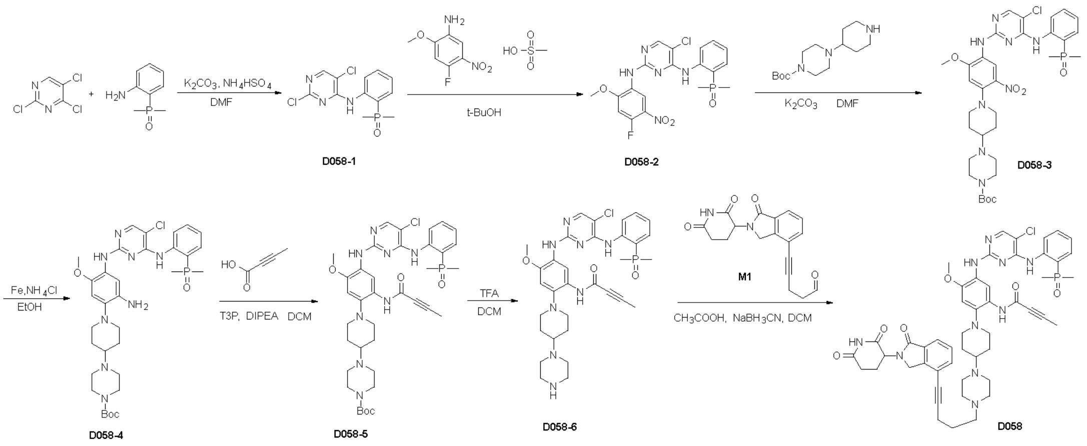


Step 1

2,4,5-Trichloropyrimidine (9.1 g, 50 mmol), (2-aminophenyl)dimethylphosphine oxide (8.45 g, 50 mmol), $NH_4HSO_4$ (1.7 g, 5 mmol) and $K_2CO_3$ (17.25 g, 125 mmol) were dispersed in 120 mL of anhydrous DMF. The reaction was heated to 65°C for 16 h. The reaction was completed as detected by TLC. The reaction solution was cooled to room temperature, and then filtered with suction. The solid residue was washed with DMF. The filtrate was concentrated under reduced pressure to give a crude product, which was flash purified to give a light yellow solid product **D058-1** (10.8 g, 34.3 mmol, 68.6%). LCMS: $[M + H]^+$ = 316.

Step 2

**D058-1** (10.8 g, 34.3 mmol) and 4-fluoro-2-methoxy-5-nitroaniline (6.38 g, 34.3 mmol) were dispersed in 200 mL of tert butanol. Methanesulfonic acid (13.18 g, 137.2 mmol) was added to the reaction solution. The reaction was heated to 90°C and refluxed for 16 h. The reaction was completed as detected by LCMS. The reaction solution was cooled to room temperature, and then concentrated under reduced pressure to remove the solvent to give a crude product, which was flash purified to give a light yellow solid product **D058-2** (11.6 g, yield 72.7%). LCMS: $[M + H]^+$ = 466.

Step 3

**D058-2** (11.6 g, 24.9 mmol), tert-butyl 4-(piperidin-4-yl)piperazine-1-carboxylate (6.7 g, 24.9 mmol) and $K_2CO_3$ (10.31 g, 74.7 mmol) were dispersed in 150 mL of anhydrous DMF. The reaction was heated to 60 °C for 12 h. The reaction was completed as detected by LCMS. The reaction solution was cooled to room temperature, and then filtered with suction. The solid residue was washed with DMF. The filtrate was concentrated under reduced pressure to give a crude product, which was flash purified to give a yellow solid product **D058-3** (13.5 g, yield 75.9%). LCMS: $[M + H]^+$ = 715.

Step 4

**D058-3** (13.5 g, 18.9 mmol), iron powder (6.35 g, 113.4 mmol) and $NH_4Cl$ (50.6 g, 945 mmol) were dispersed in a mixed solvent of 180 mL of ethanol and 60 mL of water. The reaction was heated to 90°C and refluxed for 2 h. The reaction was completed as detected by LCMS. The reaction solution was cooled to room temperature, and filtered with diatomaceous earth. The solid residue was washed with a mixed solvent of methanol and dichloromethane (150 mL). The filtrate was collected and concentrated under reduced pressure to remove ethanol. The residue was extracted twice with a mixed solvent of methanol and dichloromethane (150 mL). The organic layers were combined and washed sequentially with water (2 x 80 mL) and saturated brine (1 x 80 mL). The organic layer was dried over anhydrous magnesium sulfate, and filtered with suction. The filtrate was concentrated under reduced pressure to remove the solvent to give a crude product, which was purified by Flash to give a yellow solid product **D058-4** (11.5 g, 89.0 %). LCMS: $[M+H]^+$ = 685.

Step 5

**D058-4** (11.5 g, 16.8 mmol), 2-alkynylbutyric acid (1.41 g, 16.8 mmol) and T3P (21.37 g, 33.6 mmol) were dispersed in 115 mL of anhydrous dichloromethane. Under $N_2$ protection, diisopropylethylamine (10.8 g, 84.0 mmol) was added to the reaction solution. The mixture was reacted at room temperature for 2.0 h. The reaction was completed as detected by LCMS. The reaction solution was diluted with 115 mL of dichloromethane and silica gel was directly added to the sample. The mixture was purified by Flash to give a yellow solid product **D058-5** (11.0 g, 87.3%). LCMS: $[M + H]^+$ = 751.

Step 6

**D058-5** (11.0 g, 14.67 mmol) was dissolved in 110 mL of anhydrous dichloromethane. Under $N_2$ protection, 27.5 mL of trifluoroacetic acid was added to the reaction solution. The mixture was reacted at room temperature for 1.0 h. The reaction was completed as detected by LCMS. The reaction solution was concentrated under reduced pressure. The residue was redissolved in a mixed solvent of methanol and dichloromethane (200 mL), and then washed with saturated $NaHCO_3$ solution (2 x 80 mL) and saturated brine (1 x 80 mL) in sequence. The organic layer was dried over anhydrous magnesium sulfate, and filtered with suction. The filtrate was concentrated under reduced pressure to remove the solvent to give a crude product, which was purified by Flash to give a yellow solid product D058-6 (9.4 g). LCMS: $[M + H]^+$ = 651.

Step 7

**M1** (713 mg, 2.2 mmol) and **D058-6** (1.3 g, 2 mmol) were dissolved in a mixed solvent of 260 mL of anhydrous dichloromethane and 26 mL of anhydrous methanol. Under nitrogen protection, $CH_3COOH$ (180 mg, 3 mmol) was added and the mixture was stirred at room temperature for 0.5 h. Solid $NaBH_3CN$ (251 mg, 4 mmol) was then added to the reaction solution and the mixture was reacted at room temperature for another 2h. The reaction was completed as detected by LCMS and thin layer chromatography. Silica gel was directly added to the sample, and the mixture was purified by Flash to give a white solid pure product **D058** (1.21 g, yield 63.0%). LCMS: $[M + H]^+$ = 959.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-fluoroacrylamide (D078)**

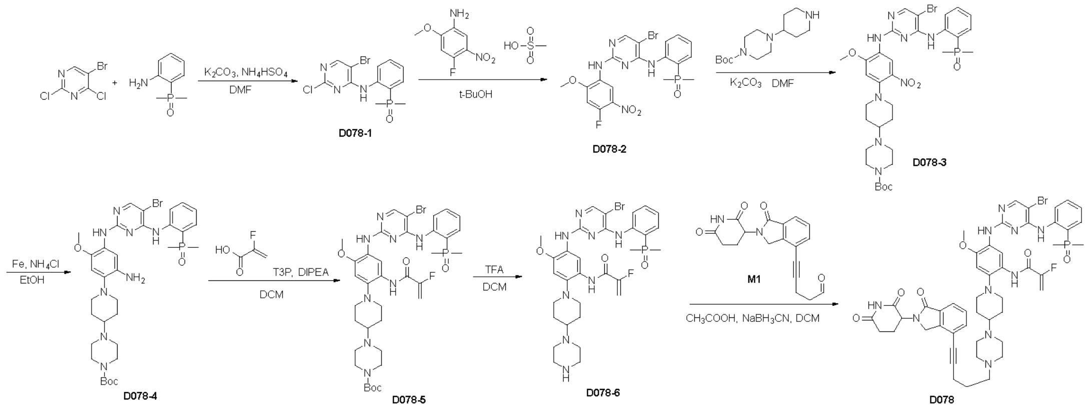


Step 1

5-Bromo-2,4-dichloropyrimidine (5.13 g, 22.5 mmol), (2-aminophenyl) dimethylphosphine oxide (3.8 g, 22.5 mmol), $NH_4HSO_4$ (0.765 g, 2.25 mmol) and $K_2CO_3$ (3.73 g, 27.0 mmol) were dispersed in 60.0 mL of anhydrous DMF. The reaction was heated to 65°C for 16 h. The reaction was completed as detected by TLC. The reaction solution was cooled to room temperature, and then filtered with suction, and the solid residue was washed with DMF. The filtrate was concentrated under reduced pressure to give a crude product, which was flash purified to give a light yellow solid product **D078-1** (8.0 g). LCMS: $[M + H]^+ = 360$.

Step 2

**D078-1** (8.0 g, 22.3 mmol) and 4-fluoro-2-methoxy-5-nitroaniline (4.14 g, 22.3 mmol) were dispersed in 160 mL of tert butanol. Methanesulfonic acid (8.56 g, 89.1 mmol) was added to the reaction solution. The reaction was heated to 90°C and refluxed for 16 h. The reaction was completed as detected by LCMS. The reaction solution was cooled to room temperature, and then concentrated under reduced pressure to remove the solvent to give a crude product, which was flash purified to give a light yellow solid product **D078-2** (9.1 g). LCMS: $[M + H]^+ = 510$.

Step 3

**D078-2** (8.4 g, 16.5 mmol), tert-butyl 4-(piperidin-4-yl)piperazine-1-carboxylate (4.44 g, 16.5 mmol) and $K_2CO_3$ (6.83 g, 49.5 mmol) were dispersed in 150 mL of anhydrous DMF. The reaction was heated to 60°C for 12 h. The reaction was completed as detected by LCMS. The reaction solution was cooled to room temperature, and then filtered with suction, and the solid residue was washed with DMF. The filtrate was concentrated under reduced pressure to give a crude product, which was flash purified to give a yellow solid product **D078-3** (7.4 g). LCMS: $[M + H]^+ = 759$.

Step 4

**D078-3** (7.4 g, 9.76 mmol), iron powder (3.28 g, 58.6 mmol) and $NH_4Cl$ (26.1 g, 488 mmol) were dispersed in a mixed solvent of 150 mL of ethanol and 50 mL of water. The reaction was heated to 90°C and refluxed for 2 h. The reaction was completed as detected by LCMS. The reaction solution was cooled to room temperature, and filtered with diatomaceous earth by suction. The solid residue was washed with 100 mL of a mixed solvent of ethanol and water. The filtrate was collected and concentrated under reduced pressure to remove ethanol. The residue was extracted twice with a mixed solvent of methanol and dichloromethane (100 mL). The organic layers were combined and washed sequentially with water (2 x 50 mL) and saturated brine (1 x 50 mL). The organic layer was dried over anhydrous magnesium sulfate, and filtered with suction. The filtrate was concentrated under reduced pressure to remove the solvent to give a crude product, which was purified by Flash to give a yellow solid product **D078-4** (5.5 g). LCMS: $[M + H]^+ = 729$.

Step 5

**D078-4** (5.0 g, 6.87 mmol), 2-fluoroacrylic acid (0.742 g, 8.24 mmol) and T3P (8.74 g, 13.74 mmol) were dispersed in 50 mL of anhydrous dichloromethane. Under $N_2$ protection, diisopropylethylamine (4.43 g, 34.35 mmol) was added to the reaction solution. The mixture was reacted at room temperature for 16 h. The reaction was completed as detected by LCMS. The reaction solution was diluted with 50 mL of dichloromethane and silica gel was directly added to the sample. The mixture was purified by Flash to give a yellow solid product **D078-5** (4.8 g). LCMS: $[M + H]^+$ = 801.

Step 6

**D078-5** (4.8 g, 6.0 mmol) was dissolved in 80 mL of anhydrous dichloromethane. Under $N_2$ protection, 20 mL of trifluoroacetic acid was added to the reaction solution. The mixture was reacted at room temperature for 1.0 h. LCMS showed that the reaction was completed. The reaction solution was concentrated under reduced pressure. The residue was redissolved in a mixed solvent of methanol and dichloromethane (150 mL), and then washed with saturated $NaHCO_3$solution (2 x 60 mL) and saturated brine (1 x 60 mL) in sequence. The organic layer was dried over anhydrous magnesium sulfate, and filtered with suction. The filtrate was concentrated under reduced pressure to remove the solvent to give a crude product, which was purified by Flash to give a yellow solid product **D078-6** (4.1 g). LCMS: $[M + H]^+$ = 701.

Step 7

**M1** (1.95 g, 6.02 mmol) and **D078-6** (3.58 g, 5.12 mmol) were dissolved in a mixed solvent of 100.0 mL of anhydrous dichloromethane and 10.0 mL of anhydrous methanol. Under nitrogen protection, $CH_3COOH$ (542 mg, 9.03 mmol) was added and the mixture was stirred at room temperature for 0.5 h. Then solid $NaBH_3CN$ (756 mg, 12.04 mmol) was added to the reaction solution and the mixture was reacted at room temperature for another 2 h. The reaction was completed as detected by LCMS and thin layer chromatography. Silica gel was directly added to the sample. The mixture was purified by Flash to obtain the pure product **D078** (3.1 g) as a white solid. LCMS: $[M + H]^+$ = 1009.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)acrylamide (D478)**

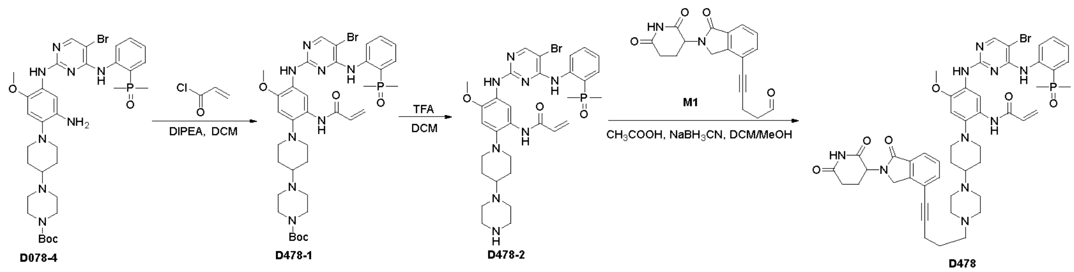


Step 1

**D078-4** (145.6 mg, 0.2 mmol) and diisopropylethylamine (77.4 mg, 0.6 mmol) were dispersed in 15 mL of anhydrous dichloromethane. Under $N_2$ protection, acryloyl chloride (19.8 mg, 0.22 mmol, dissolved in 1.0 mL of dichloromethane) was added dropwise to the reaction solution. The mixture was reacted at room temperature for 1.0 h. The reaction was completed as detected by LCMS. The reaction solution was quenched with 0.5 mL of methanol, then diluted with 20 mL of dichloromethane. Silica gel was directly added to the sample. The mixture was purified by Flash to give a yellow solid product **D478-1** (135 mg). LCMS: $[M + H]^+$ = 783.

Step 2

**D478-1** (135 mg, 0.173 mmol) was dissolved in 10 mL of anhydrous dichloromethane. Under $N_2$ protection, 2.5 mL of trifluoroacetic acid was added to the reaction solution and the mixture was reacted at room temperature for 1.0 h. The reaction was completed as detected by LCMS. The reaction solution was concentrated under reduced pressure. The residue was redissolved in a mixed solvent of methanol and dichloromethane (50 mL), then washed with saturated

$NaHCO_3$ solution (2 x 30 mL) and saturated brine (1 x 30 mL) in sequence. The organic layer was dried over anhydrous magnesium sulfate, and filtered with suction. The filtrate was concentrated under reduced pressure to remove the solvent to give a crude product, which was purified by Flash to give a yellow solid product **D478-2** (110 mg). LCMS: $[M + H]^+$ = 683.

Step 3

**D478-2** (30 mg, 0.044 mmol) and M1 (17.1 mg, 0.053 mmol) were dissolved in a mixed solvent of 10.0 mL of anhydrous dichloromethane and 1.0 mL of anhydrous methanol. Under nitrogen protection, $CH_3COOH$ (4.0 mg, 0.066 mmol) was added and the mixture was stirred at room temperature for 0.5 h. Then solid $NaBH_3CN$ (5.5 mg, 0.088 mmol) was added to the reaction solution. The mixture was reacted at room temperature for another 2 h. The reaction was completed as detected by LCMS and thin layer chromatography. Silica gel was directly added to the sample. The mixture was purified by Flash to obtain the pure product **D478** as a white solid (16 mg, yield 36.7%), LCMS: $[M + H]^+$ = 991, 993.

**Preparation of N-(5-((5-bromo-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)-4-methoxyphenyl)acrylamide (D479)**

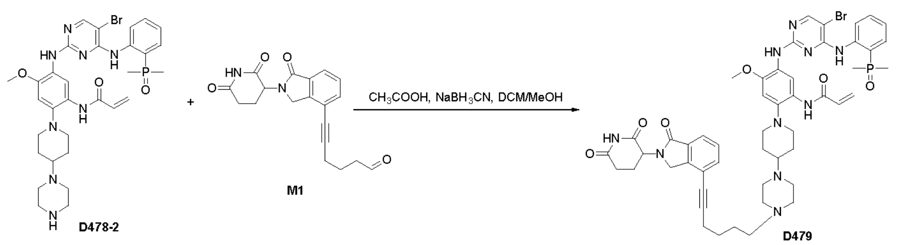


**D478-2** (30 mg, 0.044 mmol) and **M1** (17.9 mg, 0.053 mmol) were dissolved in a mixed solvent of 10.0 mL of anhydrous dichloromethane and 1.0 mL of anhydrous methanol. Under nitrogen protection, $CH_3COOH$ (4.0 mg, 0.066 mmol) was added and the mixture was stirred at room temperature for 0.5 h. Then solid $NaBH_3CN$ (5.5 mg, 0.088 mmol) was added to the reaction solution. The mixture was reacted at room temperature for another 2 h. The reaction was completed as detected by LCMS and thin layer chromatography. Silica gel was directly added to the sample. The mixture was purified by Flash to give a white solid product **D479** (18 mg, yield 40.7%), LCMS: $[M + H]^+$ = 1005, 1007.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)acrylamide (D481)**

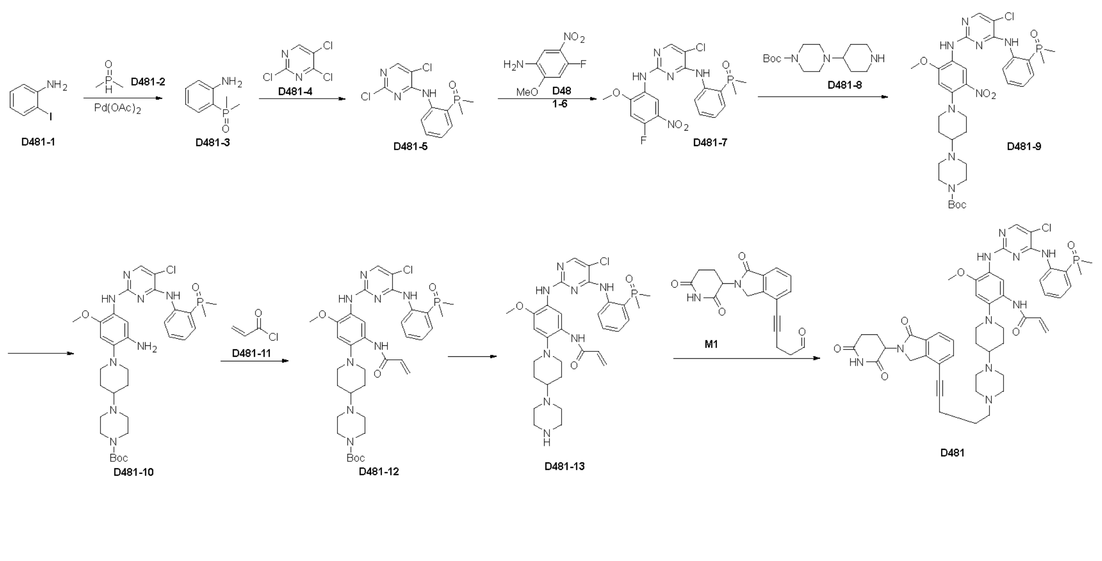

Step 1

**D481-1** (15 g, 68.51 mmol), **D481-2** (6.41 g, 82.21 mmol), palladium acetate (1.54 g, 6.85 mmol), Xantphos (3.96 g, 6.85 mmol) and potassium phosphate (16.37 g, 75.36 mmol) were dissolved in 130 mL of anhydrous DMF. Under nitrogen protection, the mixture was stirred at 120°C for 6 hours. The reaction was cooled to room temperature. Ethyl acetate was added to the reaction. The mixture was stirred for 1 hour, and filtered. The filter cake was washed twice with ethyl acetate. The mother liquor was concentrated, then dissolved in a mixed solvent of water/concentrated hydrochloric acid (4/1), and washed three times with ethyl acetate. The aqueous phase was cooled to below 10°C. The pH was adjusted to 12 with 30% aqueous sodium hydroxide solution. The temperature of the aqueous phase was maintained below 20°C, and the aqueous phase was extracted with isopropanol/dichloromethane (1/3) 4 times. The organic phases were combined, dried over anhydrous sodium sulfate, and filtered. The solvent was evaporated under reduced pressure. The crude product is recrystallized with ethyl acetate/n-hexane (1/10) to obtain **D481-3** (9.52 g, yield 82.2%), LCMS: $[M + H]^+$ = 170.

Step 2

**D481-3** (9.52 g, 56.31 mmol), **D481-4** (12.29 g, 67.57 mmol), potassium carbonate (9.34 g, 67.57 mmol) and tetrabutylammonium hydrogen sulfate (1.91 g, 5.63 mmol) were suspended in 200 mL of DMF. The mixture was stirred at 65 °C overnight under nitrogen protection. The reaction was cooled to room temperature, and filtered to remove solids. The filter cake was washed twice with DMF. The mother liquor was concentrated, and then layered in ethyl acetate/water (3.7/1). The aqueous phase was separated, and extracted twice with ethyl acetate. The organic phases were combined, washed once with saturated brine, dried over anhydrous sodium sulfate, and filtered. The filtrate was concentrated to obtain **D481-5** (10 g). LCMS: $[M + H]^+$ = 316.

Step 3

**D481-5** (1 g, 3.17 mmol), **D481-6** (0.59 g, 3.17 mmol) and methanesulfonic acid (1.22 g, 12.70 mmol) were dissolved in 10 mL of tert butanol. Under nitrogen protection, the mixture was stirred at 90 °C overnight, and cooled to room temperature. The solvent was evaporated under reduced pressure. The crude product was purified by Flash to obtain **D481-7** (1.4 g). LCMS: $[M + H]^+$ = 466.

Step 4

**D481-7** (700 mg, 1.505 mmol), **D481-8** (486 mg, 1.806 mmol) and cesium carbonate (1.47 g, 4.515 mmol) were dissolved in 10 mL of DMF. Under nitrogen protection, the mixture was stirred at 60 °C overnight, and cooled to room temperature. The solid was filtered out, and the solvent was evaporated under reduced pressure. The crude product was purified by Flash to obtain **D481-9** (0.7 g). LCMS: $[M + H]^+$ = 715.

Step 5

**D481-9** (914 mg, 1.28 mmol), iron powder (715 mg, 12.796 mmol) and ammonium chloride (411 mg, 7.678 mmol) were added to 80 mL of ethanol/water (3/1). The mixture was stirred at 100°C for 1 hour, and cooled to room temperature. The solid was filtered out. The solid residue was washed twice with a mixed solvent of ethanol and water. The solvent was evaporated under reduced pressure, and the crude product was purified by Flash to obtain **D481-10** (0.7 g). LCMS: $[M + H]^+$ = 685.

Step 6

**D481-10** (670 mg, 0.979 mmol) and TEA (297 mg, 2.937 mmol) were dissolved in 50 mL of dichloromethane. The reaction was cooled down to -15°C under nitrogen protection. A solution of **D481-11** (80 mg, 0.881 mmol) in dichloromethane (5 mL) was slowly added to the mixture dropwise with stirring. The mixutre was stirred at this temperature for another 1 hour. 1 mL of methanol was added to quench the reaction. The solvent was removed under reduced pressure. The residue was purified by Flash to obtain **D481-12** (560 mg), LCMS: $[M + H]^+$ = 739.

Step 7

**D481-12** (560 mg) was dissolved in 12 mL of dichloromethane, and trifluoroacetic acid (3 mL) was added with stirring. The mixture was stirred at room temperature for 1 hour under nitrogen protection. The solvent was removed under

reduced pressure and the residue was purified by Flash to obtain **D481-13** (460 mg, yield 95.0%), LCMS: $[M + H]^+$ = 639.

Step 8

**D481-13** (30 mg, 0.093 mmol), **M1** (71 mg, 0.111 mmol) and acetic acid (6.7 mg, 0.111 mmol) were dissolved in 3 mL of a mixed solvent of dichloromethane/methanol (2/1). The mixture was stirred at room temperature for 1 hour under nitrogen. Sodium cyanoborohydride (8.7 mg, 0.139 mmol) was added and the mixture was stirred for another 2 hours. The solvent was removed under reduced pressure below 40°C, and the crude product was purified by HPLC to give an off-white solid compound **D481** (22.6 mg, yield 50.8%), LCMS: $[M + H]^+$ = 974.

**Preparation of N-(5-((5-bromo-4-((2-(dimethylphosphoryl)phenyl)amino)pyrimidin-2-yl)amino)-2-(4-(4-(7-(2-(2,6-dioxopiperidin-3-yl)-1-oxoisoindolin-4-yl)hept-6-yn-1-yl)piperazin-1-yl)piperidin-1-yl)-4-methoxyphenyl)acrylamide (D503)**

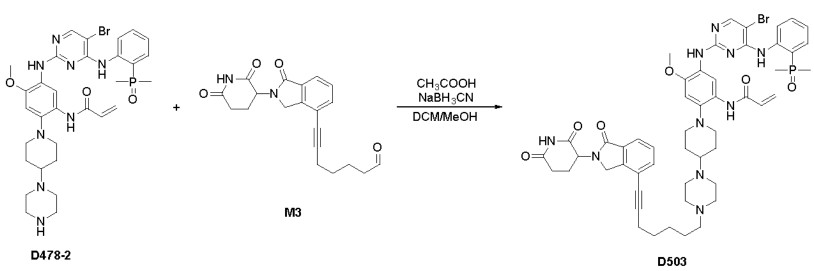


D478-2 M3 D503

**D478-2** (30 mg, 0.044 mmol) and **M3** (18.7 mg, 0.053 mmol) were dissolved in a mixed solvent of 10.0 mL of anhydrous dichloromethane and 1.0 mL of anhydrous methanol. Under nitrogen protection, $CH_3COOH$ (4.0 mg, 0.066 mmol) was added and the mixture was stirred at room temperature for 0.5 h. Then solid $NaBH_3CN$ (5.5 mg, 0.088 mmol) was added to the reaction solution and the mixture was reacted at room temperature for another 2 h. The reaction was completed as detected by LCMS and thin layer chromatography. Silica gel was directly added to the sample. The mixture was purified by Flash to give a white solid pure product D503 (17 mg, yield 37.9%). LCMS: $[M + H]^+$ = 1019, 1021.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)phenyl)acrylamide** (D549)

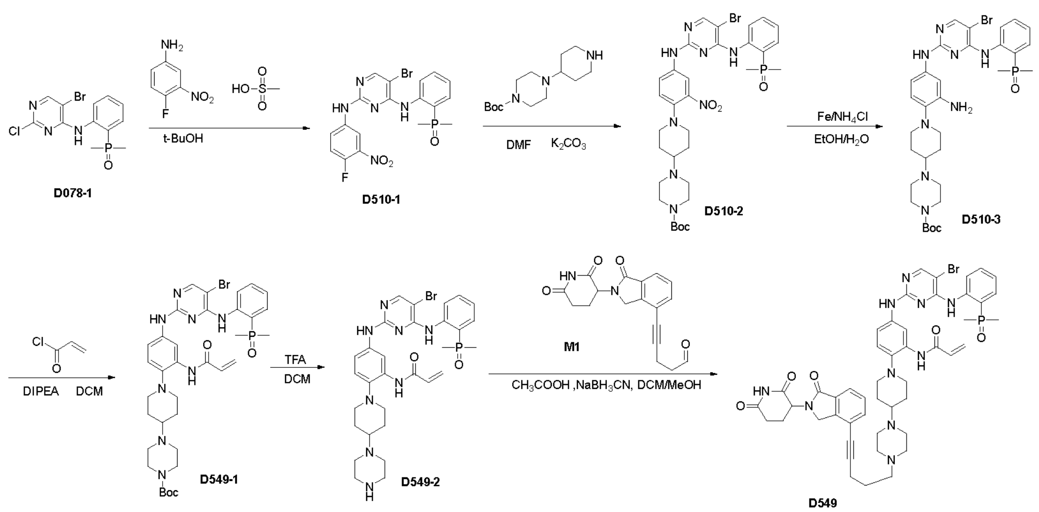


D078-1 D510-1 D510-2 D510-3 D549-1 D549-2 M1 D549

Step 1

**D078-1** (3.59 g, 10 mmol) and 4-fluoro-3-nitroaniline (1.72 g, 11 mmol) were dispersed in 70 mL of tert butanol, and methanesulfonic acid (3.84 g, 40 mmol) was added to the reaction solution. The reaction was heated to 90°C for 16 h. The reaction was completed as detected by LCMS. The reaction solution was cooled to room temperature, and then concentrated under reduced pressure to remove the solvent to give a crude product, which was flash purified to give a light yellow solid product **D510-1** (4.1 g, 85.6%). LCMS: $[M + H]^+$ = 480.

Step 2

**D510-1** (4.1 g, 8.56 mmol), tert-butyl 4-(piperidin-4-yl)piperazine-1-carboxylate (2.3 g, 8.56 mmol) and $K_2CO_3$ (3.54 g, 25.7 mmol) were dispersed in 82 mL of anhydrous DMF. The reaction was heated to 60°C for 12 h. The reaction was completed as detected by LCMS. The reaction solution was cooled to room temperature, filtered with suction, and the solid residue was washed with DMF. The filtrate was concentrated under reduced pressure to give a crude product, which was flash purified to give a yellow solid product **D510-2** (5.6 g, 89.9%). LCMS: $[M + H]^+$ = 729.

Step 3

**D510-2** (1.1 g, 1.51 mmol), iron powder (507 mg, 9.06 mmol) and $NH_4Cl$ (4.04 g, 75.5 mmol) were dispersed in a mixed solvent of 45 mL of ethanol and 15 mL of water. The reaction was heated to 90°C for 2 h. The reaction was completed as determined by LCMS. The reaction solution was cooled to room temperature, and filtered with diatomaceous earth. The solid residue was washed with a mixed solvent of ethanol and water (50 mL). The filtrate was collected and concentrated under reduced pressure to remove ethanol. The residue was extracted twice with a mixed solvent of methanol and dichloromethane (60 mL). The organic layers were combined and washed with water (2 x 30 mL) and saturated brine (1 x 30 mL) sequentially. The organic layer was dried over anhydrous magnesium sulfate, and filtered with suction. The filtrate was concentrated under reduced pressure to remove the solvent to give a crude product, which was purified by Flash to give a yellow solid product **D510-3** (760 mg, 72.1%). LCMS: $[M + H]^+$ = 699.

Step 4

**D510-3** (139.6 mg, 0.2 mmol) and diisopropylethylamine (77.4 mg, 0.6 mmol) were dispersed in 15 mL of anhydrous dichloromethane. Under $N_2$ protection, acryloyl chloride (19.8 mg, 0.22 mmol, dissolved in 1.0 mL of dichloromethane) was added dropwise to the reaction solution. The mixture was reacted at room temperature for 1.0 h. The reaction was completed as detected by LCMS. The reaction solution was quenched with 0.5 mL of methanol, and then diluted with 20 mL of dichloromethane. Silica gel was directly added to the sample. The mixture was purified by Flash to give a yellow solid product **D549-1** (135 mg). LCMS: $[M + H]^+$ = 753.

Step 5

**D549-1** (135 mg, 0.18 mmol) was dissolved in 10 mL of anhydrous dichloromethane. Under $N_2$ protection, 2.5 mL of trifluoroacetic acid was added to the reaction solution. The mixture was reacted at room temperature for 1.0 h. LCMS showed that the reaction was completed. The reaction solution was concentrated under reduced pressure. The residue was redissolved in a mixed solvent of methanol and dichloromethane (50 mL), and then washed with saturated $NaHCO_3$ solution (2 x 30 mL) and saturated brine (1 x 30 mL) in sequence. The organic layer was dried over anhydrous magnesium sulfate, and filtered with suction. The filtrate was concentrated under reduced pressure to remove the solvent to give a crude product, which was purified by Flash to give a yellow solid product **D549-2** (110 mg). LCMS: $[M + H]^+$ = 653.

Step 6

**D549-2** (28.7 mg, 0.044 mmol) and **M1** (17.1 mg, 0.053 mmol) were dissolved in a mixed solvent of 10.0 mL of anhydrous dichloromethane and 1.0 mL of anhydrous methanol. Under nitrogen protection, $CH_3COOH$ (4.0 mg, 0.066 mmol) was added and the mixture was stirred at room temperature for 0.5 h. Then solid $NaBH_3CN$ (5.5 mg, 0.088 mmol) was added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D549** (16 mg, yield 37.9%). LCMS: $[M + H]^+$ = 961, 963.

**Preparation of N-(5-((5-bromo-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)phenyl)acrylamide (D550)**

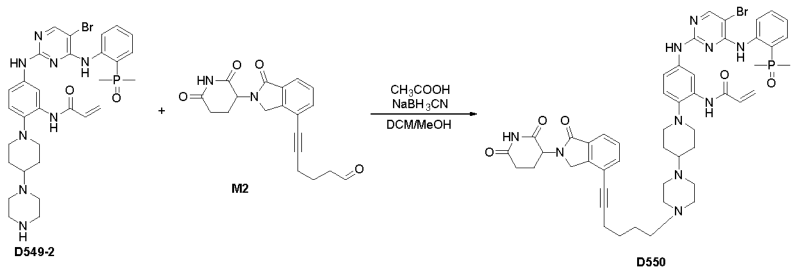


D549-2 M2 D550

**D549-2** (28.7 mg, 0.044 mmol) and **M2** (17.9 mg, 0.053 mmol) were dissolved in a mixed solvent of 10.0 mL of anhydrous dichloromethane and 1.0 mL of anhydrous methanol. Under nitrogen protection, $CH_3COOH$ (4.0 mg, 0.066 mmol) was added and the mixture was stirred at room temperature for 0.5 h. Then solid $NaBH_3CN$ (5.5 mg, 0.088 mmol) was added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D550** (18.5 mg, yield 43.2%). LCMS: $[M + H]^+$ = 975, 977.

**Preparation of N-(5-((5-bromo-4-((2-(dimethylphosphoryl)phenyl)amino)pyrimidin-2-yl)amino)-2-(4-(4-(7-(2-(2,6-dioxopiperidin-3-yl)-1-oxoisoindolin-4-yl)hept-6-yn-1-yl)piperazin-1-yl)piperidin-1-yl)phenyl)acrylamide (D551)**

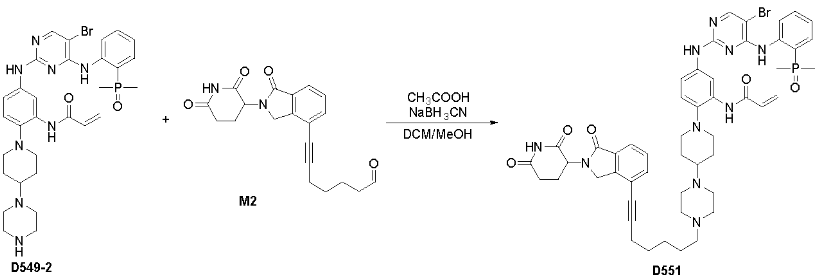


D549-2 M2 D551

**D549-2** (28.7 mg, 0.044 mmol) and **M2** (18.7 mg, 0.053 mmol) were dissolved in a mixed solvent of 10.0 mL of anhydrous dichloromethane and 1.0 mL of anhydrous methanol. Under nitrogen protection, $CH_3COOH$ (4.0 mg, 0.066 mmol) was added and the mixture was stirred at room temperature for 0.5 h. Then solid $NaBH_3CN$ (5.5 mg, 0.088 mmol) was added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D551** (19 mg, yield 43.7%). LCMS: $[M + H]^+$= 989, 991.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-fluoroacrylamide (D552)**

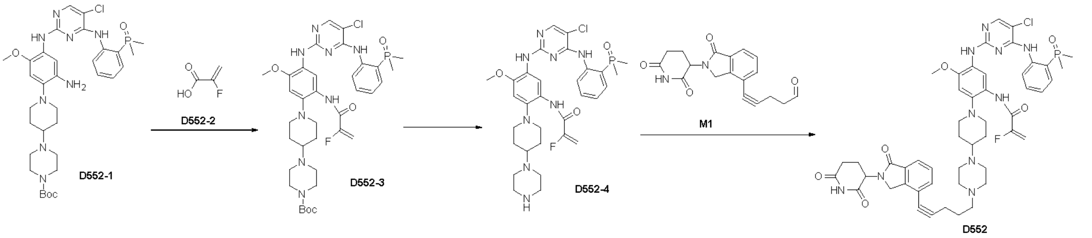


Step 1

**D552-1** (300 mg, 0.44 mmol), **D552-2** (43 mg, 0.48 mmol), T3P (1.12 mL, 1.75 mmol) and DIEA (226 mg, 1.754 mmol) were dissolved in 10 mL of dichloromethane. Under nitrogen protection, the mixture was stirred at room temperature for 4 hours. The residue was purified by Flash to give **D552-3** (318 mg, yield 96.1%), LCMS: $[M + H]^+ = 757$.

Step 2

**D552-3** (318 mg) was dissolved in 20 mL of dichloromethane, and trifluoroacetic acid (5 mL) was added with stirring. Under nitrogen protection, the mixture was stirred at room temperature for 2 hours. The solvent was removed under reduced pressure and the residue was purified by Flash to give D552-4 (276 mg, yield 100%), LCMS: $[M + H]^+ = 657$.

Step 3

**M1** (20 mg, 0.062 mmol), **D552-4** (45 mg, 0.068 mmol) and acetic acid (4.4 mg, 0.074 mmol) were dissolved in 3 mL of a mixed solvent of dichloromethane/methanol (2/1). Under nitrogen, the mixture was stirred at room temperature for 1 hour. Sodium cyanoborohydride (5.8 mg, 0.093 mmol) was added and the mixture was stirred for another 2 hours. The solvent was removed under reduced pressure below 40°C, and the crude product was purified by HPLC to give an off-white solid D552 (20 mg, yield 33.3%), LCMS: $[M + H]^+ = 965$.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)-4-methoxyphenyl)-2-fluoroacrylamide (D553)**

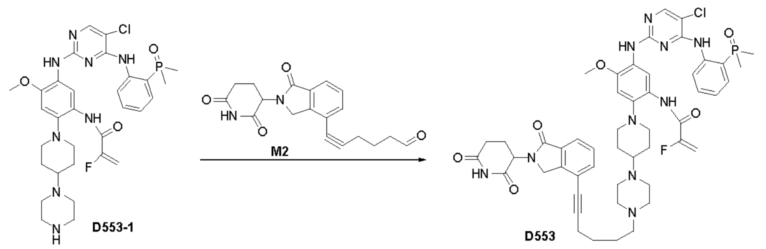


**M2** (20 mg, 0.059 mmol), **D553-1** (43 mg, 0.065 mmol) and acetic acid (4.3 mg, 0.071 mmol) were dissolved in 3 mL of a mixed solvent of dichloromethane/methanol (2/1). Under nitrogen, the mixture was stirred at room temperature for 1 hour. Sodium cyanoborohydride (5.6 mg, 0.089 mmol) was added and the mixture was stirred for another 2 hours. The solvent was removed under reduced pressure below 40°C, and the crude product was purified by HPLC to give an off-white solid compound **D553** (13 mg, yield 22.4%), LCMS: $[M + H]^+ = 979$.

**Preparation of N-(5-((5- chloro-4-((2-(dimethylphosphoryl)phenyl)amino)pyrimidin-2-yl)amino)-2-(4-(4-(7-(2-(2,6-dioxopiperidin-3-yl)-1-oxoisoindolin-4-yl)hept-6-yn-1-yl)piperazin-1-yl)piperidin-1-yl)-4-methoxyphenyl)-2-fluoroacrylamide (D554)**

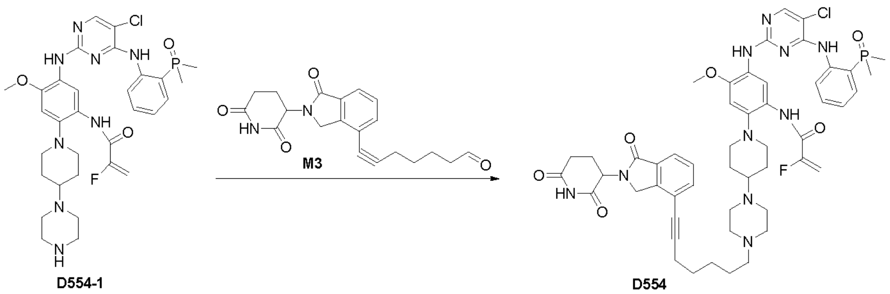


D554-1 D554

**M3** (20 mg, 0.057 mmol), **D554-1** (41 mg, 0.062 mmol) and acetic acid (4.1 mg, 0.068 mmol) were dissolved in 3 mL of a mixed solvent of dichloromethane/methanol (2/1). Under nitrogen, the mixture was stirred at room temperature for 1 hour. Sodium cyanoborohydride (5.4 mg, 0.085 mmol) was added and the mixture was stirred for another 2 hours. The solvent was removed under reduced pressure below 40°C, and the crude product was purified by HPLC to give an off-white solid compound **D554** (12 mg, yield 21.4%), LCMS: $[M + H]^+ = 993$.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4-(dimethylamino)but-2-enamide (D555)**

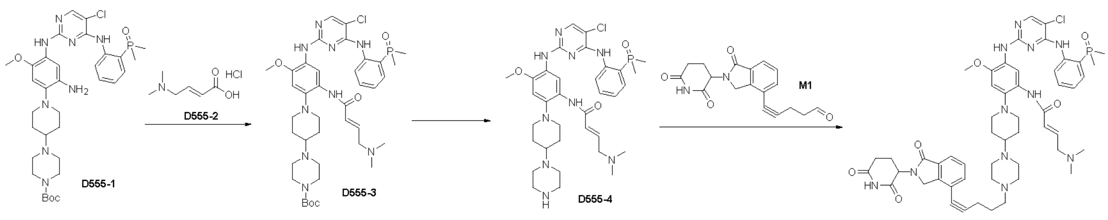


D555-1 D555-3 D555-4 D555

Step 1

**D555-1** (300 mg, 0.44 mmol), **D555-2** (80 mg, 0.48 mmol), T3P (50% EA, 1.12 mL, 1.75 mmol) and DIEA (283 mg, 2.19 mmol) were dissolved in 10 mL of dichloromethane. Under nitrogen protection, the mixture was stirred at room temperature for 4 hours. The residue was purified by Flash to give **D555-3** (303 mg, yield 86.8%), LCMS: $[M + H]^+ = 796$.

Step 2

**D555-3** (303 mg) was dissolved in 20 mL of dichloromethane. Trifluoroacetic acid (5 mL) was added with stirring. Under nitrogen protection, the mixture was stirred at room temperature for 2 hours. The solvent was removed under reduced pressure and the residue was purified by Flash to obtain **D555-4** (254 mg, yield 95.8%), LCMS: $[M + H]^+ = 696$.

Step 3

**M1** (20 mg, 0.062 mmol), **D555-4** (47 mg, 0.068 mmol) and acetic acid (4.4 mg, 0.074 mmol) were dissolved in 3 mL of a mixed solvent of dichloromethane/methanol (2/1). Under nitrogen, the mixture was stirred at room temperature for 1 hour. Sodium cyanoborohydride (5.8 mg, 0.093 mmol) was added and the mixture was stirred for another 2 hours. The solvent was removed under reduced pressure below 40°C, and the crude product was purified by HPLC to give an off-white solid compound **D555** (10 mg, yield 16.1%), LCMS: $[M + H]^+ = 1004$.

**Preparation of (*E*)-N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)-4-dimethoxyphenyl)-4-(dimethylamino)but-2-enamide (D556)**

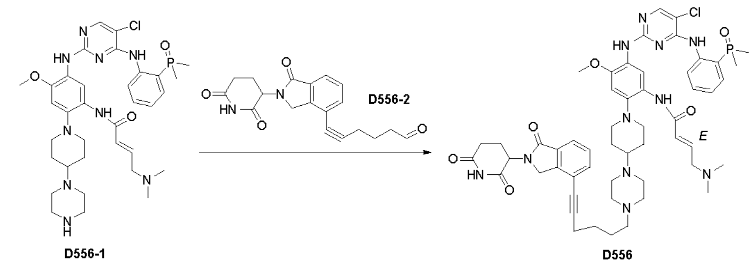

D556-1 D556-2 D556

**D556-1** (45 mg, 0.065 mmol), **D556-2** (20 mg, 0.059 mmol) and acetic acid (4.3 mg, 0.071 mmol) were dissolved in 3 mL of a mixed solvent of dichloromethane/methanol (2/1). Under nitrogen, the mixture was stirred at room temperature for 1 hour. Sodium cyanoborohydride (5.6 mg, 0.089 mmol) was added and the mixture was stirred for another 2 hours. The solvent was removed under reduced pressure below 40°C, and the crude product was purified by HPLC to give an off-white solid compound **D556** (15 mg, yield 25.0%), LCMS: $[M + H]^+$ = 1018.

**Preparation of (*E*)-N-(5-((5-chloro-4-((2-(dimethylphosphoryl)phenyl)amino)pyrimidin-2-yl)amino)-2-(4-(4-(7-(2-(2,6-dioxopiperidin-3-yl)-1-oxoisoindolin-4-yl)hept-6-yn-1-yl)piperazin-1-yl)piperidin-1-yl)-4-dimethoxyphenyl)-4-(dimethylamino)but-2-enamide (D557)**

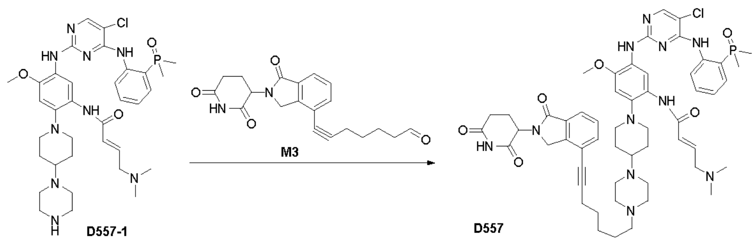

M3 D557-1 D557

**M3** (20 mg, 0.057 mmol), **D557-1** (43 mg, 0.062 mmol) and acetic acid (4.1 mg, 0.068 mmol) were dissolved in 3 mL of a mixed solvent of dichloromethane/methanol (2/1). Under nitrogen, the mixture was stirred at room temperature for 1 hour. Sodium cyanoborohydride (5.4 mg, 0.085 mmol) was added and the mixture was stirred for another 2 hours. The solvent was removed under reduced pressure below 40°C, and the crude product was purified by HPLC to give an off-white solid D557 (15 mg, yield 25.9%), LCMS: $[M + H]^+$ = 1032.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)-4-methoxyphenyl)but-2-ynamide (D559)**

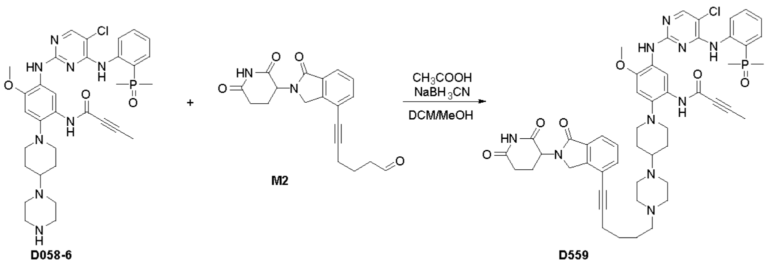


D058-6 M2 D559

**M2** (50.7 mg, 0.15 mmol) and **D058-6** (87.8 mg, 0.135 mmol) were dissolved in a mixed solvent of 15 mL of anhydrous dichloromethane and 1.5 mL of anhydrous methanol. Under nitrogen protection, $CH_3COOH$ (13.5 mg, 0.225 mmol) was added and the mixture was stirred at room temperature for 0.5 h. Then solid $NaBH_3CN$ (18.8 mg, 0.3 mmol) was added to the reaction solution and the mixture was reacted at room temperature for another 2 h. The reaction was completed as detected by LCMS and thin layer chromatography. Silica gel was directly added to the sample. The mixture was purified by Flash to obtain the pure product **D559** (36 mg, yield 24.7%) as a white solid. LCMS: $[M + H]^+$ = 973.

**Preparation of N-(5-((5-chloro-4-((2-(dimethylphosphoryl)phenyl)amino)pyrimidin-2-yl)amino)-2-(4-(4-(7-(2-(2,6-dioxopiperidin-3-yl)-1-oxoisoindolin-4-yl)hept-6-yn-1-yl)piperazin-1-yl)piperidin-1-yl)-4-methoxyphenyl)but-2-ynamide (D560)**

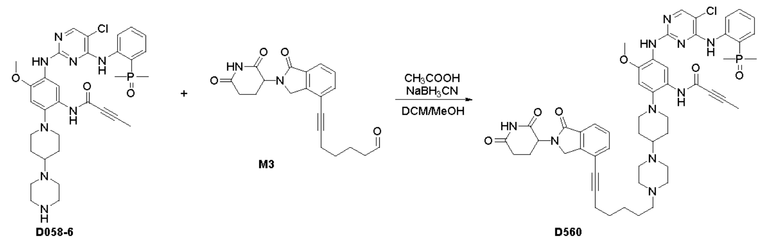


**M3** (52.8 mg, 0.15 mmol) and **D058-6** (87.8 mg, 0.135 mmol) were dissolved in a mixed solvent of 15 mL of anhydrous dichloromethane and 1.5 mL of anhydrous methanol. Under nitrogen protection, $CH_3COOH$ (13.5 mg, 0.225 mmol) was added and the mixture was stirred at room temperature for 0.5 h. Then solid $NaBH_3CN$ (18.8 mg, 0.3 mmol) was added to the reaction solution and the mixture was reacted at room temperature for another 2 h. The reaction was completed as detected by LCMS and thin layer chromatography. Silica gel was directly added to the sample. The mixture was purified by Flash to obtain the pure product **D560** (41 mg, yield 27.7%) as a white solid. LCMS: $[M + H]^+$= 987.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4-(dimethylamino)but-2-enamide (D561)**

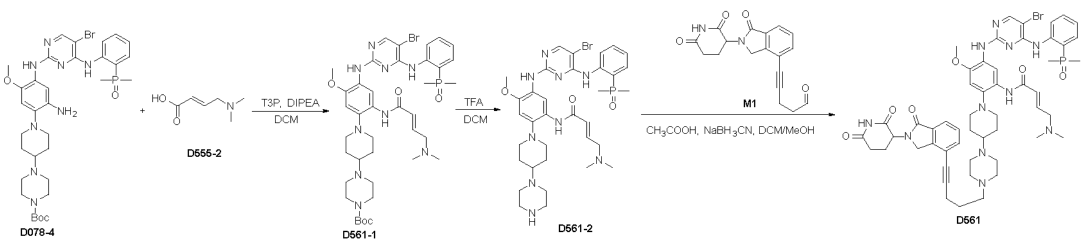


Step 1

**D078-4** (145.6 mg, 0.2 mmol), **D555-2** (25.8 mg, 0.2 mmol) and T3P (254 mg, 0.4 mmol) were dispersed in 4.0 mL of anhydrous dichloromethane. Under $N_2$ protection, diisopropylethylamine (129 g, 1.0 mmol) was added to the reaction solution. The mixture was reacted at room temperature for 2.0 h. The reaction was completed as detected by LCMS. The reaction solution was diluted with 50 mL of dichloromethane. Silica gel was directly added to the sample. The mixture was purified by Flash to give a yellow solid product **D561-1** (141 mg). LCMS: $[M + H]^+$= 840.

Step 2

**D561-1** (141 mg, 0.168 mmol) was dissolved in 10 mL of anhydrous dichloromethane. Under $N_2$ protection, 2.5 mL of trifluoroacetic acid was added to the reaction solution. The mixture was reacted at room temperature for 1.0 h. The reaction was completed as detected by LCMS. The reaction solution was concentrated under reduced pressure. The residue was redissolved in a mixed solvent of methanol and dichloromethane (50 mL), and then washed with saturated $NaHCO_3$ solution (2 x 30 mL) and saturated brine (1 x 30 mL) in sequence. The organic layer was dried over anhydrous magnesium sulfate, and filtered with suction. The filtrate was concentrated under reduced pressure to remove the solvent to give a crude product, which was purified by Flash to give a yellow solid product D561-2 (110 mg). LCMS: $[M + H]^+$= 740.

Step 3

**D561-2** (30 mg, 0.041 mmol) and **M1** (15.9 mg, 0.049 mmol) were dissolved in a mixed solvent of 10.0 mL of

anhydrous dichloromethane and 1.0 mL of anhydrous methanol. Under nitrogen protection, $CH_3COOH$ (3.7 mg, 0.062 mmol) was added and the mixture was stirred at room temperature for 0.5 h. Then solid $NaBH_3CN$ (5.1 mg, 0.082 mmol) was added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D561** (11.5 mg, yield 26.8%). LCMS: $[M + H]^+$ = 1048, 1050.

**Preparation of N-(5-((5-bromo-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)-4-dimethoxyphenyl)-4-(dimethylamino)but-2-enamide (D562)**

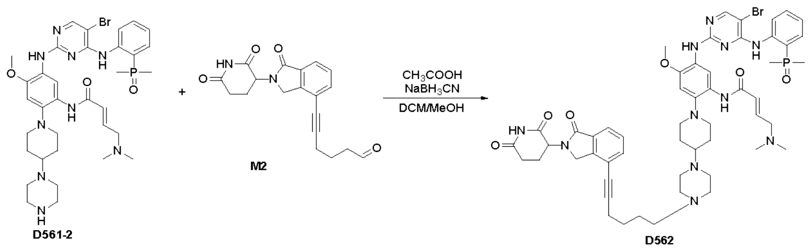


**D561-2** (30 mg, 0.041 mmol) and **M2** (16.6 mg, 0.049 mmol) were dissolved in a mixed solvent of 10.0 mL of anhydrous dichloromethane and 1.0 mL of anhydrous methanol. Under nitrogen protection, $CH_3COOH$ (3.7 mg, 0.062 mmol) was added and the mixture was stirred at room temperature for 0.5 h. Then solid $NaBH_3CN$ (5.1 mg, 0.082 mmol) was added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D562** (15.5 mg, yield 35.6%). LCMS: $[M + H]^+$ = 1062, 1064.

**Preparation of N-(5-((5-bromo-4-((2-(dimethylphosphoryl)phenyl)amino)pyrimidin-2-yl)amino)-2-(4-(4-(7-(2-(2,6-dioxopiperidin-3-yl)-1-oxoisoindolin-4-yl)hept-6-yn-1-yl)piperazin-1-yl)piperidin-1-yl)-4-dimethoxyphenyl)-4-(dimethylamino)but-2-enamide (D563)**

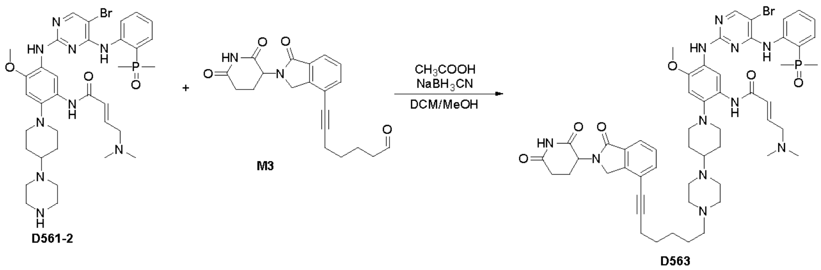


**D561-2** (30 mg, 0.041 mmol) and **M3** (17.2 mg, 0.049 mmol) were dissolved in a mixed solvent of 10.0 mL of anhydrous dichloromethane and 1.0 mL of anhydrous methanol. Under nitrogen protection, $CH_3COOH$ (3.7 mg, 0.062 mmol) was added and the mixture was stirred at room temperature for 0.5 h. Solid $NaBH_3CN$ (5.1 mg, 0.082 mmol) was then added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D563** (16.5 mg, yield 31.3%). LCMS: $[M + H]^+$ = 1076, 1078.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)methacrylamide (D579)**

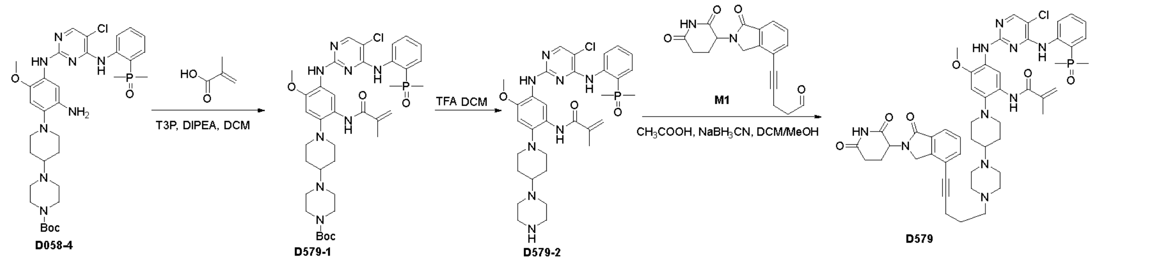


Step 1

**D058-4** (136.8 mg, 0.2 mmol), methacrylic acid (17.2 mg, 0.2 mmol) and T3P (254 mg, 0.4 mmol) were dispersed in 5.0 mL of anhydrous dichloromethane. Under $N_2$ protection, diisopropylethylamine (129 mg, 1.0 mmol) was added to the reaction solution. The mixture was reacted at room temperature for 2.0 h. The reaction was completed as detected by LCMS. The reaction solution was diluted with 15 mL of dichloromethane and silica gel was directly added to the sample. The mixture was purified by Flash to give a yellow solid product **D579-1** (120 mg). LCMS: $[M + H]^+$ = 753.

Step 2

**D579-1** (120 mg, 0.16 mmol) was dissolved in 10 mL of anhydrous DCM. Under $N_2$ protection, 2.5 mL of trifluoroacetic acid was added to the reaction solution. The mixture was reacted at room temperature for 1.0 h. The reaction was completed as detected by LCMS. The reaction solution was concentrated under reduced pressure. The residue was redissolved in a mixed solvent of methanol and dichloromethane (40 mL), and then washed with saturated $NaHCO_3$ solution (2 x 30 mL) and saturated brine (1 x 30 mL) in sequence. The organic layer was dried over anhydrous magnesium sulfate, and filtered. The filtrate was concentrated under reduced pressure to remove the solvent to give a crude product, which was purified by Flash to give a yellow solid product **D579-2** (88 mg). LCMS: $[M + H]^+$ = 653.

Step 3

**M1** (52.5 mg, 0.162 mmol) and **D579-2** (88 mg, 0.135 mmol) were dissolved in a mixed solvent of 15 mL of anhydrous dichloromethane and 1.5 mL of anhydrous methanol. Under nitrogen protection, $CH_3COOH$ (12.2 mg, 0.203 mmol) was added and the mixture was stirred at room temperature for 0.5 h. Solid $NaBH_3CN$ (17.0 mg, 0.27 mmol) was then added to the reaction solution and the mixture was reacted at room temperature for another 2 h. The reaction was completed as detected by LCMS and thin layer chromatography. Silica gel was directly added to the sample. The mixture was purified by Flash to give a white solid pure product **D579** (51 mg, yield 39.3%). LCMS: $[M + H]^+$ = 961.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)cinnamamide (D580)**

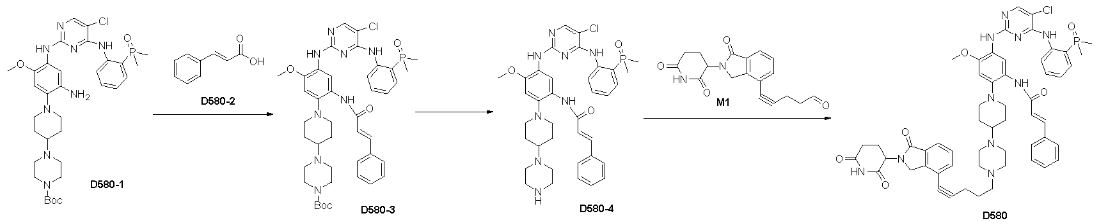


Step 1

**D580-1** (80 mg, 0.117 mmol), **D580-2** (19 mg, 0.129 mmol), T3P (50% EA, 149 mg, 0.468 mmol) and TEA (47 mg, 0.468 mmol) were dissolved in 2 mL of dichloromethane. Under nitrogen protection, the mixture was stirred at room temperature for 4 hours, and purified by thin layer chromatography to obtain **D580-3** (75 mg, yield 78.9%), LCMS: $[M + H]^+$ = 815.

Step 2

**D580-3** (75 mg) was dissolved in 4 mL of dichloromethane. Trifluoroacetic acid (1 mL) was added with stirring. Under nitrogen protection, the mixture was stirred at room temperature for 2 hours. The solvent was removed under reduced pressure and the residue was purified by Flash to obtain **D580-4** (64 mg, yield 97.0%), LCMS: $[M + H]^+ = 715$.

Step 3

**D580-4** (63.9 mg, 0.089 mmol), **M1** (26.4 mg, 0.081 mmol) and acetic acid (5.9 mg, 0.098 mmol) were dissolved in 2 mL of a mixed solvent of dichloromethane/methanol (2/1). Under nitrogen, the mixture was stirred at room temperature for 1 hour. Sodium cyanoborohydride (7.7 mg, 0.122 mmol) was added and the mixture was stirred for another 2 hours. The solvent was removed under reduced pressure below 40°C, and the crude product was purified by HPLC to give compound **D580** (30 mg, yield 33.0%), LCMS: $[M + H]^+ = 1023$.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-3-(1H-imidazol-4-yl)acrylamide (D581)**

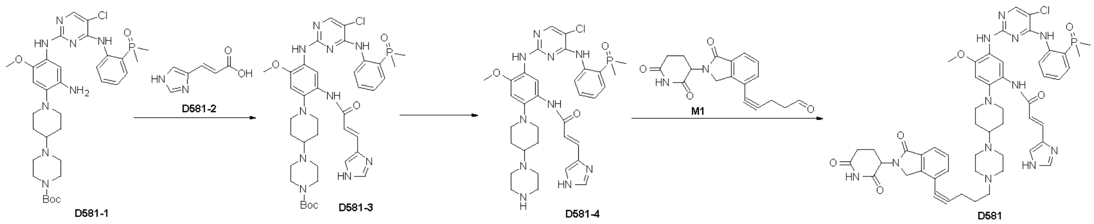

D581-1 D581-3 D581-4 D581

Step 1

**D581-2** (24 mg, 0.174 mmol) and HATU (77 mg, 0.203 mmol) were dissolved in 2 mL of tetrahydrofuran. Under nitrogen, the mixture was stirred at room temperature for 30 minutes. A solution of **D581-1** (80 mg, 0.117 mmol) and DIEA (45 mg, 0.351 mmol) in 2 mL of tetrahydrofuran was added dropwise. The mixture was heated to 60°C and stirred for 64 hours. The mixture was cooled to room temperature and the solvent was removed under reduced pressure. The residue was purified by Flash (dichloromethane/methanol) to give **D581-3** (60 mg, yield 63.8%), LCMS: $[M + H]^+ = 805$.

Step 2

**D581-3** (60 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D581-4** (38 mg, yield 52.5%), LCMS: $[M + H]^+ = 705$.

Step 3

**D581-4** (38.2 mg, 0.054 mmol), **M1** (19.3 mg, 0.060 mmol) and acetic acid (3.9 mg, 0.065 mmol) were dissolved in 2 mL of a mixed solvent of dichloromethane/methanol (2/1). Under nitrogen, the mixture was stirred at room temperature for 1 hour. Sodium cyanoborohydride (5.1 mg, 0.081 mmol) was added and the mixture was stirred for another 2 hours. The solvent was removed under reduced pressure below 40°C, and the crude product was purified by HPLC to give compound **D581** (15 mg, yield 27.3%), LCMS: $[M + H]^+ = 1013$.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-phenylacrylamide (D582)**

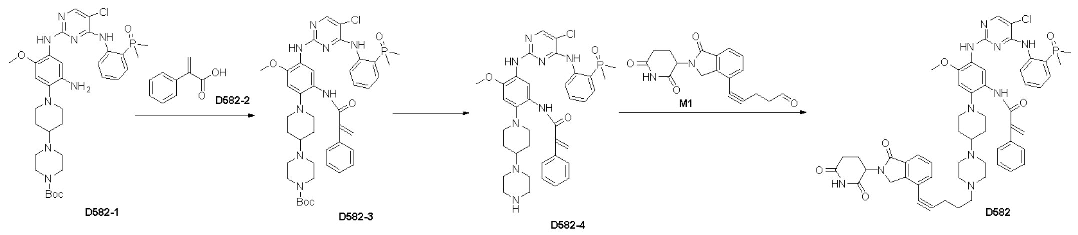


Step 1

**D582-1** (80 mg, 0.117 mmol), **D582-2** (19 mg, 0.129 mmol), T3P (50% EA, 149 mg, 0.468 mmol) and TEA (47 mg, 0.468 mmol) were dissolved in 2 mL of dichloromethane. Under nitrogen protection, the mixture was stirred at room temperature for 4 hours. The residue was purified by thin layer chromatography to obtain **D582-3** (69 mg, yield 72.6%), LCMS: $[M + H]^+$ = 815.

Step 2

**D582-3** (69 mg) was dissolved in 4 mL of dichloromethane. Trifluoroacetic acid (1 mL) was added with stirring. Under nitrogen protection, the mixture was stirred at room temperature for 2 hours. The solvent was removed under reduced pressure and the residue was purified by Flash to obtain **D582-4** (50 mg, yield 83.3%), LCMS: $[M + H]^+$ = 715.

Step 3

**D582-4** (55 mg, 0.077 mmol), **M1** (22.7 mg, 0.070 mmol) and acetic acid (5.0 mg, 0.084 mmol) were dissolved in 2 mL of a mixed solvent of dichloromethane/methanol (2/1). Under nitrogen, the mixture was stirred at room temperature for 1 hour. Sodium cyanoborohydride (6.6 mg, 0.105 mmol) was added and the mixture was stirred for another 2 hours. The solvent was removed under reduced pressure below 40°C, and the crude product was purified by HPLC to give compound **D582** (25 mg, yield 34.9%), LCMS: $[M + H]^+$ = 1023.

**Preparation of (*E*)-N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-3-(thiophen-2-yl)acrylamide (D583)**

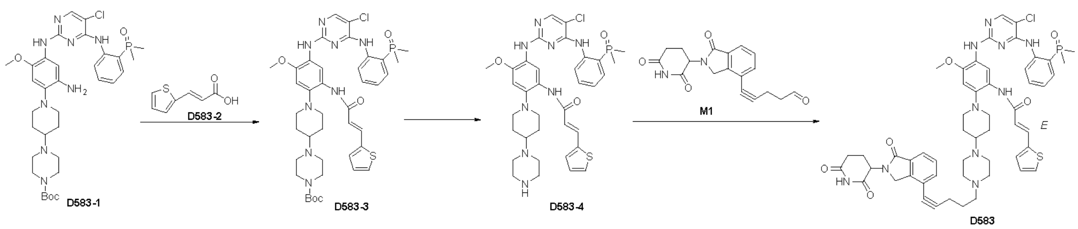


Step 1

**D583-1** (80 mg, 0.117 mmol), **D583-2** (20 mg, 0.129 mmol), T3P (149 mg, 0.468 mmol) and TEA (47 mg, 0.468 mmol) were dissolved in 2 mL of dichloromethane. Under nitrogen protection, the mixture was stirred at room temperature for 4 hours. The residue was purified by thin layer chromatography to obtain **D583-3** (66 mg, yield 68.8%), LCMS: $[M + H]^+$ = 821.

Step 2

**D583-3** (66 mg) was dissolved in 4 mL of dichloromethane. Trifluoroacetic acid (1 mL) was added with stirring. Under nitrogen protection, the mixture was stirred at room temperature for 2 hours. The solvent was removed under reduced pressure and the residue was purified by Flash to obtain **D583-4** (22 mg, yield 37.9%), LCMS: $[M + H]^+$ = 721.

Step 3

**D583-4** (53.4 mg, 0.074 mmol), **M1** (21.8 mg, 0.067 mmol) and acetic acid (4.9 mg, 0.081 mmol) were dissolved in 2 mL of a mixed solvent of dichloromethane/methanol (2/1). Under nitrogen, the mixture was stirred at room temperature for 1 hour. Sodium cyanoborohydride (6.3 mg, 0.101 mmol) was added and the mixture was stirred for another 2 hours. The solvent was removed under reduced pressure below 40°C, and the crude product was purified by HPLC to give compound **D583** (25 mg, yield 36.2%), LCMS: $[M + H]^+$ = 1029.

**Preparation of (*E*)-N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4-(piperidin-1-yl)but-2-enamide (D584)**

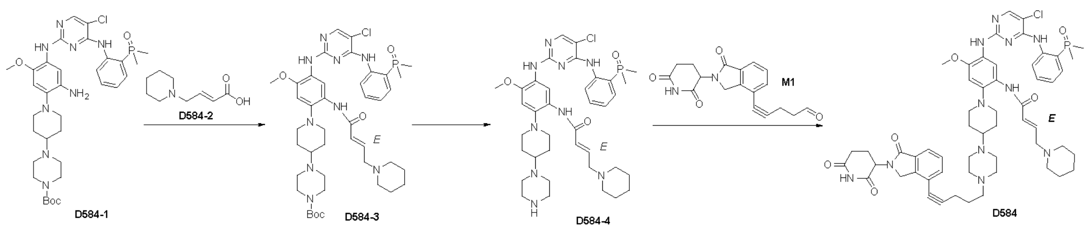


Step 1

**D584-1** (80 mg, 0.117 mmol), **D584-2** (26 mg, 0.129 mmol), T3P (149 mg, 0.468 mmol) and TEA (47 mg, 0.468 mmol) were dissolved in 2 mL of dichloromethane. Under nitrogen protection, the mixture was stirred at room temperature for 4 hours. The residue was purified by thin layer chromatography to obtain **D584-3** (68 mg, yield 70.1%), LCMS: $[M + H]^+$ = 836.

Step 2

**D584-3** (68 mg) was dissolved in 4 mL of dichloromethane. Trifluoroacetic acid (1 mL) was added with stirring. Under nitrogen protection, the mixture was stirred at room temperature for 2 hours. The solvent was removed under reduced pressure and the residue was purified by Flash to obtain **D584-**4 (15 mg, yield 25.0%), LCMS: $[M + H]^+$ = 736.

Step 3

**D584-4** (37.3 mg, 0.051 mmol), **M1** (15.0 mg, 0.046 mmol) and acetic acid (3.3 mg, 0.056 mmol) were dissolved in 2 mL of a mixed solvent of dichloromethane/methanol (2/1). Under nitrogen, the mixture was stirred at room temperature for 1 hour. Sodium cyanoborohydride (4.4 mg, 0.069 mmol) was added and the mixture was stirred for another 2 hours. The solvent was removed under reduced pressure below 40°C, and the crude product was purified by HPLC to give compound **D584** (18 mg, yield 37.5%), LCMS: $[M + H]^+$ = 1043.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4,4,4-trifluorobut-2-enamide (D596)**

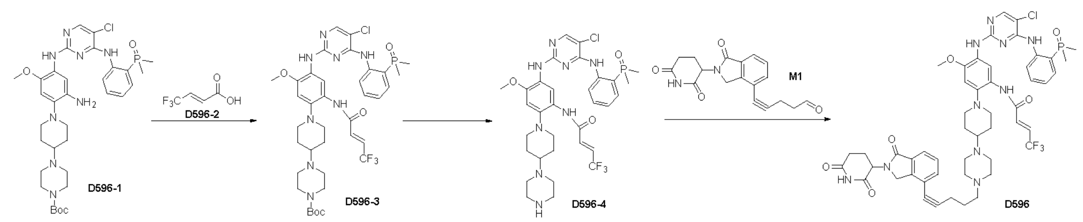

Step 1

**D596-1** (80 mg, 0.117 mmol), **D596-2** (18 mg, 0.129 mmol), T3P (149 mg, 0.468 mmol) and TEA (47 mg, 0.468 mmol) were dissolved in 2 mL of dichloromethane. Under nitrogen protection, the mixture was stirred at room temperature for 4 hours. The residue was purified by thin layer chromatography to obtain **D596-3** (62 mg, yield 66.0%), LCMS: $[M + H]^+$ = 807.

Step 2

**D596-3** (62 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D596-4** (54 mg, yield 100%), LCMS: $[M + H]^+$ = 707.

Step 3

**D596-4** (56.7 mg, 0.080 mmol), **M1** (28.6 mg, 0.088 mmol) and acetic acid (5.8 mg, 0.096 mmol) were dissolved in 2 mL of a mixed solvent of dichloromethane/methanol (2/1). Under nitrogen, the mixture was stirred at room temperature for 1 hour. Sodium cyanoborohydride (7.6 mg, 0.120 mmol) was added and the mixture was stirred for another 2 hours. The solvent was removed under reduced pressure below 40°C, and the crude product was purified by HPLC to give **D596** (15 mg, yield 18.5%), LCMS: $[M + H]^+$ = 1015.

**Preparation of 2-chloro-N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)acrylamide (D597)**

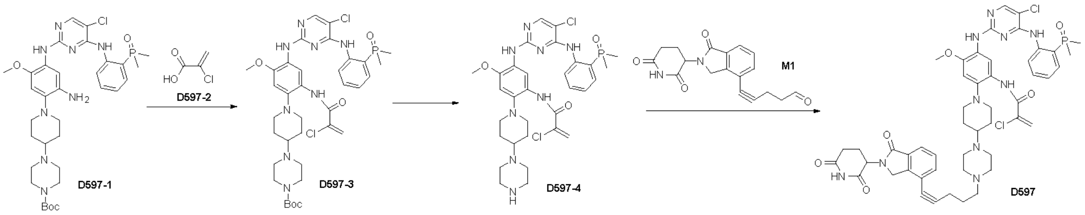


Step 1

**D597-1** (80 mg, 0.117 mmol), **D597-2** (13.6 mg, 0.129 mmol), T3P (149 mg, 0.468 mmol) and TEA (47 mg, 0.468 mmol) were dissolved in 2 mL of dichloromethane. Under nitrogen protection, the mixture was stirred at room temperature for 4 hours. The residue was purified by thin layer chromatography to obtain **D597-3** (40 mg, yield 44.4%), LCMS: $[M + H]^+$ = 773.

Step 2

**D597-3** (40 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D597-4** (29 mg, yield 85.3%), LCMS: $[M + H]^+$ = 673.

Step 3

**D597-4** (29 mg, 0.043 mmol), **M1** (15.4 mg, 0.047 mmol) and acetic acid (3.1 mg, 0.052 mmol) were dissolved in 2 mL of a mixed solvent of dichloromethane/methanol (2/1). Under nitrogen, the mixture was stirred at room temperature for 1 hour. Sodium cyanoborohydride (4.1 mg, 0.065 mmol) was added and the mixture was stirred for another 2 hours. The solvent was removed under reduced pressure below 40°C, and the crude product was purified by HPLC to obtain **D597** (13 mg, yield 31.0%), LCMS: $[M + H]^+$ = 981.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-5-yl)hex-5-yn-1-yl)piperazin-1-yl)piperidin-1-yl)-4-methoxyphenyl)-2-fluoroacrylamide (D600)**

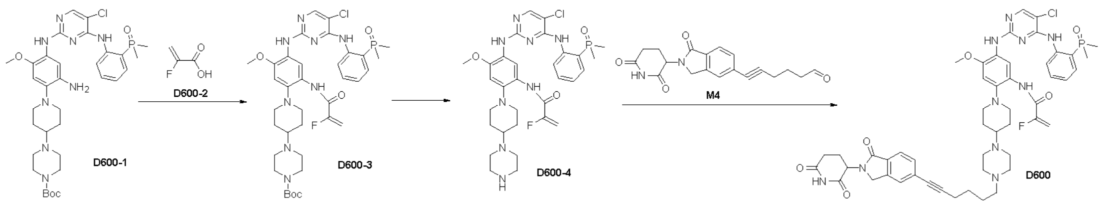


Step 1

**D600-1** (100 mg, 0.146 mmol), **D600-2** (15.8 mg, 0.175 mmol), T3P (372 mg, 0.585 mmol) and TEA (59 mg, 0.585 mmol) were dissolved in 2 mL of dichloromethane. Under nitrogen protection, the mixture was stirred at room temperature for 4 hours. The residue was purified by thin layer chromatography to obtain **D600-3** (91 mg, yield 82.4%), LCMS: $[M + H]^+$ = 757.

Step 2

**D600-3** (91 mg) was dissolved in 4 mL of dichloromethane, and trifluoroacetic acid (1 mL) was added under stirring. Under nitrogen protection, the mixture was stirred at room temperature for 1.5 hours. The solvent was removed under reduced pressure and the residue was purified by Flash to obtain **D600-4** (66 mg, yield 83.5%), LCMS: $[M + H]^+$ = 657.

Step 3

**D600-4** (66 mg, 0.101 mmol), **M4** (37 mg, 0.111 mmol) and acetic acid (7.2 mg, 0.121 mmol) were dissolved in 2 mL of a mixed solvent of dichloromethane/methanol (2/1). Under nitrogen, the mixture was stirred at room temperature for 1 hour. Sodium cyanoborohydride (9.5 mg, 0.151 mmol) was added and the mixture was stirred for another 2 hours. The solvent was removed under reduced pressure below 40°C, and the crude product was purified by HPLC to obtain **D600** (10 mg, yield 10.2%), LCMS: $[M + H]^+$ = 979.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-5-yl)hex-5-yn-1-yl)piperazin-1-yl)piperidin-1-yl)-4-methoxyphenyl)acrylamide (D601)**

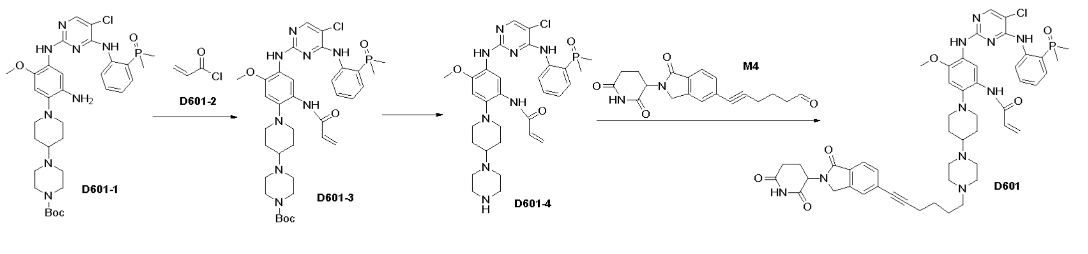


Step 1

**D601-1** (100 mg, 0.146 mmol) and TEA (44 mg, 0.438 mmol) were dissolved in 20 mL of dichloromethane. Under nitrogen protection, a solution of **D601-2** (14.5 mg, 0.161 mmol) in 1 mL of dichloromethane was added dropwise at -12 °C. The temperature was maintained and the mixture was stirred for 1.5 hours. Methanol was added to quench the reaction. The solvent was evaporated and the crude product was purified by thin layer chromatography to obtain **D601-3** (69 mg, yield 63.9%). LCMS: $[M + H]^+$ = 739.

Step 2

**D601-3** (69 mg) was dissolved in 4 mL of dichloromethane. Trifluoroacetic acid (1 mL) was added with stirring, and the mixture was stirred at room temperature for 1.5 hours under nitrogen protection. The solvent was removed under reduced pressure and the residue was purified by Flash to give **D601-4** (51 mg, yield 60.0%), LCMS: $[M + H]^+ = 639$.

Step 3

**D601-4** (51 mg, 0.080 mmol), **M4** (30 mg, 0.088 mmol) and acetic acid (5.8 mg, 0.096 mmol) were dissolved in 3 mL of a mixed solvent of dichloromethane/methanol (2/1). Under nitrogen, the mixture was stirred at room temperature for 1 hour. Sodium cyanoborohydride (7.5 mg, 0.120 mmol) was added and the mixture was stirred for another 2 hours. The solvent was removed under reduced pressure below 40°C, and the crude product was purified by HPLC to give compound **D601** (25 mg, yield 32.5%), LCMS: $[M + H]^+ = 961$.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-5-yl)hex-5-yn-1-yl)piperazin-1-yl)piperidin-1-yl)-4-methoxyphenyl)but-2-ynamide (D602)**

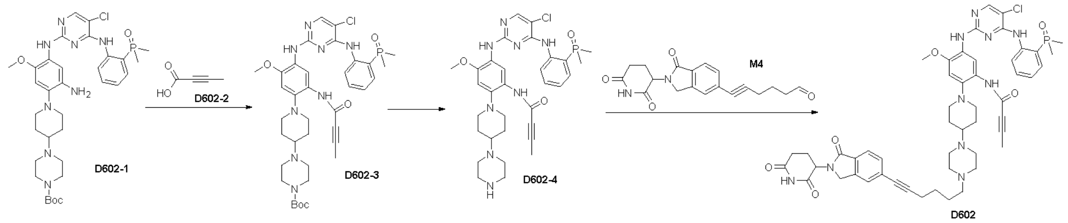


Step 1

**D602-1** (100 mg, 0.146 mmol), **D602-2** (14.7 mg, 0.175 mmol), T3P (50% EA, 372 mg, 0.585 mmol) and TEA (59 mg, 0.585 mmol) were dissolved in 2 mL of dichloromethane. Under nitrogen protection, the mixture was stirred at room temperature for 4 hours. The crude product was purified by thin layer chromatography to give **D602-3** (75 mg, yield 68.2%), **LCMS:** $[M + H]^+ = 751$.

Step 2

**D602-3** (75 mg) was dissolved in 4 mL of dichloromethane, and trifluoroacetic acid (1 mL) was added with stirring. The mixture was stirred at room temperature for 1.5 hours under nitrogen protection. The solvent was removed under reduced pressure and the residue was purified by Flash to obtain **D602-4** (55 mg, yield 84.6%), LCMS: $[M + H]^+ = 651$.

Step 3

**D602-4** (55 mg, 0.085 mmol), **M4** (32 mg, 0.093 mmol) and acetic acid (6.1 mg, 0.101 mmol) were dissolved in 3 mL of a mixed solvent of dichloromethane/methanol (2/1). Under nitrogen, the mixture was stirred at room temperature for 1 hour. Sodium cyanoborohydride (8.0 mg, 0.127 mmol) was added and the mixture was stirred for another 2 hours. The solvent was removed under reduced pressure below 40°C, and the crude product was purified by HPLC to give compound **D602** (20 mg, yield 24.4%), LCMS: $[M + H]^+ = 973$.

**Preparation** of **(*E*)-N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4,4,4-trifluorobut-2-enamide (D603)**

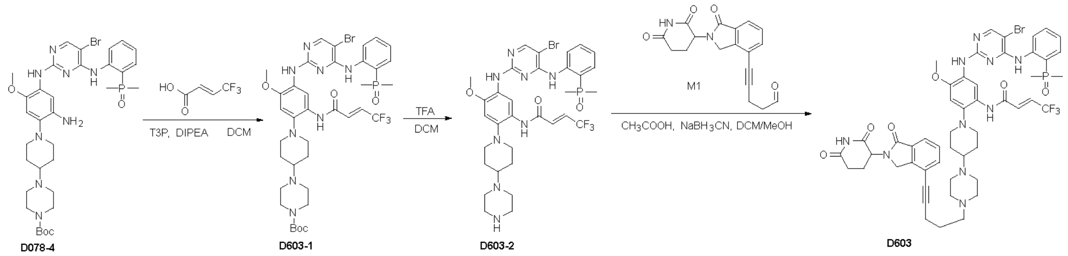


Step 1

**D078-4** (145.6 mg, 0.2 mmol), (E)-4,4,4-trifluoro-2-butenoic acid (42 mg, 0.3 mmol) and T3P (254 mg, 0.4 mmol) were dispersed in 5.0 mL of anhydrous dichloromethane. Under $N_2$ protection, diisopropylethylamine (129 mg, 1.0 mmol) was added to the reaction solution and reacted at room temperature for 2.0 h. The reaction was completed as detected by LCMS. The reaction solution was diluted with 30 mL of dichloromethane. Silica gel was directly added to the sample. The mixture was purified by Flash to give a yellow solid product **D603-1** (128 mg, yield 75.0%). LCMS: $[M + H]^+$ = 851.

Step 2

**D603-1** (128 mg, 0.15 mmol) was dissolved in 10 mL of anhydrous dichloromethane. Under $N_2$ protection, 2.5 mL of trifluoroacetic acid was added to the reaction solution and the mixture was reacted at room temperature for 1.0 h. The reaction was completed as detected by LCMS. The reaction solution was concentrated under reduced pressure. The residue was redissolved in 30 mL of a mixed solvent (MeOH:DCM = 1:20), then washed with saturated $NaHCO_3$ solution (2 x 20 mL) and saturated brine (1 x 20 mL) in sequence. The organic layer was dried over anhydrous magnesium sulfate, and filtered. The filtrate was concentrated under reduced pressure to remove the solvent to give a crude product, which was purified by Flash to give a yellow solid product **D603-2** (95 mg, yield 84.4%). LCMS: $[M + H]^+$ = 751.

Step 3

**D603-2** (95 mg, 0.127 mmol) and **M1** (53.4 mg, 0.165 mmol) were dissolved in a mixed solvent of 10.0 mL of anhydrous dichloromethane and 1.0 mL of anhydrous methanol. Under nitrogen protection, $CH_3COOH$ (11.4 mg, 0.191 mmol) was added and the mixture was stirred at room temperature for 0.5 h. Solid $NaBH_3CN$ (16.0 mg, 0.254 mmol) was then added to the reaction solution and the mixture was reacted at room temperature for another 2 h. LCMS and TLC showed that the reaction was completed. Silica gel was directly added to the sample. The mixture was purified by Flash to give a white solid pure product **D603** (35 mg, yield 26.1%). LCMS: $[M + H]^+$ = 1059, 1061.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)but-2-ynamide (D604)**

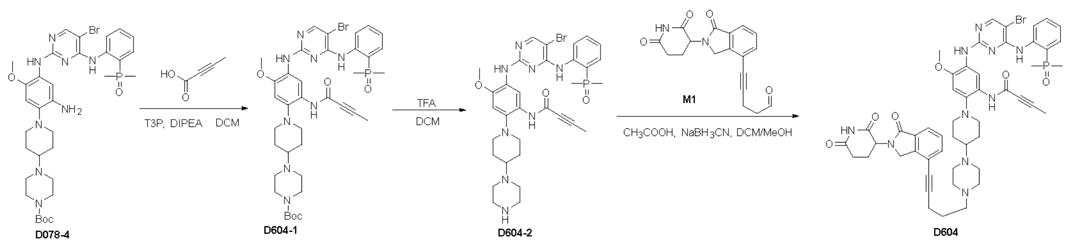


Step 1

**D078-4** (3.2 g, 4.4 mmol), but-2-ynoic acid (0.37 g, 4.4 mmol) and T3P (4.47 g, 8.8 mmol) were dispersed in 32 mL of anhydrous dichloromethane. Under $N_2$ protection, diisopropylethylamine (2.84 g, 22.0 mmol) was added to the reaction solution. The mixture was reacted at room temperature for 2.0 h. The reaction was completed as detected by LCMS. The reaction solution was diluted with 50 mL of dichloromethane and silica gel was directly added to the sample. The mixture

was purified by Flash to give a yellow solid product **D604-1** (3.1 g). LCMS: $[M + H]^+ = 795$.

Step 2

**D604-1** (3.1 g, 3.9 mmol) was dissolved in 60 mL of anhydrous dichloromethane. Under $N_2$ protection, 15 mL of trifluoroacetic acid was added to the reaction solution and the mixture was reacted at room temperature for 1.0 h. The reaction was completed as detected by LCMS. The reaction solution was concentrated under reduced pressure. The residue was redissolved in a mixed solvent of methanol and dichloromethane (100 mL), then washed with saturated $NaHCO_3$ solution (2 x 60 mL) and saturated brine (1 x 60 mL) in sequence. The organic layer was dried over anhydrous magnesium sulfate, and filtered. The filtrate was concentrated under reduced pressure to remove the solvent to give a crude product, which was purified by Flash to give a yellow solid product **D604-2** (2.1 g). LCMS: $[M + H]^+ = 701$.

Step 3

**D604-2** (2.1 g, 3.03 mmol) and **M1** (0.94 g, 3.33 mmol) were dissolved in a mixed solvent of 160.0 mL of anhydrous dichloromethane and 16.0 mL of anhydrous methanol. Under nitrogen protection, $CH_3COOH$ (238 mg, 4.55 mmol) was added and the mixture was stirred at room temperature for 0.5 h. Solid $NaBH_3CN$ (332 mg, 6.06 mmol) was then added to the reaction solution and the mixture was reacted at room temperature for another 2 h. LCMS and TLC showed that the reaction was completed. Silica gel was directly added to the sample. The mixture was purified by Flash to give a white solid pure product **D604** (1.6 g, yield 52.8%). LCMS: $[M + H]^+ = 1003, 1005$.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-4-yl)hex-5-yn-1-yl)piperazin-1-yl)piperidin-1-yl)-4-methoxyphenyl)-4,4,4-trifluorobut-2-enamide (D620)**

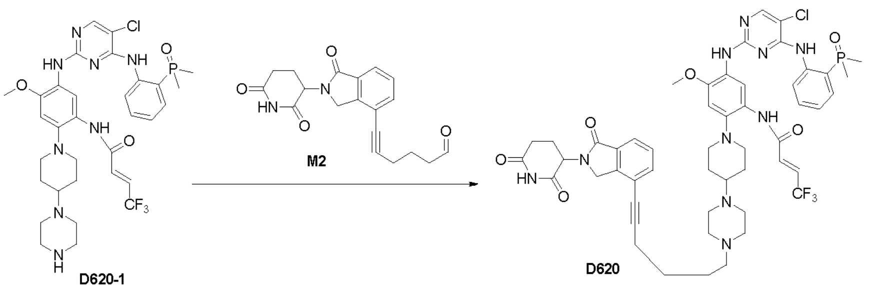

**D620-1** (50 mg, 0.071 mmol), **M2** (29 mg, 0.085 mmol) and acetic acid (5.1 mg, 0.085 mmol) were dissolved in 3 mL of a mixed solvent of dichloromethane/methanol (2/1). Under nitrogen, the mixture was stirred at room temperature for 1 hour. Sodium cyanoborohydride (6.7 mg, 0.106 mmol) was added and the mixture was stirred for another 2 hours. The solvent was removed under reduced pressure below 40°C, and the crude product was purified by HPLC to give compound **D620** (24 mg, yield 32.9%), LCMS: $[M + H]^+ = 1029$.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-5-yl)pent-4-yn-1-yl)piperazin-1-yl)piperidin-1-yl)-4-methoxyphenyl)-4,4,4-trifluorobut-2-enamide (D622)**

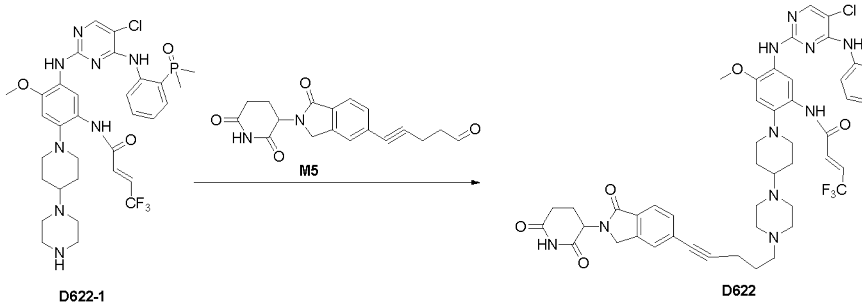


**D622-1** (22 mg, 0.031 mmol), **M5** (12 mg, 0.037 mmol) and acetic acid (2.2 mg, 0.037 mmol) were dissolved in 3 mL of a mixed solvent of dichloromethane/methanol (2/1). Under nitrogen, the mixture was stirred at room temperature for 1 hour. Sodium cyanoborohydride (2.9 mg, 0.047 mmol) was added and the mixture was stirred for another 2 hours. The solvent was removed under reduced pressure below 40°C, and the crude product was purified by HPLC to give compound **D622** (12 mg, yield 37.5%), LCMS: $[M + H]^+ = 1015$.

**Preparation of N-(5-((5-chloro-4-((2-(dimethylphosphoryl)phenyl)amino)pyrimidin-2-yl)amino)-2-(4-(4-(6-(2-(2,6-dioxopiperidin-3-yl)-1-oxoisoindolin-5-yl)hex-5-yn-1-yl)piperazin-1-yl)piperidin-1-yl)-4-methoxyphenyl)-4,4,4-trifluorobut-2-enamide (D623)**

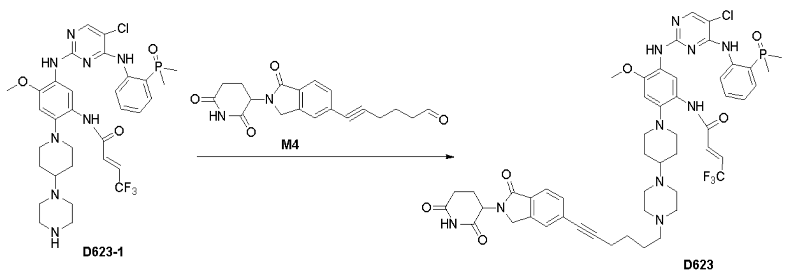


**D623-1** (50 mg, 0.071 mmol), **M4** (29 mg, 0.085 mmol) and acetic acid (5.1 mg, 0.037 mmol) were dissolved in 3 mL of a mixed solvent of dichloromethane/methanol (2/1). Under nitrogen, the mixture was stirred at room temperature for 1 hour. Sodium cyanoborohydride (6.7 mg, 0.106 mmol) was added and the mixture was stirred for another 2 hours. The solvent was removed under reduced pressure below 40°C, and the crude product was purified by HPLC to give compound **D623** (20 mg, yield 27.5%), LCMS: $[M + H]^+ = 1029$.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4-hydroxybut-2-ynamide (D628)**

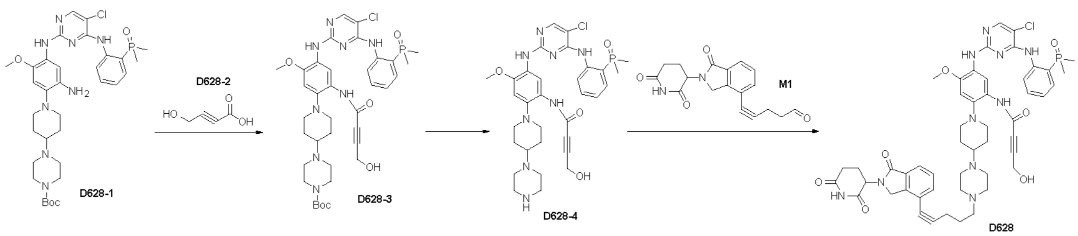


Step 1

**D628-1** (200 mg, 0.292 mmol), **D628-2** (35.1 mg, 0.351 mmol), pyridine (92 mg, 1.170 mmol) and EDCI (224 mg, 1.170 mmol) were dissolved in 3 mL of DMF. Under nitrogen protection, the mixutre was stirred at room temperature overnight. The solvent was removed under reduced pressure and the residue was purified by Flash to give **D628-3** (100

mg, yield 44.6%), LCMS: [M + H]+ = 767.

Step 2

**D628-3** (100 mg) was dissolved in 8 mL of dichloromethane. Trifluoroacetic acid (2 mL) was added with stirring. Under nitrogen protection, the mixture was stirred at room temperature for 1.5 hours. The solvent was removed under reduced pressure and the residue was purified by Flash to obtain **D628-4** (50 mg, yield 57.5%), LCMS: [M + H]+ = 667.

Step 3

**D628-4** (50 mg, 0.075 mmol), **M1** (27 mg, 0.083 mmol) and acetic acid (5.4 mg, 0.090 mmol) were dissolved in 2 mL of a mixed solvent of dichloromethane/methanol (2/1). Under nitrogen, the mixture was stirred at room temperature for 1 hour. Sodium cyanoborohydride (7.1 mg, 0.113 mmol) was added and the mixture was stirred for another 2 hours. The solvent was removed under reduced pressure below 40°C, and the crude product was purified by HPLC to give compound **D628** (11 mg, yield 15.1%), LCMS: [M + H]+ = 975.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)propynamide (D630)**

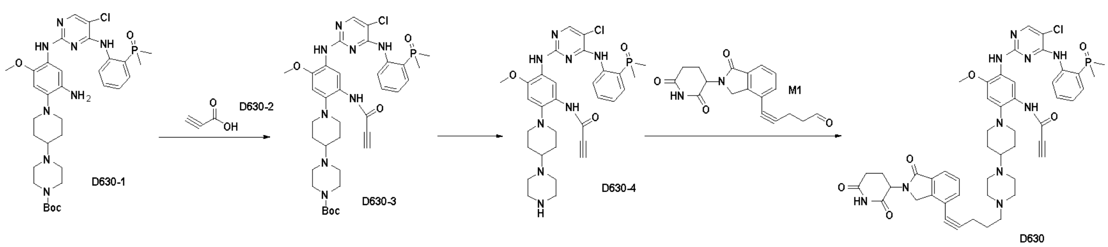


Step 1

**D630-1** (80 mg, 0.117 mmol), **D630-2** (9.8 mg, 0.140 mmol), T3P (296 mg, 0.468 mmol) and TEA (47 mg, 0.468 mmol) were dissolved in 2 mL of dichloromethane. Under nitrogen protection, the mixutre was stirred at room temperature overnight. The mixture was purified by Flash to give **D630-3** (57 mg, yield 66.3%), LCMS: [M + H]+ = 737.

Step 2

**D630-3** (57 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D630-4** (47 mg, yield 95.9%), LCMS: [M + H]+ = 637.

Step 3

**D630-4** (47 mg, 0.067 mmol), **M1** (24 mg, 0.073 mmol) and acetic acid (4.8 mg, 0.080 mmol) were dissolved in 3 mL of a mixed solvent of dichloromethane/methanol (2/1). Under nitrogen, the mixture was stirred at room temperature for 1 hour. Sodium cyanoborohydride (6.3 mg, 0.100 mmol) was added and the mixture was stirred for another 2 hours. The solvent was removed under reduced pressure below 40°C, and the crude product was purified by HPLC to give compound **D630** (2.14 mg, yield 3.1%), LCMS: [M + H]+ = 945.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)hex-2-ynamide (D631)**

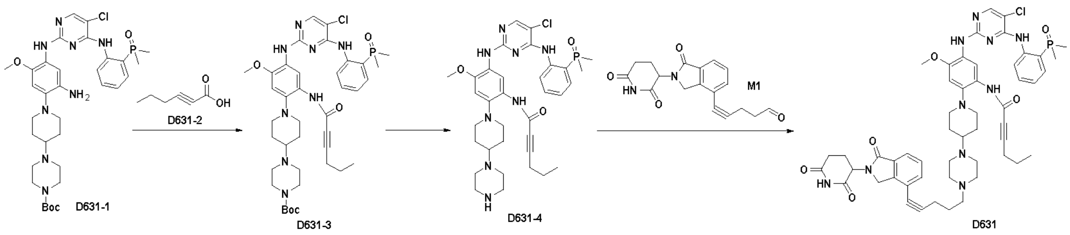


Step 1

**D631-1** (100 mg, 0.146 mmol), **D631-2** (20 mg, 0.175 mmol), T3P (372 mg, 0.585 mmol) and TEA (59 mg, 0.585 mmol) were dissolved in 2 mL of dichloromethane. Under nitrogen protection, the mixutre was stirred at room temperature overnight. The mixture was purified by Flash to give D631-3 (70 mg, yield 61.4%), LCMS: $[M + H]^+$ = 779.

Step 2

**D631-3** (70 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D631-4** (56 mg, yield 91.8%), LCMS: $[M + H]^+$ = 679.

Step 3

**D631-4** (56 mg, 0.083 mmol), **M1** (29 mg, 0.091 mmol) and acetic acid (5.9 mg, 0.099 mmol) were dissolved in 2 mL of a mixed solvent of dichloromethane/methanol (2/1). Under nitrogen, the mixture was stirred at room temperature for 1 hour. Sodium cyanoborohydride (7.8 mg, 0.124 mmol) was added and the mixture was stirred for another 2 hours. The solvent was removed under reduced pressure below 40°C, and the crude product was purified by HPLC to obtain **D631** (26 mg, yield 32.1%), LCMS: $[M + H]^+$ = 987.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)pent-2-enamide (D632)**

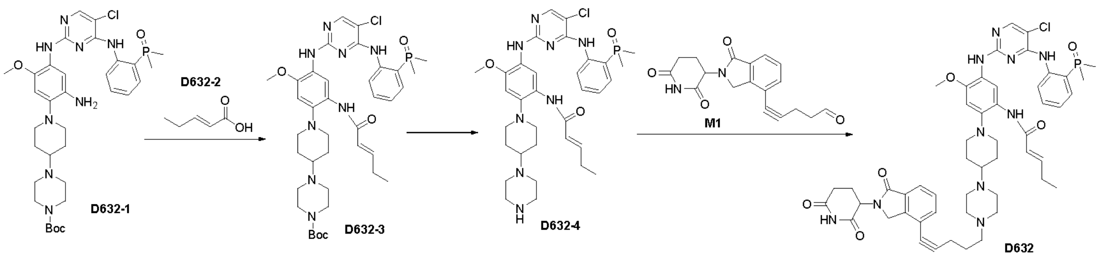


Step 1

**D632-1** (100 mg, 0.146 mmol), **D632-2** (17.5 mg, 0.175 mmol), T3P (372 mg, 0.585 mmol) and TEA (59 mg, 0.585 mmol) were dissolved in 2 mL of dichloromethane. Under nitrogen protection, the mixutre was stirred at room temperature overnight. The mixture was purified by Flash to give **D632-3** (77 mg, yield 68.8%), Mass: 767.

Step 2

**D632-3** (77 mg) was dissolved in 4 mL of dichloromethane, and trifluoroacetic acid (1 mL) was added with stirring. Under nitrogen protection, the mixture was stirred at room temperature for 1.5 hours. The solvent was removed under reduced pressure and the residue was purified by Flash to obtain **D632-4** (62 mg, yield 92.5%), LCMS: $[M + H]^+$ = 667.

Step 3

**D632-4** (62 mg, 0.093 mmol), **M1** (33 mg, 0.102 mmol) and acetic acid (6.7 mg, 0.112 mmol) were dissolved in 2 mL of a mixed solvent of dichloromethane/methanol (2/1). Under nitrogen, the mixture was stirred at room temperature for 1 hour. Sodium cyanoborohydride (8.8 mg, 0.140 mmol) was added and the mixture was stirred for another 2 hours. The solvent was removed under reduced pressure below 40°C, and the crude product was purified by HPLC to obtain **D632** (28 mg, yield 30.8%), LCMS: [M + H]$^+$ = 975.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-3-methylbut-2-enamide (D633)**

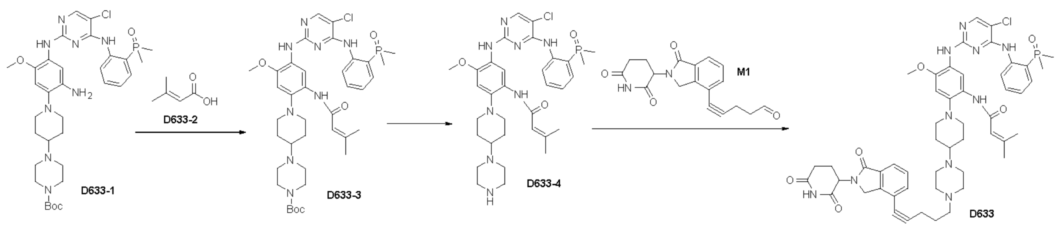


Step 1

**D633-1** (100 mg, 0.146 mmol), **D633-2** (17.5 mg, 0.175 mmol), T3P (372 mg, 0.585 mmol) and TEA (59 mg, 0.585 mmol) were dissolved in 2 mL of dichloromethane. Under nitrogen protection, the mixutre was stirred at room temperature overnight. The mixture was purified by Flash to give **D633-3** (75 mg, yield 67.0%), LCMS: [M + H]$^+$ = 767.

Step 2

**D633-3** (75 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D633-4** (50 mg, yield 76.9%), LCMS: [M + H]$^+$ = 667.

Step 3

**D633-4** (50 mg, 0.075 mmol), **M1** (27 mg, 0.083 mmol) and acetic acid (5.4 mg, 0.090 mmol) were dissolved in 2 mL of a mixed solvent of dichloromethane/methanol (2/1). Under nitrogen, the mixture was stirred at room temperature for 1 hour. Sodium cyanoborohydride (7.1 mg, 0.113 mmol) was added and the mixture was stirred for another 2 hours. The solvent was removed under reduced pressure below 40°C, and the crude product was purified by HPLC to obtain **D633** (6 mg, yield 8.2%), LCMS: [M + H]$^+$ = 975.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)phenyl)but-2-ynamide (D665)**

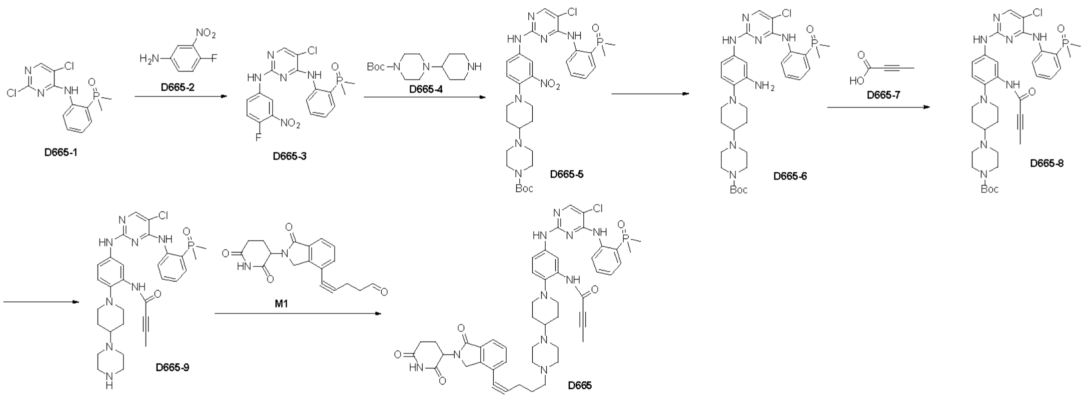


Step 1

**D665-1** (200 mg, 0.635 mmol), **D665-2** (99 mg, 0.635 mmol), and methanesulfonic acid (244 mg, 2.540 mmol) were dissolved in 5 mL of tert-butanol. Under nitrogen protection, the mixture was stirred at 90 °C overnight. The solvent was removed under reduced pressure and the residue was purified by Flash to obtain **D665-3** (248 mg, yield 89.9%), LCMS: $[M + H]^+$ = 436.

Step 2

**D665-3** (496 mg, 1.14 mmol), **D665-4** (368 mg) and cesium carbonate (1.115 g, 3.42 mmol) were added to 10 mL of DMF. The mixture was stirred at 70 °C overnight under nitrogen protection. The mixture was cooled to room temperature and the solvent was removed under reduced pressure. The residue was purified by Flash to give **D665-5** (613 mg, yield 78.6%), LCMS: $[M + H]^+$ = 685.

Step 3

**D665-5** (613 mg, 0.896 mmol), iron powder (500 mg, 8.958 mmol) and ammonium chloride (288 mg, 5.375 mmol) were added to 8 mL of a mixed solvent of ethanol/water (3/1). The mixture was stirred at 100°C for 1 hour. The reaction was cooled to room temperature. The solid was filtered out. The solid residue was washed twice with a mixed solvent of ethanol and water. The solvent was evaporated under reduced pressure, and the crude product was purified by Flash to obtain **D665-6** (425 mg). LCMS: $[M + H]^+$ = 655.

Step 4

**D665-6** (80 mg, 0.122 mmol), **D665-7** (12 mg, 0.147 mmol), T3P (50% EA, 311 mg, 0.489 mmol) and TEA (49 mg, 0.489 mmol) were dissolved in 2 mL of dichloromethane. Under nitrogen protection, the mixutre was stirred at room temperature overnight. The mixture was purified by Flash to give **D665-8** (64 mg, yield 76.0%), LCMS: $[M + H]^+$ = 721.

Step 5

**D665-8** (64 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D665-9** (41 mg, yield 74.5%), LCMS: $[M + H]^+$ = 621.

Step 6

**D665-9** (41 mg, 0.066 mmol), **M1** (26 mg, 0.079 mmol) and acetic acid (4.8 mg, 0.080 mmol) were dissolved in 3 mL of a mixed solvent of dichloromethane/methanol (2/1). Under nitrogen, the mixture was stirred at room temperature for 1 hour. Sodium cyanoborohydride (6.2 mg, 0.099 mmol) was added and the mixture was stirred for another 2 hours. The solvent was removed under reduced pressure below 40°C, and the crude product was purified by HPLC to give compound **D665** (20 mg, yield 32.8%), LCMS: $[M + H]^+$ = 929.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)phenyl)-2-fluoroacrylamide (D666)**

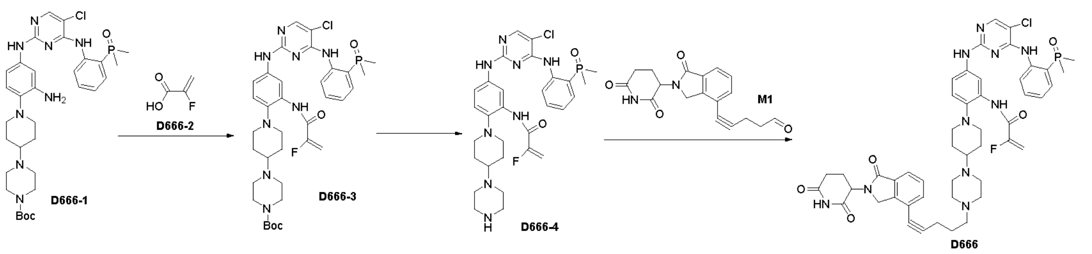


Step 1

**D666-1** (80 mg, 0.122 mmol), **D666-2** (13 mg, 0.147 mmol), T3P (311 mg, 0.489 mmol) and TEA (49 mg, 0.489 mmol) were dissolved in 2 mL of dichloromethane. Under nitrogen protection, the mixutre was stirred at room temperature overnight. The mixture was purified by Flash to give **D666-3** (56 mg, yield 63.1%), LCMS: $[M + H]^+ = 727$.

Step 2

**D666-3** (56 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D666-4** (48 mg, yield 100%), LCMS: $[M + H]^+ = 627$.

Step 3

**D666-4** (55 mg, 0.088 mmol), **M1** (34 mg, 0.105 mmol) and acetic acid (6.3 mg, 0.105 mmol) were dissolved in 3 mL of a mixed solvent of dichloromethane/methanol (2/1). Under nitrogen, the mixture was stirred at room temperature for 1 hour. Sodium cyanoborohydride (8.3 mg, 0.132 mmol) was added and the mixture was stirred for another 2 hours. The solvent was removed under reduced pressure below 40°C, and the crude product was purified by HPLC to obtain **D666** (35.6 mg, yield 43.4%), LCMS: $[M + H]^+ = 935$.

**Preparation of (*E*)-N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)phenyl)-4,4,4-trifluorobut-2-enamide (D667)**

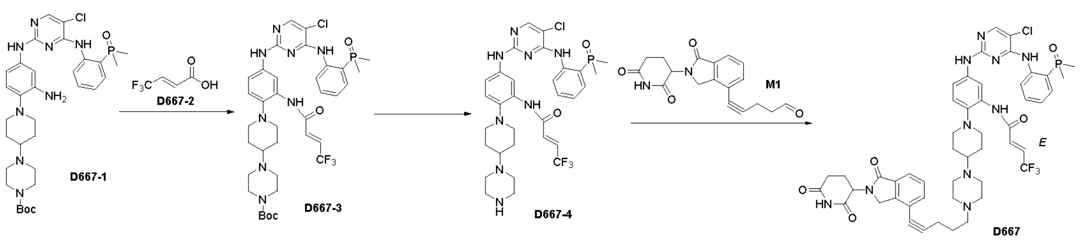


Step 1

**D667-1** (80 mg, 0.122 mmol), **D667-2** (20 mg, 0.147 mmol), T3P (311 mg, 0.489 mmol) and TEA (49 mg, 0.489 mmol) were dissolved in 2 mL of dichloromethane. Under nitrogen protection, the mixutre was stirred at room temperature overnight. The mixture was purified by Flash to give **D667-3** (47 mg, yield 49.5%), LCMS: $[M + H]^+ = 777$.

Step 2

**D667-3** (47 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D667-4** (40 mg, yield 97.8%), LCMS: $[M + H]^+ = 677$.

Step 3

**D667-4** (46 mg, 0.068 mmol), **M1** (26 mg, 0.082 mmol) and acetic acid (4.9 mg, 0.082 mmol) were dissolved in 3 mL of a mixed solvent of dichloromethane/methanol (2/1). Under nitrogen, the mixture was stirred at room temperature for 1 hour. Sodium cyanoborohydride (6.4 mg, 0.102 mmol) was added and the mixture was stirred for another 2 hours. The solvent was removed under reduced pressure below 40°C, and the crude product was purified by HPLC to give compound **D667** (30 mg, yield 44.8%), LCMS: $[M + H]^+ = 985$.

**Preparation of N-(5-((5-chloro-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)phenyl)hex-2-ynamide (D668)**

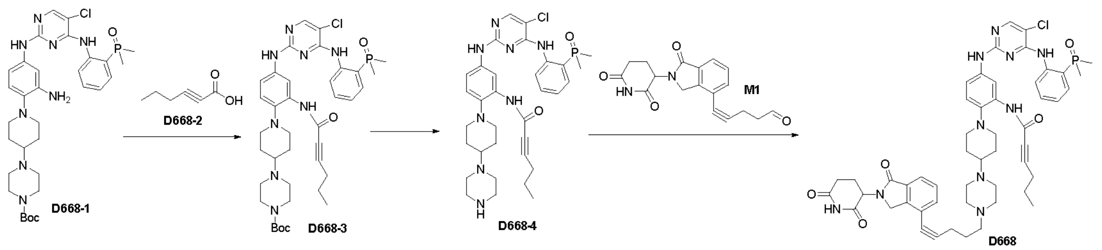


Step 1

**D668-1** (80 mg, 0.122 mmol), **D668-2** (16 mg, 0.147 mmol), T3P (311 mg, 0.489 mmol) and TEA (49 mg, 0.489 mmol) were dissolved in 2 mL of dichloromethane. Under nitrogen protection, the mixutre was stirred at room temperature overnight. The mixture was purified by Flash to give **D668-3** (36 mg, yield 39.6%), LCMS: $[M + H]^+ = 749$.

Step 2

**D668-3** (36 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D668-4** (31 mg, yield 100%), LCMS: $[M + H]^+ = 649$.

Step 3

**D668-4** (44 mg, 0.068 mmol), **M1** (26 mg, 0.082 mmol) and acetic acid (4.9 mg, 0.082 mmol) were dissolved in 3 mL of a mixed solvent of dichloromethane/methanol (2/1). Under nitrogen, the mixture was stirred at room temperature for 1 hour. Sodium cyanoborohydride (6.4 mg, 0.102 mmol) was added and the mixture was stirred for another 2 hours. The solvent was removed under reduced pressure below 40°C, and the crude product was purified by HPLC to obtain **D668** (24 mg, yield 37.0%), LCMS: $[M + H]^+ = 957$.

**Preparation of N-(5-((5-chloro-4-((5-(dimethylphosphoryl)quinoxalin-6-yl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)acrylamide (D670)**

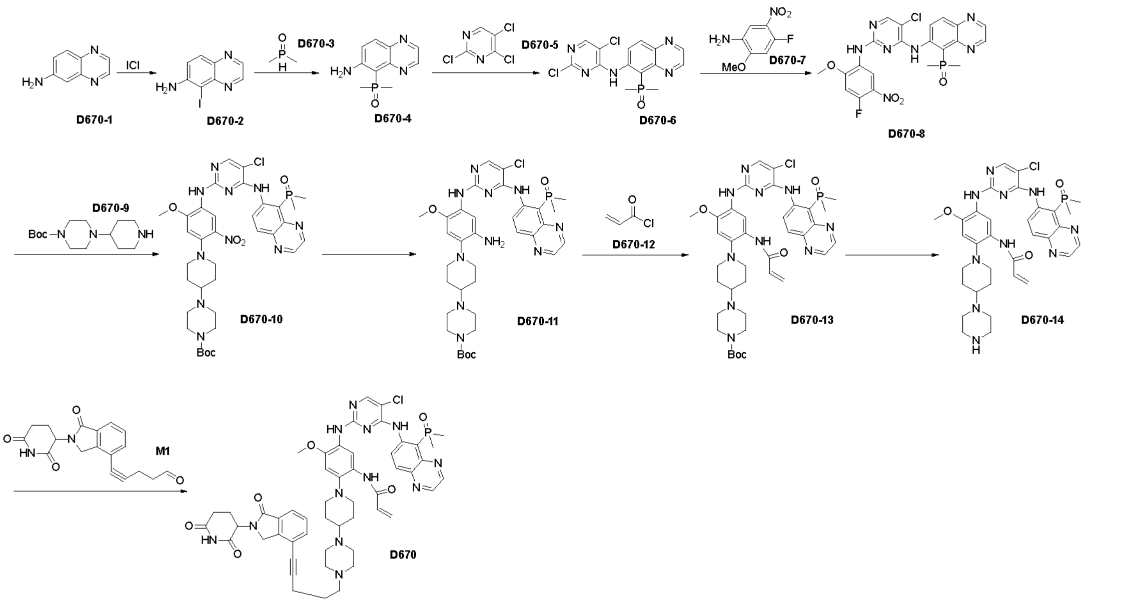


Step 1

**D670-1** (6.87 g, 47.36 mmol) was dissolved in 210 mL of glacial acetic acid. To the mixture was added a solution of iodine monochloride (8.43 g, 60.66 mmol) in glacial acetic acid (75 mL) dropwise under stirring at room temperature. After the addition was completed, the mixture was stirred for another 1 hour. The solvent was removed by concentration under reduced pressure, and the mixture was added to 300 mL of ethyl acetate. The pH was adjusted to about 9 with saturated aqueous sodium bicarbonate solution. The organic phase was washed twice with saturated aqueous sodium thiosulfate solution, dried over anhydrous sodium sulfate, and filtered. The filtrate was concentrated. The crude product was purified by Flash to obtain **D670-2** (8.43 g, yield 65.6%), LCMS: $[M + H]^+$ = 272.

Step 2

**D670-2** (8.32 g, 30.71 mmol), dimethylphosphine oxide **(D670-2,** 3.59 g, 46.06 mmol), potassium phosphate (15.21 g, 70.00 mmol), palladium acetate (1.38 g, 6.14 mmol) and Xantphos (3.55 g, 6.14 mmol) were added to 80 mL of 1,4-dioxane. Under nitrogen protection, the mixture was stirred at 100 °C overnight. The reaction solution was cooled to room temperature. Water was added. The mixture was then extracted three times with dichloromethane, dried over anhydrous sodium sulfate, and filtered. The filtrate was concentrated. The crude product was purified by Flash to obtain **D670-4** (5.1 g, yield 75.1%), LCMS: $[M + H]^+$ = 222.

Step 3

**D670-4** (4.83 g, 21.85 mmol) was dissolved in 100 mL of N,N-dimethylformamide and the temperature was lowered to 0°C. Sodium hydride (60%, 1.92 g, 48.08 mmol) was added in batches with stirring. Under nitrogen protection, the mixutre was stirred at this temperature for another 30 minutes. Then **D670-5** (4.77 g, 26.22 mmol) was slowly added dropwise. After the addition was completed, the reaction was warmed to room temperature and stirred overnight. Saturated aqueous ammonium chloride was added to quench the reaction. The mixture was diluted with water. The mixture was extracted with ethyl acetate three times, dried over anhydrous sodium sulfate, and filtered. The filtrate was concentrated. The crude product was purified by Flash to obtain **D670-6** (7.52 g, yield 93.8%), LCMS: $[M + H]^+$ = 368.

Step 4

**D670-6** (7.32 g, 19.94 mmol), **D670-7** (3.71 g, 19.94 mmol) and methanesulfonic acid (7.67 g, 79.78 mmol) were dissolved in 300 mL of tert-butanol. The mixture was stirred at 90 °C overnight under nitrogen. The reaction was cooled down. The solvent was removed. The residue was dissolved in dichloromethane and washed once with aqueous sodium bicarbonate solution. The organic phase was dried over anhydrous sodium sulfate, and filtered. The filtrate was concentrated. The crude product was purified by Flash to give **D670-8** (8.0 g, yield 77.7%), LCMS: $[M + H]^+$ = 518.

Step 5

**D670-8** (4.0 g, 0.097 mmol), **D670-9** (2.5 g, 9.28 mmol) and cesium carbonate (7.57 g, 23.21 mmol) were added to 60 mL of N,N-dimethylformamide. The mixture was stirred at 70 °C overnight under nitrogen. The solvent was removed under reduced pressure and the residue was purified by Flash to give **D670-10** (3.9 g, yield 66.1%), LCMS: $[M + H]^+ = 767$.

Step 6

**D670-10** (3.9 g, 5.09 mmol), iron powder (2.8 g, 50.89 mmol) and ammonium chloride (1.6 g, 30.54 mmol) were added to a mixed solvent of 120 mL ethanol/water (3/1). The mixture was stirred at 100 °C for 4.5 hours. The reaction was cooled to room temperature. The solid was filtered out. The solid residue was rinsed twice with a large amount of dichloromethane, and the filtrate was allowed to stand for stratification. The aqueous phase was washed once with dichloromethane. The organic phases were combined, washed once with saturated brine, dried over anhydrous sodium sulfate, and filtered. The solvent was evaporated under reduced pressure. The crude product was purified by Flash to obtain **D670-11** (3.2 g, yield 85.3%). LCMS: $[M + H]^+ = 737$.

Step 7

**D670-11** (150 mg, 0.204 mmol) and TEA (62 mg, 0.611 mmol) were dissolved in 20 mL of dichloromethane. A solution of **D670-12** (18 mg, 0.204 mmol) in 1 mL of dichloromethane was added dropwise at -12 °C under nitrogen protection. The temperature was maintained and the mixture was stirred for 1.5 hours. Methanol was added to quench the reaction. The solvent was evaporated and the crude product was purified by thin layer chromatography (dichloromethane/methanol = 10/1) to give **D670-13** (123 mg, yield 76.4%), LCMS: $[M + H]^+ = 791$.

Step 8

**D670-13** (123 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D670-14** (72 mg, yield 67.3%), LCMS: $[M + H]^+ = 691$.

Step 9

**D670-14** (40 mg, 0.058 mmol), **M1** (23 mg, 0.070 mmol) and acetic acid (4.2 mg, 0.070 mmol) were dissolved in 3 mL of a mixed solvent of dichloromethane/methanol (2/1). Under nitrogen, the mixture was stirred at room temperature for 1 hour. Sodium cyanoborohydride (5.5 mg, 0.087 mmol) was added and the mixture was stirred for another 2 hours. The solvent was removed under reduced pressure below 40°C, and the crude product was purified by HPLC to give compound **D670** (29 mg, yield 50.0%), LCMS: $[M + H]^+ = 999$.

**Preparation of N-(5-((5-chloro-4-((5-(dimethylphosphoryl)quinoxalin-6-yl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-fluoroacrylamide (D671)**

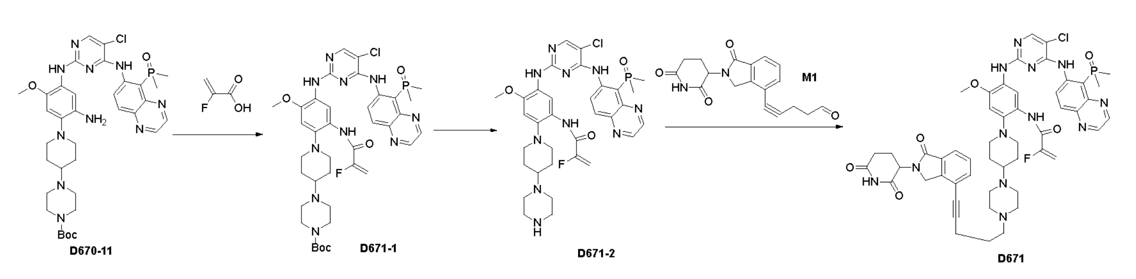

D670-11 D671-1 D671-2 D671

Step 1

**D670-11** (150 mg, 0.20 mmol), 2-fluoroacrylic acid (18.3 mg, 0.20 mmol), T3P (518 mg, 0.81 mmol) and TEA (82 mg, 0.81 mmol) were dissolved in 3 mL of dichloromethane. Under nitrogen protection, the mixture was stirred at room temperature for 4 hours. The mixture was purified by Flash to give compound **D671-1** (83 mg, yield 50.6%), LCMS: $[M + H]^+$

= 809.

Step 2

**D671-1** (83 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D671-2** (68 mg, yield 93.2%), LCMS: $[M + H]^+$ = 709.

Step 3

**D671-2** (34 mg, 0.048 mmol), **M1** (19 mg, 0.058 mmol) and acetic acid (3.4 mg, 0.058 mmol) were dissolved in 3 mL of a mixed solvent of dichloromethane/methanol (2/1). Under nitrogen, the mixture was stirred at room temperature for 1 hour. Sodium cyanoborohydride (4.5 mg, 0.072 mmol) was added and the mixture was stirred for another 2 hours. The solvent was removed under reduced pressure below 40°C, and the crude product was purified by HPLC to give compound **D671** (21 mg, yield 43.0%), LCMS: $[M + H]^+$ = 1017.

**Preparation of N-(5-((5-chloro-4-((5-(dimethylphosphoryl)quinoxalin-6-yl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)but-2-ynamide (D672)**

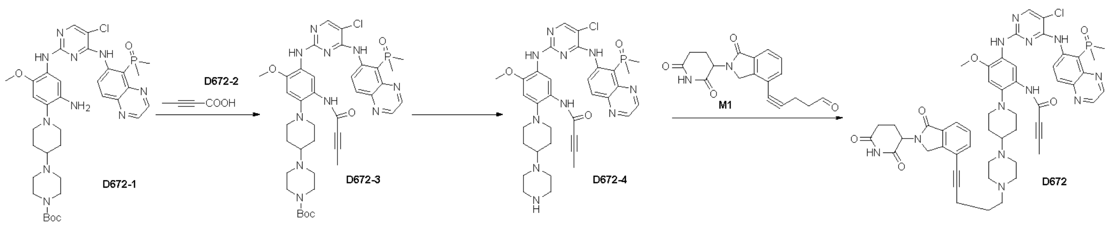


Step 1

**D672-1** (150 mg, 0.20 mmol), **D672-2** (17.1 mg, 0.20 mmol), T3P (518 mg, 0.81 mmol) and TEA (82 mg, 0.81 mmol) were dissolved in 3 mL of dichloromethane. Under nitrogen protection, the mixture was stirred at room temperature for 4 hours. The mixture was purified by Flash to give **D672-**3 (76 mg, yield 46.6%), LCMS: $[M + H]^+$ = 803.

Step 2

**D672-3** (76 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D672-4** (66 mg, yield 100%), LCMS: $[M + H]^+$ = 703.

Step 3

**D672-4** (34 mg, 0.048 mmol), **M1** (19 mg, 0.058 mmol) and glacial acetic acid (3.5 mg, 0.058 mmol) were dissolved in 3 mL of a mixed solvent of dichloromethane/methanol (2/1). Under nitrogen, the mixture was stirred at room temperature for 1 hour. Sodium cyanoborohydride (4.6 mg, 0.073 mmol) was added and the mixture was stirred for another 2 hours. The solvent was removed under reduced pressure below 40°C, and the crude product was purified by HPLC to give **D672** (4 mg, yield 8.2%), LCMS: $[M + H]^+$ = 1011.

**Preparation of (*E*)-N-(5-((5-chloro-4-((5-(dimethylphosphoryl)quinoxalin-6-yl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4,4,4-trifluorobut-2-enamide (D673)**

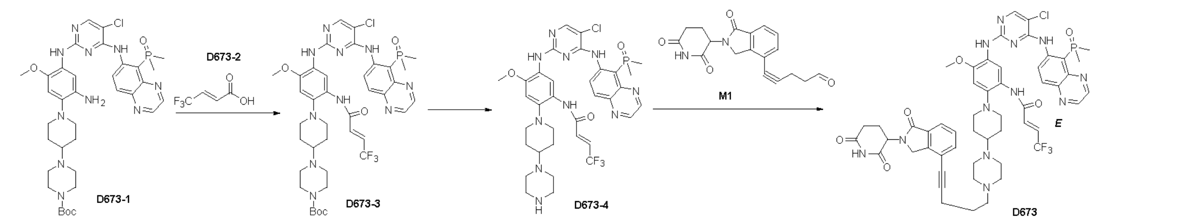


Step 1

**D673-1** (150 mg, 0.20 mmol), **D673-2** (28.5 mg, 0.20 mmol), T3P (518 mg, 0.81 mmol) and TEA (82 mg, 0.81 mmol) were dissolved in 3 mL of dichloromethane. Under nitrogen protection, the mixture was stirred at room temperature for 4 hours. The residue was purified by Flash to give **D673-**3 (116 mg, yield 66.3%), LCMS: $[M + H]^+ = 859$.

Step 2

**D673-3** (116 mg) was dissolved in 4 mL of dichloromethane. Trifluoroacetic acid (1 mL) was added with stirring. Under nitrogen protection, the mixture was stirred at room temperature for 1.5 hours. The solvent was removed under reduced pressure and the residue was purified by Flash to obtain **D673-4** (86 mg, yield 84.3%), LCMS: $[M + H]^+ = 759$.

Step 3

**D673-4** (43 mg, 0.057 mmol), **M1** (22 mg, 0.068 mmol) and glacial acetic acid (4.1 mg, 0.068 mmol) were dissolved in 3 mL of a mixed solvent of dichloromethane/methanol (2/1). Under nitrogen, the mixture was stirred at room temperature for 1 hour. Sodium cyanoborohydride (5.3 mg, 0.085 mmol) was added and the mixture was stirred for another 2 hours. The solvent was removed under reduced pressure below 40°C, and the crude product was purified by HPLC to give compound **D673** (26 mg, yield 43.3%), LCMS: $[M + H]^+ = 1067$.

**Preparation of N-(6-((5-bromo-2-((4-(4-(4-(5-(2-(2,6-dioxopiperidin-3-yl)-1-oxoisoindolin-4-yl)pent-4-yn-1-yl)piperazin-1-yl)piperidin-1-yl)-2-methoxy-5-methylphenyl)amino)pyrimidin-4-yl)amino)quinoxalin-5-yl)methanesulfonamide (D741)**

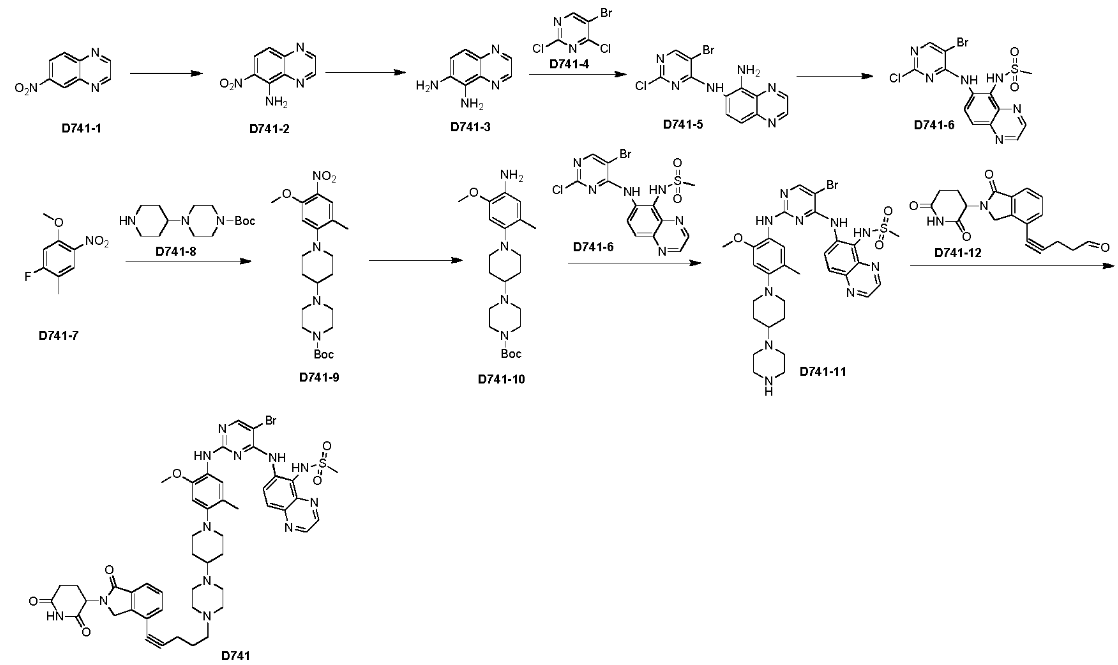

Step 1

**D741-1** (5.00 g, 28.6 mmol) was added to methanol (240 mL). The reaction was heated to 80°C with continuous stirring. Hydroxylamine hydrochloride (2.98 g, 42.9 mmol) was dissolved in methanol (150 mL). Sodium methoxide (5.09 g, 94.3 mmol) was added under stirring at 0°C, and the mixture was allowed to stand. The supernatant was added dropwise to the above solution. The mixture was stirred at 80°C for 1.5 hours. The reaction was allowed to stand. The reaction was cooled to 0°C, and filtered. The mother liquor was concentrated. The reaction was cooled to room temperature, and filtered. The solid was added to 8 mL of acetic acid/water (3/1). The mixture was stirred at 100°C for ten minutes. The reaction was cooled down, and filtered. The residue was dried and collected to give **D741-2** (2.445 g, yield 45.0%), LCMS: $[M + H]^+$ = 191.

Step 2

**D741-2** (100 mg, 0.53 mmol) and Pd/C (10%, 11 mg) were added to 5 mL of anhydrous ethanol. The mixture was heated and stirred at 78°C for 1 hour. Hydrazine hydrate (527 mg, 10.53 mmol) was added and the mixture was stirred for another 1 hour. The mixture was cooled to room temperature. The solid was filtered out. The mother liquor was concentrated, and the crude product was used directly in the next step. LCMS: $[M + H]^+$ = 161.

Step 3

**D741-3** (76 mg, 0.475 mmol), **D741-4** (107 mg, 0.475 mmol) and DIEA (122 mg, 0.946 mmol) were added to 6 mL of n-butanol. The mixture was reacted in a microwave oven at 110 °C for 1 hour under nitrogen protection. The solvent was removed under reduced pressure. The residue was purified by thin layer chromatography to obtain **D741-5** (55 mg, 33.1%), LCMS: $[M + H]^+$ = 351.

Step 4

**D741-5** (55 mg, 0.157 mmol) was dissolved in 3 mL of pyridine. Methanesulfonyl chloride (125 mg, 1.100 mmol) was added dropwise under nitrogen protection at 0°C with stirring. After the addition was completed, the mixture was stirred at room temperature overnight. The reaction solution was added to 0°C ice water. The mixture was extracted three times with ethyl acetate. The organic phases were combined, dried over anhydrous sodium sulfate, and filtered. The solvent was evaporated under reduced pressure. The residue was purified by thin layer chromatography to obtain **D741-6** (18 mg, 26.9%), LCMS: $[M + H]^+$ = 429.

Step 5

**D741-7** (500 mg, 2.70 mmol), **D741-8** (1.09 g, 4.05 mmol), and potassium carbonate (745 mg, 5.40 mmol) were added to 15 mL of DMF. The mixture was stirred at 80 °C overnight under nitrogen protection. The reaction was cooled, and filtered. The residue was purified by flash to give **D741-9** (660 mg, yield 56.3%), LCMS: $[M + H]^+$ = 435.

Step 6

**D741-9** (660 mg, 1.52 mmol) and Pd/C (10%, 132 mg) were added to 20 mL of methanol. Hydrogen was introduced, and the mixture was stirred at room temperature for 3 hours. The mixture was filtered and concentrated to give a crude product **D741-10** (600 mg, 97.7%), which was used directly in the next step. LCMS: $[M + H]^+$ = 405.

Step 7

**D741-10** (11.8 mg, 0.028 mmol), **D741-6** (22 mg, 0.055 mmol) and methanesulfonic acid (11 mg, 0.110 mmol) were dissolved in 2 mL of n-butanol. The mixture was stirred at 90 °C for 20 hours under nitrogen protection. The residue was purified by Flash to give **D741-11** (7.5 mg, yield 39.5%), LCMS: $[M + H]^+$ = 697.

Step 8

**D741-11** (12 mg, 0.017 mmol) was dissolved in 3 mL of dichloromethane/methanol (2/1). **D741-12** (6.7 mg, 0.021 mmol) and acetic acid (1.2 mg, 0.021 mmol) were added. Under nitrogen, the mixture was stirred at room temperature for 1 hour. Sodium cyanoborohydride (4.8 mg, 0.076 mmol) was added in batches over 2 hours. The solvent was removed under reduced pressure below 40°C, and the crude product was purified by HPLC to give compound **D741** (8 mg, yield

47.1%), LCMS: [M + H]+ = 1005.

**Preparation of N-(5-((4-((2-(dimethylphosphoryl)phenyl)amino)-5-(trifluoromethyl)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-fluoroacrylamide (D746)**

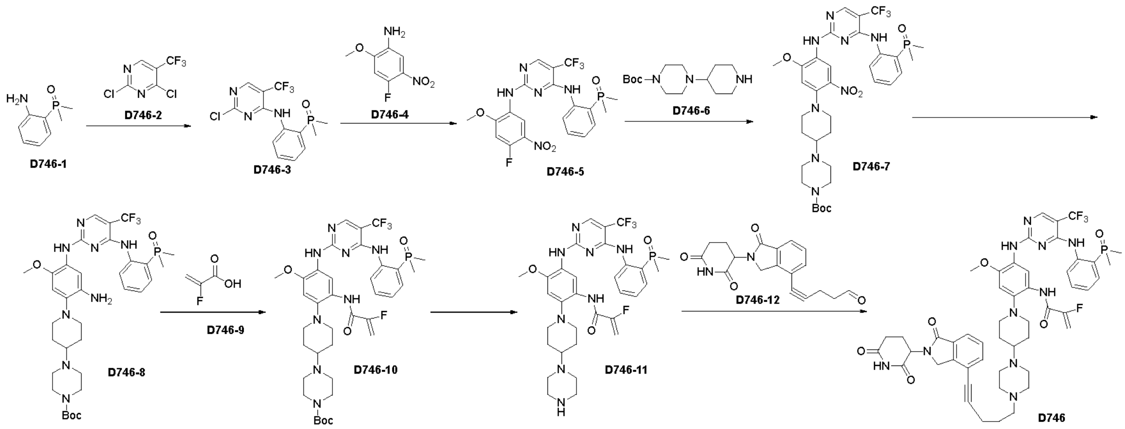


Step 1

Compound **D746-1** (5 g, 29.6 mmol) was dissolved in n-butanol (50 mL). Compound **D746-**2 (6.45 g, 29.9 mmol) was added dropwise under stirring at 0°C, followed by DIEA (4.59 g, 35.5 mmol). The mixture was stirred at 0-5°C for 1 hour. The mixture was heated to room temperature and stirred for 4 hours. The solvent was removed under reduced pressure. The residue was purified by Flash to give compound **D746-3** (2.68 g, 26.0%). LCMS: [M + H]+ = 350.

Step 2

**D746-3** (2.68 g, 7.68 mmol), **D746-4** (1.57 g, 8.45 mmol) and methanesulfonic acid (2.95 g, 30.71 mmol) were dissolved in 70 mL of tert-butanol. The mixture was stirred at 90°C overnight under nitrogen protection. The mixture was cooled to room temperature. The solvent was evaporated under reduced pressure. The pH was adjusted to 7 with aqueous sodium bicarbonate solution. The mixture was extracted three times with dichloromethane, dried over anhydrous sodium sulfate, and filtered. The filtrate was concentrated to give a crude product. The crude product was purified by Flash to obtain **D746-5** (2.48 g, 64.8%). LCMS: [M + H]+ = 500.

Step 3

**D746-5** (2.48 g, 4.97 mmol), **D746-6** (1.61 g, 5.96 mmol) and cesium carbonate (4.86 g, 14.91 mmol) were dissolved in 30 mL of DMF. The mixture was stirred at 70°C overnight under nitrogen protection. The mixture was cooled to room temperature. The solid was filtered out. The solvent was evaporated under reduced pressure, and the crude product was purified by Flash to obtain **D746-7** (3.7 g, yield 99.5%). LCMS: [M + H]+ = 749.

Step 4

Under stirring at room temperature, a solution of sodium hydrosulfite (3.64 g, 24.7 mmol) in water (100 mL) was added to a solution of **D746-7** (3.70 g, 4.94 mmol) in ethanol (100 mL). The mixture was stirred for 10 minutes. Dichloromethane was added, and the layers were seperated. The organic layer was dried over anhydrous sodium sulfate, and filtered. The filtrate was concentrated to give a crude product **D746-8** (3.5 g, yield 98.6%), which was used directly in the next step. LCMS: [M + H]+ = 719.

Step 5

**D746-8** (3.50 g, 4.87 mmol), **D746-9** (351 mg, 3.390 mmol), T3P (12.40 g, 19.49 mmol) and TEA (1.97 g, 19.49 mmol) were dissolved in 140 mL of dichloromethane. The mixture was stirred overnight at room temperature under

nitrogen protection. The mixture was washed once with aqueous ammonium chloride solution, and the organic phase was dried over anhydrous sodium sulfate, and filtered. The filtrate was concentrated. The crude product was purified by Flash to obtain **D746-10** (3.67 g, yield 95.3%), LCMS: [M + H]$^+$ = 791.

Step 6

**D746-10** (3.67 g) was dissolved in 24 mL of dichloromethane. TFA (6 mL) was added under stirring. Under nitrogen protection, the mixture was stirred at room temperature for 1.5 hours. The solvent was removed under reduced pressure, and an aqueous sodium bicarbonate solution was added dropwise under stirring to adjust the pH to 7.5. The mixture was extracted three times with a large amount of dichloromethane. The organic phase was dried over anhydrous sodium sulfate, allowed to stand, and filtered. The solvent was removed under reduced pressure to obtain **D746-11** (3.06 g, yield 95.3%), which was used directly in the next step. LCMS: [M + H]$^+$ = 691.

Step 7

**D746-11** (3.06 g, 4.43 mmol), **D746-12** (1.87 g, 5.76 mmol) and acetic acid (0.32 g, 5.32 mmol) were dissolved in 60 mL of dichloromethane/methanol (2/1, v/v). The mixture was stirred at room temperature under nitrogen for 1 hour. Sodium cyanoborohydride (1.26 g, 20.05 mmol) was added in batches over two hours. The solvent was removed under reduced pressure below 40°C, and the crude product was flash purified to give an off-white solid compound **D746** (2.4 g, yield 54.2%), LCMS: [M + H]$^+$ = 999.

**Preparation of (E)-N-(5-((4-((2-(dimethylphosphoryl)phenyl)amino)-5-(trifluoromethyl)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-4,4,4-trifluorobut-2-enamide (D747)**

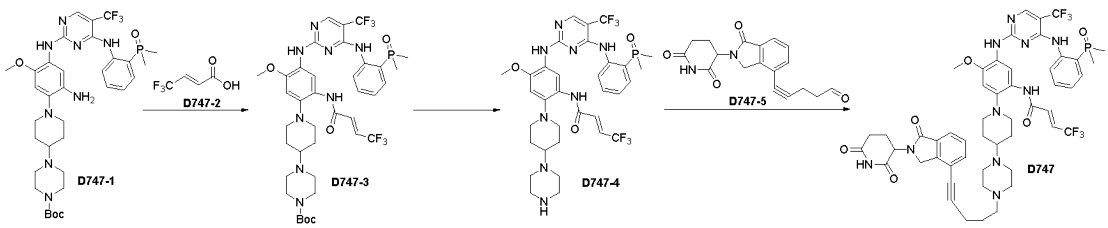


D747-1 D747-3 D747-4 D747

Step 1

**D747-1** (50 mg, 0.070 mmol), **D747-2** (7.8 mg, 0.056 mmol), T3P (50% EA, 177 mg, 0.278 mmol) and TEA (28 mg, 0.278 mmol) were dissolved in 3 mL of dichloromethane. Under nitrogen protection, the mixutre was stirred at room temperature overnight. The mixture was purified by Flash to give **D747-3** (39 mg, yield 67.2%), LCMS: [M + H]$^+$ = 841.

Step 2

**D747-3** (39 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: [M + H]$^+$ = 741.

Step 3

**D747-4** (34.4 mg, 0.046 mmol) and **D747-5** (19.6 mg, 0.060 mmol) were dissolved in 3 mL of dichloromethane/methanol (2/1). TEA was added dropwise, and the pH was adjusted to 7.5. Acetic acid (3.3 mg, 0.056 mmol) was added. The mixture was stirred at room temperature under nitrogen for 1 hour. Sodium cyanoborohydride (13.2 mg, 0.210 mmol) was added in batches over 2 hours. The solvent was removed under reduced pressure below 40°C, and the crude product was purified by HPLC to obtain **D747** (23 mg, yield 47.2%), LCMS: [M + H]$^+$ = 1049.

**Preparation of N-(5-((4-((2-(dimethylphosphoryl)phenyl)amino)-5-(trifluoromethyl)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)but-2-ynamide (D748)**

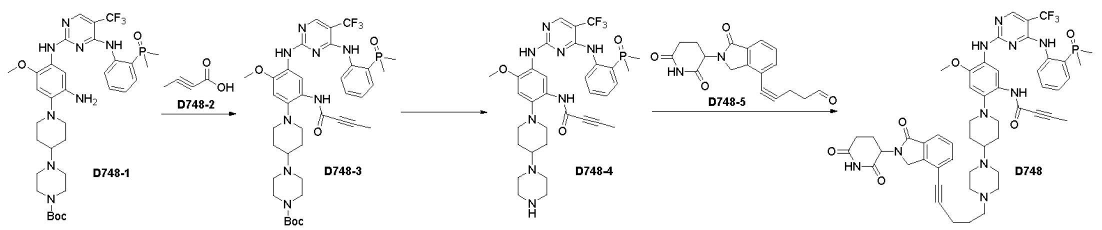


Step 1

**D748-1** (50 mg, 0.070 mmol), **D748-2** (4.7 mg, 0.056 mmol), T3P (50% EA, 177 mg, 0.278 mmol) and TEA (28 mg, 0.278 mmol) were dissolved in 3 mL of dichloromethane. Under nitrogen protection, the mixutre was stirred at room temperature overnight. The mixture was purified by Flash to give **D748-3** (27 mg, yield 49.1%), LCMS: $[M + H]^+ = 785$.

Step 2

**D748-3** (27 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: $[M + H]^+ = 685$.

Step 3

**D748-4** (23.6 mg, 0.034 mmol) and **D748-5** (14.5 mg, 0.045 mmol) were dissolved in 3 mL of dichloromethane/methanol (2/1, v/v). TEA was added dropwise, and the pH was adjusted to 7.5. Acetic acid (2.5 mg, 0.041 mmol) was added, and the mixture was stirred at room temperature under nitrogen for 1 hour. Sodium cyanoborohydride (9.9 mg, 0.156 mmol) was added in batches over 2 hours. The solvent was removed under reduced pressure below 40°C, and the crude product was purified by HPLC to obtain **D748** (12 mg, yield 35.3%), LCMS: $[M + H]^+ = 993$.

**Preparation of N-(5-((4-((2-(dimethylphosphoryl)phenyl)amino)-5-(trifluoromethyl)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)hex-2-ynamide (D835)**

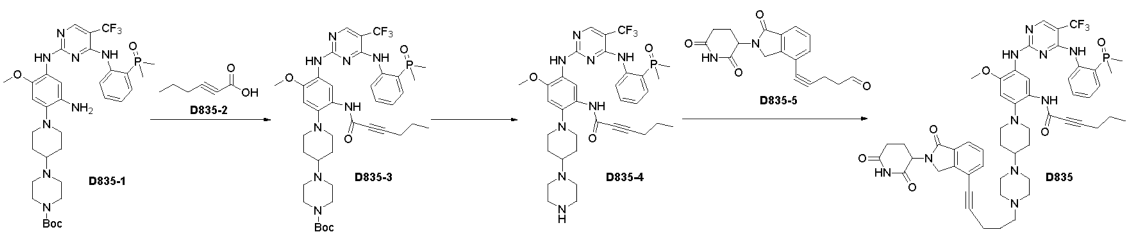


Step 1

**D835-1** (100 mg, 0.139 mmol), **D835-2** (12.5 mg, 0.111 mmol), T3P (50% EA, 354 mg, 0.557 mmol) and TEA (56 mg, 0.557 mmol) were dissolved in 3 mL of dichloromethane. Under nitrogen protection, the mixutre was stirred at room temperature overnight. The mixture was purified by Flash to give **D835-3** (65.6 mg, yield 58.1%), LCMS: $[M + H]^+ = 813$.

Step 2

**D835-3** (44.4 mg) was dissolved in 4 mL of dichloromethane. TFA (1 mL) was added with stirring. Under nitrogen

protection, the mixture was stirred at room temperature for 1.5 hours. The solvent was removed under reduced pressure, and the crude product was used directly in the next step, LCMS: $[M + H]^+$ = 713.

Step 3

**D835-4** (38.9 mg, 0.055 mmol) and **D835-5** (23 mg, 0.071 mmol) were dissolved in 3 mL of dichloromethane/-methanol (2/1, v/v). TEA was added dropwise, and the pH was adjusted to 7.5. Acetic acid (3.9 mg, 0.066 mmol) was added, and the mixture was stirred at room temperature under nitrogen for 1 hour. Sodium cyanoborohydride (15.3 mg, 0.246 mmol) was added in batches over 2 hours. The solvent was removed under reduced pressure below 40°C, and the crude product was purified by HPLC to give compound **D835** (26 mg, yield 46.7%), LCMS: $[M + H]^+$ = 1021.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-(2,2,2-trifluoroethoxy)phenyl)but-2-ynamide (D845)**

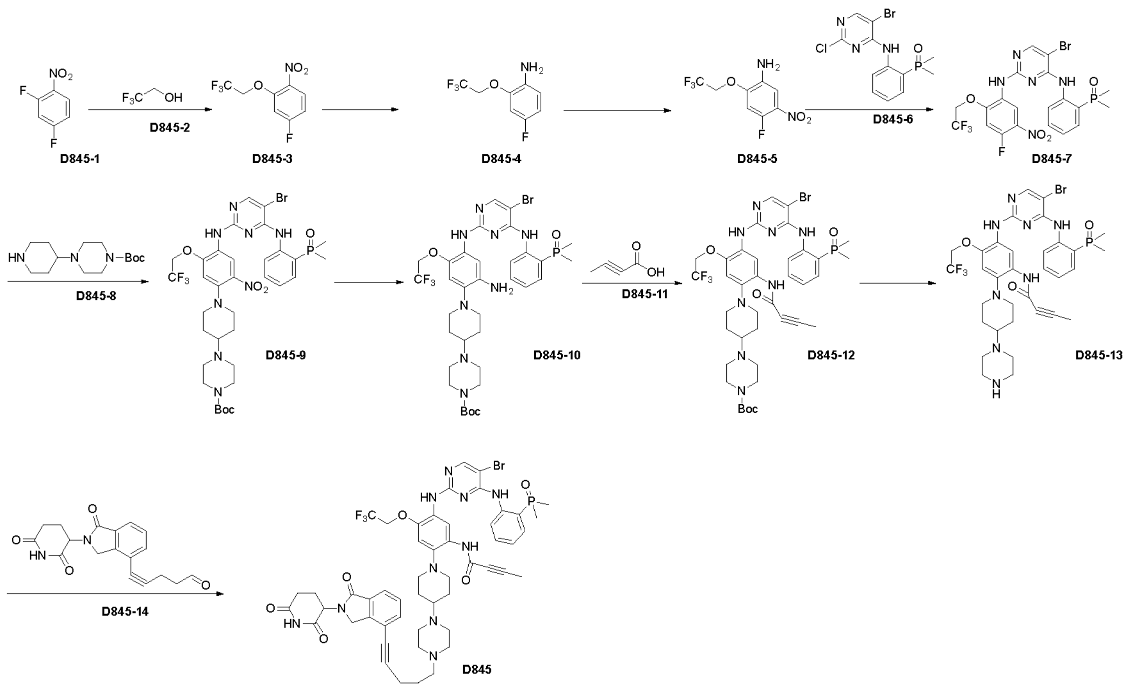


Step 1

**D845-1** (10 g, 62.9 mmol) and cesium carbonate (20.5 g, 62.9 mmol) were added to tetrahydrofuran (100 mL). **D845-2** (6.29 g, 62.9 mmol) was added dropwise under stirring. The mixture was stirred at room temperature overnight, and filtered. The solvent was removed under reduced pressure. The residue was purified by Flash to obtain **D845-3** (14.35 g, yield 95.7%). LCMS: $[M + H]^+$ = 240.

Step 2

**D845-3** (14.35 g, 60 mmol), iron powder (20.12 g, 360 mmol) and ammonium chloride (9.63 g, 180 mmol) were added to 100 mL of water/ethanol (1/4, V/V). The mixture was heated and stirred at 100°C for 1 hour. The mixture was filtered while hot and the filtrate was cooled. The filtrate was extracted with ethyl acetate three times. The organic phases were combined. The solvent was evaporated under reduced pressure. The residue was purified by Flash to obtain **D845-4** (9.76 g, yield 78.1%). LCMS: $[M + H]^+$ = 210.

Step 3

**D845-4** (9.76 g, 47 mmol) was added to 40 mL of concentrated sulfuric acid under stirring at 0°C. Potassium

nitrate (5.66 g, 56 mmol) was added in batches. The temperature was maintained and the mixture was stirred for 1 hour. The reaction solution was poured into ice water and extracted twice with ethyl acetate. The aqueous phase was adjusted to pH = 7 with dilute hydrochloric acid, and then extracted twice with ethyl acetate. The organic phases were combined, dried over anhydrous sodium sulfate, and filtered. The filtrate was concentrated to give a crude product. The crude product was purified by Flash to obtain **D845-5** (8.58 g, 72.3%). LCMS: $[M + H]^+$ = 255.

Step 4

Compound **D845-5** (1 g, 2.79 mmol), **D845-6** (708 mg, 2.79 mmol) and methanesulfonic acid (803 mg, 8.36 mmol) were dissolved in 20 mL of tert butanol. The mixture was stirred at 90°C for 24 hours under nitrogen protection. The temperature was cooled to room temperature. The solvent was evaporated under reduced pressure. The pH was adjusted to 7 with sodium bicarbonate aqueous solution. The mixture was extracted with dichloromethane three times. The organic phase was dried over anhydrous sodium sulfate, and filtered. The filtrate was concentrated to give a crude product. The crude product was purified by Flash to give compound **D845-7** (1.30 g, yield 80.7%). LCMS: $[M + H]^+$ = 578.

Step 5

**D845-7** (900 mg, 1.56 mmol), **D845-8** (504 mg, 1.87 mmol), and cesium carbonate (1.53 g, 4.68 mmol) were added to 10 mL of DMF. The mixture was stirred at 70 °C overnight under nitrogen protection. The mixture was cooled, and filtered. The filtrate was concentrated. The crude product was purified by Flash to obtain **D845-9** (1.055 g, yield 81.4%), LCMS: $[M + H]^+$ = 827.

Step 6

Under stirring at room temperature, a solution of sodium hydrosulfite (1.05 g, 6.05 mmol) in water (40 mL) was added to a solution of **D845-9** (1.0 g, 1.21 mmol) in ethanol (40 mL). The mixture was stirred and reacted for 10 minutes. Dichloromethane was added, and the layers were separated. The mixture was dried over anhydrous sodium sulfate, and filtered. The filtrate was concentrated to give a crude product **D845-10** (0.7 g, 72.9%), which was directly used in the next step. LCMS: $[M + H]^+$ = 797.

Step 7

**D845-10** (50 mg, 0.063 mmol), **D845-11** (4.2 mg, 0.050 mmol), T3P (50% EA, 160 mg, 0.251 mmol) and TEA (25 mg, 0.251 mmol) were dissolved in 2 mL of dichloromethane. Under nitrogen protection, the mixutre was stirred at room temperature overnight. The mixture was purified by Flash to give **D845-12** (36.3 mg, yield 67.1%), LCMS: $[M + H]^+$ = 863.

Step 8

**D845-12** (36.3 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: $[M + H]^+$ = 763.

Step 9

**D845-13** (32 mg, 0.042 mmol) was dissolved in 3 mL of dichloromethane/methanol (2/1, v/v). TEA was added dropwise. The pH was adjusted to 7.5. Compound **D845-14** (17.7 mg, 0.055 mmol) and acetic acid (3.0 mg, 0.050 mmol) were added. The mixture was stirred at room temperature under nitrogen for 1 hour. Sodium cyanoborohydride (12.0 mg, 0.189 mmol) was added in batches over 2 hours. The solvent was removed under reduced pressure below 40°C, and the crude product was purified by HPLC to give compound **D845** (15 mg, yield 33.4%), LCMS: $[M + H]^+$ = 1071.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-(2,2,2-trifluoroethoxy)phenyl)-2-fluoroacrylamide (D846)**

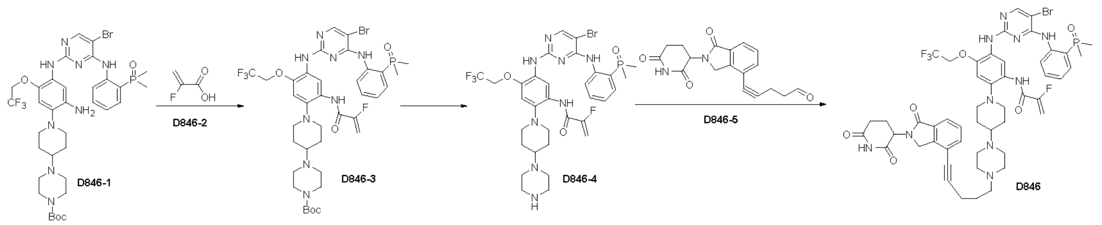


Step 1

**D846-1** (50 mg, 0.063 mmol), **D846-2** (4.5 mg, 0.050 mmol), T3P (50% EA, 160 mg, 0.251 mmol) and TEA (25 mg, 0.251 mmol) were dissolved in 2 mL of dichloromethane. Under nitrogen protection, the mixutre was stirred at room temperature overnight. The mixture was purified by Flash to give **D846-3** (41.5 mg, yield 76.1%), LCMS: $[M + H]^+ = 869$.

Step 2

**D846-3** (41.5 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: $[M + H]^+ = 769$.

Step 3

**D846-4** (36.7 mg, 0.048 mmol) was dissolved in 3 mL of dichloromethane/methanol (2/1, v/v). TEA was added dropwise. The pH was adjusted to 7.5. **D846-5** (20.1 mg, 0.062 mmol) and acetic acid (3.4 mg, 0.057 mmol) were added, and the mixture was stirred at room temperature under nitrogen for 1 hour. Sodium cyanoborohydride (13.5 mg, 0.216 mmol) was added in batches over 2 hours. The solvent was removed under reduced pressure below 40°C, and the crude product was purified by HPLC to obtain **D846** (17 mg, yield 33.3%), LCMS: $[M + H]^+ = 1077$.

**Preparation of (E)-N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-(2,2,2-trifluoroethoxy)phenyl)-4,4,4-trifluorobut-2-enamide (D847)**

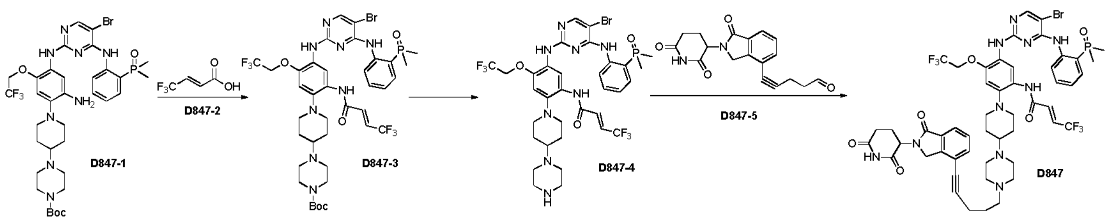


Step 1

**D847-1** (50 mg, 0.063 mmol), **D847-2** (7.0 mg, 0.050 mmol), T3P (50% EA, 160 mg, 0.251 mmol) and TEA (25 mg, 0.251 mmol) were dissolved in 2 mL of dichloromethane. Under nitrogen protection, the mixutre was stirred at room temperature overnight. The mixture was purified by Flash to give **D847-3** (53.6 mg, yield 93.1%), LCMS: $[M + H]^+ = 919$.

Step 2

**D847-3** (53.6 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: $[M + H]^+ = 819$.

Step 3

**D847-4** (51.8 mg, 0.063 mmol) was dissolved in 3 mL of dichloromethane/methanol (2/1, v/v). TEA was added

dropwise. The pH was adjusted to 7.5. **D847-5** (26.7 mg, 0.082 mmol) and acetic acid (4.6 mg, 0.076 mmol) were added, and the mixture was stirred at room temperature under nitrogen for 1 hour. Sodium cyanoborohydride (18.0 mg, 0.285 mmol) was added in batches over 2 hours. The solvent was removed under reduced pressure below 40°C, and the crude product was purified by HPLC to obtain **D847** (28 mg, yield 36.4%), LCMS: $[M + H]^+$ = 1127.

**Preparation of N-(5-((5-bromo-4-((2-(dimethylphosphoryl)phenyl)amino)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-(2,2,2-trifluoroethoxy)phenyl)hex-2-ynamide (D856)**

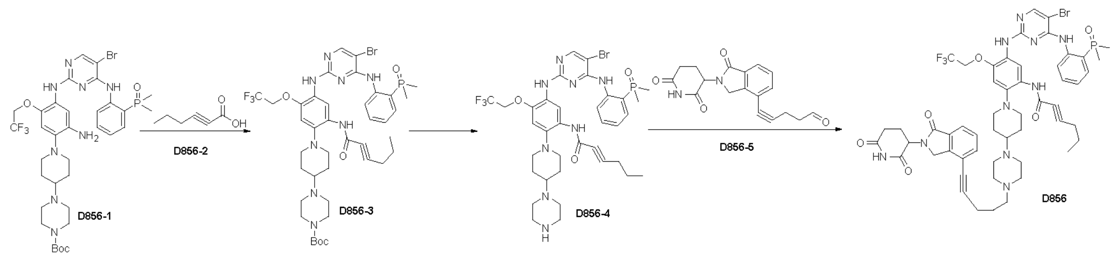


Step 1

**D856-1** (50 mg, 0.063 mmol), **D856-2** (5.6 mg, 0.050 mmol), T3P (50% EA, 160 mg, 0.251 mmol) and TEA (25 mg, 0.251 mmol) were dissolved in 2 mL of dichloromethane. Under nitrogen protection, the mixutre was stirred at room temperature overnight. The mixture was purified by Flash to give **D856-3** (39.4 mg, yield 70.5%), LCMS: $[M + H]^+$ = 891.

Step 2

**D856-3** (39.4 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: $[M + H]^+$ = 791.

Step 3

**D856-4** (34.9 mg, 0.044 mmol) was dissolved in 3 mL of dichloromethane/methanol (2/1, v/v). TEA was added dropwise. The pH was adjusted to 7.5. **D856-5** (18.6 mg, 0.057 mmol) and acetic acid (3.2 mg, 0.053 mmol) were added, and the mixture was stirred at room temperature under nitrogen for 1 hour. Sodium cyanoborohydride (12.6 mg, 0.198 mmol) was added in batches over 2 hours. The solvent was removed under reduced pressure below 40°C, and the crude product was purified by HPLC to give compound **D856** (16 mg, yield 33.3%), LCMS: $[M + H]^+$ = 1099.

**Preparation of N-(5-((4-((2-(dimethylphosphoryl)phenyl)amino)-5-(trifluoromethyl)pyrimidin-2-yl)amino)-2-(4-(4-(5-(2-(2,6-dioxopiperidin-3-yl)-1-oxoisoindolin-4-yl)pent-4-yn-1-yl)piperazin-1-yl)piperidin-1-yl)-4-(2,2,2-trifluoroethoxy)phenyl)-2-fluoroacrylamide (D870)**

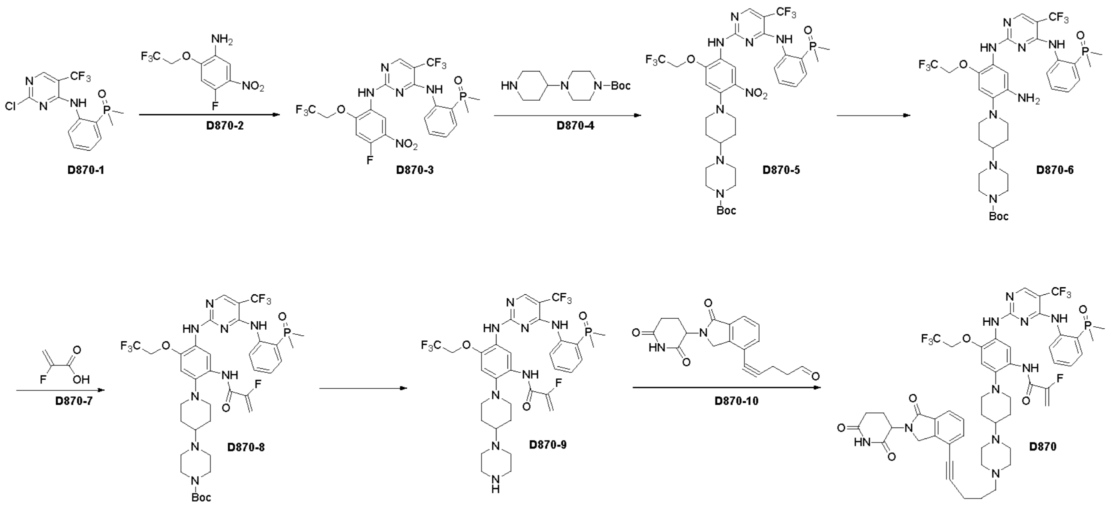


Step 1

**D870-1** (401 mg, 1.15 mmol), **D870-2** (321 mg, 1.26 mmol) and methanesulfonic acid (442 mg, 4.60 mmol) were dissolved in 20 mL of tert butanol. The mixture was stirred at 90 °C for 24 hours under nitrogen protection. The temperature was cooled to room temperature, the solvent was evaporated under reduced pressure, and the crude product was purified by Flash to obtain **D870-3** (505 mg, 77.0%). LCMS: $[M + H]^+$ = 568.

Step 2

**D870-3** (300 mg, 0.529 mmol), **D870-4** (171 mg, 0.635 mmol), and cesium carbonate (517 mg, 1.587 mmol) were added to 5 mL of DMF. The mixture was stirred at 70 °C overnight under nitrogen protection. The mixture was cooled, and filtered. The filtrate was concentrated. The crude product was purified by Flash to obtain **D870-5** (416 mg, yield 96.5%), LCMS: $[M + H]^+$ = 817.

Step 3

Under stirring at room temperature, a solution of sodium hydrosulfite (444 g, 2.55 mmol) in water (20 mL) was added to a solution of **D870-5** (416 mg, 0.51 mmol) in ethanol (20 mL). The mixture was stirred and reacted for 10 minutes. Dichloromethane was added, and the layers were separated. The mixture was dried over anhydrous sodium sulfate, and filtered. The filtrate was concentrated to give a crude product **D870-6** which was directly used in the next step. LCMS: $[M + H]^+$ = 787.

Step 4

**D870-6** (60 mg, 0.076 mmol), **D870-7** (5.5 mg, 0.061 mmol), T3P (50% EA, 194 mg, 0.305 mmol) and TEA (31 mg, 0.305 mmol) were dissolved in 2 mL of dichloromethane. Under nitrogen protection, the mixutre was stirred at room temperature overnight. The mixture was purified by Flash to give **D870-8** (30 mg, yield 46.2%), LCMS: $[M + H]^+$ = 859.

Step 5

**D870-8** (30 mg) was dissolved in 4 mL of dichloromethane. TFA (1 mL) was added with stirring. Under nitrogen protection, the mixture was stirred at room temperature for 1.5 hours. The solvent was removed under reduced pressure, and the crude product was used directly in the next step, LCMS: $[M + H]^+$ = 759.

Step 6

**D870-9** (27 mg, 0.036 mmol) was dissolved in 3 mL of dichloromethane/methanol (2/1, v/v). TEA was added dropwise. The pH was adjusted to 7.5. **D870-10** (15 mg, 0.046 mmol) and acetic acid (2.6 mg, 0.043 mmol) were added, and the mixture was stirred at room temperature under nitrogen for 1 hour. Sodium cyanoborohydride (10.2 mg, 0.159 mmol) was added in batches over 2 hours. The solvent was removed under reduced pressure below 40°C, and the crude product was purified by HPLC to obtain **D870** (16 mg, yield 42.1%), LCMS: $[M + H]^+$ = 1067.

**Preparation of N-(6-((5-bromo-2-((4-(4-(5-(2-(2,6-dioxopiperidin-3-yl)-1-oxoisoindolin-4-yl)pent-4-yn-1-yl)piperazin-1-yl)piperidin-1-yl)-2-methoxy-5-methylphenyl)amino)pyrimidin-4-yl)amino)quinoxalin-5-yl)-N-methylmethanesulfonamide (D932)**

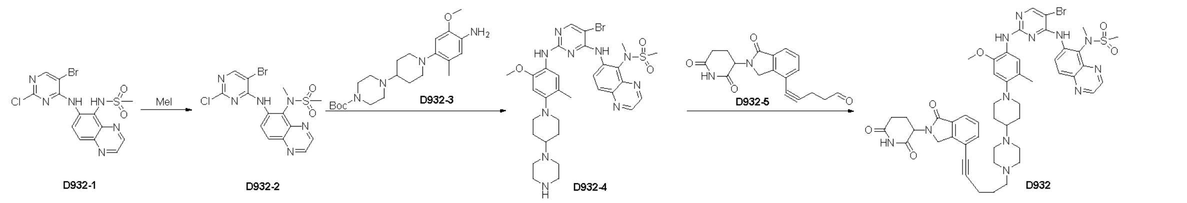


Step 1:

Compound **D932-1** (or **D741-6,** 120 mg, 0.280 mmol), iodomethane (60 mg, 0.421 mmol), and potassium carbonate (58 mg, 0.421 mmol) were added to 2 ml of acetonitrile. The mixture was stirred at 50 °C overnight under nitrogen protection. The residue was purified by Flash to give **D932-2** (29 mg, yield 23.6%), LCMS: $[M + H]^+$ = 443.

Step 2:

**D932-2** (10 mg, 0.0226 mmol), **D932-3** (18 mg, 0.0453 mmol) and methanesulfonic acid (8.7 mg, 0.0905 mmol) were dissolved in 2 ml of n-butanol. The mixture was stirred at 90 °C overnight under nitrogen protection. The residue was purified by Flash to give **D932-4** (12 mg, yield 75%), LCMS: $[M + H]^+$ = 711.

Step 3:

**D932-4** (26 mg, 0.037 mmol), **D932-5** (14 mg, 0.044 mmol) and acetic acid (2.6 mg, 0.04 mmol) were dissolved in 2 ml of dichloromethane/methanol (2/1, v/v). The mixture was stirred at room temperature for 1 hour under nitrogen. Sodium cyanoborohydride (10.5 mg, 0.156 mmol) was added in batches over 2 hours. The solvent was removed under reduced pressure below 40°C, and the crude product was purified by HPLC to give compound **D932** (3.7 mg, yield 10.0%), LCMS: $[M + H]^+$ = 1019.

**Preparation of N-(5-((5-bromo-4-((5-(methylsulfonamido)quinoxalin-6-yl)amino)pyrimidin-2-yl)amino-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-fluoroacrylamide (D933)**

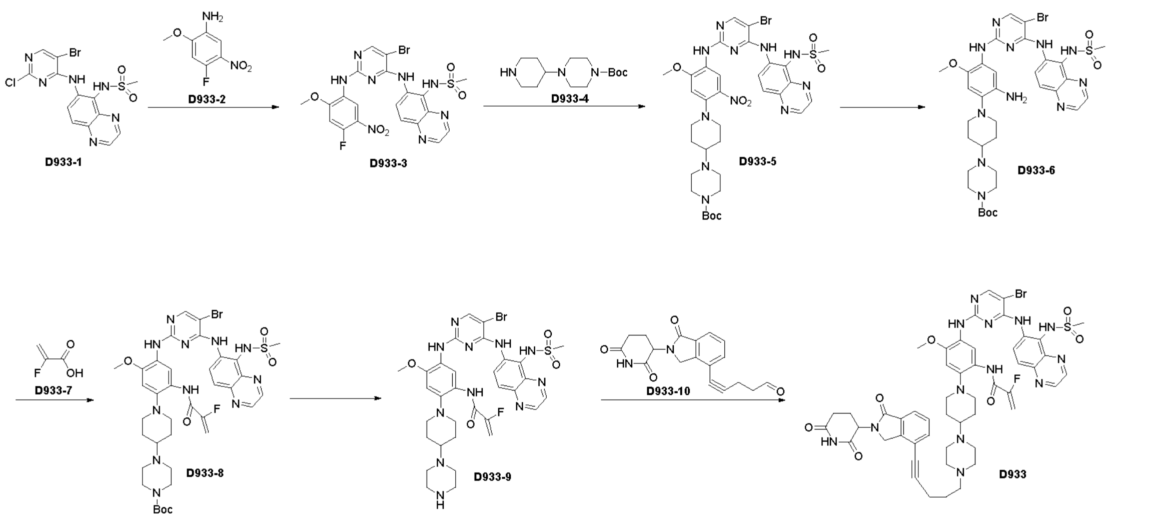

Step 1

Compound **D933-1** (or **D741-6,** 50 mg, 0.117 mmol), **D933-2** (23 mg, 0.123 mmol) and methanesulfonic acid (45 mg, 0.467 mmol) were dissolved in 2 ml of n-butanol. The mixture was stirred at 90 °C overnight under nitrogen protection. The mixture was cooled to room temperature. The solvent was evaporated under reduced pressure, and the crude product was purified by Flash to obtain **D933-3** (27 mg, 40.0%). LCMS: $[M + H]^+$ = 579.

Step 2

**D933-3** (27 mg, 0.047 mmol), **D933-4** (15 mg, 0.056 mmol), and cesium carbonate (46 mg, 0.140 mmol) were added to 2 ml of DMF. The mixture was stirred at 70 °C overnight under nitrogen protection. The mixture was cooled, and filtered. The filtrate was concentrated. The crude product was purified by Flash to obtain **D933-5** (28 mg, yield 72.5%), LCMS: $[M + H]^+$ = 828.

Step 3

Under stirring at room temperature, a solution of sodium hydrosulfite (59 mg, 0.338 mmol) in water (10 ml) was added to a solution of **D933-5** (28 mg, 0.34 mmol) in ethanol (10 ml). The mixture was stirred and reacted for 10 minutes. Dichloromethane was added, and the layers were separated. The mixture was dried over anhydrous sodium sulfate, and filtered. The filtrate was concentrated to give a crude product **D933-6** which was directly used in the next step. LCMS: [M + H]+ = 798.

Step 4

**D933-6** (32.6 mg, 0.041 mmol), **D933-7** (2.94 mg, 0.033 mmol), T3P (104 mg, 0.164 mmol) and TEA (16.5 mg, 0.164 mmol) were dissolved in 2 ml of dichloromethane. Under nitrogen protection, the mixutre was stirred at room temperature overnight. The mixture was purified by Flash to give **D933-8** (5 mg, yield 14.1%), LCMS: $[M + H]^+$ = 870.

Step 5

**D933-8** (5 mg) was dissolved in 4 ml of dichloromethane. TFA (1 ml) was added with stirring. Under nitrogen protection, the mixture was stirred at room temperature for 1 hour. The solvent was removed under reduced pressure, and the crude product was directly used in the next step. LCMS: $[M + H]^+$ = 770.

Step 6

**D933-9** (4.4 mg, 0.006 mmol), **D933-10** (2.2 mg, 0.007 mmol) and acetic acid (0.4 mg, 0.007 mmol) were dissolved in 3 ml of dichloromethane/methanol (2/1, v/v). The mixture was stirred at room temperature for 1 hour under nitrogen. Sodium cyanoborohydride (1.5 mg, 0.027 mmol) was added in batches over 2 hours. The solvent was removed under reduced pressure below 40°C, and the crude product was purified by HPLC to give compound **D933** (2 mg, yield 32.5%), LCMS: $[M + H]^+$ = 1078.

**Preparation** of **N-(5-((5-bromo-4-((5-(N-methylmethanesulfonamido)quinoxalin-6-yl)amino)pyrimidin-2-yl)amino-2-(4-(4-(5-(2-(2,6-dioxopiperidin-3-yl)-1-oxoisoindolin-4-yl)pent-4-yn-1-yl)piperazin-1-yl)piperidin-1-yl)-4-methoxyphenyl)-2-fluoroacrylamide (D934)**

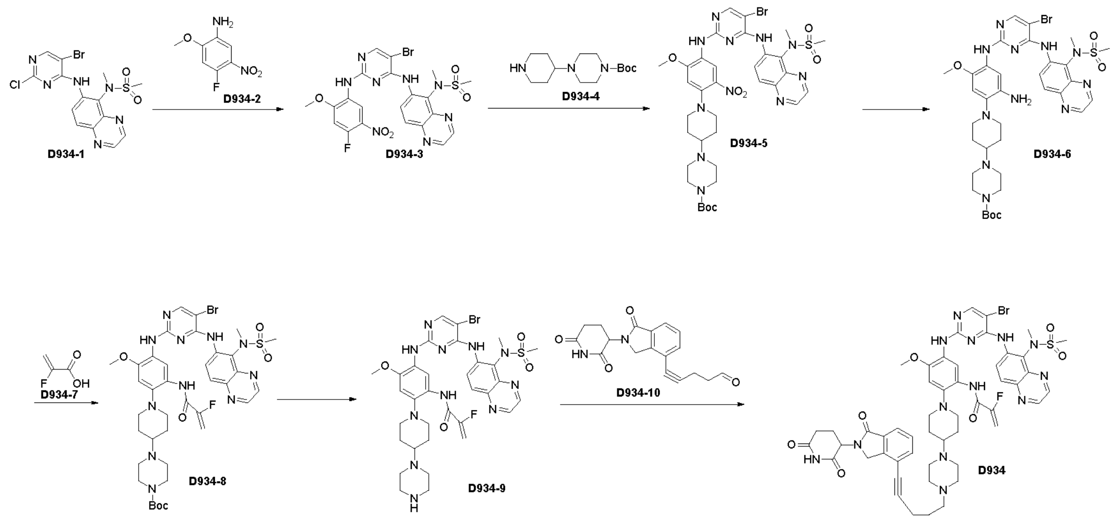


Step 1

**D934-1** (or **D932-2,** 19 mg, 0.043 mmol), **D934-2** (8.4 mg, 0.045 mmol) and methanesulfonic acid (16 mg, 0.172 mmol) were dissolved in 2 ml of n-butanol and the mixture was stirred overnight at 90 °C under nitrogen protection. The mixture was cooled to room temperature and the solvent was evaporated under reduced pressure. The crude product was purified by thin layer chromatography to obtain **D934-3** (14 mg, 56.0%). LCMS: $[M + H]^+ = 593$.

Step 2

**D934-3** (14 mg, 0.024 mmol), **D934-4** (8 mg, 0.028 mmol), and cesium carbonate (23 mg, 0.071 mmol) were added to 2 ml of DMF. The mixture was stirred at 70 °C overnight under nitrogen protection. The mixture was cooled, and filtered. The filtrate was concentrated and purified by thin layer chromatography to obtain **D934-5** (12 mg, yield 60.0%), LCMS: $[M + H]^+ = 842$.

Step 3

Under stirring at room temperature, a solution of sodium hydrosulfite (25 mg, 0.143 mmol) in water (10 ml) was added to a solution of **D934-5** (12 mg, 0.014 mmol) in ethanol (10 ml). The mixture was stirred and reacted for 10 minutes. Dichloromethane was added, and the layers were separated. The mixture was dried over anhydrous sodium sulfate, and filtered. The filtrate was concentrated to give a crude product **D934-6** which was directly used in the next step. LCMS: $[M + H]^+ = 812$.

Step 4

**D934-6** (13.7 mg, 0.017 mmol), **D934-7** (1.21 mg, 0.014 mmol), T3P (50% EA, 43 mg, 0.068 mmol) and TEA (6.8 mg, 0.068 mmol) were dissolved in 2 ml of dichloromethane. The mixture was stirred at room temperature overnight under nitrogen protection. The residue was purified by thin layer chromatography to obtain **D934-8** (5 mg, yield 33.6%), LCMS: $[M + H]^+ = 884$.

Step 5

**D934-8** (5 mg) was dissolved in 4 ml of dichloromethane. TFA (1 ml) was added with stirring. Under nitrogen protection, the mixture was stirred at room temperature for 1 hour. The solvent was removed under reduced pressure, and the crude product was directly used in the next step. LCMS: $[M + H]^+ = 784$.

Step 6

**D934-9** (4.4 mg, 0.006 mmol), **D934-10** (2.2 mg, 0.007 mmol) and acetic acid (0.4 mg, 0.007 mmol) were dissolved in 3 ml of dichloromethane/methanol (2/1, v/v). The mixture was stirred at room temperature for 1 hour under nitrogen. Sodium cyanoborohydride (1.5 mg, 0.024 mmol) was added in batches over 2 hours. The solvent was removed

under reduced pressure below 40°C, and the crude product was purified by HPLC to obtain **D934** (2 mg, yield 32.8%), LCMS: $[M + H]^+ = 1092$.

Assay **of activity on Ba/F3 ($EGFR^{L858R/T790M/C797S}$) cells**

Osimertinib is a small molecule EGFR inhibitor used in the clinical treatment of non-small cell lung cancer. Due to the mutation of EGFR C797S, patients develop resistance to Osimertinib. In order to explore the potential of the compounds of the present disclosure to overcome Osimertinib resistance, Ba/F3 ($EGFR^{L858R/T790M/C797S}$) cells were used as the test cell line to test the antiproliferative activity of the compounds of the present disclosure.

1. Assay Methods

1) Ba/F3 ($EGFR^{L858R/T790M/C797S}$) cells were cultured according to the requirements of ATCC, incubated in an incubator at 37°C and 5% $CO_2$, and analyzed by index; cells with viability of > 90% can be used in the assay, and cells were seeded in a 384-well plate (PerkinElmer, 6007680) with 700 cells/well, 30 μL/well.
2) A compound stock solution was prepared, and then diluted 3x to give a compound dilution. 30 nL of the compound dilution was added to a 384-well plate by Echo (Labcyte, Echo550). The cells were incubated in an incubator at 37°C and 5% $CO_2$ for 72h.
3) 30 μL of CTG was added to each well, and the 384-well plate was shaken on a Plate shaker (QILINBEIER, QB-9002). The 384-well plate was incubated at 37°C and 5% $CO_2$ in the dark for 30 min, and the chemiluminescence value was read by Envision (PerkinElmer, EnVision 2104).

2. Data Analysis

The percent inhibition rate (% inhibition) was calculated by the following formula

$$\text{Inhibition}(\%) = \left[1 - \frac{LUM_{cmpd} - \overline{LUM}_{positive}}{\overline{LUM}_{vehicle} - \overline{LUM}_{positive}}\right] \times 100$$

$LUM_{cmpd}$: Luminescence value of assay compound.
$\overline{LUM}_{positive}$ : Average LUM value of positive drug with a concentration of 10 μM.
$\overline{LUM}_{vehicle}$ : Average LUM value of negative control group without drug treatment.

The nonlinear regression curve (dose response-variable slope) between the value of inhibition rate (%) and the logarithm of compound concentration was fitted with graphpad prism 8.0, the effect dose curve of compound was drawn, and the $IC_{50}$ value was calculated.

$Y = Bottom + (Top-Bottom)/(1+10^{\wedge}((LogIC_{50}-X)*HillSlope))$
X-axis: logarithm of compound concentration; Y axis: inhibition rate (% inhibition).

3. Assay result

$IC_{50}$ values of the antiproliferative activity of the compounds of the present disclosure on Ba/F3 ($EGFR^{L858R/T790M/C797S}$) cells are shown in Table 1.

Table 1: Antiproliferative activity of compounds on Ba/F3 ($EGFR^{L858R/T790M/C797S}$) cells

| Compound No. | Structural formula | $IC_{50}$ (nM) |
|---|---|---|
| D058 | 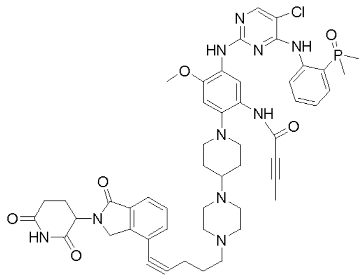 | 35.63 |
| D078 | 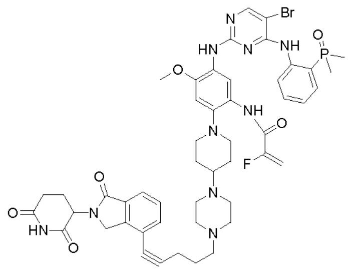 | 19.20 |
| D478 | 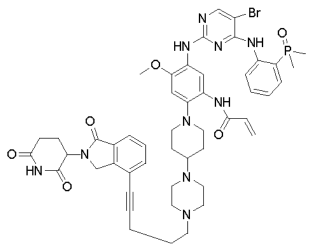 | 30.27 |
| D479 | 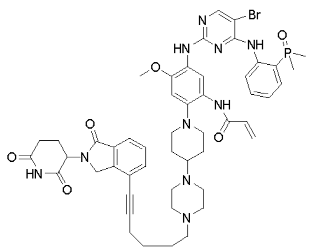 | 86.76 |
| D481 | 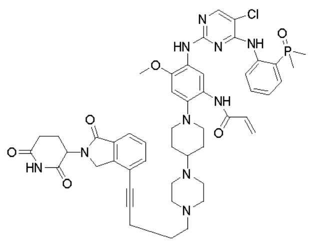 | 68.73 |
| D503 | 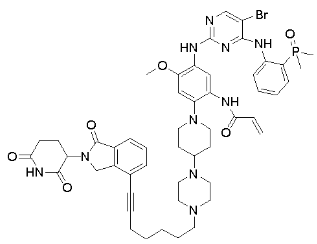 | 51.19 |

(continued)

| Compound No. | Structural formula | $IC_{50}$ (nM) |
|---|---|---|
| D549 | 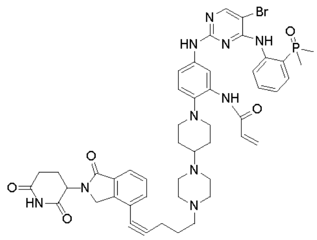 | 7.409 |
| D550 | 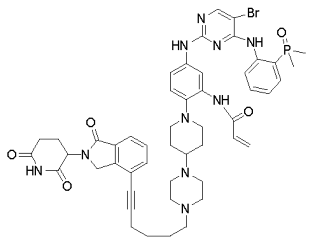 | 14.36 |
| D551 | 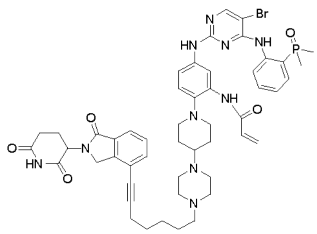 | 14.54 |
| D552 | 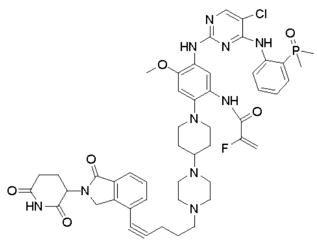 | 37.34 |
| D553 | 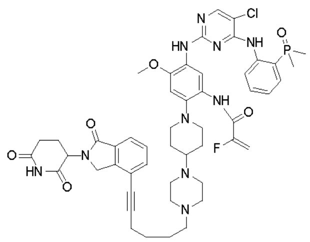 | 49.45 |
| D554 | 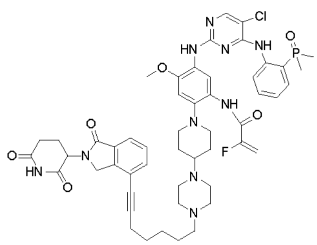 | 45.25 |

(continued)

| Compound No. | Structural formula | $IC_{50}$ (nM) |
|---|---|---|
| D555 | 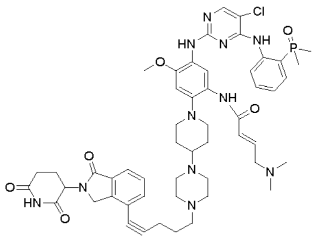 | 67.60 |
| D556 | 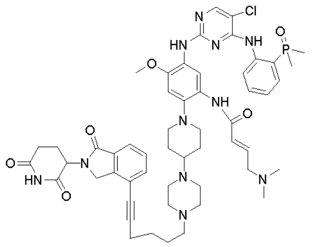 | 582.0 |
| D557 | 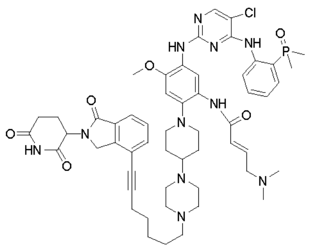 | 382.6 |
| D559 | 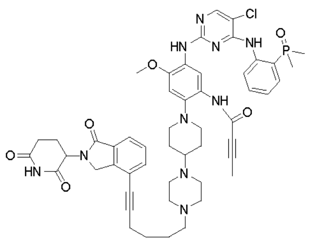 | 50.45 |
| D560 | 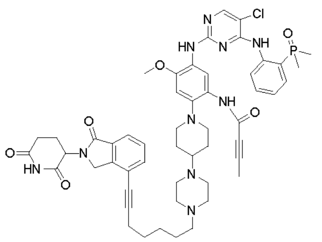 | 112.2 |
| D561 | 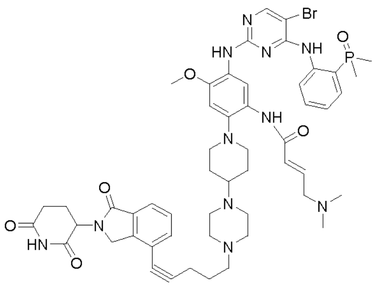 | |

(continued)

| Compound No. | Structural formula | $IC_{50}$ (nM) |
|---|---|---|
| D562 | 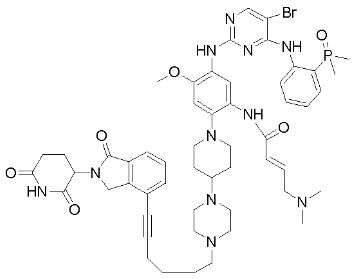 | |
| D563 | 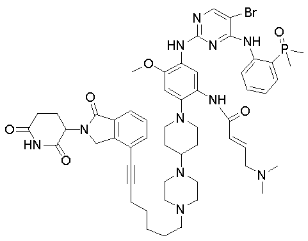 | 305.5 |
| D579 | 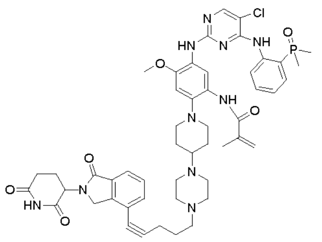 | 41.38 |
| D580 | 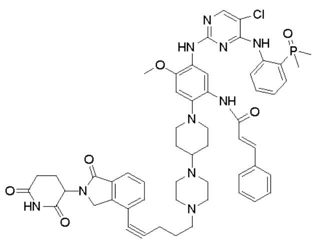 | |
| D581 | 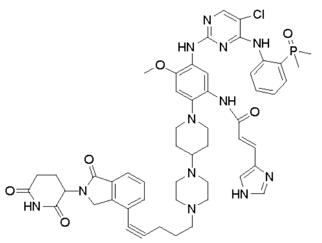 | 2598 |
| D582 | 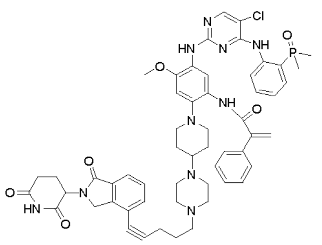 | |

(continued)

| Compound No. | Structural formula | $IC_{50}$ (nM) |
|---|---|---|
| D583 | 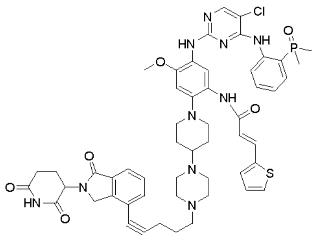 | 45.58 |
| D584 | 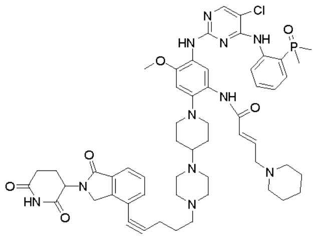 | 216.2 |
| D596 | 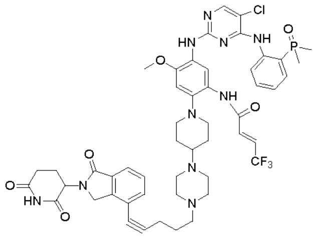 | 31.74 |
| D597 | 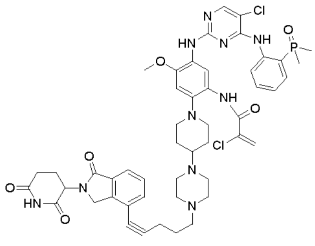 | 459.7 |
| D600 | 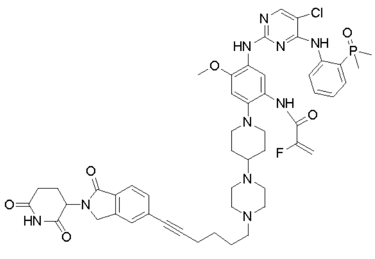 | |
| D601 | 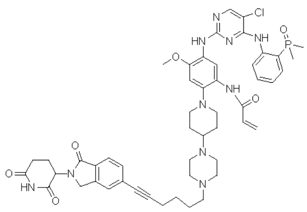 | 144.4 |

(continued)

| Compound No. | Structural formula | $IC_{50}$ (nM) |
|---|---|---|
| D602 | 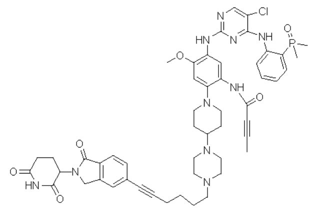 | 57.25 |
| D603 | 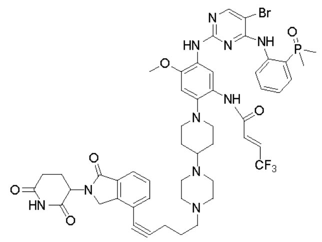 | 37.07 |
| D604 | 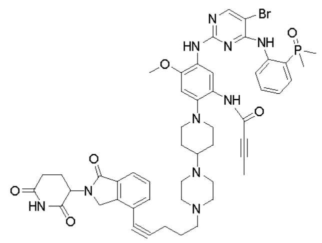 | 44.41 |
| D620 | 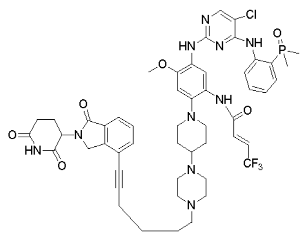 | 46.68 |
| D622 | 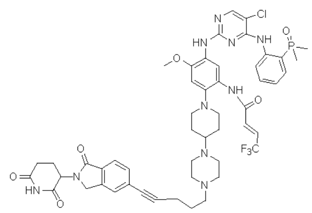 | 257.6 |
| D623 | 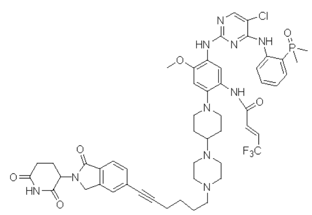 | 94.31 |
| D628 | 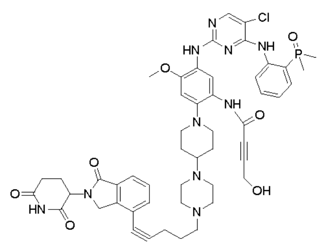 | 325.3 |

(continued)

| Compound No. | Structural formula | $IC_{50}$ (nM) |
|---|---|---|
| D630 | 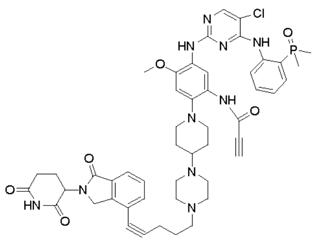 | |
| D631 | 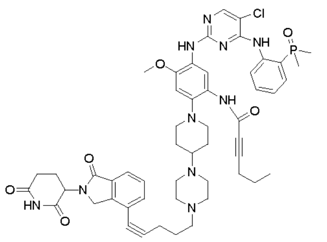 | 48.15 |
| D632 | 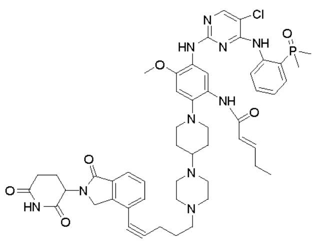 | |
| D633 | 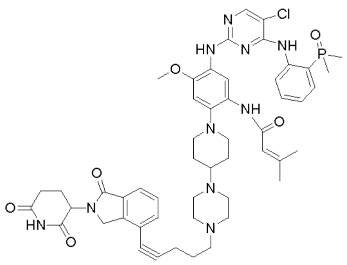 | 56.48 |
| D665 | 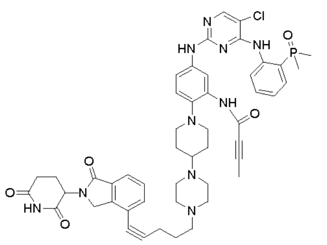 | 10.34 |
| D666 | 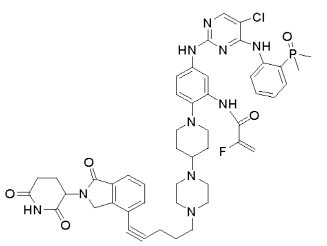 | 13.74 |

(continued)

| Compound No. | Structural formula | $IC_{50}$ (nM) |
|---|---|---|
| D667 | 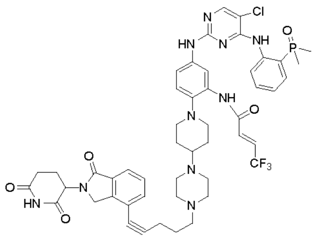 | 18.15 |
| D668 | 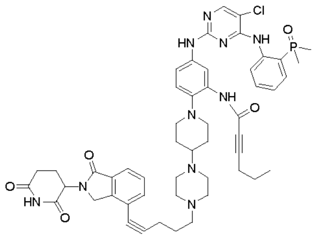 | 21.16 |
| D670 | 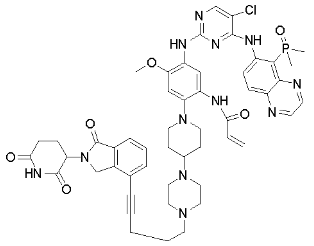 | 25.74 |
| D671 | 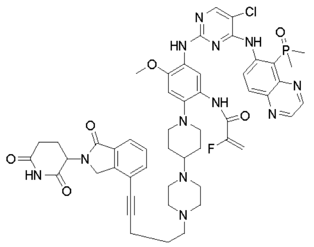 | 14.51 |
| D672 | 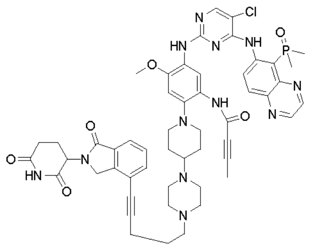 | 15.05 |
| D673 | 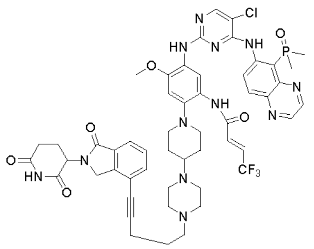 | 20.63 |

(continued)

| Compound No. | Structural formula | $IC_{50}$ (nM) |
|---|---|---|
| D682 | 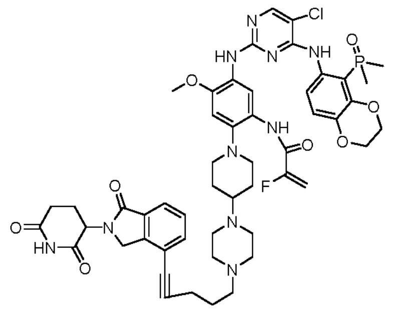 | 14.63 |
| D683 | 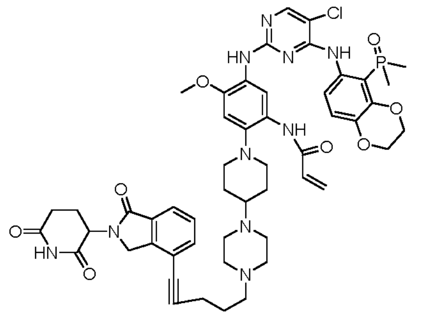 | 22.26 |
| D684 | 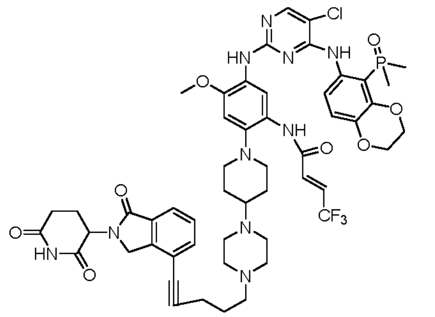 | 24.05 |
| D685 | 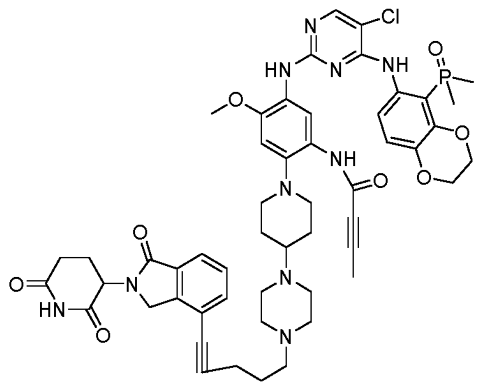 | 22.47 |
| D741 | 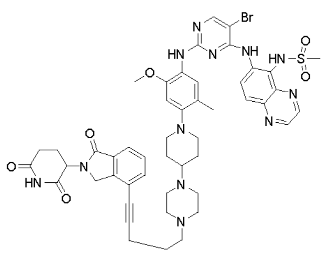 | 10.17 |

(continued)

| Compound No. | Structural formula | IC$_{50}$ (nM) |
|---|---|---|
| D743 | 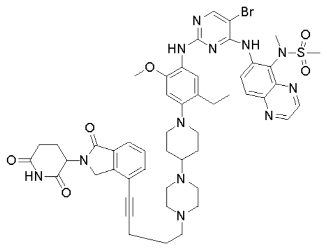 | |
| D744 | 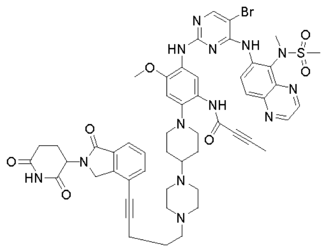 | |
| D746 | 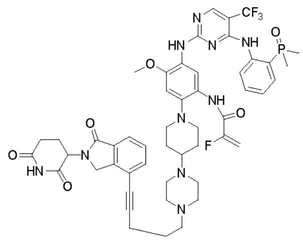 | 3.361 |
| D747 | 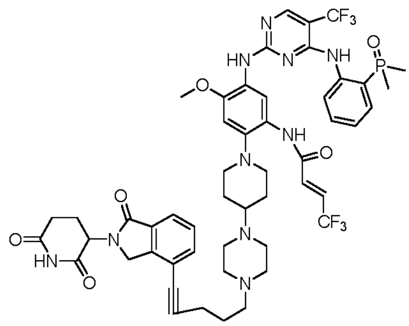 | 8.771 |
| D748 | 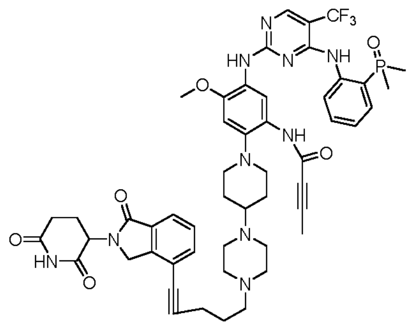 | 7.757 |

(continued)

| Compound No. | Structural formula | $IC_{50}$ (nM) |
|---|---|---|
| D755 | 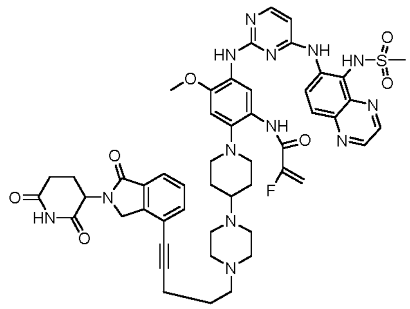 | |
| D756 | 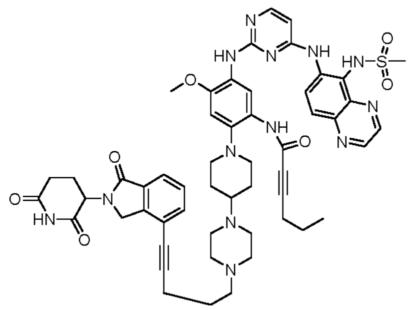 | |
| D757 | 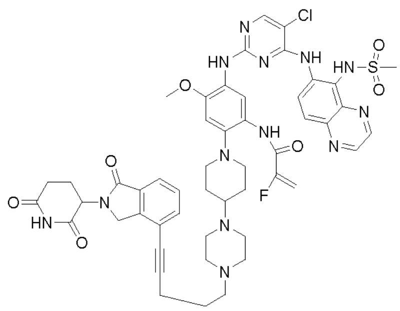 | |
| D758 | 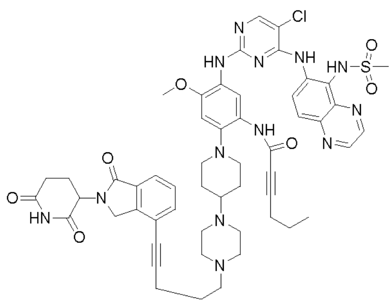 | |
| D835 | 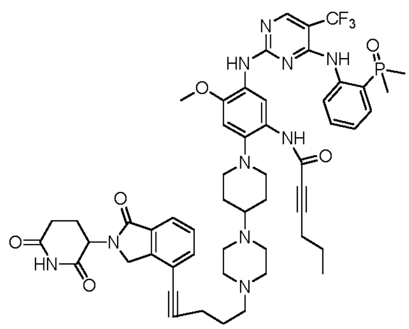 | 11.85 |

(continued)

| Compound No. | Structural formula | $IC_{50}$ (nM) |
|---|---|---|
| D845 | 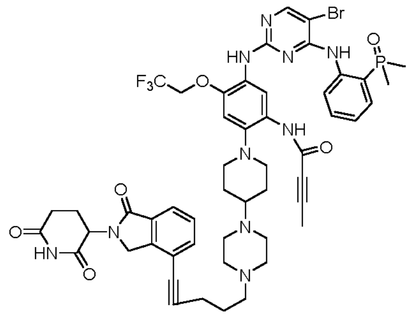 | 16.92 |
| D846 | 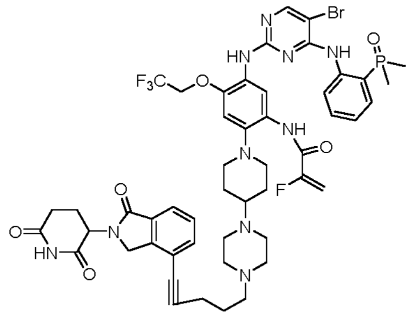 | 11.89 |
| D847 | 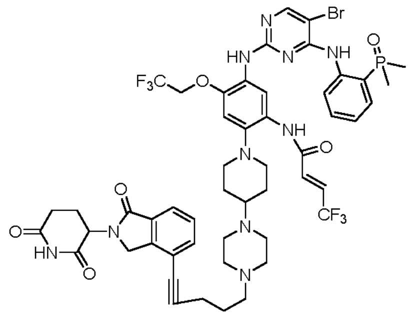 | 16.26 |
| D856 | 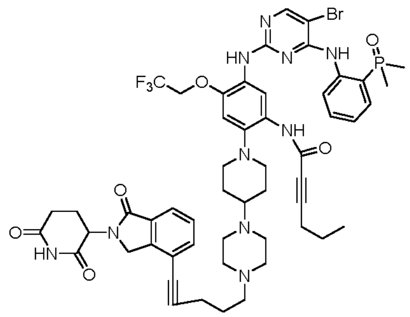 | 27.43 |
| D870 | 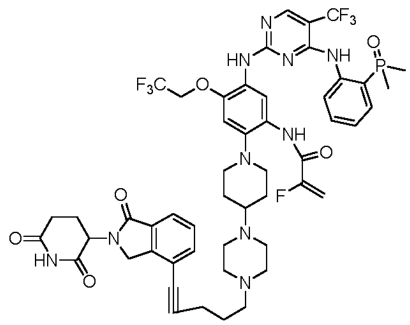 | 8.870 |

(continued)

| Compound No. | Structural formula | $IC_{50}$ (nM) |
|---|---|---|
| D932 | 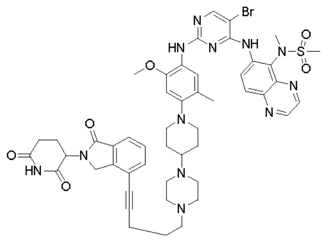 | 36.73 |
| D933 | 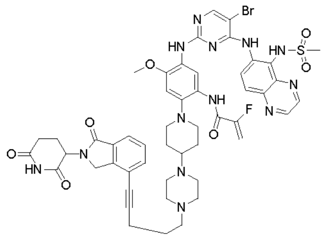 | 12.29 |
| D934 | 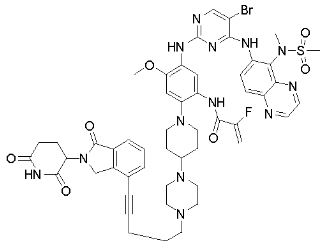 | 31.87 |
| D966 | 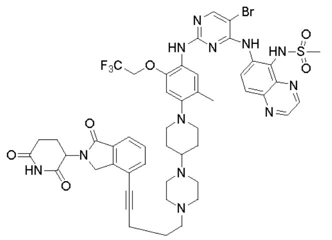 | |
| D967 | 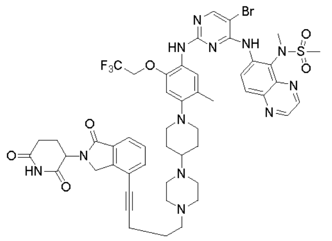 | |
| D968 | 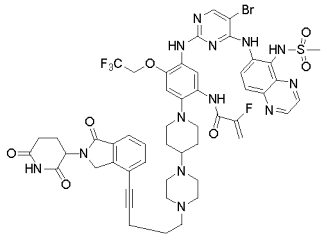 | |

(continued)

| Compound No. | Structural formula | $IC_{50}$ (nM) |
|---|---|---|
| D969 | 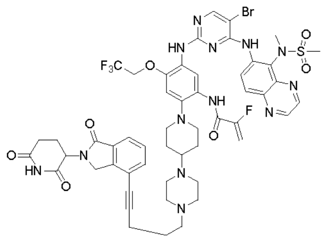 | |
| D970 | 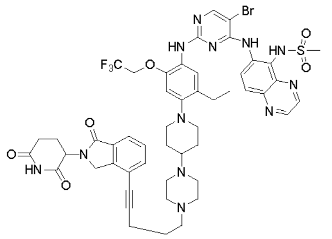 | |
| D971 | 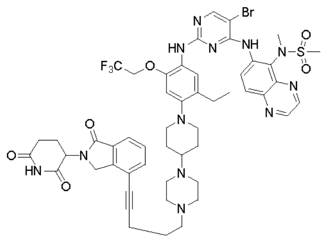 | |
| D972 | 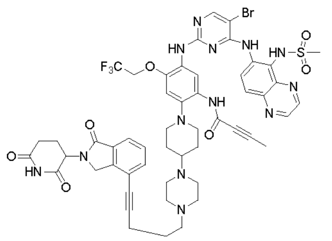 | |
| D973 | 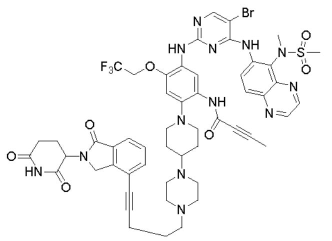 | |
| D974 | 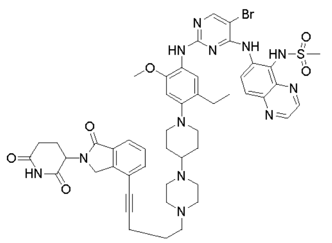 | |

(continued)

| Compound No. | Structural formula | $IC_{50}$ (nM) |
|---|---|---|
| D975 | 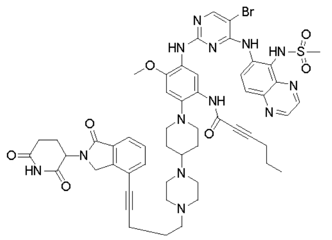 | |
| D976 | 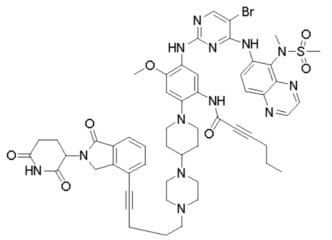 | |
| D977 | 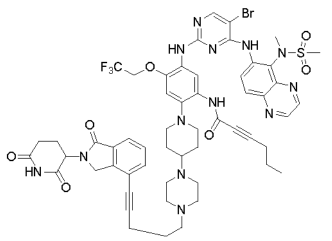 | |
| D978 | 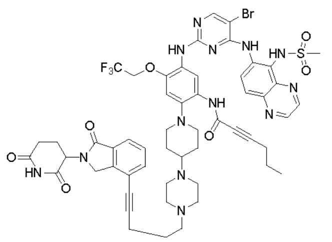 | |
| D979 | 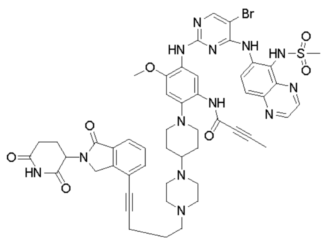 | |
| D980 | 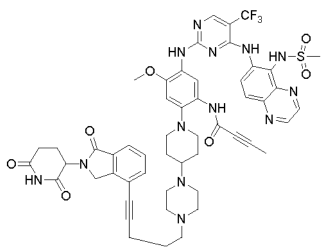 | |

(continued)

| Compound No. | Structural formula | $IC_{50}$ (nM) |
|---|---|---|
| D981 | 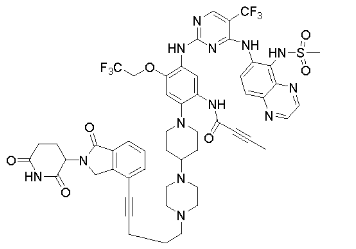 | |
| D982 | 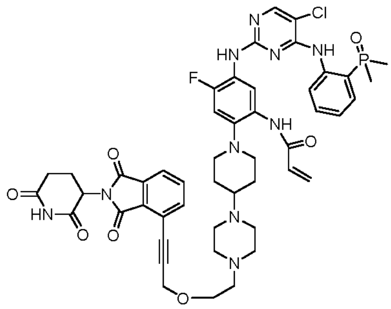 | |
| D983 | 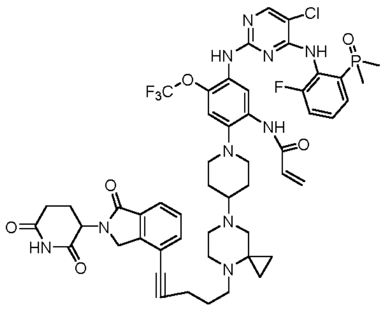 | |
| D984 | 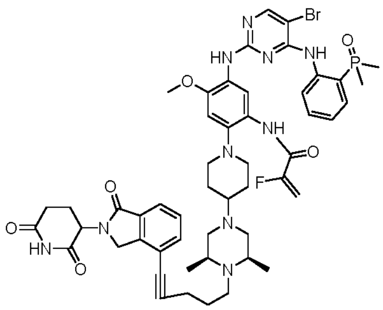 | |
| D985 | 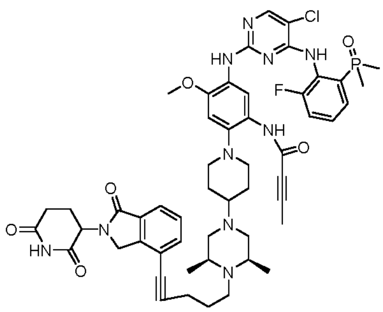 | |

(continued)

| Compound No. | Structural formula | $IC_{50}$ (nM) |
|---|---|---|
| Comparative compound L495 | 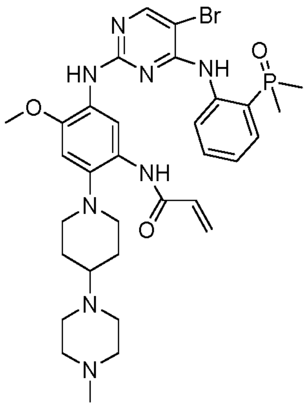 | 3742 |
| Comparative compound L521 | 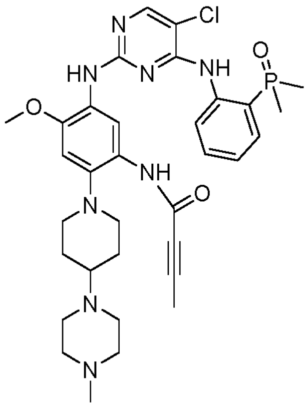 | 3048 |
| Comparative compound L527 | 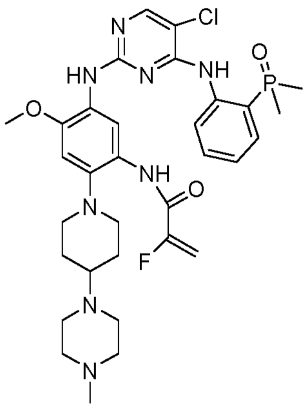 | 457.9 |
| Brigatinib | 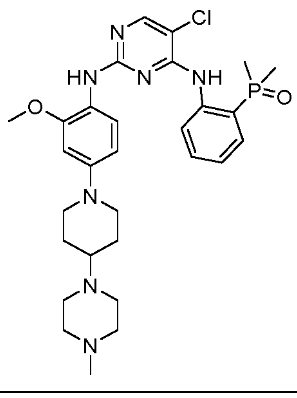 | 354.7 |

(continued)

| Compound No. | Structural formula | $IC_{50}$ (nM) |
|---|---|---|
| Osimertinib | 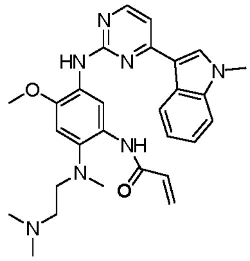 | 1875 |

Conclusion: From the assay results in Table 1, it can be seen that the compounds of the present disclosure have better anti-proliferative activity on Ba/F3 ($EGFR^{L858R/T790M/C797S}$) cells, and the anti-proliferative activity of most compounds is better than that of the positive drug Brigatinib. By comparing the representative compounds D058 and L521, it was found that the anti-proliferative activity of D058 on Ba/F3 ($EGFR^{L858R/T790M/C797S}$) cells was significantly better than that of L521; by comparing the representative compounds D478 and L495, it was found that the anti-proliferative activity of D478 on Ba/F3 ($EGFR^{L858R/T790M/C797S}$) cells was significantly better than that of L495. It can be seen that the compound of the present disclosure, that is, the EGFR degrader, has potent anti-proliferative activity on Ba/F3 ($EGFR^{L858R/T790M/C797S}$) cells, and is superior to the anti-proliferative activity of its corresponding small molecule ligand, providing a strong basis for overcoming Osimertinib resistance.

**Assay of H1975 ($EGFR^{L858R/T790M}$) cell activity**

In order to further illustrate that the compounds of the present disclosure can also overcome the drug resistance caused by EGFR T790M mutation, H1975 ($EGFR^{L858R/T790M}$) cells were used as the test cell line, and representative compounds D058 and D478 were selected to test the anti-proliferative activity of the compounds of the present disclosure.

1. Assay method

1) H1975 ($EGFR^{L858R/T790M}$) cells were cultured according to the requirements of ATCC, incubated in an incubator at 37°C and 5% $CO_2$, and analyzed by index; cells with viability of > 90% can be used in the assay, and cells were seeded in a 384-well plate (PerkinElmer, 6007680) with 500 cells/well, 30 μL/well.
2) A compound stock solution was prepared, and then diluted 3x to give a compound dilution. 30 nL of the compound dilution was added to a 384-well plate by Echo (Labcyte, Echo550). The cells were incubated in an incubator at 37°C and 5% $CO_2$ for 72h.
3) 30 μL of CTG was added to each well, and the 384-well plate was shaken on a Plate shaker (QILINBEIER, QB-9002). The 384-well plate was incubated at 37°C and 5% $CO_2$ in the dark for 30 min, and the chemiluminescence value was read by Envision (PerkinElmer, EnVision 2104).

2. Data Analysis

The percent inhibition rate (% inhibition) was calculated by the following formula

$$\text{Inhibition}(\%) = \left[1 - \frac{LUM_{cmpd} - \overline{LUM}_{positive}}{\overline{LUM}_{vehicle} - \overline{LUM}_{positive}}\right] \times 100$$

$LUM_{cmpd}$: Luminescence value of assay compound.
$\overline{LUM}_{positive}$ : Average LUM value of positive drug with a concentration of 10 μM.
$\overline{LUM}_{vehicle}$ : Average LUM value of negative control group without drug treatment.

The nonlinear regression curve (dose response-variable slope) between the value of inhibition rate (%) and the logarithm of compound concentration was fitted with graphpad prism 8.0, the effect dose curve of compound was drawn,

and the $IC_{50}$ value was calculated.

$$Y = Bottom + (Top-Bottom)/(1+10^((LogIC_{50}-X)*HillSlope))$$

X-axis: logarithm of compound concentration; Y axis: inhibition rate (% inhibition).

3. Assay result

$IC_{50}$ values of the antiproliferative activity of the representative compounds of the present disclosure on H1975 ($EGFR^{L858R/T790M}$) cells are shown in the table 2.

Table 2: Antiproliferative activity of compounds on H1975 ($EGFR^{L858R/T790M}$) cells

| Compound No. | Structural formula | $IC_{50}$ (nM) |
|---|---|---|
| D058 | 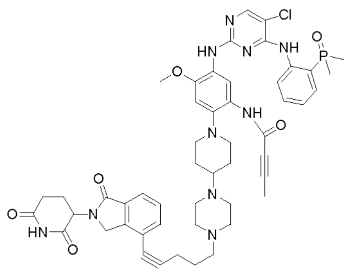 | 14.60 |
| D478 | 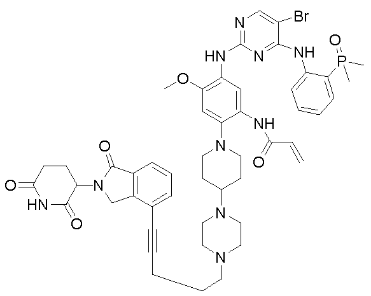 | 17.64 |
| Comparative compound C126 | 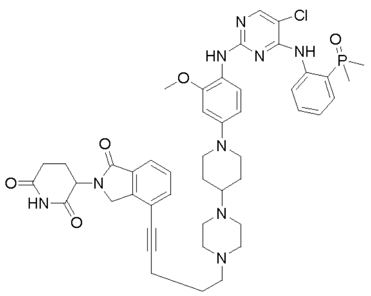 | 1388 |
| Brigatinib | 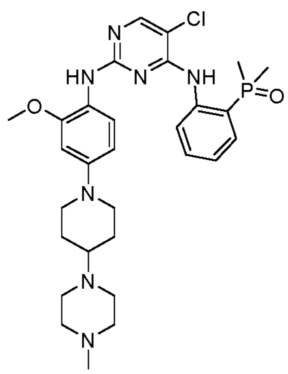 | 747.0 |

(continued)

| Compound No. | Structural formula | $IC_{50}$ (nM) |
|---|---|---|
| Gefitinib | 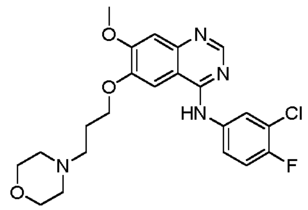 | >10000 |
| Osimertinib | 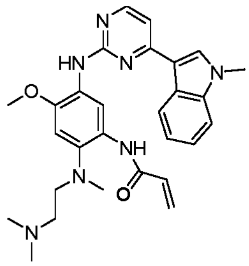 | 10.73 |

Conclusion: From the assay results in Table 2, it can be seen that the representative compounds D058 and D478 of the present disclosure have better anti-proliferative activity against H1975 ($EGFR^{L858R/T790M}$), and their anti-proliferative activities are significantly better than that of the comparative compound C126. It can be seen that the compound of the present disclosure, that is, the EGFR degrader, can overcome the drug resistance caused by the EGFR T790M mutation.

**Assay of activity of compounds for inducing $EGFR^{L858R/T790M/C797S}$ and $EGFR^{Del19/T790M/C797S}$ protein degradation**

In order to verify that the compounds of the present disclosure can induce the degradation of EGFR, Ba/F3 ($EGFR^{L858R/T790M/C797S}$) and Ba/F3 ($EGFR^{Del19/T790M/C797S}$) cells were used as test cell lines, and representative compounds D058, D078 and D552 were selected to study the mechanism of action of the compounds and observe their effects on the protein levels of $EGFR^{L858R/T790M/C797S}$ and $EGFR^{Del19/T790M/C797S}$.

(1) Cell culture:

Ba/F3 ($EGFR^{L858R/T790M/C797S}$) and Ba/F3 ($EGFR^{Del19/T790M/C797S}$) cells were cultured according to the culture conditions recommended by ATCC, and analyzed by index.
Complete medium: 1640 medium, 10% FBS, 1x glutamine, 1x penicillin-streptomycin.
Culture conditions: incubated at 37°C, 95% air, 5% $CO_2$ incubator.

(2) Compound stock solution: 10 mM stock solution in DMSO, stored at -20°C.
(3) Preparation of cell suspension:
The cells in the cell culture bottle were collected, and the cells with viability of > 90% can be used in the assay. Cells were seeded in a 96-well plate with 40 μL cells, $1*10^5$ cells/well.
(4) Compound treatment:
The compounds were serially diluted 3x with DMSO, with the starting concentrations of the compounds being 1.0 mM ($EGFR^{L858R/T790M/C797S}$ assay) and 10 mM ($EGFR^{Del19/T790M/C797S}$ assay), respectively, into 10 concentrations to prepare working solutions.
(5) Compound was pipetted into the 96-well plate to treat cells in a 37°C, 95% air, 5% $CO_2$ incubator for 24h.
(6) Detection:

1) 1 μg/mL EGF stimulated cells for 10 min;
2) After the compound treatment was completed, lysis buffer was added to lyse cells; 10 μL of cell lysate was transferred to the 384-well plate; in addition, control lysate, negative control and 5 μL of acceptor mix were added to the 384-well plate; The plate was shaken on a shaker for 1-2 min;
3) 5 μL of donor mix was added to each well; the 384-well plate was sealed, shaken on a shaker for 1-2 min, and

left in the dark at room temperature overnight; then the plate was read with ELISA reader.

(7) Data analysis:
Alpha counts were fitted by logarithmic treatment of compound concentration with Graphpad Prism 8.0.

$Y=Bottom + (Top-Bottom)/(1+10^{((LogIC_{50}-X)*HillSlope)})$
X: logarithm of compound concentration; Y: Alpha Counts.

(8) Assay result:
The effects of the compounds of the present disclosure on the protein levels of mutant $EGFR^{L858R/T790M/C797S}$ and $EGFR^{Del19/T790M/C797S}$ are shown in Figs. 1, 2 and 3.

As shown in Fig. 1, the assay results show that in the range of 1 nM to 1000 nM, as the concentration of compound D078 increases, the level of $EGFR^{L858R/T790M/C797S}$ protein decreases. Compound D078 significantly reduces the level of $EGFR^{L858R/T790M/C797S}$ protein with $DC_{50}$ of 84.74 nM, proving that compound D078 has a significant degradation effect on $EGFR^{L858R/T790M/C797S}$ protein in a dose-dependent manner; while Osimertinib and Brigatinib have no degradation effect on $EGFR^{L858R/T790M/C797S}$ protein.

As shown in Fig. 2, the assay results show that in the range of 1 nM to 1000 nM, as the concentration of compound D552 increases, the level of $EGFR^{L858R/T790M/C797S}$ protein decreases. Compound D552 significantly reduces the level of $EGFR^{L858R/T790M/C797S}$ protein with $DC_{50}$ of 117.1 nM, proving that compound D552 has a significant degradation effect on $EGFR^{L151R/T790M/C797S}$ protein in a dose-dependent manner; while the comparative compound L527 has no degradation effect on $EGFR^{L858R/T790M/C797S}$ protein.

As shown in Fig. 3, the assay results show that in the range of 1 nM to 10000 nM, as the concentration of compound D058 or D552 increases, the level of $EGFR^{Del19/T790M/C797S}$ protein decreases. Compounds D058 and D552 significantly decrease the levels of $EGFR^{Del19/T790M/C797S}$ protein, respectively, with $DC_{50}$ of 25.06 nM and 29.71 nM respectively, proving that compounds D058 and D552 respectively have significant degradation effects on $EGFR^{Del19/T790M/C797S}$ protein in a dose-dependent manner, while Osimertinib has no degradation effect on $EGFR^{Del19/T790M/C797S}$ protein.

Conclusion: The compound of the present disclosure can significantly induce the degradation of $EGFR^{Del19/T790M/C797S}$ and $EGFR^{L858R/T790M/C797S}$ proteins in cells in a dose-dependent manner.

## Claims

1. A compound of formula (A), or a pharmaceutically acceptable salt, enantiomer, diastereomer, racemate, solvate, hydrate, polymorph, prodrug or isotopic variant thereof, or a mixture thereof:

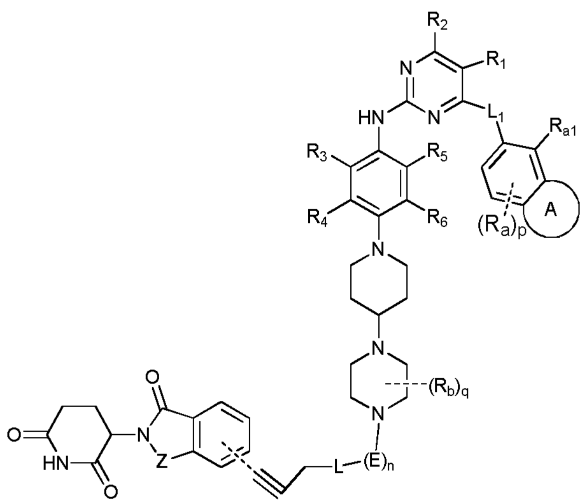

(A)

wherein

----- represents that the point of attachment to the rest of the molecule can be located at an available point of a ring

where it is located;
Ring A is absent, or is selected from $C_{4-10}$ cycloalkyl, 5-8 membered heterocyclyl, $C_{6-10}$ aryl and 5-14 membered heteroaryl;
$R_1$ is selected from H, halogen, -OH, -$NH_2$, -CN, $C_{1-6}$ alkyl and $C_{1-6}$ haloalkyl;
$R_2$ is selected from H, halogen, -OH, -$NH_2$, -CN, $C_{1-6}$ alkyl and $C_{1-6}$ haloalkyl;
$R_3$ is selected from H, halogen, -OH, -$NH_2$, -CN, $C_{1-6}$ alkyl, $C_{1-6}$ haloalkyl, -$OC_{1-6}$ alkyl, -$OC_{1-6}$ haloalkyl, $C_{2-6}$ alkenyl and $C_{2-6}$ alkynyl;
$R_4$ is selected from H, halogen, -OH, -$NH_2$, -CN, $C_{1-6}$ alkyl and $C_{1-6}$ haloalkyl;
$R_5$ is selected from H, halogen, -OH, -$NH_2$, -CN, $C_{1-6}$ alkyl and $C_{1-6}$ haloalkyl;
$R_6$ is selected from $C_{1-6}$ alkyl, $C_{1-6}$ haloalkyl, -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, -$OR_c$, -$NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_{a1}$ is selected from

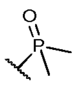
and
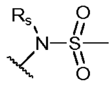
;

$R_s$ is selected from H, $C_{1-6}$ alkyl and $C_{1-6}$ haloalkyl;
$R_a$ is selected from H, halogen, -OH, $C_{1-6}$ alkyl, $C_{1-6}$ haloalkyl, $C_{2-6}$ alkenyl, $C_{2-6}$ alkynyl, -CN, - $NR_cR_d$, -$NH(CH_2)_{0-5}R_c$ and -$NHC(O)(CH_2)_{0-5}R_c$;
alternatively, two $R_a$ on the same carbon atom or on different carbon atoms are taken together to form a $C_{4-10}$ cycloalkyl, a 5-8 membered heterocyclyl, a $C_{6-10}$ aryl, or a 5-10 membered heteroaryl;
$R_b$ is selected from H, halogen, -OH, -$NH_2$, -CN, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl, a 3-10 membered heterocyclyl, a $C_{6-10}$ aryl, or a 5-10 membered heteroaryl;
$R_c$ is selected from H, -OH, -$NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, -$NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;
L is selected from a chemical bond, -O-, -NR#-, -CR#R#'-, -S-, -SO- and -$S(O)_2$-;
$L_1$ is selected from a chemical bond, -O-, -NR#-, -CR#R#'-, -S-, -SO- and -$S(O)_2$-;
E is selected from a chemical bond and -CR#R#'-;
wherein R# and R#' are independently selected from H, halogen, -OH, -$NH_2$, -CN, $C_{1-6}$ alkyl, $C_{1-6}$ haloalkyl, $C_{2-6}$ alkenyl, and $C_{2-6}$ alkynyl;
p is 0, 1, 2, 3, or 4;
q is 0, 1, 2, 3, or 4;
n is 0, 1, 2, 3, 4, or 5.

**2.** A compound of formula (X), or a pharmaceutically acceptable salt, enantiomer, diastereomer, racemate, solvate, hydrate, polymorph, prodrug or isotopic variant thereof, or a mixture thereof:

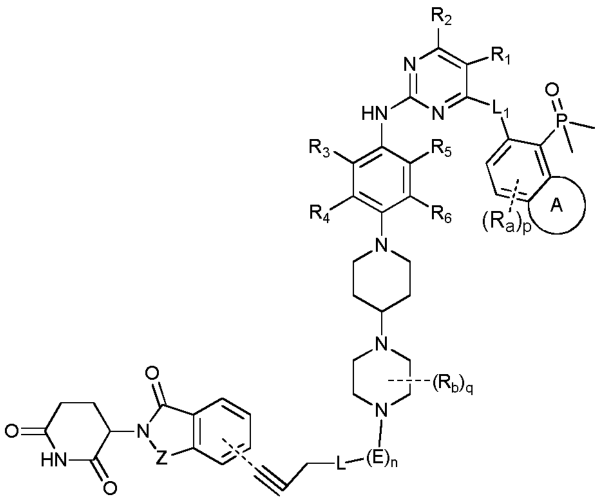

(X)

wherein

-----represents that the point of attachment to the rest of the molecule can be located at an available point of a ring where it is located;
Ring A is absent, or is selected from $C_{4-10}$ cycloalkyl, 5-8 membered heterocyclyl, $C_{6-10}$ aryl and 5-14 membered heteroaryl;
$R_1$ is selected from H, halogen, -OH, $-NH_2$, -CN, $C_{1-6}$ alkyl and $C_{1-6}$ haloalkyl;
$R_2$ is selected from H, halogen, -OH, $-NH_2$, -CN, $C_{1-6}$ alkyl and $C_{1-6}$ haloalkyl;
$R_3$ is selected from H, halogen, -OH, $-NH_2$, -CN, $C_{1-6}$ alkyl, $C_{1-6}$ haloalkyl, $-OC_{1-6}$ alkyl, $-OC_{1-6}$ haloalkyl, $C_{2-6}$ alkenyl and $C_{2-6}$ alkynyl;
$R_4$ is selected from H, halogen, -OH, $-NH_2$, -CN, $C_{1-6}$ alkyl and $C_{1-6}$ haloalkyl;
$R_5$ is selected from H, halogen, -OH, $-NH_2$, -CN, $C_{1-6}$ alkyl and $C_{1-6}$ haloalkyl;
$R_6$ is selected from -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, $-OR_c$, $-NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_a$ is selected from H, halogen, -OH, $C_{1-6}$ alkyl, $C_{1-6}$ haloalkyl, $C_{2-6}$ alkenyl, $C_{2-6}$ alkynyl, -CN, - $NR_cR_d$, $-NH(CH_2)_{0-5}R_c$ and $-NHC(O)(CH_2)_{0-5}R_c$;
alternatively, two $R_a$ on the same carbon atom or on different carbon atoms are taken together to form a $C_{4-10}$ cycloalkyl, a 5-8 membered heterocyclyl, a $C_{6-10}$ aryl, or a 5-10 membered heteroaryl;
$R_b$ is selected from H, halogen, -OH, $-NH_2$, -CN, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl, a 3-10 membered heterocyclyl, a $C_{6-10}$ aryl, or a 5-10 membered heteroaryl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;
L is selected from a chemical bond, -O-, -NR#-, -CR#R#'-, -S-, -SO- and $-S(O)_2-$;
$L_1$ is selected from a chemical bond, -O-, -NR#-, -CR#R#'-, -S-, -SO- and $-S(O)_2-$;
E is selected from a chemical bond and -CR#R#'-;
wherein R# and R#' are independently selected from H, halogen, -OH, $-NH_2$, -CN, $C_{1-6}$ alkyl, $C_{1-6}$ haloalkyl, $C_{2-6}$ alkenyl, and $C_{2-6}$ alkynyl;
p is 0, 1, 2, 3, or 4;

q is 0, 1, 2, 3, or 4;
n is 0, 1, 2, 3, 4, or 5.

**3.** The compound of claim 2, or a pharmaceutically acceptable salt, enantiomer, diastereomer, racemate, solvate, hydrate, polymorph, prodrug or isotopic variant thereof, or a mixture thereof, wherein the compound has the following general structure:

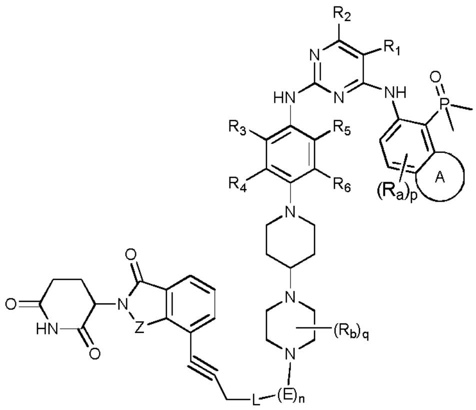
(I),

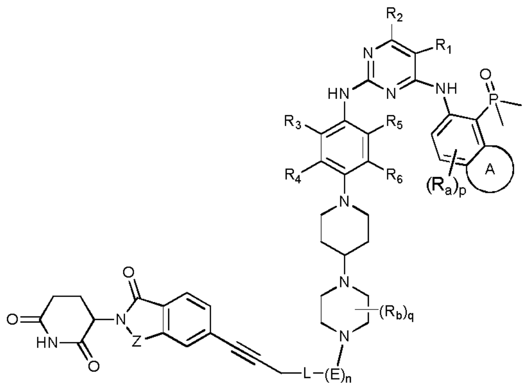
(II),

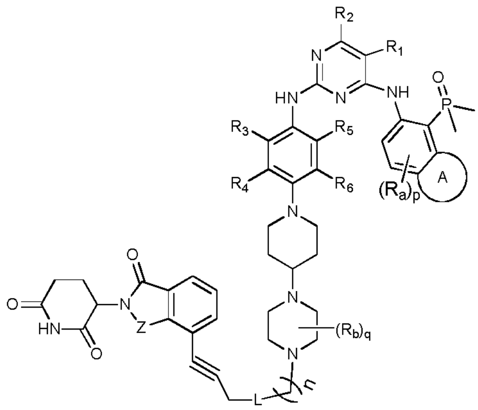
(III),

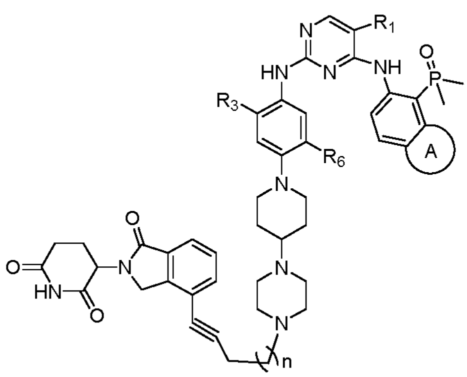
(IV),

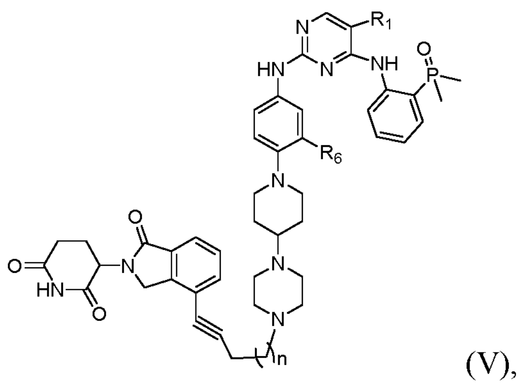
(V),

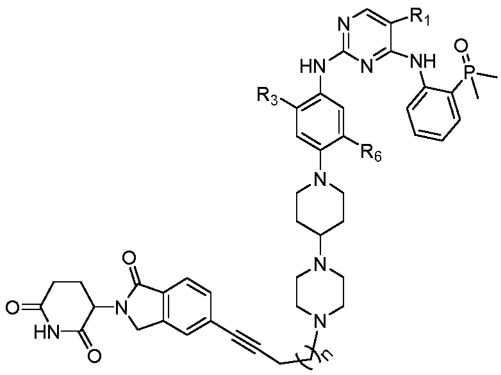
(VI),

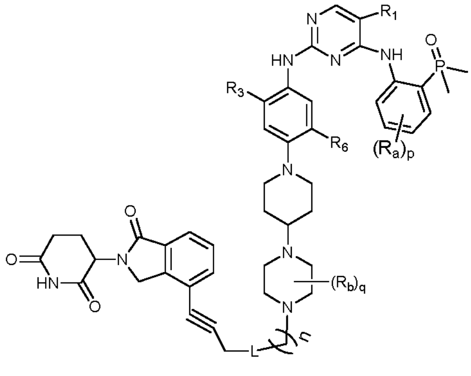

(VII),

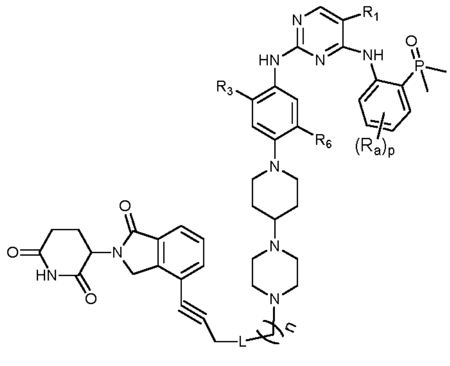

(VIII),

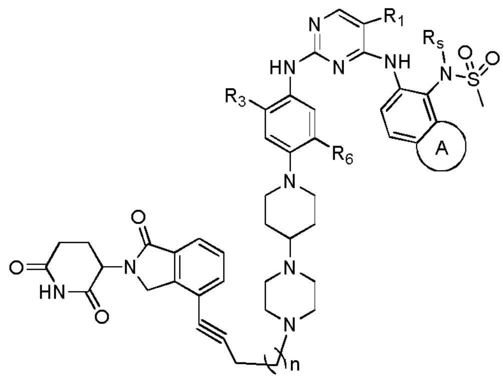

(IX),

wherein each group is as defined in claim 2.

**4.** The compound of claim 3, or a pharmaceutically acceptable salt, enantiomer, diastereomer, racemate, solvate, hydrate, polymorph, prodrug or isotopic variant thereof, or a mixture thereof, which has a structure of formula (III):

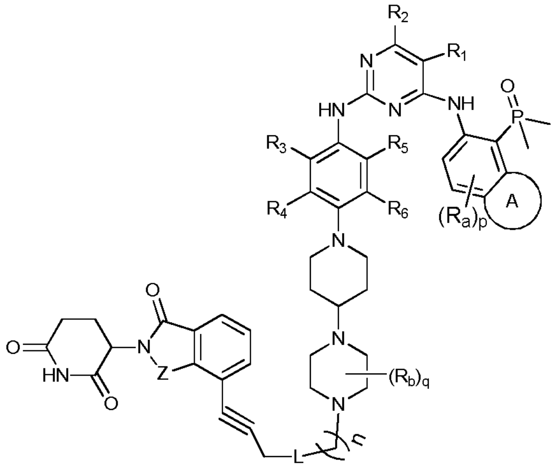

(III)

wherein

Ring A is absent, or is selected from $C_{4-10}$ cycloalkyl, 5-8 membered heterocyclyl, $C_{6-10}$ aryl and 5-10 membered heteroaryl;
$R_1$ is selected from H, halogen, $C_{1-6}$ alkyl and $C_{1-6}$ haloalkyl;
$R_2$ is selected from H, halogen, $C_{1-6}$ alkyl and $C_{1-6}$ haloalkyl;
$R_3$ is selected from H, halogen, $C_{1-6}$ alkyl, $C_{1-6}$ haloalkyl, $-OC_{1-6}$ alkyl, $-OC_{1-6}$ haloalkyl, $C_{2-6}$ alkenyl and $C_{2-6}$ alkynyl,
$R_4$ is selected from H, halogen, $C_{1-6}$ alkyl and $C_{1-6}$ haloalkyl;

$R_5$ is selected from H, halogen, $C_{1-6}$ alkyl and $C_{1-6}$ haloalkyl;
$R_6$ is selected from -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl, wherein the $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl are each optionally substituted with 0, 1, 2, 3, 4 or 5 R*, as long as the valence permits;
R* is selected from H, halogen, $-OR_c$, $-NR_cR_d$, -CN, $C_{1-6}$ alkyl, $C_{1-6}$ haloalkyl, $C_{2-6}$ alkenyl, $C_{2-6}$ alkynyl, $C_{3-10}$ cycloalkyl, 3-10 membered heterocyclyl, $C_{6-10}$ aryl and 5-10 membered heteroaryl;
Z is $CH_2$, C=O, C=S or C=NH;
$R_a$ is selected from H, halogen, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$;
$R_b$ is selected from H, halogen, $C_{1-6}$ alkyl, $C_{1-6}$ haloalkyl, $C_{2-6}$ alkenyl and $C_{2-6}$ alkynyl;
alternatively, two $R_b$ on the same carbon atom or on different carbon atoms are taken together to form a $C_{3-10}$ cycloalkyl or a 3-10 membered heterocyclyl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
alternatively, $R_c$ and $R_d$ are taken together with the N atom to which they are attached to form 3-10 membered heterocyclyl;
L is selected from a chemical bond, -O-, -NR#-, -S-, -SO- and $-S(O)_2-$,
wherein R# is selected from H, halogen, $C_{1-6}$ alkyl, $C_{1-6}$ haloalkyl, $C_{2-6}$ alkenyl and $C_{2-6}$ alkynyl;
p is 0, 1, 2, 3, or 4;
q is 0, 1, 2, 3, or 4;
n is 0, 1, 2, 3, 4, or 5.

**5.** The compound of claim 4, or a pharmaceutically acceptable salt, enantiomer, diastereomer, racemate, solvate, hydrate, polymorph, prodrug or isotopic variant thereof, or a mixture thereof, wherein:

Ring A is absent, or is selected from $C_{4-10}$ cycloalkyl, 5-8 membered heterocyclyl, $C_{6-10}$ aryl and 5-10 membered heteroaryl;
$R_1$ is selected from H, halogen and $C_{1-6}$ haloalkyl;
$R_2$ is selected from H and halogen;
$R_3$ is selected from H, halogen, $-OC_{1-6}$ alkyl and $-OC_{1-6}$ haloalkyl;
$R_4$ is selected from H and halogen;
$R_5$ is selected from H and halogen;
$R_6$ is selected from -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{3-10}$ cycloalkyl, 3-7 membered heterocyclyl, $C_{6-10}$ aryl and 5-10 membered heteroaryl, wherein the $C_{1-6}$ alkyl, $C_{1-6}$ haloalkyl, $C_{3-10}$ cycloalkyl, 3-7 membered heterocyclyl, $C_{6-10}$ aryl and 5-10 membered heteroaryl are each optionally substituted with 0, 1, 2 or 3 R*, as long as the valence permits;
R* is selected from H, halogen, $-OR_c$, $-NR_cR_d$, -CN, $C_{1-6}$ alkyl, $C_{1-6}$ haloalkyl, $C_{3-7}$ cycloalkyl, 5-6 membered heterocyclyl, $C_{6-10}$ aryl and 5-6 membered heteroaryl;
Z is selected from $CH_2$, C=O and C=S;
$R_a$ is selected from H, halogen, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$;
$R_b$ is selected from H, $C_{1-6}$ alkyl and $C_{1-6}$ haloalkyl;
alternatively, two $R_b$ on the same carbon atom are taken together to form a $C_{3-7}$ cycloalkyl or a 5-6 membered heterocyclyl;
$R_c$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
$R_d$ is selected from H, -OH, $-NH_2$, halogen, $C_{1-6}$ alkyl, $C_{1-6}$ haloalkyl, $C_{3-10}$ cycloalkyl, 3-10 membered heterocyclyl, $C_{6-10}$ aryl and 5-10 membered heteroaryl;
or $R_c$ and $R_d$ are taken together with the N atom to which they are attached to form 3-7 membered heterocyclyl;
L is selected from a chemical bond, -O-, -S- and -NR#-;
wherein R# is selected from H, halogen, $C_{1-4}$ alkyl and $C_{1-4}$ haloalkyl;
p is 0, 1, 2 or 3;

q is 0, 1, 2 or 3;
n is 1, 2, 3, 4 or 5.

**6.** The compound of claim 4, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from a 5-6 membered heteroaryl and a 5-6 membered heterocyclyl;
$R_1$ is selected from halogen and $C_{1-4}$ haloalkyl;
$R_2$ is H;
$R_3$ is selected from H, halogen, $-OC_{1-4}$ alkyl and $-OC_{1-4}$ haloalkyl;
$R_4$ is H;
$R_5$ is H;
$R_6$ is selected from -NH-C(O)-CR=CR'R" and -NH-C(O)-C≡CR';
wherein R is selected from H, halogen, $C_{1-4}$ alkyl and $C_{6-10}$ aryl, wherein the $C_{1-4}$ alkyl and $C_{6-10}$ aryl are each optionally substituted with 0, 1, or 2 R*;
R' and R" are each independently selected from H, $C_{1-4}$ alkyl, $C_{1-4}$ haloalkyl, $C_{3-7}$ cycloalkyl, 5-6 membered heterocyclyl, $C_{6-10}$ aryl and 5-6 membered heteroaryl, wherein the $C_{1-4}$ alkyl, $C_{1-4}$ haloalkyl, $C_{3-7}$ cycloalkyl, 5-6 membered heterocyclyl, $C_{6-10}$ aryl and 5-6 membered heteroaryl are each optionally substituted with 0, 1 or 2 R*, as long as the valence permits;
R* is selected from H, halogen, $-OR_c$, $-NR_cR_d$, $C_{1-4}$ alkyl, $C_{1-4}$ haloalkyl, $C_{3-7}$ cycloalkyl, 5-6 membered heterocyclyl, $C_{6-10}$ aryl and 5-6 membered heteroaryl;
Z is $CH_2$ or C=O;
$R_a$ is selected from H, halogen, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$, alternatively H, halogen, $-NR_cR_d$ and $-NH(CH_2)_{0-5}R_c$, still alternatively H and halogen;
$R_b$ is selected from H and $C_{1-4}$ alkyl;
alternatively, two $R_b$ on the same carbon atom are taken together to form a $C_{3-7}$ cycloalkyl group;
$R_c$ is selected from H, -OH, $-NH_2$, halogen, $C_{1-4}$ alkyl, $C_{3-10}$ cycloalkyl, 3-10 membered heterocyclyl, $C_{6-10}$ aryl and 5-10 membered heteroaryl, alternatively H, $C_{1-4}$ alkyl, $C_{3-10}$ cycloalkyl and 3-10 membered heterocyclyl, still alternatively H and $C_{1-4}$ alkyl;
$R_d$ is selected from H, -OH, $-NH_2$, halogen, $C_{1-4}$ alkyl, $C_{3-10}$ cycloalkyl, 3-10 membered heterocyclyl, $C_{6-10}$ aryl and 5-10 membered heteroaryl, alternatively H, $C_{1-4}$ alkyl, $C_{3-10}$ cycloalkyl and 3-10 membered heterocyclyl, still alternatively H and $C_{1-4}$ alkyl;
or $R_c$ and $R_d$ are taken together with the N atom to which they are attached to form 5-6 membered heterocyclyl;
L is selected from a chemical bond and -O-;
p is 0, 1, or 2;
q is 0, 1, or 2;
n is 1, 2, 3, 4 or 5.

**7.** The compound of claim 4, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from

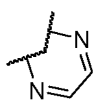 and 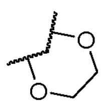 ;

$R_1$ is selected from Cl, Br and $-CF_3$;
$R_2$ is H;
$R_3$ is selected from H, F, Cl, $-OCH_3$ and $-OCF_3$; alternatively, $R_3$ is selected from H, F, $-OCH_3$ and $-OCF_3$;
$R_4$ is H;
$R_5$ is H;
$R_6$ is selected from

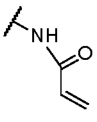, 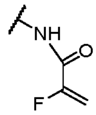, 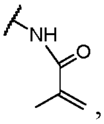, 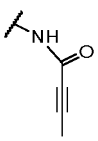, 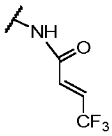, 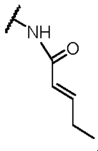, 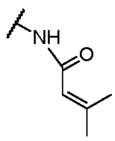, 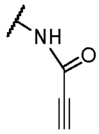,

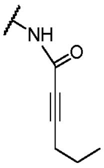, 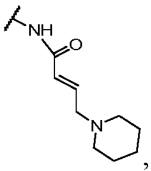, 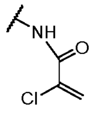, 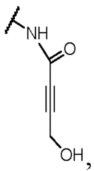, 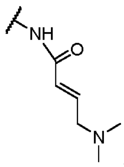, 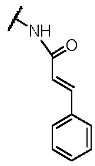, 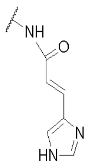, 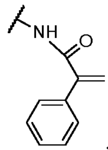, and 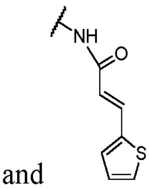;

Z is $CH_2$ or C=O;
$R_a$ is selected from H, halogen, $C_{1-6}$ alkyl, $C_{1-6}$ haloalkyl, $C_{2-6}$ alkenyl, $C_{2-6}$ alkynyl, -CN, $-NR_cR_d$, $-NH(CH_2)_{0-5}R_c$ and $-NHC(O)(CH_2)_{0-5}R_c$, alternatively H, F, Cl, $-NR_cR_d$ and $-NH(CH_2)_{0-5}R_c$, still alternatively H and F;
$R_b$ is selected from H and $-CH_3$;
alternatively, two $R_b$ on the same carbon atom are taken together to form a cyclopropyl group;
$R_c$ is selected from H, -OH, $-NH_2$, halogen, $C_{1-4}$ alkyl, $C_{3-7}$ cycloalkyl, 5-6 membered heterocyclyl, $C_{6-10}$ aryl and 5-6 membered heteroaryl, alternatively H, $C_{1-4}$ alkyl, $C_{3-7}$ cycloalkyl and 5-6 membered heterocyclyl, still alternatively H and $C_{1-4}$ alkyl;
$R_a$ is selected from H, -OH, $-NH_2$, halogen, $C_{1-4}$ alkyl, $C_{3-7}$ cycloalkyl, 5-6 membered heterocyclyl, $C_{6-10}$ aryl and 5-6 membered heteroaryl, alternatively H, $C_{1-4}$ alkyl, $C_{3-7}$ cycloalkyl and 5-6 membered heterocyclyl, still alternatively H and $C_{1-4}$ alkyl;
L is selected from a chemical bond and -O-;
p is 0 or 1;
q is 0, 1, or 2;
n is 2, 3 or 4.

**8.** The compound of claim 3, or a pharmaceutically acceptable salt, enantiomer, diastereomer, racemate, solvate, hydrate, polymorph, prodrug or isotopic variant thereof, or a mixture thereof, which has a structure of formula (IV):

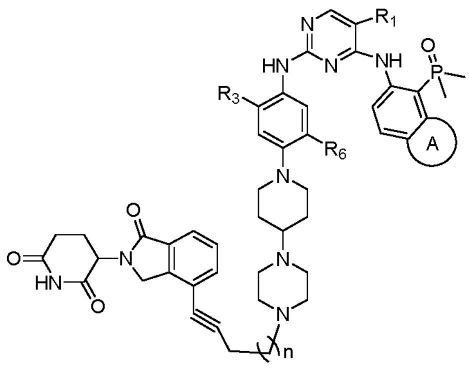

(IV)

wherein

Ring A is absent, or is selected from $C_{4-10}$ cycloalkyl, 5-8 membered heterocyclyl, $C_{6-10}$ aryl and 5-10 membered heteroaryl;
$R_1$ is selected from H, halogen and $C_{1-4}$ haloalkyl, alternatively H and halogen;
$R_3$ is selected from H, halogen, $-OC_{1-6}$ alkyl and $-OC_{1-6}$ haloalkyl, alternatively H and $-OC_{1-6}$ alkyl;
$R_6$ is selected from -NH-C(O)-CR=CR'R" and -NH-C(O)-C≡CR';
wherein R, R' and R" are each independently selected from H, halogen, $C_{1-6}$ alkyl, $C_{1-6}$ haloalkyl, $C_{2-6}$ alkenyl, $C_{2-6}$ alkynyl, 3-10 membered heterocyclyl and $C_{3-10}$ cycloalkyl, wherein the $C_{1-6}$ alkyl, $C_{1-6}$ haloalkyl, $C_{2-6}$ alkenyl, $C_{2-6}$ alkynyl, 3-10 membered heterocyclyl and $C_{3-10}$ cycloalkyl are each optionally substituted with 0, 1, 2 or 3 R*;
R* is selected from H, halogen, -OH, $-NH_2$, -CN, 3-10 membered heterocyclyl, and $C_{3-10}$ cycloalkyl;

n is 1, 2, 3 or 4.

**9.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-10 membered heteroaryl and 5-8 membered heterocyclyl;
$R_1$ is selected from halogen and $C_{1-4}$ haloalkyl, alternatively halogen;
$R_3$ is selected from H, $-OC_{1-6}$ alkyl and $-OC_{1-6}$ haloalkyl, alternatively H and $-OC_{1-6}$ alkyl;
$R_6$ is selected from -NH-C(O)-CR=CR'R" and -NH-C(O)-C≡CR';
wherein R, R' and R" are each independently selected from H, halogen, $C_{1-6}$ alkyl and $C_{1-6}$ haloalkyl, wherein the $C_{1-6}$ alkyl and $C_{1-6}$ haloalkyl are each optionally substituted with 0, 1 or 2 R*;
R* is selected from H, halogen, -OH and 3-10 membered heterocyclyl;
n is 1, 2, 3 or 4.

**10.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-6 membered heteroaryl and 5-6 membered heterocyclyl;
$R_1$ is selected from halogen and $C_{1-4}$ haloalkyl, alternatively halogen;
$R_3$ is selected from H, $-OC_{1-4}$ alkyl and $-OC_{1-4}$ haloalkyl, alternatively H and $-OC_{1-4}$ alkyl;
$R_6$ is selected from -NH-C(O)-CR=CR'R" and -NH-C(O)-C≡CR';
wherein R is selected from H, halogen and $C_{1-4}$ alkyl;
R' and R" are each independently selected from H, $C_{1-4}$ alkyl and $C_{1-4}$ haloalkyl, wherein the $C_{1-4}$ alkyl and $C_{1-4}$ haloalkyl are each optionally substituted with 0, 1 or 2 R*;
R* is selected from H, halogen, -OH and 5-6 membered heterocyclyl;
n is 1, 2, 3 or 4.

**11.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from

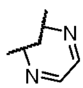 and 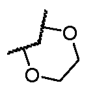;

$R_1$ is selected from Cl, Br and $-CF_3$, alternatively Cl and Br;
$R_3$ is selected from H and $-OCH_3$;
$R_6$ is selected from

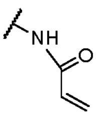, 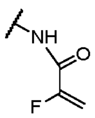, 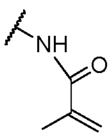, 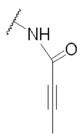, 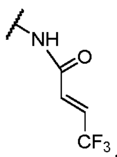, 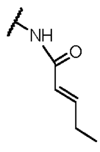, 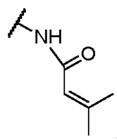, 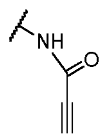,

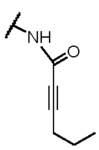, 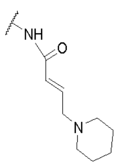, 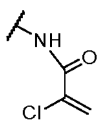 and 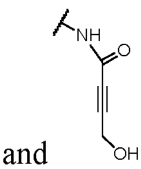;

n is 2, 3 or 4.

**12.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-10 membered heteroaryl and 5-8 membered heterocyclyl;
$R_1$ is selected from halogen and $C_{1-4}$ haloalkyl, alternatively halogen;
$R_3$ is selected from H, $-OC_{1-6}$ alkyl and $-OC_{1-6}$ haloalkyl, alternatively H and $-OC_{1-6}$ alkyl;
$R_6$ is selected from -NH-C(O)-CR=CR'R" and -NH-C(O)-C≡CR';
wherein R, R' and R" are each independently selected from H, F, $C_{1-6}$ alkyl and $C_{1-6}$ haloalkyl;
n is 1, 2, 3, 4 or 5.

**13.** The compound of claim 12, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from 5-6 membered heteroaryl and 5-6 membered heterocyclyl;
$R_1$ is selected from halogen and $C_{1-4}$ haloalkyl, alternatively halogen;
$R_3$ is selected from H and $-OC_{1-4}$alkyl;
$R_6$ is selected from -NH-C(O)-CR=CR'R" and -NH-C(O)-C≡CR';
wherein R is selected from H, F and $C_{1-4}$ alkyl;
R' and R" are each independently selected from H, $C_{1-4}$ alkyl and $C_{1-4}$ haloalkyl;
n is 1, 2, 3 or 4.

**14.** The compound of claim 12, or a pharmaceutically acceptable salt, enantiomer, diastereomer, racemate, solvate, hydrate, polymorph, prodrug or isotopic variant thereof, or a mixture thereof, wherein:

Ring A is absent, or Ring A is selected from

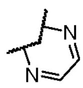 and 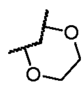;

$R_1$ is selected from Cl, Br and $-CF_3$, alternatively Cl and Br;
$R_3$ is selected from H and $-OCH_3$;
$R_6$ is selected from

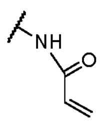, 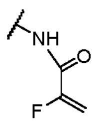, 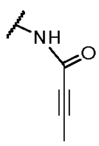, 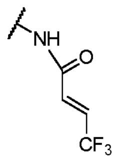, 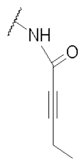, 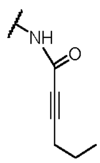, 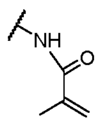, 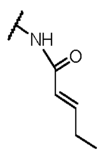,

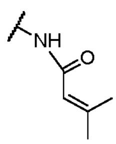 and 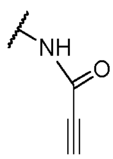;

n is 1, 2 or 3.

**15.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is selected from 5-10 membered heteroaryl and 5-8 membered heterocyclyl;
$R_1$ is selected from halogen and $C_{1-4}$ haloalkyl, alternatively halogen;
$R_3$ is selected from $-OC_{1-6}$ alkyl and $-OC_{1-6}$ haloalkyl, alternatively $-OC_{1-6}$ alkyl;
$R_6$ is selected from -NH-C(O)-CR=CR'R" and -NH-C(O)-C≡CR';
wherein R, R' and R" are each independently selected from H, halogen, $C_{1-6}$ alkyl and $C_{1-6}$ haloalkyl;
n is 1, 2 or 3.

**16.** The compound of claim 15, or a pharmaceutically acceptable salt, enantiomer, diastereomer, racemate, solvate, hydrate, polymorph, prodrug or isotopic variant thereof, or a mixture thereof, wherein:

Ring A is selected from 5-6 membered heteroaryl and 5-6 membered heterocyclyl;
$R_1$ is selected from halogen and $C_{1-4}$ haloalkyl, alternatively halogen;
$R_3$ is $-OC_{1-4}$alkyl;
$R_6$ is selected from -NH-C(O)-CR=CR'R" and -NH-C(O)-C≡CR';
wherein R is selected from H and halogen;
R' and R" are each independently selected from H, $C_{1-4}$ alkyl and $C_{1-4}$ haloalkyl;
n is 1, 2 or 3.

**17.** The compound of claim 15, or a pharmaceutically acceptable salt, enantiomer, diastereomer, racemate, solvate, hydrate, polymorph, prodrug or isotopic variant thereof, or a mixture thereof, wherein:

Ring A is selected from

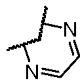 and 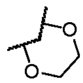;

$R_1$ is selected from Cl, Br and $-CF_3$, alternatively Cl and Br, still alternatively Cl;
$R_3$ is $-OCH_3$;
$R_6$ is selected from

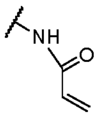, 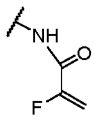, 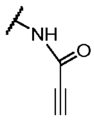, 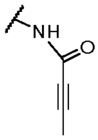, 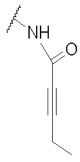, 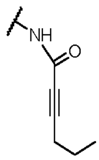 and 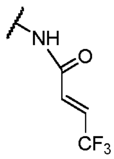;

n is 2.

**18.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is halogen, $C_{1-4}$ alkyl or $C_{1-4}$ haloalkyl;
$R_3$ is selected from $-OC_{1-6}$ alkyl and $-OC_{1-6}$ haloalkyl;
$R_6$ is selected from -NH-C(O)-CR=CR'R" and -NH-C(O)-C≡CR';
wherein R is H, F or Me;
R' and R" are each independently selected from H, $C_{1-6}$ alkyl and $C_{1-6}$ haloalkyl;
n is 2.

**19.** The compound of claim 18, or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is Br or $C_{1-2}$ haloalkyl;
$R_3$ is selected from $-OC_{1-2}$ alkyl and $-OC_{1-2}$ haloalkyl;
$R_6$ is selected from -NH-C(O)-CR=CR'R" and -NH-C(O)-C≡CR';
wherein R is H or F; R' is H, $C_{1-4}$ alkyl or $C_{1-4}$ haloalkyl; R" is H;
n is 2.

**20.** The compound of claim 18, or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is Br or $CF_3$;
$R_3$ is selected from -OMe and $-OCH_2CF_3$;
$R_6$ is selected from -NH-C(O)-CR=CR'R" and -NH-C(O)-C≡CR';

wherein R is H or F; R' is H, Me, iPr or $CF_3$; R" is H;
n is 2.

**21.** The compound of claim 8, or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is $C_{1-4}$ haloalkyl;
$R_3$ is selected from $-OC_{1-6}$ alkyl and $-OC_{1-6}$ haloalkyl;
$R_6$ is selected from -NH-C(O)-CR=CR'R" and -NH-C(O)-C≡CR';
wherein R is H or halogen; R' is H, $C_{1-2}$ alkyl or $C_{1-2}$ haloalkyl; R" is H;
n is 2.

**22.** The compound of claim 21, or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is $C_{1-2}$ haloalkyl;
$R_3$ is selected from $-OC_{1-2}$ alkyl and $-OC_{1-2}$ haloalkyl;
$R_6$ is selected from -NH-C(O)-CR=CR'R" and -NH-C(O)-C≡CR';
wherein R is H, F or Br; R' is H, $C_{1-2}$ alkyl or $C_{1-2}$ haloalkyl; R" is H;
n is 2.

**23.** The compound of claim 21, or a pharmaceutically acceptable salt, enantiomer, diastereomer, racemate, solvate, hydrate, polymorph, prodrug or isotopic variant thereof, or a mixture thereof, wherein:

Ring A is absent;
$R_1$ is $CF_3$;
$R_3$ is selected from -OMe and $-OCH_2CF_3$;
$R_6$ is selected from -NH-C(O)-CR=CR'R" and -NH-C(O)-C≡CR';
wherein R is H or F; R' is H, Me or $CF_3$; R" is H;
n is 2.

**24.** The compound of claim 3, or a pharmaceutically acceptable salt, enantiomer, diastereomer, racemate, solvate, hydrate, polymorph, prodrug or isotopic variant thereof, or a mixture thereof, which has a structure of formula (V) :

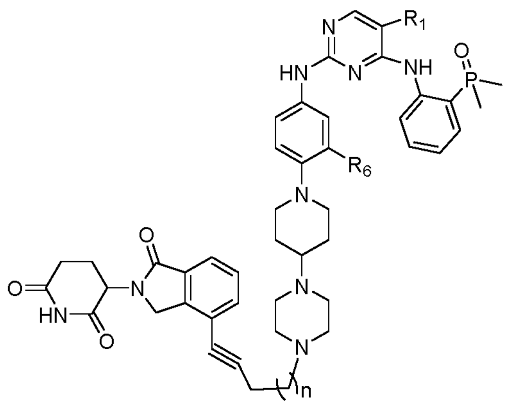

(V)

wherein

$R_1$ is halogen;
$R_6$ is selected from -NH-C(O)-CR=CR'R" and -NH-C(O)-C≡CR';
wherein R, R' and R" are each independently selected from H, halogen, $C_{1-6}$ alkyl and $C_{1-6}$ haloalkyl;
n is 0, 1, 2, 3, 4, or 5.

**25.** The compound of claim 24, or a pharmaceutically acceptable salt, enantiomer, diastereomer, racemate, solvate, hydrate, polymorph, prodrug or isotopic variant thereof, or a mixture thereof, wherein:

$R_1$ is halogen;
$R_6$ is selected from -NH-C(O)-CR=CR'R" and -NH-C(O)-C≡CR';
wherein R is selected from H and halogen;
R' and R" are each independently selected from H, $C_{1-4}$ alkyl and $C_{1-4}$ haloalkyl;
n is 1, 2, 3 or 4.

**26.** The compound of claim 24, or a pharmaceutically acceptable salt, enantiomer, diastereomer, racemate, solvate, hydrate, polymorph, prodrug or isotopic variant thereof, or a mixture thereof, wherein:

$R_1$ is selected from Br and Cl;
$R_6$ is selected from

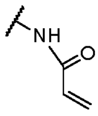 , 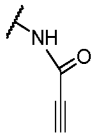 , 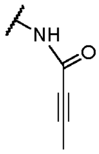 , 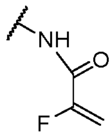 , 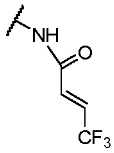 , 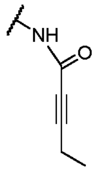 and 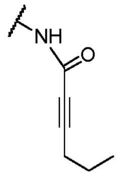 ;

n is 2, 3 or 4.

**27.** The compound of claim 3, or a pharmaceutically acceptable salt, enantiomer, diastereomer, racemate, solvate, hydrate, polymorph, prodrug or isotopic variant thereof, or a mixture thereof, which has the structure of formula (VI) :

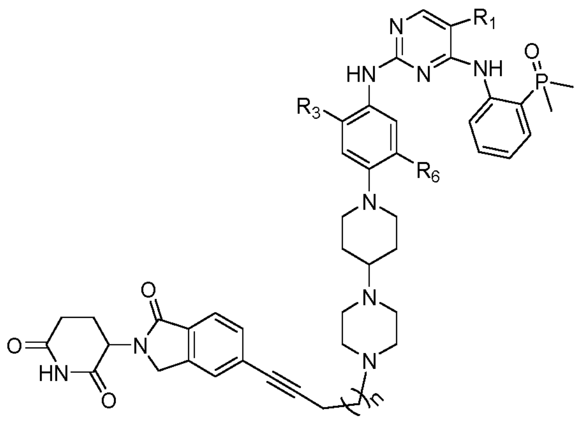

(VI)

wherein

$R_1$ is halogen;
$R_3$ is $-OC_{1-6}$ alkyl;
$R_6$ is selected from -NH-C(O)-CR=CR'R" and -NH-C(O)-C≡CR';
wherein R, R' and R" are independently selected from H, halogen, $C_{1-6}$ alkyl and $C_{1-6}$ haloalkyl;
n is 1, 2, 3 or 4.

**28.** The compound of claim 27, or a pharmaceutically acceptable salt, enantiomer, diastereomer, racemate, solvate, hydrate, polymorph, prodrug or isotopic variant thereof, or a mixture thereof, wherein:

$R_1$ is halogen;
$R_3$ is $-OC_{1-6}$ alkyl;
$R_6$ is selected from -NH-C(O)-CR=CR'R" and -NH-C(O)-C≡CR';
wherein R, R' and R" are independently selected from H, $C_{1-6}$ alkyl and $C_{1-6}$ haloalkyl;
n is 2, 3 or 4.

**29.** The compound of claim 27, or a pharmaceutically acceptable salt, enantiomer, diastereomer, racemate, solvate, hydrate, polymorph, prodrug or isotopic variant thereof, or a mixture thereof, wherein:

$R_1$ is halogen;
$R_3$ is -$OC_{1-4}$alkyl;
$R_6$ is selected from -NH-C(O)-CR=CR'R" and -NH-C(O)-C≡CR';
wherein R is H;
R' and R" are independently selected from H, $C_{1-4}$ alkyl and $C_{1-4}$ haloalkyl;
n is 3.

**30.** The compound of claim 27, or a pharmaceutically acceptable salt, enantiomer, diastereomer, racemate, solvate, hydrate, polymorph, prodrug or isotopic variant thereof, or a mixture thereof, wherein:

$R_1$ is Cl;
$R_3$ is -$OCH_3$;
$R_6$ is selected from

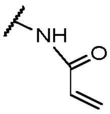, 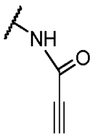, 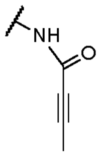, 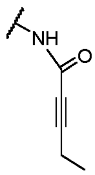, 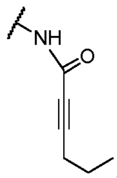, 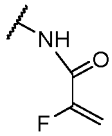 and 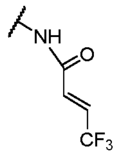;

n is 3.

**31.** The compound of claim 3, or a pharmaceutically acceptable salt, enantiomer, diastereomer, racemate, solvate, hydrate, polymorph, prodrug or isotopic variant thereof, or a mixture thereof, which has a structure of formula (IX) :

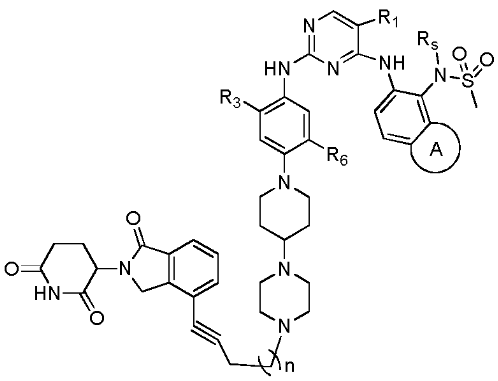

(IX)

wherein

Ring A is selected from $C_{4-10}$ cycloalkyl, 5-8 membered heterocyclyl, $C_{6-10}$ aryl and 5-14 membered heteroaryl;
$R_1$ is H, halogen, $C_{1-4}$ alkyl or $C_{1-4}$ haloalkyl; alternatively, $R_1$ is halogen, $C_{1-4}$ alkyl or $C_{1-4}$ haloalkyl;
$R_3$ is selected from -$OC_{1-6}$ alkyl and -$OC_{1-6}$ haloalkyl;
$R_6$ is selected from $C_{1-6}$ alkyl, $C_{1-6}$ haloalkyl, -NH-C(O)-CR=CR'R" and -NH-C(O)-C≡CR';
wherein R is H, halogen, $C_{1-6}$ alkyl or $C_{1-6}$ haloalkyl;
R' and R" are each independently selected from H, $C_{1-6}$ alkyl and $C_{1-6}$ haloalkyl;
$R_s$ is selected from H, $C_{1-6}$ alkyl and $C_{1-6}$ haloalkyl;
n is 2.

**32.** The compound of claim 31, or a pharmaceutically acceptable salt, enantiomer, diastereomer, racemate, solvate, hydrate, polymorph, prodrug or isotopic variant thereof, or a mixture thereof, wherein:

Ring A is selected from $C_{6-10}$ aryl and 5-6 membered heteroaryl;

$R_1$ is H, halogen, $C_{1-4}$ alkyl or $C_{1-4}$ haloalkyl; alternatively, $R_1$ is halogen, $C_{1-4}$ alkyl or $C_{1-4}$ haloalkyl;
$R_3$ is selected from $-OC_{1-4}$ alkyl and $-OC_{1-4}$ haloalkyl;
$R_6$ is selected from $C_{1-4}$ alkyl, $C_{1-4}$ haloalkyl, -NH-C(O)-CR=CR'R" and -NH-C(O)-C=CR';
wherein R is H or halogen; R' is H, $C_{1-4}$ alkyl or $C_{1-4}$ haloalkyl; R" is H;
$R_s$ is selected from H, $C_{1-4}$ alkyl and $C_{1-4}$ haloalkyl;
n is 2.

**33.** The compound of claim 31, or a pharmaceutically acceptable salt, enantiomer, diastereomer, racemate, solvate, hydrate, polymorph, prodrug or isotopic variant thereof, or a mixture thereof, wherein:

Ring A is selected from phenyl and 5-6 membered heteroaryl;
$R_1$ is H, halogen or $C_{1-4}$ haloalkyl; alternatively, $R_1$ is halogen or $C_{1-4}$ haloalkyl;
$R_3$ is selected from $-OC_{1-4}$ alkyl and $-OC_{1-4}$ haloalkyl;
$R_6$ is selected from $C_{1-4}$ alkyl, $C_{1-4}$ haloalkyl, -NH-C(O)-CR=CR'R" and -NH-C(O)-C=CR';
wherein R is H or halogen; R' is H, $C_{1-4}$ alkyl or $C_{1-4}$ haloalkyl; R" is H;
$R_s$ is selected from H, $C_{1-4}$ alkyl and $C_{1-4}$ haloalkyl;
n is 2.

**34.** The compound of claim 33, or a pharmaceutically acceptable salt, enantiomer, diastereomer, racemate, solvate, hydrate, polymorph, prodrug or isotopic variant thereof, or a mixture thereof, wherein:

Ring A is a 5-6 membered heteroaryl group;
$R_1$ is H, Cl, Br or $CF_3$; alternatively, $R_1$ is Br or $CF_3$;
$R_3$ is selected from $-OC_{1-2}$ alkyl and $-OC_{1-2}$ haloalkyl;
$R_6$ is selected from $C_{1-4}$ alkyl, $C_{1-4}$ haloalkyl and -NH-C(O)-CR=CR'R";
wherein R is H or halogen; R' and R" are H;
$R_s$ is selected from H and $C_{1-4}$ alkyl;
n is 2.

**35.** The compound of claim 31, or a pharmaceutically acceptable salt, enantiomer, diastereomer, racemate, solvate, hydrate, polymorph, prodrug or isotopic variant thereof, or a mixture thereof, wherein:

Ring A is pyrazinyl;
$R_1$ is H, Cl, Br or $CF_3$; alternatively, $R_1$ is Br or $CF_3$;
$R_3$ is selected from $-OC_{1-2}$ alkyl and $-OC_{1-2}$ haloalkyl;
$R_6$ is selected from $C_{1-4}$ alkyl and -NH-C(O)-CR=CR'R";
wherein R is H or F; R' and R" are H;
$R_s$ is H;
n is 2.

**36.** A compound, or a pharmaceutically acceptable salt, enantiomer, diastereomer, racemate, solvate, hydrate, polymorph, prodrug or isotopic variant thereof, or a mixture thereof, wherein the compound is selected from:

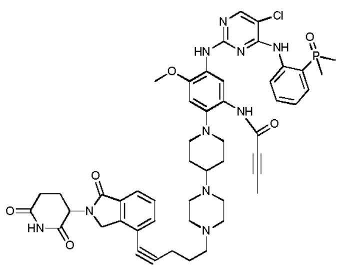
,
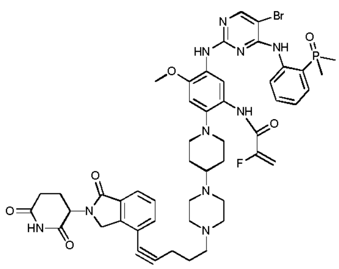
,
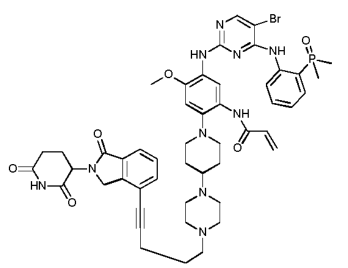
,

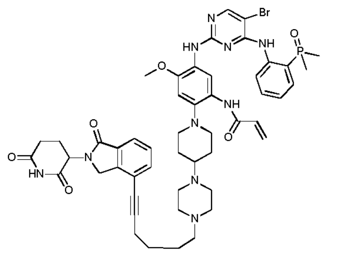,
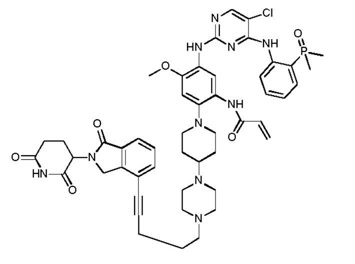,
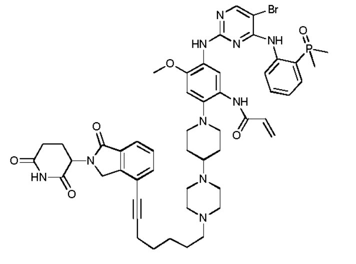,

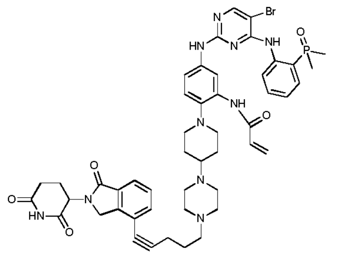,
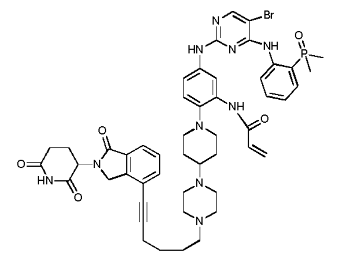,
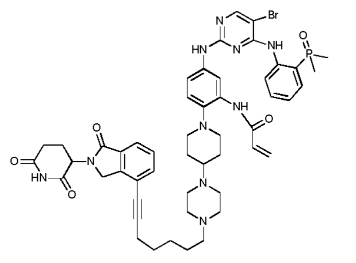,

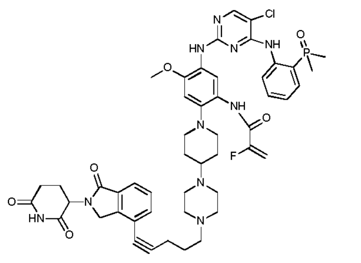,
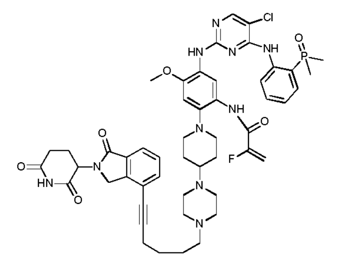,
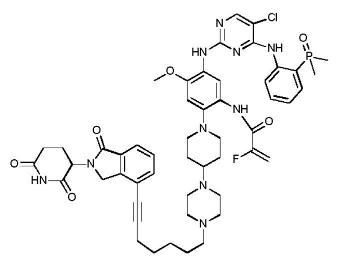,

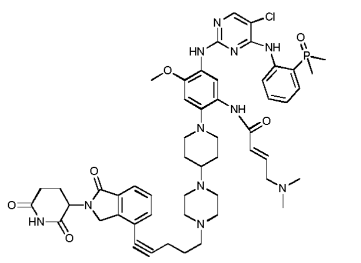,
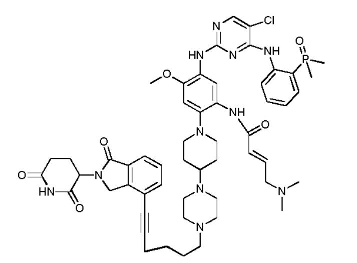,
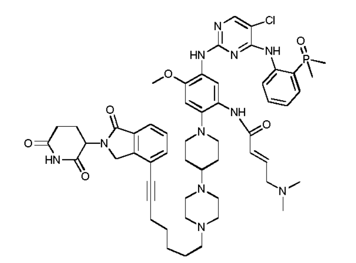,

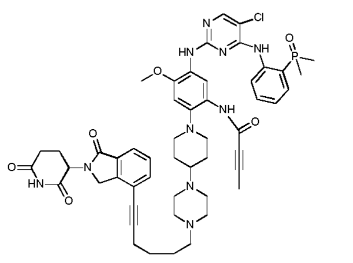,
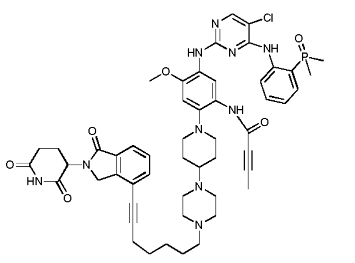,
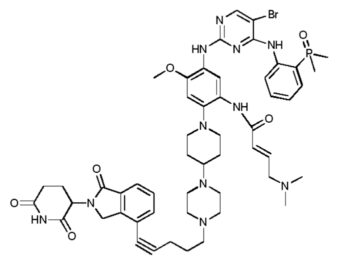,

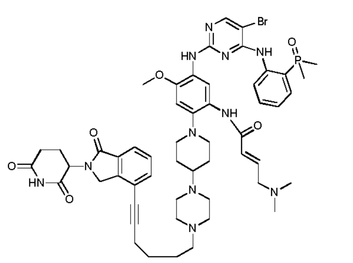,
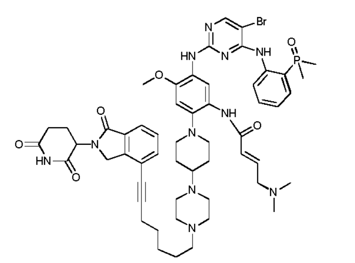,
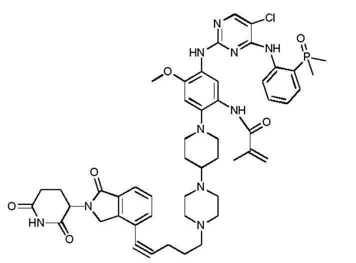,

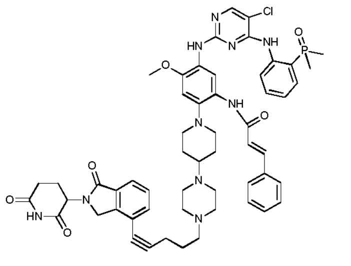
,
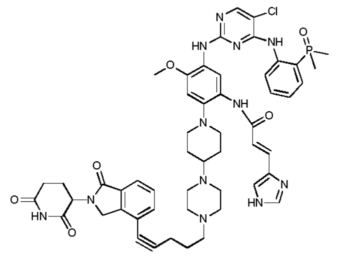
,
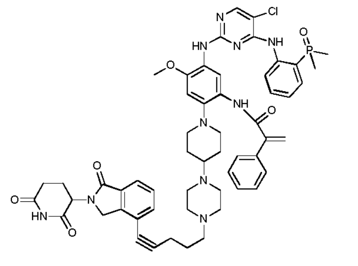
,
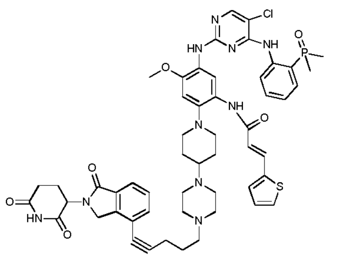
,
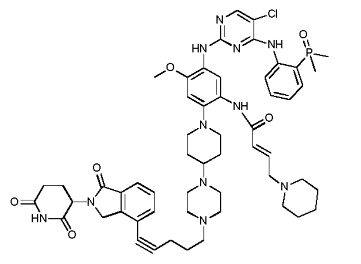
,
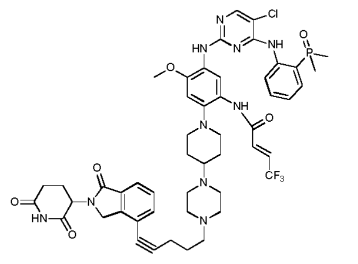
,
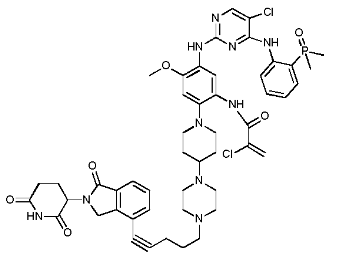
,
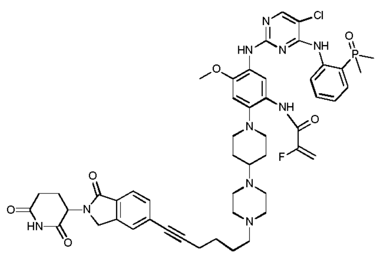
,
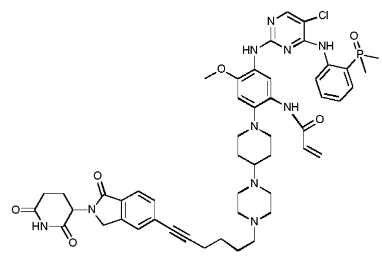
,
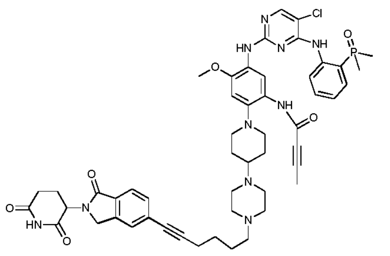
,
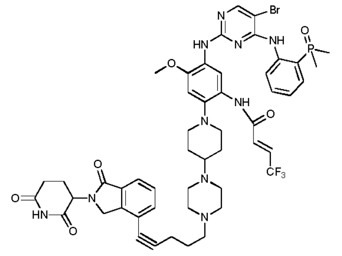
,
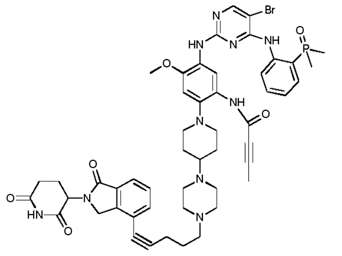
,
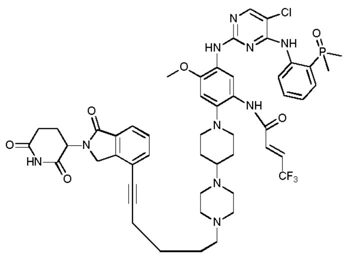
,
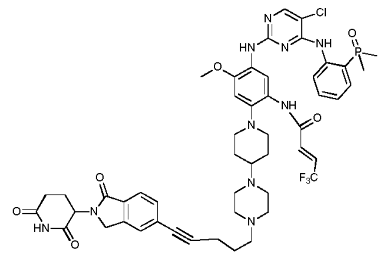
,
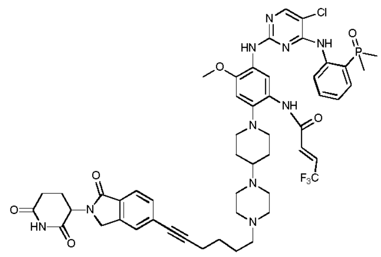
,
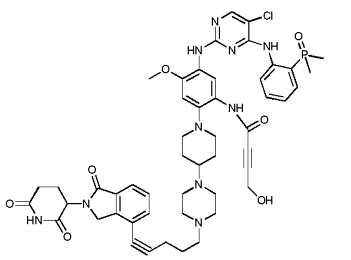
,
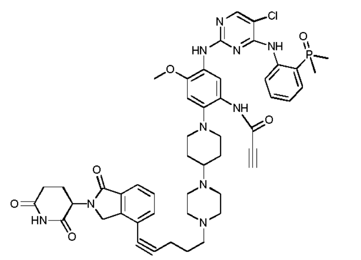
,
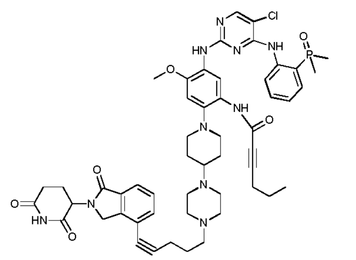
,

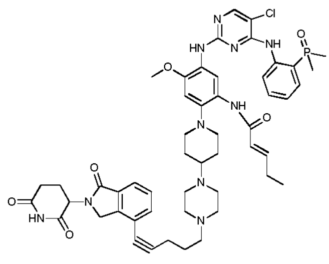,
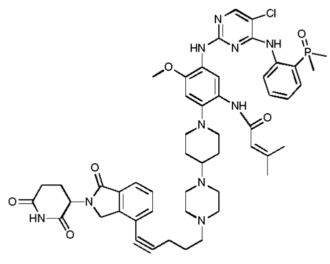,
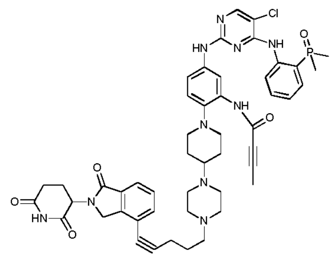,
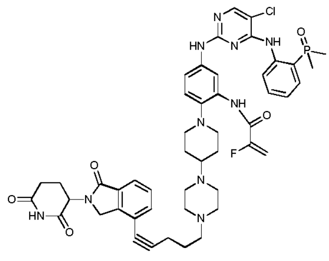,
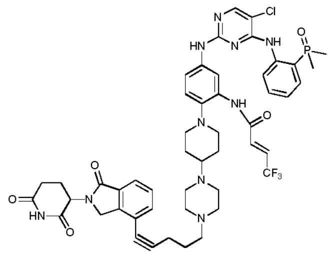,
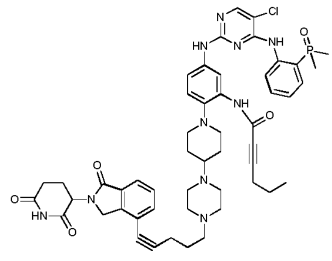,
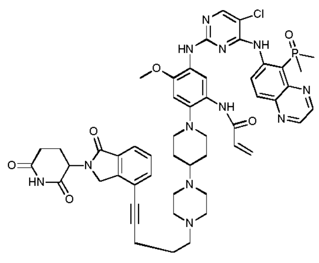,
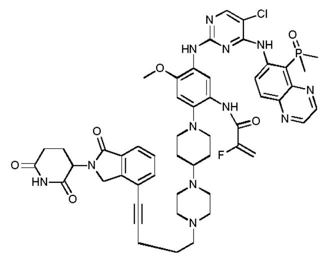,
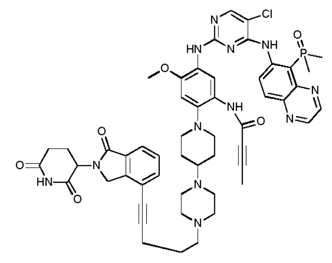,
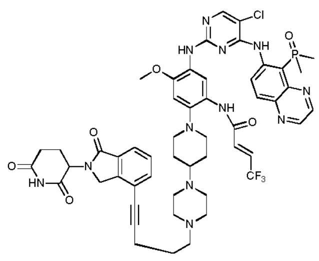,
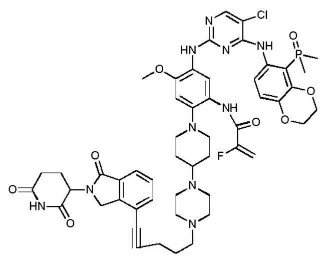,
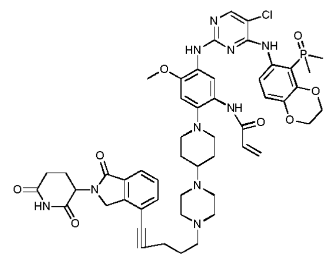,
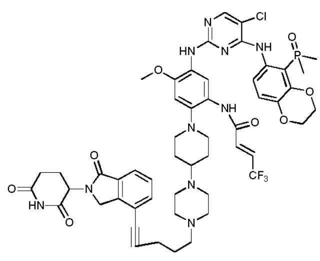,
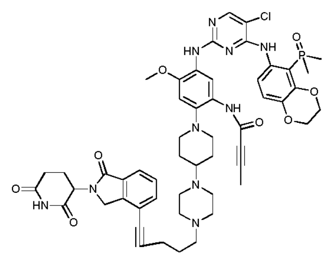,
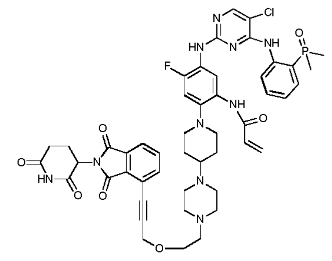,
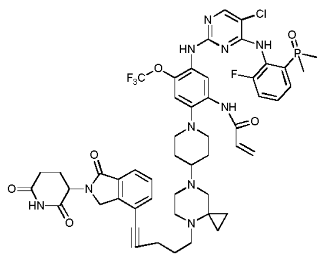,
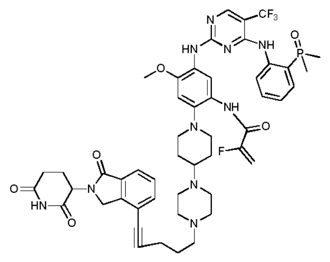,
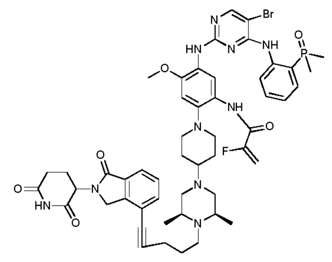,

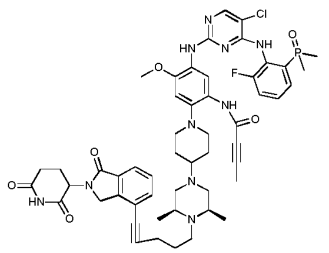
,
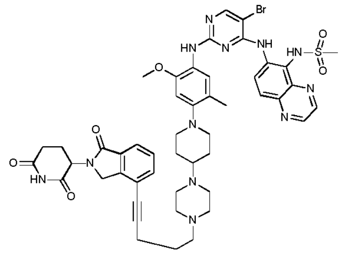
,
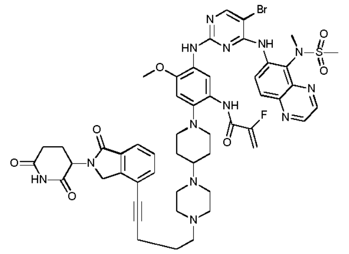
,
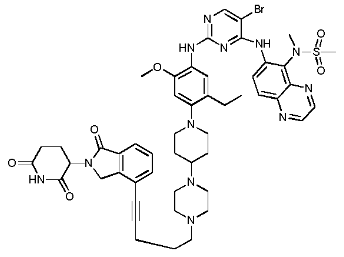
,
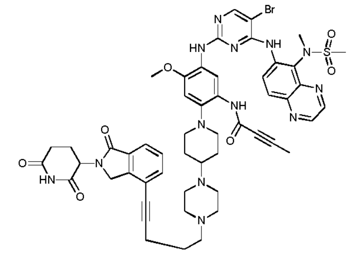
,
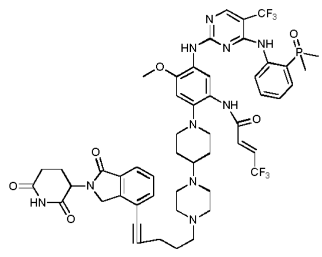
,
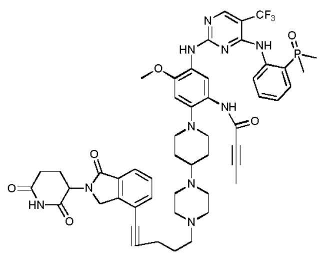
,
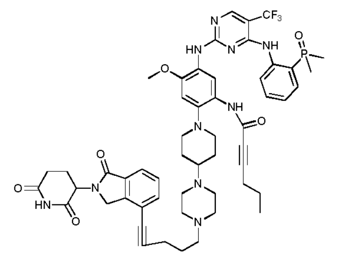
,
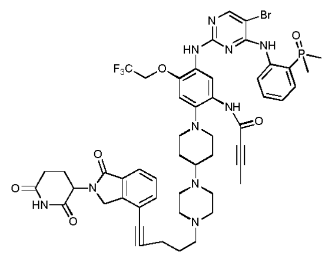
,
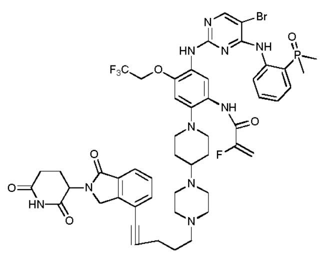
,
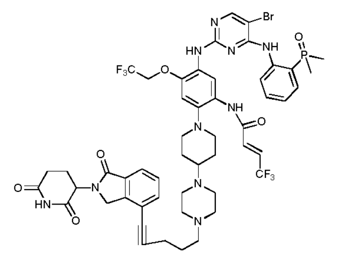
,
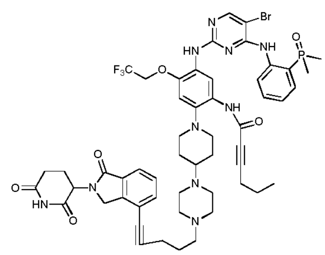
,
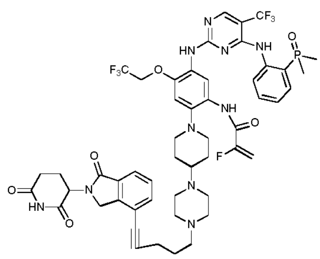
,
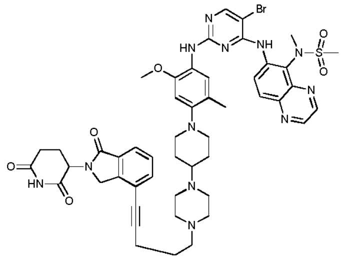
,
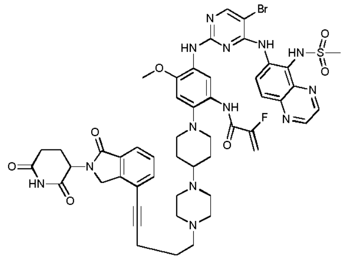
,
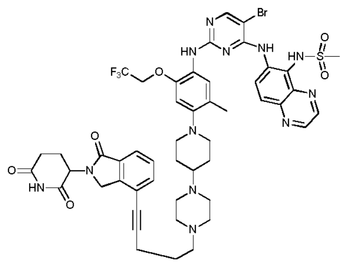
,
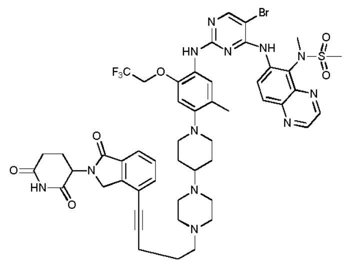
,
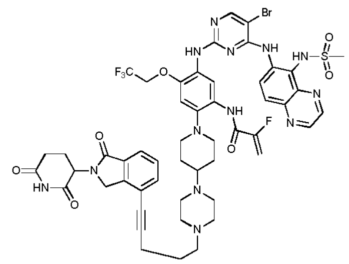
,

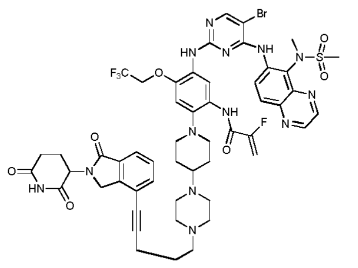,
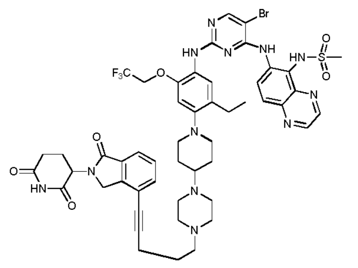,
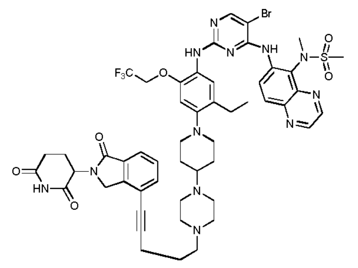,
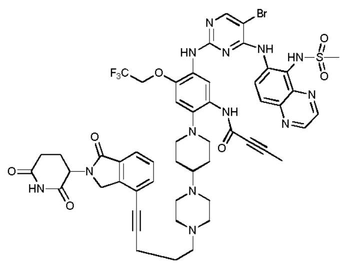,
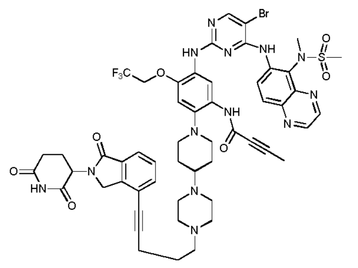,
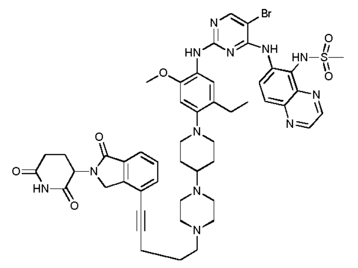,
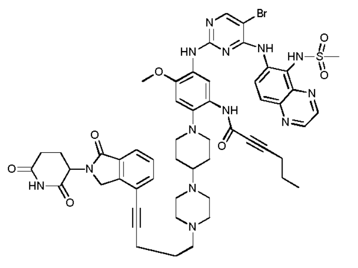,
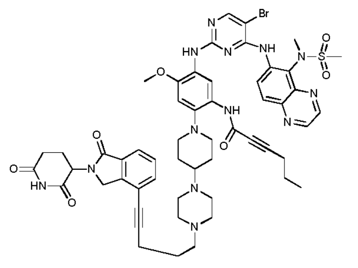,
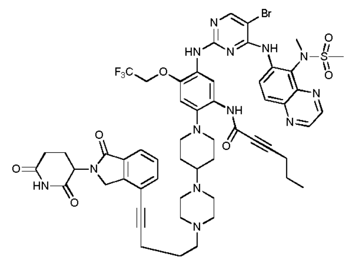,
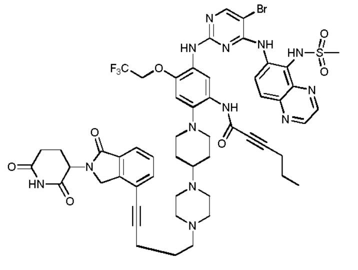,
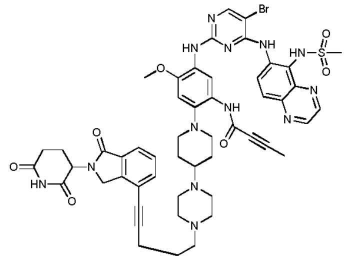,
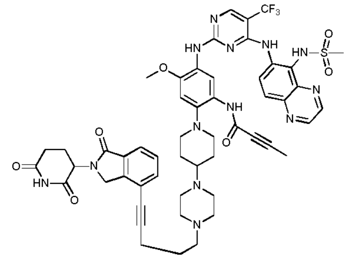,
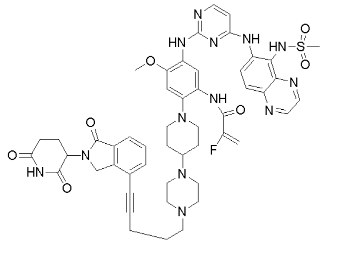,
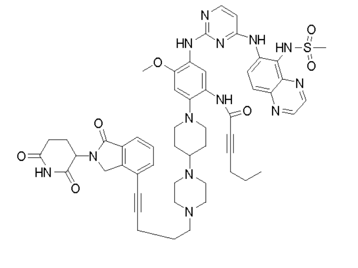,
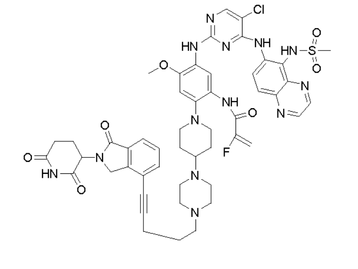,
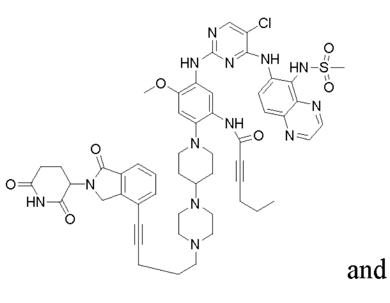
and
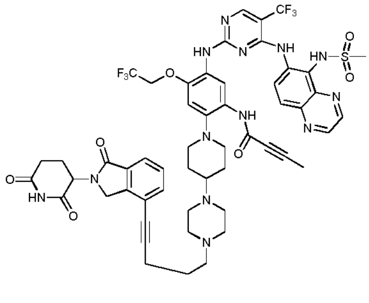.

**37.** A pharmaceutical composition comprising a compound according to any one of claims 1 to 36, or a pharmaceutically acceptable salt, enantiomer, diastereomer, racemate, solvate, hydrate, polymorph, prodrug or isotopic variant thereof, and a pharmaceutically acceptable excipient; optionally, further comprising other therapeutic agents.

**38.** Use of a compound according to any one of claims 1 to 36 or a pharmaceutically acceptable salt, enantiomer, diastereomer, racemate, solvate, hydrate, polymorph, prodrug or isotopic variant thereof in the manufacture of a medicament for the treatment and/or prevention of an EGFR kinase-mediated disease.

**39.** A method for treating and/or preventing an EGFR kinase-mediated disease in a subject, the method comprising administering to the subject a compound of any one of claims 1 to 36 or a pharmaceutically acceptable salt, enantiomer, diastereomer, racemate, solvate, hydrate, polymorph, prodrug or isotopic variant thereof, or a pharmaceutical composition of claim 37.

**40.** A compound according to any one of claims 1 to 36 or a pharmaceutically acceptable salt, enantiomer, diastereomer, racemate, solvate, hydrate, polymorph, prodrug or isotopic variant thereof, or a pharmaceutical composition according to claim 37 for use in the treatment and/or prevention of an EGFR kinase-mediated disease.

**41.** The use of claim 38 or the method of claim 39 or the compound or composition for use according to claim 40, wherein the EGFR kinase-mediated disease comprises cancer, such as ovarian cancer, cervical cancer, colorectal cancer, breast cancer, pancreatic cancer, glioma, glioblastoma, melanoma, prostate cancer, leukemia, lymphoma, non-Hodgkin lymphoma, gastric cancer, lung cancer, hepatocellular carcinoma, gastric cancer, gastrointestinal stromal tumor (GIST), thyroid cancer, bile duct cancer, endometrial cancer, renal cancer, anaplastic large cell lymphoma, acute myeloid leukemia (AML), multiple myeloma, melanoma, mesothelioma.

**42.** The use of claim 38 or the method of claim 39 or the compound or composition for use according to claim 40, wherein the EGFR kinase-mediated disease is lung cancer, particularly non-small cell lung cancer.

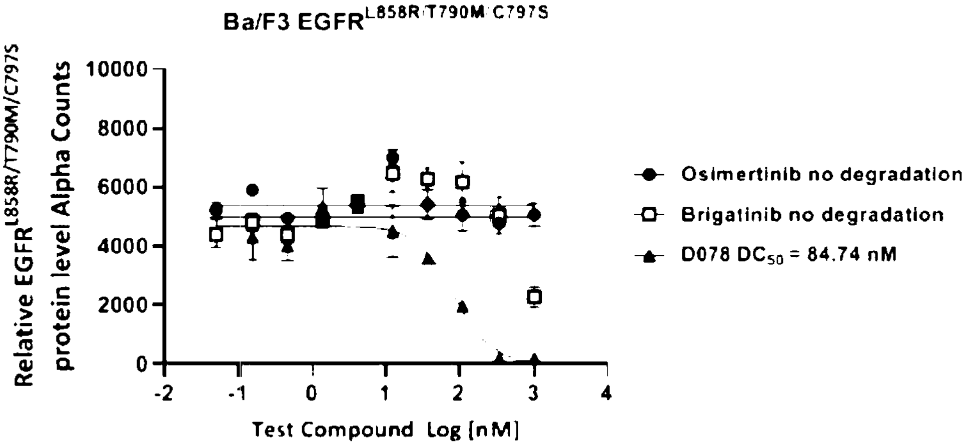


Fig. 1

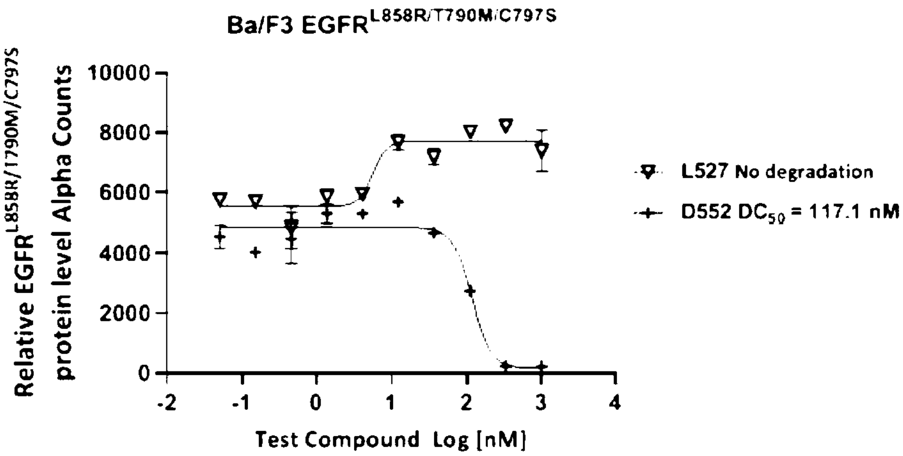


Fig. 2

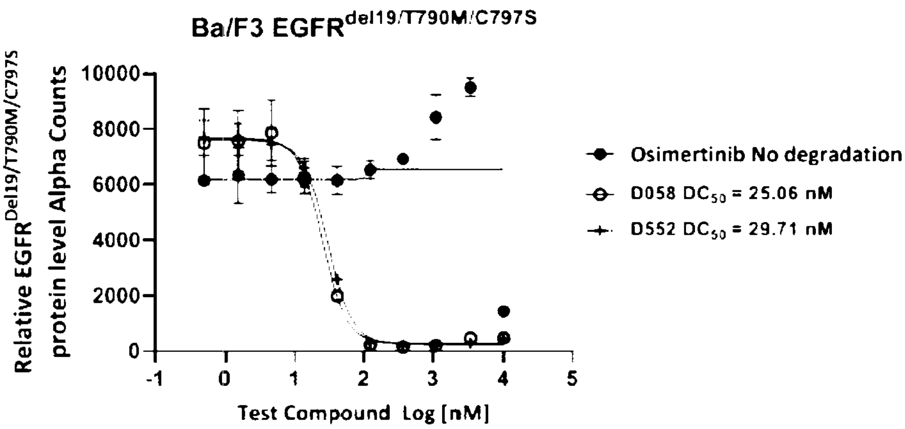


Fig. 3

**INTERNATIONAL SEARCH REPORT**

International application No.
**PCT/CN2023/088336**

**A. CLASSIFICATION OF SUBJECT MATTER**

C07F 9/6558(2006.01)i; C07D401/14(2006.01)i; C07D417/14(2006.01)i; A61K31/675(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07F C07D A61K A61P

Documentation searched other than minimum documentation to the extent that such documents are included in the fields searched

Electronic data base consulted during the international search (name of data base and, where practicable, search terms used)

CNABS; DWPI; VEN; CNKI; CNTXT; USTXT; WOTXT; EPTXT; STN-REGISTRY; STN-CAPLUS; PATENTICS; 超星读秀, CHAOXING DUXIU; ISI-Web of Science: 北京泰德制药股份有限公司, 王红军, 王业明, 王晓倩, 赵焰平, 结构式检索, structural formula search, 表皮生长因子, 癌症, 肿瘤, 瘤, 降解, EGFR, cancer, tumor, tumour, degradat+

**C. DOCUMENTS CONSIDERED TO BE RELEVANT**

| Category* | Citation of document, with indication, where appropriate, of the relevant passages | Relevant to claim No. |
|---|---|---|
| X | WO 2021036922 A1 (BEIJING TIDE PHARMACEUTICAL CO., LTD.) 04 March 2021 (2021-03-04)<br>claims 1, 28, 30-33, description page 2 paragraph 2 | 1-42 |
| PX | WO 2022268229 A1 (JING MEDICINE TECHNOLOGY LTD.) 29 December 2022 (2022-12-29)<br>claims 1, 34-38, description page 193 embodiment 42, page 264 table 6 | 1-42 |
| PX | CN 114805303 A (HAISCO PHARMACEUTICAL GROUP INC.) 29 July 2022 (2022-07-29)<br>claims 1-20 | 1-42 |
| A | CN 112079866 A (SHANGHAITECH UNIVERSITY) 15 December 2020 (2020-12-15)<br>entire document | 1-42 |
| A | WO 2022055181 A1 (J2H BIOTECH INC.) 17 March 2022 (2022-03-17)<br>entire document | 1-42 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 June 2023** | **24 July 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/088336**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **39, 41 (in part), 42 (in part)**
because they relate to subject matter not required to be searched by this Authority, namely:

Claim 39 sets forth a method for treating and/or preventing EGFR kinase-mediated diseases in a subject, and claims 41 and 42 refer to the method of claim 39. The subject matter mentioned above falls within the category of methods for treatment of a living human or animal body, and falls within the cases set out in PCT Rule 39.1(iv) for which an international search is not required. Nevertheless, a search is performed on the basis of the subject matter of claim 39 setting forth the use of the compound of any one of claims 1-36 or the pharmaceutical composition of claim 37 in the preparation of a drug for treating and/or preventing EGFR kinase-mediated diseases, and claims 41 and 42 referring to the use of claim 39.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/088336**

| Patent document cited in search report | | | Publication date (day/month/year) | Patent family member(s) | | | Publication date (day/month/year) |
|---|---|---|---|---|---|---|---|
| WO | 2021036922 | A1 | 04 March 2021 | TW | 202115026 | A | 16 April 2021 |
| | | | | TW | 758832 | B1 | 21 March 2022 |
| | | | | CN | 114286678 | A | 05 April 2022 |
| | | | | KR | 20220051244 | A | 26 April 2022 |
| | | | | HK | 40063816 | A0 | 24 June 2022 |
| | | | | EP | 4019021 | A1 | 29 June 2022 |
| | | | | US | 2022306659 | A1 | 29 September 2022 |
| | | | | EP | 4019021 | A4 | 02 November 2022 |
| | | | | JP | 2022546375 | A | 04 November 2022 |
| WO | 2022268229 | A1 | 29 December 2022 | | None | | |
| CN | 114805303 | A | 29 July 2022 | | None | | |
| CN | 112079866 | A | 15 December 2020 | JP | 2022542761 | A | 07 October 2022 |
| | | | | EP | 4029499 | A1 | 20 July 2022 |
| | | | | KR | 20220024507 | A | 03 March 2022 |
| | | | | WO | 2020249048 | A1 | 17 December 2020 |
| | | | | CA | 3141413 | A1 | 17 December 2020 |
| | | | | US | 2022257776 | A1 | 18 August 2022 |
| | | | | HK | 40041927 | A0 | 27 August 2021 |
| WO | 2022055181 | A1 | 17 March 2022 | KR | 20220035014 | A | 21 March 2022 |
| | | | | CA | 3191532 | A1 | 17 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210398246
- CN 202310317846
- US 5376645 A


### Non-patent literature cited in the description


- **BERGE S. M. et al.** Pharmaceutical Salts. *J. Pharm. Sci.*, 1977, vol. 66, 1-19
- Bioreversible Carriers in Drug Design. **T. HIGUCHI** ; **V STELLA**. Prodrugs as Novel Delivery Systems, A.C.S. Symposium Series. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130